(19) Europäisches Patentamt
European Patent Office
Office européen des brevets

(11) EP 2 681 337 B1

(12) EUROPEAN PATENT SPECIFICATION

(45) Date of publication and mention of the grant of the patent:
**25.04.2018 Bulletin 2018/17**

(21) Application number: **12752998.0**

(22) Date of filing: **01.03.2012**

(51) Int Cl.:
***C12Q 1/68*** *(2018.01)*

(86) International application number:
**PCT/IS2012/050004**

(87) International publication number:
**WO 2012/117424 (07.09.2012 Gazette 2012/36)**

(54) **BRIP1 VARIANTS ASSOCIATED WITH RISK FOR CANCER**

MIT KREBSRISIKO ASSOZIIERTE BRIP1-VARIANTEN

VARIANTS À RISQUE POUR LE CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.03.2011 IS 50009**
**22.07.2011 IS 50017**

(43) Date of publication of application:
**08.01.2014 Bulletin 2014/02**

(73) Proprietors:
- **Decode Genetics EHF**
  **101 Reykjavik (IS)**
- **Illumina, Inc.**
  **San Diego, California 92121 (US)**

(72) Inventors:
- **SULEM, Patrick**
  **IS-105 Reykjavik (IS)**
- **RAFNAR, Thorunn**
  **IS-108 Reykjavik (IS)**

(74) Representative: **Arnason Faktor Intellectual Property Consulting**
**Gudridarstig 2-4**
**113 Reykjavik (IS)**

(56) References cited:
**WO-A1-2008/035102**

- **T. R. REBBECK ET AL: "Modification of Ovarian Cancer Risk by BRCA1/2-Interacting Genes in a Multicenter Cohort of BRCA1/2 Mutation Carriers", CANCER RESEARCH, vol. 69, no. 14, 7 July 2009 (2009-07-07), pages 5801-5810, XP055163986, ISSN: 0008-5472, DOI: 10.1158/0008-5472.CAN-09-0625**
- **MIKI Y ET AL: "A STRONG CANDIDATE FOR THE BREAST AND OVARIAN CANCER SUSCEPTIBILITY GENE BRCA1", SCIENCE, AMERICAN ASSOCIATION FOR THE ADVANCEMENT OF SCIENCE, US, vol. 266, 7 October 1994 (1994-10-07), pages 66-71, XP000202410, ISSN: 0036-8075, DOI: 10.1126/SCIENCE.7545954**
- **A. DE NICOLO ET AL: "A Novel Breast Cancer-Associated BRIP1 (FANCJ/BACH1) Germ-line Mutation Impairs Protein Stability and Function", CLINICAL CANCER RESEARCH, vol. 14, no. 14, 15 July 2008 (2008-07-15) , pages 4672-4680, XP055120596, ISSN: 1078-0432, DOI: 10.1158/1078-0432.CCR-08-0087**
- **THORUNN RAFNAR ET AL: "Mutations in BRIP1 confer high risk of ovarian cancer", NATURE GENETICS, vol. 43, no. 11, 2 October 2011 (2011-10-02), pages 1104-1107, XP055120607, ISSN: 1061-4036, DOI: 10.1038/ng.955**
- **DE NICOLO A ET AL.: 'A novel breast cancer-associated BRIP1 (FANCJ/BACH1) germ-line mutation impairs protein stability and function' CLIN CANCER RES. vol. 14, no. 14, 15 July 2008, pages 4672 - 4680, XP055120596**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KARPPINEN SM ET AL.: 'Nordic collaborative study of the BARD1 Cys557Ser allele in 3956 patients with cancer: enrichment in familial BRCA1/BRCA2 mutation-negative breast cancer but not in other malignancies' J MED GENET. vol. 43, no. 11, November 2006, pages 856 - 862, XP055120597
- SONG H ET AL.: 'Tagging single nucleotide polymorphisms in the BRIP1 gene and susceptibility to breast and ovarian cancer' PLOS ONE. vol. 2, no. 3, 07 March 2007, page E268, XP055120599
- BARROSO E ET AL.: 'The Fanconi anemia family of genes and its correlation with breast cancer susceptibility and breast cancer features' BREAST CANCER RES TREAT. vol. 118, no. 3, December 2009, pages 655 - 660, XP019746629
- WALSH T ET AL.: 'Ten genes for inherited breast cancer' CANCER CELL. vol. 11, no. 2, February 2007, pages 103 - 105, XP055120600
- JOHNATTY SE ET AL.: 'The BARD Cys557Ser polymorphism and breast cancer risk: an Australian case-control and family analysis' BREAST CANCER RES TREAT. vol. 115, no. 1, May 2009, pages 145 - 150, XP019671243
- KUUSISTO KM ET AL.: 'Screening for BRCA1, BRCA2, CHEK2, PALB2, BRIP1, RAD50, and CDH1 mutations in high-risk Finnish BRCA1/2-founder mutation-negative breast and/or ovarian cancer individuals' BREAST CANCER RES. vol. 13, no. 1, 28 February 2011, page R20, XP021099291
- RUTTER JL ET AL.: 'Mutational analysis of the BRCA1-interacting genes ZNF350/ZBRK1 and BRIP1/BACH1 among BRCA1 and BRCA2-negative probands from breast-ovarian cancer families and among early-onset breast cancer cases and reference individuals' HUM MUTAT. vol. 22, no. 2, August 2003, pages 121 - 128, XP002467675
- VAHTERISTO P ET AL.: 'BACH1 Ser919Pro variant and breast cancer risk' BMC CANCER. vol. 6, 24 January 2006, page 19, XP021004869
- REBBECK TR ET AL.: 'Modification of BRCA1-Associated Breast and Ovarian Cancer Risk by BRCA1-Interacting Genes' CANCER RES. vol. 71, no. 17, 01 September 2011, pages 5792 - 5805, XP055120603
- RAFNAR T ET AL.: 'Mutations-in BRIP1 confer high risk of ovarian cancer' NAT GENET. vol. 43, no. 11, 02 October 2011, pages 1104 - 1107, XP055120607
- PENNINGTON KP ET AL.: 'Hereditary ovarian cancer: beyond the usual suspects' GYNECOL ONCOL. vol. 124, no. 2, February 2012, pages 347 - 353, XP028445105
- WONG MW ET AL.: 'BRIP1, PALB2, and RAD51C mutation analysis reveals their relative importance as genetic susceptibility factors for breast cancer' BREAST CANCER RES TREAT. vol. 127, no. 3, June 2011, pages 853 - 859, XP055120608
- RAY AM ET AL.: 'Absence of truncating BRIP1 mutations in chromosome 17q-linked hereditary prostate cancer families' BR J CANCER. vol. 101, no. 12, 15 December 2009, pages 2043 - 2047, XP055120612
- BRADBURY AR ET AL.: 'Genetic susceptibility to breast cancer' REV ENDOCR METAB DISORD. vol. 8, no. 3, September 2007, pages 255 - 267, XP027106316

## Description

Malignant cancer is characterized by uncontrolled growth within specific cell groups, invasion that intrudes upon and destroys adjacent tissues. Cancer often metastasizes, wherein tumor cells spread to other locations in the body via the lymphatic system or through the bloodstream.

Ovarian cancer is the fifth most common cause of cancer death in women in the US. Five-year relative survival rate is less than 45% with the stage at diagnosis being the major prognostic factor. Only 19% of ovarian cancer cases are diagnosed while the cancer is still localized and chances of cure are over 90%. A striking 68% are diagnosed after the cancer has already metastasized.

Ovarian cancer causes non-specific symptoms. Most women with ovarian cancer report one or more symptoms such as abdominal pain or discomfort, an abdominal mass, bloating, back pain, urinary urgency, constipation, tiredness and a range of other non-specific symptoms, as well as more specific symptoms such as pelvic pain, abnormal vaginal bleeding or involuntary weight loss.

In the absence of effective treatment for advanced ovarian cancer, the major emphasis is on developing screening programs that will detect the disease at an early stage. Ovarian cancer screening with transvaginal ultrasound (TVU) and CA-125 was evaluated in the Prostate, Lung, Colorectal and Ovarian (PLCO) Trial, including almost 40,000 women. Screening identified both early- and late-stage neoplasms; however, the predictive value of both tests was relatively low and the effect of screening on ovarian cancer mortality still needs a longer follow-up. This trial, along with other studies, has led the U.S. Preventive Services Task Force to conclude that despite evidence that screening with serum CA-125 level or TVU can detect ovarian cancer at an earlier stage than it can be detected in the absence of screening, earlier detection would likely have a small effect on mortality from ovarian cancer.

Given that approximately 1 in 72 women will be diagnosed with cancer of the ovary during their lifetime, repeated screening of the whole population with costly procedures like ultrasound is not a feasible strategy. This is particularly true considering the large number of false positive cases that need follow-up by surgical procedures with the associated risks of side effects. Management strategies that aim to identify those individuals at highest risk of the disease could be used to focus screening efforts on women who will benefit the most from them while minimizing unnecessary interventions and anxiety amongst those at lower risk. Clearly, the implementation of such strategies depends on the development of a risk model that includes all known risk factors.

The strongest factors affecting ovarian cancer risk are family history (genetic factors), age, race, the number of children and endocrine history. Presently, all these factors can be evaluated except for the genetic factors. The role of inherited factors in ovarian cancer has been firmly established in epidemiological and family studies. Studies on 44,788 pairs of twins in the Swedish, Danish, and Finnish twin registries estimated that genetic factors can explain about 22% of ovarian cancer. Furthermore, a meta-analysis of data from 15 observational studies estimated that the relative risk of developing ovarian cancer for a woman with a single first-degree relative affected with ovarian cancer is 3.1. Two cancer syndromes include ovarian cancer as a part of their phenotype; the hereditary breast/ovarian cancer syndrome and Lynch syndrome. The majority of families with extensive family history of breast and ovarian cancer harbour mutations in the breast cancer genes, *BRCA1* and *BRCA2,* while Lynch syndrome is caused by mutations in DNA mismatch repair genes. However, these two cancer syndromes constitute a small fraction (5-15%) of ovarian cancer cases. It has been found that the Icelandic founder *BRCA2 999del5* mutation is present in 6% of ovarian cancer cases in Iceland and is associated with a 20-fold increase in the risk of the disease. The remaining genetic risk of ovarian cancer is likely due to the combined effects of multiple variants yet to be identified.

REBBECK ET AL ("Modification of Ovarian Cancer Risk by BRCA1/2-Interacting Genes in a Multicenter Cohort of BRCA1/2 Mutation Carriers", CANCER RESEARCH, vol. 69, no. 14, pages 5801-5810) disclose that two haplotypes in BRIP1 are associated to ovarian cancer risk.

## SUMMARY OF THE INVENTION

The present inventors have discovered that variants on chromosome 17q23.2 in the human *BRIP1* gene are associated with increased risk of ovarian cancer. The present invention relates to the utilization of such variants in the risk management of ovarian cancer. For simplicity, many details of the invention, including details related to *BRIP1* or techniques or materials for practicing the invention are described in the context of predicting susceptibility to ovarian cancer.

In one aspect, the invention provides a method of determining a susceptibility to ovarian cancer, the method comprising analyzing sequence data from a human individual for at least one frameshift or nonsense polymorphic marker in the human *BRIP1* gene, or an encoded BRIP1 protein, wherein different alleles of the at least one polymorphic marker are associated with different susceptibilities to cancer in humans, and determining a susceptibility to ovarian cancer from the sequence data. In certain embodiments, the sequence data is nucleic acid sequence data. In a preferred embodiment, the at least one polymorphic marker is an -/AA insertion/deletion polymorphism at position 57,208,601 in NCBI Build 36 (SEQ ID NO:12) (chr17:57208601 ins+AA) or a TT deletion at position 57213073 in NCBI Build 36 (chr17: 57213073 delTT).

Another aspect relates to a method of method of determining a susceptibility to a ovarian cancer, the method comprising analyzing data representative of at least one frameshift or nonsense allele of a *BRIP1* gene in a human subject, wherein different alleles of the human *BRIP1* gene are associated with different susceptibilities to ovarian cancer in humans, and determining a susceptibility to a ovarian cancer for the human subject from the data.

The invention also provides a method of determining a susceptibility to Ovarian Cancer, the method comprising analyzing sequence data from a human subject for at least one frameshift or nonsense variant in the human *BRIP1* gene, or in an encoded human BRIP1 protein, wherein different alleles of the at least one variant are associated with different susceptibilities to Ovarian Cancer in humans, and determining a susceptibility to Ovarian Cancer for the human subject from the sequence data.

Further provided is a method of analyzing nucleic acid sequence data from a human individual for at least one polymorphic marker selected from the group consisting of: an -/AA insertion/deletion polymorphism between position 57,208,601 and 57,208,602 in NCBI Build 36 (SEQ ID NO:12); rs34289250 (SEQ ID NO:1); rs12938171 (SEQ ID NO:2); an A/T polymorphism at position 55,422,245 in NCBI Build 36 (SEQ ID NO:3); a C/T polymorphism at position 55,217,320 in NCBI Build 36 (SEQ ID NO:4); rs12451939 (SEQ ID NO:5); a G/T polymorphism at position 56,567,990 in NCBI Build 36 (SEQ ID NO:6); an A/C polymorphism at position 56,478,611 in NCBI Build 36 (SEQ ID NO:7); a C/T polymorphism at position 56,505,864 in NCBI Build 36 (SEQ ID NO:8); and rs12937080 (SEQ ID NO:9), and markers in linkage disequilibrium therewith, wherein different alleles of the at least one polymorphic marker are associated with different susceptibilities to Ovarian Cancer in humans, and determining a susceptibility to Ovarian Cancer from the nucleic acid sequence data.

Also provided is a method of determining whether an individual is at increased risk of developing ovarian cancer, the method comprising steps of determining, in a biological sample from an individual, nucleic acid sequence about the *BRIP1* gene; and comparing the wild-type sequence of BRIP1 (SEQ ID NO:10); wherein an identification of a nonsense mutation or a frameshift mutation in BRIP1 in the individual is indicative that the individual is at increased risk of developing ovarian cancer.

The disclosure further provides a method of identification of a marker for use in assessing susceptibility to Ovarian cancer in human individuals, the method comprising (a) identifying at least one polymorphic marker in the human *BRIP1* gene; (b) obtaining sequence information about the at least one polymorphic marker in a group of individuals diagnosed with ovarian cancer; and (c) obtaining sequence information about the at least one polymorphic marker in a group of control individuals; wherein determination of a significant difference in frequency of at least one allele in the at least one polymorphism in individuals diagnosed with ovarian cancer as compared with the frequency of the at least one allele in the control group is indicative of the at least one polymorphism being useful for assessing susceptibility to ovarian cancer.Further provided are prognostic methods and methods of assessing probability of response to treatment. Thus, the disclosure provides a method of predicting prognosis of an individual diagnosed with Ovarian Cancer, the method comprising obtaining sequence data about a human individual about at least one variant in the human *BRIP1* gene, wherein different alleles of the at least one variant are associated with different susceptibilities to Ovarian Cancer in humans, and predicting prognosis of Ovarian Cancer from the sequence data. The disclosure also relates to a method of predicting prognosis of an individual diagnosed with ovarian cancer, the method comprising obtaining sequence data about a human individual about at least one polymorphic marker in the human *BRIP1* gene, wherein different alleles of the at least one polymorphic marker are associated with different susceptibilities to ovarian cancer in humans, and predicting prognosis of ovarian cancer from the sequence data. Also disclosed is a method of assessing probability of response of a human individual to a therapeutic agent for preventing, treating and/or ameliorating symptoms associated with ovarian cancer, comprising obtaining sequence data about a human individual identifying at least one allele of at least one polymorphic marker in the human *BRIP1* gene, wherein different alleles of the at least one polymorphic marker are associated with different probabilities of response to the therapeutic agent in humans, and determining the probability of a positive response to the therapeutic agent from the sequence data.

Another aspect of the invention relates to methods of selecting treatment regimens. Thus, one aspect of the invention provides a method of selecting a treatment regimen for a human subject with ovarian cancer, the method comprising analyzing data representative of at least one allele of a *BRIP1* gene in a human subject with ovarian cancer to identify the presence or absence of a loss-of-function *BRIP1* mutant allele, and selecting a therapeutic regimen of a therapeutic agent for treating ovarian cancer for a subject identified from the data as having the loss-of-function *BRIP1* mutant allele. Another such aspect related to a method comprising determining the presence or absence of at least one allele of at least one polymorphic marker in a nucleic acid sample obtained from the individual, wherein the at least one allele causes a loss of function or loss of expression of a *BRIP1,* and selecting for treatment with a therapeutic agent for ovarian cancer a subject identified as having the at least one allele in the nucleic acid sample.

The disclosure also provides kits. In one such aspect, the disclosure relates to a kit for assessing susceptibility to ovarian cancer in human individuals, the kit comprising reagents for selectively detecting at least one at-risk variant for ovarian cancer in the individual, wherein the at least one at-risk variant is a marker in the human *BRIP1* gene or an amino acid substitution in an encoded BRIP1 protein, and a collection of data comprising correlation data between the at least one at-risk variant and susceptibility to ovarian cancer.

Further disclosed is the use of an oligonucleotide probe in the manufacture of a diagnostic reagent for diagnosing and/or assessing a susceptibility to ovarian cancer, wherein the probe is capable of hybridizing to a segment of the human *BRIP1* gene with sequence as given by SEQ ID NO:10, and wherein the segment is 15-400 nucleotides in length.

The invention also provides computer-implemented methods and applications. In one such application, the invention relates to a system comprising a computer implemented method for identifying susceptibility to ovarian cancer in a human subject, the system comprising at least one processor, at least one computer-readable medium, a susceptibility database operatively coupled to a computer-readable medium of the system and containing population information correlating the presence or absence of one or more frameshift or nonsense alleles of the human *BRIP1* gene and susceptibility to ovarian cancer in a population of humans, a measurement tool that receives an input about the human subject and generates information from the input about the presence or absence of at least one mutant *BRIP1* allele indicative of a *BRIP1* defect in the human subject; and an analysis tool that is operatively coupled to the susceptibility database and the the measurement tool, is stored on a computer-readable medium of the system, and is adapted to be executed on a processor of the system, to compare the information about the human subject with the population information in the susceptibility database and generate a conclusion with respect to susceptibility to the cancer for the human subject.

Another system is provided for assessing or selecting a treatment protocol for a subject diagnosed with ovarian cancer, comprising at least one processor, at least one computer-readable medium, a medical treatment database operatively connected to a computer-readable medium of the system and containing information correlating the presence or absence of at least one mutant frameshift or nonsense *BRIP1* allele and efficacy of treatment regimens for the cancer, a measurement tool to receive an input about the human subject and generate information from the input about the presence or absence of the at least one mutant *BRIP1* allele indicative of a *BRIP1* defect in a human subject diagnosed with the cancer; and a medical protocol tool operatively coupled to the medical treatment database and the measurement tool, stored on a computer-readable medium of the system, and adapted to be executed on a processor of the system, to compare the information with respect to presence or absence of the at least one mutant *BRIP1* allele for the subject and the medical treatment database, and generate a conclusion with respect to at least one of (1) the probability that one or more medical treatments will be efficacious for treatment of the cancer for the patient, and (2) which of two or more medical treatments for the cancer will be more efficacious for the patient.

Also provided is a computer-readable medium having computer executable instructions for determining susceptibility to Ovarian Cancer in a human individual, the computer readable medium comprising sequence data identifying at least one allele of at least one polymorphic marker in the individual; a routine stored on the computer readable medium and adapted to be executed by a processor to determine risk of developing Ovarian Cancer for the at least one polymorphic marker; wherein the at least one polymorphic marker is a frameshift or nonsense marker in the human *BRIP1* gene, or an encoded BRIP1 protein, that is associated with susceptibility of Ovarian Cancer in humans.

Also disclosed is an apparatus for determining a susceptibility to Ovarian Cancer in a human individual, comprising a processor; a computer readable memory having computer executable instructions adapted to be executed on the processor to analyze sequence information about at least one human individual with respect to at least one marker in the human *BRIP1* gene or an encoded human BRIP1 protein that is associated with susceptibility of Ovarian Cancer in humans, and generate an output based on the marker sequence information, wherein the output comprises at least one measure of susceptibility to Ovarian Cancer for the human individual.

Further details of these and other aspects of the inventions are described in the following detailed description of the invention.

It should be understood that all combinations of features described herein are contemplated, even if the combination of features is not specifically found in the same sentence or paragraph as set forth in the following detailed description.

## BRIEF DESCRIPTION OF THE DRAWINGS

The foregoing and other objects, features and advantages of the invention will be apparent from the following more particular description of preferred embodiments of the invention.

**FIG 1** provides a diagram illustrating a system comprising computer implemented methods utilizing risk variants as described herein.

**FIG 2** shows an exemplary system for determining risk of cancer as described further herein.

**FIG 3** shows a system for selecting a treatment protocol for a subject diagnosed with a cancer.

**FIG 4** shows a schematic representation of the location and consequences of the two indels found in exon 12 (Spain) and exon 14 (Iceland) of *BRIP1.* The mRNA is transcribed of the minus strand.

**FIG 5** shows loss-of-heterozygosity (LOH) in tumor samples from carriers of Chr17:57208601 ins+AA. Sequence of the region around Chr17:57208601 ins+AA in 10 tumor samples from heterozygous carriers of the insert. The tumors are ordered with the tumor showing no LOH first, followed by one tumor with partial LOH and 8 tumors with significant or complete loss of the wild-type allele.

**FIG 6** shows sequence traces of the region over Chr17:57208601 ins+AA in a heterozygous carrier of the insert showing loss of the wild-type allele and mRNA expression in the tumor. Top panel: Sequence of germline DNA from blood, Center panel: Sequence of tumor DNA, Bottom panel: Sequence of cDNA from tumor.

**FIG. 7** depicts a multi-species alignment of BRIP1 amino acid sequences from H.sapiens, P.troglodytes, C.familiaris, M.musculus, R.norvegicus, and G.gallus (SEQ ID NOs:13 (H. sapiens) and 16-20, respectively). Symbols below the sequence alignment highlight residues that are fully (*) or partially (: or .) conserved between the species.

## DETAILED DESCRIPTION

*Definitions*

Unless otherwise indicated, nucleic acid sequences are written left to right in a 5' to 3' orientation. Numeric ranges recited within the specification are inclusive of the numbers defining the range and include each integer or any non-integer fraction within the defined range. Unless defined otherwise, all technical and scientific terms used herein have the same meaning as commonly understood by the ordinary person skilled in the art to which the invention pertains.

The following terms shall, in the present context, have the meaning as indicated:

A "polymorphic marker", sometime referred to as a "marker", as described herein, refers to a genomic polymorphic site. Each polymorphic marker has at least two sequence variations characteristic of particular alleles at the polymorphic site. Thus, genetic association to a polymorphic marker implies that there is association to at least one specific allele of that particular polymorphic marker. The marker can comprise any allele of any variant type found in the genome, including SNPs, mini- or microsatellites, insertion-deletions, translocations and copy number variations (insertions, deletions, duplications). Polymorphic markers can be of any measurable frequency in the population. For mapping of disease genes, polymorphic markers with population frequency higher than 5-10% are in general most useful. However, polymorphic markers may also have lower population frequencies, such as 1-5% frequency, or even lower frequency. The term shall, in the present context, be taken to include polymorphic markers with any population frequency.

An "allele" refers to the nucleotide sequence of a given locus (position) on a chromosome. A polymorphic marker allele thus refers to the composition (i.e., sequence) of the marker on a chromosome. Genomic DNA from an individual contains two alleles (*e.g.*, allele-specific sequences) for any given polymorphic marker, representative of each copy of the marker on each chromosome. Sequence codes for nucleotides used herein are: A = 1, C = 2, G = 3, T = 4.

Sequence conucleotide ambiguity as described herein is according to WIPO ST.25:

| IUB code | Meaning |
|---|---|
| A | Adenosine |
| C | Cytidine |
| G | Guanine |
| T | Thymidine |
| R | G or A |
| Y | T or C |
| K | G or T |
| M | A or C |
| S | G or C |
| W | A or T |

(continued)

| IUB code | Meaning |
|---|---|
| B | C, G or T |
| D | A, G or T |
| H | A, C or T |
| V | A, C or G |
| N | A or G or C or T, unknown or other |

A nucleotide position at which more than one sequence is possible in a population (either a natural population or a synthetic population, *e.g.*, a library of synthetic molecules) is referred to herein as a "polymorphic site".

A "Single Nucleotide Polymorphism" or "SNP" is a DNA sequence variation occurring when a single nucleotide at a specific location in the genome differs between members of a species or between paired chromosomes in an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The SNP nomenclature as reported herein refers to the official Reference SNP (rs) ID identification tag as assigned to each unique SNP by the National Center for Biotechnological Information (NCBI).

A "variant", as described herein, refers to a segment of DNA that comprises a polymorphic site. A "marker" or a "polymorphic marker", as defined herein, is a variant.

A "microsatellite" is a polymorphic marker that has multiple small repeats of bases that are 2-8 nucleotides in length (such as CA repeats) at a particular site, in which the number of repeat lengths varies in the general population.

An "indel", or an "insertion-deletion" is a common form of polymorphism comprising a small insertion or deletion that is typically only a few nucleotides long. One example of an indel is the -/AA polymorphism in the *BRIP1* gene described herein (chr17:57208601 ins+AA). This indel introduces two A nucleotides (AA) between position 57,208,601 and 57,208,602 in NCBI Build 36 of the human genome assembly, as further shown in SEQ ID NO:12 herein. At this position, there are thus two possible alleles: (a) no insertion, thus the sequence is the wild-type sequence (SEQ ID NO:10 and SEQ ID NO:11); and (b) an insertion of AA between position 57,208,601 and 57,208,602 in NCBI Build 36, corresponding to a TT insertion between position 2345 and 2346 in SEQ ID NO:10 (complementary sequence, since gene is transcribed from the minus strand).

A "haplotype," as described herein, refers to a segment of genomic DNA that is characterized by a specific combination of alleles arranged along the segment. For diploid organisms such as humans, a haplotype comprises one member of the pair of alleles for two or more polymorphic markers or loci along the segment. In a certain embodiment, the haplotype can comprise two or more alleles, three or more alleles, four or more alleles, or five or more alleles.

Allelic identities are described herein in the context of the marker name and the particular allele of the marker, *e.g.*, "2 rs34289250" refers to the 2 allele of marker rs34289250, and is equivalent to "rs34289250 allele 2". Furthermore, allelic codes are as for individual markers, i.e. 1 = A, 2 = C, 3 = G and 4 = T.

The term *"BRIP1",* as described herein, refers to the BRCA1-interacting protein 1 gene on chromosome 17q22. This gene is sometimes also referred to as BRCA1-associated C-terminal helicase 1 (*BACH1*). The nucleotide sequence of the gene is shown in SEQ ID NO:10 herein (corresponding to accession number NM_032043.2). The -/AA insertion/deletion polymorphism (chr17:57208601 ins+AA) corresponds to a -/TT indel at position 2345 (i.e., between position 2345 and 2346) in the sequence of a *BRIP1* transcript (cDNA) as set forth in SEQ ID NO:10. Species homologs for human *BRIP1* have been identified and characterized. Deduced amino acid sequences for human and exemplary other vertebrate species homologs are shown in Fig. 7.

The term "susceptibility", as described herein, refers to the proneness of an individual towards the development of a certain state (*e.g.*, a certain trait, phenotype or disease), or towards being less able to resist a particular state than the average individual. The term encompasses both increased susceptibility and decreased susceptibility. Thus, particular alleles at polymorphic markers may be characteristic of increased susceptibility (i.e., increased risk) of Ovarian cancer, as characterized by a relative risk (RR) or odds ratio (OR) of greater than one for the particular allele. Alternatively, the markers and/or haplotypes of the invention are characteristic of decreased susceptibility (i.e., decreased risk) of Ovarian cancer, as characterized by a relative risk of less than one.

The term "and/or" shall in the present context be understood to indicate that either or both of the items connected by it are involved. In other words, the term herein shall be taken to mean "one or the other or both".

The term "look-up table", as described herein, is a table that correlates one form of data to another form, or one or more forms of data to a predicted outcome to which the data is relevant, such as phenotype or trait. For example, a look-up table can comprise a correlation between allelic data for at least one polymorphic marker and a particular trait or phenotype, such as a particular disease diagnosis, that an individual who comprises the particular allelic data is likely to display, or is more likely to display than individuals who do not comprise the particular allelic data. Look-up tables can be multidimensional, *i.e.* they can contain information about multiple alleles for single markers simultaneously, or they can contain information about multiple markers, and they may also comprise other factors, such as particulars about diseases diagnoses, racial information, biomarkers, biochemical measurements, therapeutic methods or drugs, etc.

The term "database" refers to a collection of data organized for one or more purposes. In the context of the invention, databases may be organized in a digital format for access, analysis, or processing by a computer. The data are typically organized to model features relevant to the invention. For instance, one component of data in a database may be information about variations in a population, such as genetic variation with respect to *BRIP1,* but also variation with respect to other medically informative parameters, including other genetic loci, race, ethnicity, sex, age, behaviors and lifestyle (tobacco consumption (smoking), alcohol consumption (drinking), exercise, body mass indices), glucose tolerance/diabetes, and any other factors that medical personnel may measure in the context of standard medical care or specific diagnoses. Other components of the database may include one or more sets of data relating to susceptibility to a disease in a population, and/or suitability or success of a disease treatment, and/or suitability or success of a protocol for screening for or presenting a disease. Preferably the data is organized to permit analysis of how the biological variation in the population correlates with the susceptibility to disease and/or the suitability or success of the treatment, protocol etc. A look-up datable (or the information in a look-up table) may be stored in a database to facilitate aspects of the invention.

A "computer-readable medium", is an information storage medium that can be accessed by a computer using a commercially available or custom-made interface. Exemplary computer-readable media include memory (*e.g.*, RAM, ROM, flash memory, etc.), optical storage media (*e.g.*, CD-ROM), magnetic storage media (*e.g.*, computer hard drives, floppy disks, etc.), punch cards, or other commercially available media. Information may be transferred between a system of interest and a medium, between computers, or between computers and the computer-readable medium for storage or access of stored information. Such transmission can be electrical, or by other available methods, such as IR links, wireless connections, etc.

The term "biological sample" refers to a sample obtained from an individual that contains nucleic acid and/or protein and/or fluid containing organic and/or inorganic metabolites and substances. In many variations of the invention, the biological sample comprises nucleic acid suitable for genetic analysis.

A "nucleic acid sample" as described herein, refers to a sample obtained from an individual that contains nucleic acid (DNA or RNA). In certain embodiments, i.e. the detection of specific polymorphic markers and/or haplotypes, the nucleic acid sample comprises genomic DNA. Such a nucleic acid sample can be obtained from any source that contains genomic DNA, including a blood sample, sample of amniotic fluid, sample of cerebrospinal fluid, or tissue sample from skin, muscle, buccal or conjunctival mucosa, placenta, gastrointestinal tract or other organs.

The term "antisense agent" or "antisense oligonucleotide" refers, as described herein, to molecules, or compositions comprising molecules, which include a sequence of purine an pyrimidine heterocyclic bases, supported by a backbone, which are effective to hydrogen bond to corrresponding contiguous bases in a target nucleic acid sequence. The backbone is composed of subunit backbone moieties supporting the purine and pyrimidine hetercyclic bases at positions which allow such hydrogen bonding. These backbone moieties are cyclic moieties of 5 to 7 atoms in size, linked together by phosphorous-containing linkage units of one to three atoms in length. In certain preferred embodiments, the antisense agent comprises an oligonucleotide molecule.

*Variants on chromosome 17q23.2 associate with cancer*

It has been discovered that variants on chromosome 17q23.2 are associated with risk of cancer. In particular, it has been discovered that mutations in the *BRIP1* gene have a large effect on the risk of cancer.

The inventors identified a large number of SNP variants in this chromosomal region that confer significant risk of ovarian cancer. Further sequencing analysis revealed a frame-shift two basepair insertion (-/AA; chr17: 57208601 ins+AA) in exon 14 of the gene encoding BRCA1-interacting protein 1 (BRIP1). This two-basepair insertion confers a risk of over 7, and has a carrier frequency of about 0.7% in the population, but about 5% in individuals with ovarian cancer. Further analysis of this variant revealed its association with other cancers, including Pancreatic cancer, Colorectal Cancer, Upper Airways Cancer and Rectal cancer.

A second frameshift mutation, a TT insertion/deletion polymorphism (-/TT) at position 57,213,073 in NCBI Build 36 (chr17: 57213073 delTT; corresponds to AA deletion in position 2008 - 2009 in the cDNA sequence set forth in SEQ ID NO:10) was further identified. Subsequent analysis showed that this variant confers risk of both ovarian and breast cancer.

Ovarian tumors from *BRIP1* mutation carriers show loss of the wild type allele suggesting that the *BRIP1* gene behaves like a classical tumor suppressor gene in ovarian cancer; one copy of the gene is lost or defective due a heterozygous germline mutation, and the second wild-type (normal) copy is lost in the tumor.

Due to the relatively low prevalence of ovarian cancer, apparent sporadic cases of ovarian cancer may thus actually be due to rare mutations with large effects. These findings, which are described in more detail in the following, show that BRIP1 variants are predictive of risk of cancer.

*Variants in BRIP1 are predictive of cancer risk*

The *BRIP1* gene (*BRCA1* interacting protein 1; also called *BACH1* and *FANCJ*) was identified by screening for proteins that interact with the C-terminal BRCT domain in BRCA1 (reviewed in Cantor & Suillemette, Future Oncol 7:253-261 (2011)).

BRIP1 interacts with the BRCT domain of BRCA1 and has several BRCA1-dependent, as well as independent, functions in preserving the integrity of the genome. It is required for homologous recombination (HR)-mediated double strand break repair (Litman, R., et al. Cancer Cell 8:255 (2005)), the execution of a G2/M cell-cycle checkpoint (Yu,X. et al., Science 302:639 (2003)) and for normal progression through S-phase by assisting in the resolution of stalled replication forks (Kumaraswamy, E., et al. Mol Cell Biol 27:6733 (2007)). Furthermore, mutations in BRIP1 that impair its helicase function render cells highly sensitive to crosslinking agents such as cisplatin (Bridge, W.L., et al. Nat Genet 37:953 (2005)). *BRIP1* is located about 20 Mb telomeric to *BRCA1,* in a region that is frequently lost in ovarian tumors and previous studies have suggested that a tumor suppressor, distinct from *BRCA1,* may reside in this region (Godwin, A.K., et al. Am J Hum Genet 55:666 (1994)).

BRIP1 contains two major structural domains, a helicase domain spanning residues 1 - 888 (Cantor, et al. Cell 105:149-160 (2001)), and a BRCA1 binding domain that spans residues 979 - 1063 (Cantor, et al. PNAS 101:2357-2362 (2004)). The helicase domain further contains a nuclear localization signal (residues 158-175). Loss of function of BRIP1 may thus occur through mutations that affect the helicase domain, the BRCA1 binding domain, or both. Loss of function may also be a result of reduced or complete obliteration of expression of BRIP1, or loss of BRIP1 transcript.

The -/AA insertion/deletion polymorphism (chr17: 57208601 ins+AA) introduces two nucleotides in exon 14 of the human *BRIP1* gene, between position 57,208,601 and 57,208,602, as set forth in SEQ ID NO:12 (between position 59 and 60), corresponding to position 94835 and 94836 in the genomic sequence of *BRIP1* as set forth in SEQ ID NO:15 herein. The result of this insertion is that a stretch of four A residues is increased in size to six consecutive A residues. The chr17: 57213073 delTT mutation leads to a two basepair deletion at position 57213073 (position 99307 in SEQ ID NO:15; *i.e.* position 99308-99309 is deleted), which results in a frameshift and premature termination of protein translation at codon 576.

The present inventors have further shown that the wild-type allele of *BRIP1* is lost in tumors of heterozygous carriers of frameshift mutations, thus showing that *BRIP1* behaves like a tumor suppressor gene (Example 5). This has important clinical implications, as it shows a direct functional relationship between mutations in the gene and loss of function in tumors.

The *BRIP1* gene interacts with the breast cancer *BRCA1* gene and functions in regulating DNA double strand break repair pathways. Variants in the *BRCA1* and *BRCA2* genes are known to confer significant increased risk of breast and ovarian cancer. However, variants in the *BRIP1* gene have to date not been significantly implicated in risk of other cancers, in particular have no high risk variants in this gene been associated with ovarian cancer.

The loss-of-function effect of *BRIP1* mutants in tumors shows that an underlying biological may be the loss of activity of one copy *BRIP1* in germline DNA. Therefore, it is likely that other germline variants in the gene also lead to ovarian cancer, in particular loss-of-function and loss of expression variants. In other words, other variants in the human *BRIP1* gene that lead to loss of function of one copy of the gene, (*e.g.* nonsense and frameshift variants), or variants that lead to reduced or no expression of the gene, are also predictive of risk of ovarian cancer. Such variants are thus also within scope of the invention as described further herein.

A number of factors permit prediction of which *BRIP1* mutations result in loss of function. For example, nonsense mutations that introduce a stop codon are expected to cause loss of function, with earlier introduction of the stop codon (closer to start codon, resulting in elimination of more of the protein) being more likely to cause loss of function. Similarly, frameshift mutations usually cause drastic changes to the amino acid sequence of the encoded protein, and often further result in introduction of a premature stop codon, and therefore are expected to cause loss of function.

In addition, many missense mutations are predicted to cause loss of function, and the character of the missense mutation can be analyzed to improve the prediction. For example, missense mutations that occur in highly conserved regions of BRIP1, as assessed by inter-species alignments such as shown in Fig. 7, are expected to be more likely to cause loss of function than mutations in highly variable regions. Missense mutations that occur in residues recognized as important for the structure or activity of a functional domain of BRIP1 are more likely to cause loss of function

The wild-type BRIP1 polypeptide sequence is set forth in SEQ ID NO: 13. The *BRIP1* gene is conserved over a range of species, and a multiple species alignment is depicted in Figure 7. The alignment shows that the helicase domain is highly conserved across species ranging from humans to chicken. Accordingly, a mutation in the helicase domain is contemplated to affect the translation of the polypeptide or the activity of the translated polypeptide. In addition, mutations in the iron-sulfur (Fe-S) domain, the Nuclear Localization Signal (NLS) domain, the ATP-binding domain and serine 990, which is reported to be required for binding to *BRCA1* (Cantor et al., Future Oncol. 7: 253-261 (2011)), are also contemplated to affect the activity of the BRIP1 polypeptide. Various domains are identified in the SwissProt database, and are listed in the table below. Mutations in any of these domains, or outside of these domains, are contemplated to affect the activity of the BRIP1 polypeptide. In various embodiments of the methods of the disclosure, the residues of particular interest are those that are conserved across the species identified in Figure 7. This is because the conservation of one or more amino acids suggests an evolutionary significance, and loss or mutation of the one or more amino acids can lead to a loss in overall BRIP1 activity.

Table of BRIP1 domains.

| Domain Type | Start | End | Description |
|---|---|---|---|
| Superfamily | 5 | 408 | |
| Superfamily | 656 | 869 | |
| Smart | 17 | 441 | DEAD-like_helicase |
| Pfam | 248 | 415 | DEAD_2 |
| TIGRfam | 149 | 884 | DNA_helicase_DNA-repair_Rad3 |
| Prosite_profiles | 11 | 442 | Helic_SF1/SF2_ATP-bd_DinG/Rad3 |
| Smart | 13 | 437 | Helicase-like_DEXD_c2 |
| Smart | 698 | 851 | Helicase_ATP-dep_c2 |

Missense mutations that result in non-conservative substitutions that introduce new amino acids with different side chain characteristics are more likely to cause loss of function than conservative mutations. Mutations that alter a promoter region or splice site are more likely to affect transcription and expression levels than mutations in other noncoding regions of the gene.

For any mutation that is identified, the mutation's effect on various BRIP1 functions can be confirmed with in vitro experiments as described in Examples below pertaining to BRIP1 functional assays.

*Methods of determining susceptibility to cancer*

Accordingly, in one aspect, the disclosure provides a method of analyzing data representative of at least one allele of a *BRIP1* gene (SEQ ID NO:15) in a human subject, wherein different alleles of the human *BRIP1* gene are associated with different susceptibilities to at least one cancer in humans, and determining a susceptibility to a cancer for the human subject from the data. In certain embodiments, the method is predictive of susceptibility of a cancer selected from ovarian cancer, pancreatic cancer, colorectal cancer, upper airways cancer and breast cancer. In certain preferred embodiments, the cancer is ovarian cancer.

The data can be any type of data that is representative of polymorphic alleles in the *BRIP1* gene. In certain embodiments, the data is nucleic acid sequence data. The sequence data is data that is sufficient to provide information about particular alleles. In certain embodiments, the nucleic acid sequence data is obtained from a biological sample comprising or containing nucleic acid from the human individual. The nucleic acids sequence may suitably be obtained using a method that comprises at least one procedure selected from (i) amplification of nucleic acid from the biological sample; (ii) hybridization assay using a nucleic acid probe and nucleic acid from the biological sample; (iii) hybridization assay using a nucleic acid probe and nucleic acid obtained by amplification of the biological sample, and (iv) sequencing, in particular high-throughput sequencing. The nucleic acid sequence data may also be obtained from a preexisting record. For example, the preexisting record may comprise a genotype dataset for at least one polymorphic marker. In certain embodiments, the determining comprises comparing the sequence data to a database containing correlation data between the at least one polymorphic marker and susceptibility to ovarian cancer. In certain embodiments, the sequence data is provided as genotype data, identifying the presence or absence of particular alleles at polymorphic locations.

In some embodiments, the analyzing comprises analyzing the data for the presence or absence of at least one mutant allele indicative of a *BRIP1* defect. The *BRIP1* defect may for example be a premature truncation or frameshift of an encoded BRIP1 protein, relative to a wild-type amino acid sequence, such as the wild-type amino acid sequence presented in SEQ ID NO:13 herein. The *BRIP1* defect may also be expression of a BRIP1 protein with reduced activity compared to a wild-type BRIP1 protein. The activity can for example be BRIP1 binding to the C-terminal BRCT domain of BRCA1 (*e.g.*, the BRCT domain of wild-type BRCA1). The activity can also be DNA-dependent ATPase activity, and the activity may also be DNA helicase activity. In one embodiment, the *BRIP1* defect is selected from defects that impair any of these activities.

Determination of BRIP1 binding to BRCT domain of BRCA1, ATPase activity and DNA helicase activity can be performed using standard assays well known to the skilled person, some of which are described herein. As noted above, such assays can be used to confirm that a particular *BRIP1* mutation impairs or eliminates a BRIP1 activity and therefor would be expected to carry an increased susceptibility for cancers as described herein.

The data to be analyzed by the method of the invention is suitably obtained by analysis of a biological sample from a human subject to obtain information about particular alleles in the genome of the individual. In certain embodiments, the information is nucleic acid information which comprises sufficient sequence to identify the presence or absence of at least one allele in the subject (*e.g*. a mutant allele). The information can also be nucleic acid information that identifies at least one allele of a polymorphic marker that is in linkage disequilibrium with a mutant allele. Linkage disequilibrium may suitably be determined by the correlation coefficient between polymorphic sites. In one embodiment, the sites are correlated by values of the correlation coefficient $r^2$ of greater than 0.5. Other suitable values of $r^2$ that are also appropriate to characterize polymorphic sites in LD are however also contemplated, as discussed further herein. The information may also be information about measurement of quantity of length of *BRIP1* mRNA, wherein the measurement is indicative of the presence or absence of the mutant allele. For example, mutant alleles may result in premature truncation of transcribed mRNA which can be detected by measuring the length of mRNA. The information may further be measurement of quantity of BRIP1 protein, wherein the measurement of protein is indicative of the presence or absence of a mutant allele. Truncated transcripts will result in truncated forms of translated polypeptides, which can be measured using standard methods known in the art. For example, truncated proteins or proteins arising from a frameshift may have fewer or different epitopes from wildtype protein and can be distinguished with immunoassays. Truncated proteins or proteins altered in other ways may migrate differently and be distinguished with electrophoresis. The information obtained may also be measurement of BRIP1 activity, wherein the measurement is indicative of the mutant allele. The activity is suitably selected from DNA helicase activity, ATPase acitivity and ability to bind to the BRCT domain of BRCA1. In one embodiment, the information is selected from any one of the above mentioned types of information.

In a further embodiment of the invention, a biological sample is obtained from the human subject prior to the analyzing steps. The analyzing may also suitably be performed by analyzing data from a preexisting record about the human subject. The preexisting record may for example include sequence information or genotype information about the individual, which can identify the presence or absence of mutant alleles.

In certain embodiments, information about risk for the human subject can be determined using methods known in the art. Some of these methods are described herein. For example, information about odds ratio (OR), relative risk (RR) or lifetime risk (LR) can be determined from information about the presence or absence of particular mutant alleles of *BRIP1.*

In certain embodiments, the mutant allele of *BRIP* is a frameshift mutation or a nonsense mutation. In one preferred embodiment, the mutant allele is a frameshift mutation. In certain embodiments, the frameshift mutation is selected from the group consisting of chr17:57208601 ins+AA and chr17: 57213073 delTT. In another embodiment, the mutant allele is a missense mutation in *BRIP1* that results in expression of a BRIP1 protein with reduced or no activity compared to a wild-type BRIP1 protein. The mutant allele may also be a promoter polymorphism that leads to decreased expression of *BRIP1.*

In certain embodiments, the mutant allele in *BRIP1* is not one, or a combination of, the following mutant alleles: P47A, G69fs8x, R173C, V193I, R251C, Q255H, M299I, A349P, H291D, R543fs 12x, W647C, R707C, K752fs 11x, R798x, Y800X, R831fs3x, G859fs3x, Q944E, K998fs59x and P1034L.

In certain embodiments, the mutant allele is not one of, or a combination of, the following mutant allele: P47A, R173C, V193I, M299I, R798x, Q944E, K998fs59x and P1034L.

In this context, frameshift mutations are indicated by "fs", and the following number indicates the position of the first stop codon created by the new reading frame. Thus, "G69fs8x" indicates that a frameshift occurs starting at codon 69, and that a stop codon is introduced 8 codons downstream, counting from codon 69.

It should be apparent from the foregoing that another aspect of the invention may relate to a method of determining whether an individual is at increased risk of developing ovarian cancer, the method comprising steps of (a) obtaining a biological sample containing nucleic acid from the individual; (b) determining, in the biological sample, nucleic acid sequence about the *BRIP1* gene, and (c) comparing the sequence information to the wild-type sequence of *BRIP1,* as set forth in SEQ ID NO:10 herein, wherein the identification of a mutation in *BRIP1* in the individual is indicative that the individual is at increased risk of developing ovarian cancer.

Alternatively, the invention provides a method of determining whether an individual is at increased risk of developing ovarian cancer, the method comprising steps of determining, in a biological sample from the individual, nucleic acid sequence about the *BRIP1* gene, and comparing the sequence information to the wild-type sequence of *BRIP1,* as set forth in SEQ ID NO:10 herein, wherein the identification of a mutation in *BRIP1* in the individual is indicative that the individual is at increased risk of developing ovarian cancer.

The mutation may be a a nonsense mutation or a frameshift mutation in *BRIP1.* The mutation may further result in a *BRIP1* defect as described in the above.

In any of the methods described herein, the human subject or human individual whose susceptibility of cancer is being assessed may be a male or a female. It will be readily apparent that risk for, e.g., ovarian cancer will be assessed in females, although assays of the invention, when practiced on males, may have informative value for female relatives in the context of ovarian cancer risk.

In another aspect, the invention provides a method of determining a susceptibility to Ovarian Cancer, the method comprising analyzing sequence data from a human subject for at least one frameshift or nonsense variant in the human *BRIP1* gene, or in an encoded human BRIP1 protein, wherein different alleles of the at least one variant are associated with different susceptibilities to Ovarian Cancer in humans, and determining a susceptibility to Ovarian Cancer for the human subject from the sequence data. In a preferred embodiment, the variant is the - /AA insertion/deletion polymorphism between position 57,208,601 and 57,208,602 in NCBI Build 36 (SEQ ID NO:12). In another embodiment, the variant is a variant in linkage disequilibrium with the -/AA insertion/deletion polymorphism.

The -/AA insertion/deletion results in an increase in length of a stretch of A residues in the human *BRIP1* gene. Thus, the wild-type sequence has a stretch of AAAA beginning at position 57,208,602 in NCBI Build 36, and the insertion of two A residues results in a stretch of six consecutive A nucleotides (AAAAAA). The skilled person will thus appreciate that in principle the location of the indel may be anywhere within the stretch of four A residues; the resulting stretch of nucleotides would always be that of six consecutive A residues. The present inventors have for the sake of convenience, placed the indel at the first position in the stretch, *i.e*. between position 57,208,601 and 57,208,602.

In certain embodiments, the data that is obtained is nucleic acid sequence data. In certain embodiments, the nucleic acid sequence data is obtained from a biological sample comprising or containing nucleic acid from the human individual. The nucleic acids sequence may suitably be obtained using a method that comprises at least one procedure selected from (i) amplification of nucleic acid from the biological sample; (ii) hybridization assay using a nucleic acid probe and nucleic acid from the biological sample; (iii) hybridization assay using a nucleic acid probe and nucleic acid obtained by amplification of the biological sample, and (iv) sequencing, in particular high-throughput sequencing. The nucleic acid sequence data may also be obtained from a preexisting record. For example, the preexisting record may comprise a genotype dataset for at least one polymorphic marker. In certain embodiments, the determining comprises comparing the sequence data to a database containing correlation data between the at least one polymorphic marker and susceptibility to ovarian cancer.

Certain risk alleles have been found to be predictive of increased risk of ovarian cancer. Thus, in certain embodiments, determination of the presence of at least one allele selected from the group consisting of an AA insertion between position 57,208,601 and 57,208,602 in NCBI Build 36 (SEQ ID NO:12); a C allele of rs34289250 (SEQ ID NO:1); an A allele of rs12938171(SEQ ID NO:2); an A allele of an A/T polymorphism at position 55,422,245 in NCBI Build 36 (SEQ ID NO:3); a C allele of an C/T polymorphism at position 55,217,320 in NCBI Build 36 (SEQ ID NO:4); a G allele of rs12451939 (SEQ ID NO:5); a G allele of a G/T polymorphism at position 56,567,990 in NCBI Build 36 (SEQ ID NO:6); an A allele of an A/C polymorphism at position 56,478,611 in NCBI Build 36 (SEQ ID NO:7); a C allele of a C/T polymorphism at position 56,505,864 in NCBI Build 36 (SEQ ID NO:8); and an G allele of rs12937080 (SEQ ID NO:9) is indicative of an increased susceptibility of Ovarian Cancer for the human subject

The AA insertion is indicative of increased risk of ovarian cancer. Thus, in certain embodiment, determination of the presence of the AA insertion is indicative of increased risk of ovarian cancer for the individual. Determination of the absence of the AA insertion, or another variant allele conferring increased risk of ovarian cancer is indicative that the individual does not have the increased risk conferred by the allele.

Alternatively, the allele that is detected can be the allele of the complementary strand of DNA, such that the nucleic acid sequence data identifies at least one allele which is complementary to any of the alleles of the polymorphic markers referenced above. For example, the allele that is detected may be the complementary TT allele of the at-risk AA allele of the -/AA insertion/deletion polymorphism.

It is contemplated that in certain embodiments of the invention, it may be convenient to prepare a report of results of risk assessment. Thus, certain embodiments of the methods of the invention comprise a further step of preparing a report containing results from the determination of risk, wherein said report is written in a computer readable medium, printed on paper, or displayed on a visual display. In certain embodiments, it may be convenient to report results of susceptibility to at least one entity selected from the group consisting of the individual, a guardian of the individual, a genetic service provider, a physician, a medical organization, and a medical insurer.

*Obtaining nucleic acid sequence data*

Sequence data can be nucleic acid sequence data, which may be obtained by means known in the art. Sequence data is suitably obtained from a biological sample of genomic DNA, RNA, or cDNA (a "test sample") from an individual ("test subject). For example, nucleic acid sequence data may be obtained through direct analysis of the sequence of the polymorphic position (allele) of a polymorphic marker. Suitable methods, some of which are described herein, include, for instance, whole genome sequencing methods, whole genome analysis using SNP chips (e.g., Infinium HD BeadChip), cloning for polymorphisms, non-radioactive PCR-single strand conformation polymorphism analysis, denaturing high pressure liquid chromatography (DHPLC), DNA hybridization, computational analysis, single-stranded conformational polymorphism (SSCP), restriction fragment length polymorphism (RFLP), automated fluorescent sequencing; clamped denaturing gel electrophoresis (CDGE); denaturing gradient gel electrophoresis (DGGE), mobility shift analysis, restriction enzyme analysis; heteroduplex analysis, chemical mismatch cleavage (CMC), RNase protection assays, use of polypeptides that recognize nucleotide mismatches, such as E. coli mutS protein, allele-specific PCR, and direct manual and automated sequencing. These and other methods are desribed in the art (see, for instance, Li et al., Nucleic Acids Research, 28(2): e1 (i-v) (2000); Liu et al., Biochem Cell Bio 80:17-22 (2000); and Burczak et al., Polymorphism Detection and Analysis, Eaton Publishing, 2000; Sheffield et al., Proc. Natl. Acad. Sci. USA, 86:232-236 (1989); Orita et al., Proc. Natl. Acad. Sci. USA, 86:2766-2770 (1989); Flavell et al., Cell, 15:25-41 (1978); Geever et al., Proc. Natl. Acad. Sci. USA, 78:5081-5085 (1981); Cotton et al., Proc. Natl. Acad. Sci. USA, 85:4397-4401 (1985); Myers et al., Science 230:1242-1246 (1985); Church and Gilbert, Proc. Natl. Acad. Sci. USA, 81:1991-1995 (1988); Sanger et al., Proc. Natl. Acad. Sci. USA, 74:5463-5467 (1977); and Beavis et al., U.S. Patent No. 5,288,644).

Recent technological advances have resulted in technologies that allow massive parallel sequencing to be performed in relatively condensed format. These technologies share sequencing-by-synthesis principle for generating sequence information, with different technological solutions implemented for extending, tagging and detecting sequences. Exemplary technologies include 454 pyrosequencing technology (Nyren, P. et al. Anal Biochem 208:171-75 (1993); http://www.454.com), Illumina Solexa sequencing technology (Bentley, D.R. Curr Opin Genet Dev 16:545-52 (2006); http://www.illumina.com), and the SOLiD technology developed by Applied Biosystems (ABI) (http://www.appliedbiosystems.com; see also Strausberg, R.L., et al. Drug Disc Today 13:569-77 (2008)). Other sequencing technologies include those developed by Pacific Biosciences (http://www.pacificbiosciences.com), Complete Genomics (http://www.completegenomics.com), Intelligen Bio-Systems (http://www.intelligentbiosystems.com), Genome Corp (http://www.genomecorp.com), ION Torrent Systems (http://www.iontorrent.com) and Helicos Biosciences (http://www.helicosbio.som). It is contemplated that sequence data useful for performing the present invention may be obtained by any such sequencing method, or other sequencing methods that are developed or made available. Thus, any sequence method that provides the allelic identity at particular polymorphic sites (*e.g.*, the absence or presence of particular alleles at particular polymorphic sites) is useful in the methods described and claimed herein.

Alternatively, hybridization methods may be used (see Current Protocols in Molecular Biology, Ausubel et al., eds., John Wiley & Sons, including all supplements). For example, a biological sample of genomic DNA, RNA, or cDNA (a "test sample") may be obtained from a test subject. The subject can be an adult, child, or fetus. The DNA, RNA, or cDNA sample is then examined. The presence of a specific marker allele can be indicated by sequence-specific hybridization of a nucleic acid probe specific for the particular allele. The presence of more than one specific marker allele or a specific haplotype can be indicated by using several sequence-specific nucleic acid probes, each being specific for a particular allele. A sequence-specific probe can be directed to hybridize to genomic DNA, RNA, or cDNA. A "nucleic acid probe", as used herein, can be a DNA probe or an RNA probe that hybridizes to a complementary sequence. One of skill in the art would know how to design such a probe so that sequence specific hybridization will occur only if a particular allele is present in a genomic sequence from a test sample.

In certain embodiments, determination of a susceptibility to ovarian cancer comprises forming a hybridization sample by contacting a test sample, such as a genomic DNA sample, with at least one nucleic acid probe. A non-limiting example of a probe for detecting mRNA or genomic DNA is a labeled nucleic acid probe that is capable of hybridizing to mRNA or genomic DNA sequences described herein. The nucleic acid probe can be, for example, a full-length nucleic acid molecule, or a portion thereof, such as an oligonucleotide of at least 10, 15, 30, 50, 100, 250 or 500 nucleotides in length that is sufficient to specifically hybridize under stringent conditions to appropriate mRNA or genomic DNA. For example, the nucleic acid probe can comprise all or a portion of the nucleotide sequence of the *BRIP1* gene, or the probe can be the complementary sequence of such a sequence. Hybridization can be performed by methods well known to the person skilled in the art (see, e.g., Current Protocols in Molecular Biology, Ausubel et al., eds., John Wiley & Sons, including all supplements). In one embodiment, hybridization refers to specific hybridization, i.e., hybridization with no mismatches (exact hybridization). In one embodiment, the hybridization conditions for specific hybridization are high stringency.

Specific hybridization, if present, is detected using standard methods. If specific hybridization occurs between the nucleic acid probe and the nucleic acid in the test sample, then the sample contains the allele that is complementary to the nucleotide that is present in the nucleic acid probe.

Additionally, or alternatively, a peptide nucleic acid (PNA) probe can be used in addition to, or instead of, a nucleic acid probe in the hybridization methods described herein. A PNA is a DNA mimic having a peptide-like, inorganic backbone, such as N-(2-aminoethyl)glycine units, with an organic base (A, G, C, T or U) attached to the glycine nitrogen via a methylene carbonyl linker (see, for example, Nielsen et al., Bioconjug. Chem. 5:3-7 (1994)). The PNA probe can be designed to specifically hybridize to a molecule in a sample suspected of containing one or more of the marker alleles shown herein to be associated with risk of ovarian cancer.

In one embodiment of the invention, a test sample containing genomic DNA obtained from the subject is collected and the polymerase chain reaction (PCR) is used to amplify a fragment comprising one or more polymorphic marker. As described herein, identification of particular marker alleles can be accomplished using a variety of methods. In another embodiment, determination of susceptibility is accomplished by expression analysis, for example using quantitative PCR (kinetic thermal cycling). This technique can, for example, utilize commercially available technologies, such as TaqMan® (Applied Biosystems, Foster City, CA) . The technique can for example assess the presence of an alteration in the expression or composition of a polypeptide or splicing variant(s) that is encoded by an associated nucleic acid described herein. Alternatively, this technique may assess expression levels of genes or particular splice variants of genes, that are affected by one or more of the variants described herein. Further, the expression of the variant(s) can be quantified as physically or functionally different.

Allele-specific oligonucleotides can also be used to detect the presence of a particular allele in a nucleic acid. An "allele-specific oligonucleotide" (also referred to herein as an "allele-specific oligonucleotide probe") is an oligonucleotide of any suitable size, for example an oligonucleotide of approximately 10-50 base pairs or approximately 15-30 base pairs, that specifically hybridizes to a nucleic acid which contains a specific allele at a polymorphic site (e.g., a polymorphic marker). An allele-specific oligonucleotide probe that is specific for one or more particular alleles at polymorphic markers can be prepared using standard methods (see, e.g., *Current Protocols in Molecular Biology, supra*). PCR can be used to amplify the desired region. Specific hybridization of an allele-specific oligonucleotide probe to DNA from a subject is indicative of the presence of a specific allele at a polymorphic site (see, e.g., Gibbs et al., Nucleic Acids Res. 17:2437-2448 (1989) and WO 93/22456).

With the addition of analogs such as locked nucleic acids (LNAs), the size of primers and probes can be reduced to as few as 8 bases. LNAs are a novel class of bicyclic DNA analogs in which the 2' and 4' positions in the furanose ring are joined via an O-methylene (oxy-LNA), S-methylene (thio-LNA), or amino methylene (amino-LNA) moiety. Common to all of these LNA variants is an affinity toward complementary nucleic acids, which is by far the highest reported for a DNA analog. For example, particular all oxy-LNA nonamers have been shown to have melting temperatures (Tm) of 64°C and 74°C when in complex with complementary DNA or RNA, respectively, as opposed to 28°C for both DNA and RNA for the corresponding DNA nonamer. Substantial increases in Tm are also obtained when LNA monomers are used in combination with standard DNA or RNA monomers. For primers and probes, depending on where the LNA monomers are included (e.g., the 3' end, the 5' end, or in the middle), the Tm could be increased considerably. It is therefore contemplated that in certain embodiments, LNAs are used to detect particular alleles at polymorphic sites associated with particular vascular conditions, as described herein.

In certain embodiments, arrays of oligonucleotide probes that are complementary to target nucleic acid sequence segments from a subject can be used to identify polymorphisms in a nucleic acid. For example, an oligonucleotide array can be used. Oligonucleotide arrays typically comprise a plurality of different oligonucleotide probes that are coupled to a surface of a substrate in different known locations. These arrays can generally be produced using mechanical synthesis methods or light directed synthesis methods that incorporate a combination of photolithographic methods and solid phase oligonucleotide synthesis methods, or by other methods known to the person skilled in the art (see, e.g., Bier et al., Adv Biochem Eng Biotechnol 109:433-53 (2008); Hoheisel, Nat Rev Genet 7:200-10 (2006); Fan et al., Methods Enzymol 410:57-73 (2006); Raqoussis & Elvidge, Expert Rev Mol Diagn 6:145-52 (2006); Mockler et al., Genomics 85:1-15 (2005), and references cited therein, the entire teachings of each of which are incorporated by reference herein). Many additional descriptions of the preparation and use of oligonucleotide arrays for detection of polymorphisms can be found, for example, in US 6,858,394, US 6,429,027, US 5,445,934, US 5,700,637, US 5,744,305, US 5,945,334, US 6,054,270, US 6,300,063, US 6,733,977, US 7,364,858, EP 619 321, and EP 373 203, the entire teachings of which are incorporated by reference herein.

Also, standard techniques for genotyping can be used to detect particular marker alleles, such as fluorescence-based techniques (*e.g.*, Chen et al., Genome Res. 9(5): 492-98 (1999); Kutyavin et al., Nucleic Acid Res. 34:e128 (2006)), utilizing PCR, LCR, Nested PCR and other techniques for nucleic acid amplification. Specific commercial methodologies available for SNP genotyping include, but are not limited to, TaqMan genotyping assays and SNPlex platforms (Applied Biosystems), gel electrophoresis (Applied Biosystems), mass spectrometry (e.g., MassARRAY system from Sequenom), minisequencing methods, real-time PCR, Bio-Plex system (BioRad), CEQ and SNPstream systems (Beckman), array hybridization technology(e.g., Affymetrix GeneChip; Perlegen), BeadArray Technologies (e.g., Illumina GoldenGate and Infinium assays), array tag technology (*e.g.*, Parallele), and endonuclease-based fluorescence hybridization technology (Invader; Third Wave).

Suitable biological sample in the methods described herein can be any sample containing nucleic acid (e.g., genomic DNA) and/or protein from the human individual. For example, the biological sample can be a blood sample, a serum sample, a leukapheresis sample, an amniotic fluid sample, a cerbrospinal fluid sample, a hair sample, a tissue sample from skin, muscle, buccal, or conjuctival mucosa, placenta, gastrointestinal tract, or other organs, a semen sample, a urine sample, a saliva sample, a nail sample, a tooth sample, and the like. Preferably, the sample is a blood sample, a saliva sample or a buccal swab.

*Protein analysis*

Missense, nonsense and frameshift nucleic acid variations may lead to an altered amino acid sequence, as compared to the non-variant (e.g., wild-type) protein, due to amino acid substitutions, deletions, or insertions, or truncations. Variations at splice sites may also lead to splice variation. In such instances, detection of an amino acid substitution or a truncated amino acid sequence of the variant protein may be useful. Thus, nucleic acid sequence data may be obtained through indirect analysis of the nucleic acid sequence of the allele of the polymorphic marker, *i.e.* by detecting a protein variation.

The variants described herein result in altered BRIP1 protein. Accordingly, one aspect of the invention relates to a method of determining whether a human subject is at increased risk of developing cancer, the method comprising analyzing amino acid sequence data about a BRIP1 polypeptide from the subject, wherein a determination of the presence of an altered BRIP1 polypeptide compared with a wild-type BRIP1 polypeptide with sequence as set forth in SEQ ID NO:13 is indicative that the subject is at increased risk of developing cancer. In certain

In certain embodiment, the altered BRIP1 polypeptide is a truncated BRIP1 polypeptide compared with wild-type BRIP1. In certain embodiments, the altered BRIP1 polypeptide has a reduced activity compared with wild-type BRIP1, wherein the activity is selected from (1) BRIP1 binding to C-terminal BRCT domain of BRCA1, (2) DNA-dependent ATPase activity and (3) DNA helicase activity.

Methods of detecting variant proteins are known in the art. For example, direct amino acid sequencing of the variant protein followed by comparison to a reference amino acid sequence can be used. Alternatively, SDS-PAGE followed by gel staining can be used to detect variant proteins of different molecular weights. Also, Immunoassays, e.g., antibody assays, e.g., immunofluorescent immunoassays, immunoprecipitations, radioimmunoasays, ELISA, and Western blotting, in which an antibody specific for an epitope comprising the variant sequence among the variant protein and non-variant or wild-type protein can be used. In certain embodiments, the amino acid sequence data about BRIP1 protein is obtained or deduced from a preexisting record.

In certain embodiments of the present invention, an amino acid substitution in the human BRIP1 protein is detected. In another embodiment, a truncated polypeptide encoded by an altered *BRIP1* gene sequence is detected. In one embodiment, the truncated polypeptide is encoded by the -/AA insertion deletion polymorphism between position 57,208,601 and 57,208,602 in NCBI Build 36 (SEQ ID NO:12) (chr17:57208601 ins+AA). In another embodiment, the truncated polypeptide is encoded by the 57213073 delTT polymorphism. In one embodiment, the truncated polypeptide is a BRIP1 polypeptide that is truncated at codon 687, with an alternate sequence starting at codon 680, as shown in SEQ ID NO:14 herein. In one embodiment, the truncated polypeptide is a BRIP1 polypeptide that is truncated at codon 576. The detection may be suitably performed, for example using any of the methods described in the above, or any other suitable method known to the skilled artisan.

Methods of detecting expression levels are known in the art. For example, ELISA, radioimmunoassays, immunofluorescence, and Western blotting can be used to compare the expression of protein levels. Alternatively, Northern blotting can be used to compare the levels of mRNA. These processes are described in Sambrook et al., Molecular Cloning: A Laboratory Manual, 3rd ed. Cold Spring Harbor Laboratory Press, Cold Spring Harbor, New York (2001).

Any of these methods may be performed using a nucleic acid (e.g., DNA, mRNA) or protein of a biological sample obtained from the human individual for whom a susceptibility is being determined. The biological sample can be any nucleic acid or protein containing sample obtained from the human individual. For example, the biological sample can be any of the biological samples described herein.

*Number of Polymorphic Markers/Genes Analyzed*

With regard to the methods of determining a susceptibility described herein, the methods can comprise obtaining sequence data about any number of polymorphic markers and/or about any number of genes. For example, the method can comprise obtaining sequence data for about at least 1, 2, 3, 4, 5, 6, 7, 8, 9, 10, 15, 20, 25, 30, 40, 50, 100, 500, 1000, 10,000 or more polymorphic markers. In certain embodiments, the sequence data is obtained from a microarray comprising probes for detecting a plurality of markers. The polymorphic markers can be the ones of the group specified herein or they can be different polymorphic markers that are not specified herein. In a specific embodiment, the method comprises obtaining sequence data about at least two polymorphic markers. In certain embodiments, each of the markers may be associated with a different gene. For example, in some instances, if the method comprises obtaining nucleic acid data about a human individual identifying at least one allele of a polymorphic marker, then the method comprises identifying at least one allele of at least one polymorphic marker. Also, for example, the method can comprise obtaining sequence data about a human individual identifying alleles of multiple, independent markers, which are not in linkage disequilibrium.

*Linkage Disequilibrium*

Linkage Disequilibrium (LD) refers to a non-random assortment of two genetic elements. For example, if a particular genetic element (e.g., an allele of a polymorphic marker, or a haplotype) occurs in a population at a frequency of 0.50 (50%) and another element occurs at a frequency of 0.50 (50%), then the predicted occurrance of a person's having both elements is 0.25 (25%), assuming a random distribution of the elements. However, if it is discovered that the two elements occur together at a frequency higher than 0.25, then the elements are said to be in linkage disequilibrium, since they tend to be inherited together at a higher rate than what their independent frequencies of occurrence (e.g., allele or haplotype frequencies) would predict. Roughly speaking, LD is generally correlated with the frequency of recombination events between the two elements. Allele or haplotype frequencies can be determined in a population by genotyping individuals in a population and determining the frequency of the occurence of each allele or haplotype in the population. For populations of diploids, e.g., human populations, individuals will typically have two alleles for each genetic element (e.g., a marker, haplotype or gene).

Many different measures have been proposed for assessing the strength of linkage disequilibrium (LD; reviewed in Devlin, B. & Risch, N., Genomics 29:311-22 (1995)). Most capture the strength of association between pairs of biallelic sites. Two important pairwise measures of LD are $r^2$ (sometimes denoted $\Delta^2$) and |D'| (Lewontin, R., Genetics 49:49-67 (1964); Hill, W.G. & Robertson, A. Theor. Appl. Genet. 22:226-231 (1968)). Both measures range from 0 (no disequilibrium) to 1 ('complete' disequilibrium), but their interpretation is slightly different. |D'| is defined in such a way that it is equal to 1 if just two or three of the possible haplotypes are present, and it is <1 if all four possible haplotypes are present. Therefore, a value of |D'| that is <1 indicates that historical recombination may have occurred between two sites (recurrent mutation can also cause |D'| to be <1, but for single nucleotide polymorphisms (SNPs) this is usually regarded as being less likely than recombination). The measure $r^2$ represents the statistical correlation between two sites, and takes the value of 1 if only two haplotypes are present. Markers which are correlated by an $r^2$ value of 1 are said to be perfectly correlated. In such an instance, the genotype of one marker perfectly predicts the genotype of the other.

The $r^2$ measure is arguably the most relevant measure for association mapping, because there is a simple inverse relationship between $r^2$ and the sample size required to detect association between susceptibility loci and SNPs. These measures are defined for pairs of sites, but for some applications a determination of how strong LD is across an entire region that contains many polymorphic sites might be desirable (*e.g.*, testing whether the strength of LD differs significantly among loci or across populations, or whether there is more or less LD in a region than predicted under a particular model). Measuring LD across a region is not straightforward, but one approach is to use the measure r, which was developed in population genetics. Roughly speaking, r measures how much recombination would be required under a particular population model to generate the LD that is seen in the data. This type of method can potentially also provide a statistically rigorous approach to the problem of determining whether LD data provide evidence for the presence of recombination hotspots.

A significant $r^2$ indicative of markers being in linkage disequilibrium may be at least 0.1, such as at least 0.15, 0.2, 0.25, 0.3, 0.35, 0.4, 0.45, 0.5, 0.55, 0.6, 0.65, 0.7, 0.75, 0.8, 0.85, 0.9, 0.91, 0.92, 0.93, 0.94, 0.95, 0.96, 0.97, 0.98, 0.99 or 1.0. A significant $r^2$ indicates that the markers are highly correlated, and therefore in linkage disequilibrium. Highly correlated markers must, be definition, show highly comparable results in association mapping, since the genotypes for one marker predicts the genotype for another, correlated, marker. In one specific embodiment of invention, the significant $r^2$ value can be at least 0.2. In another specific embodiment of invention, the significant $r^2$ value can be at least 0.5. In one specific embodiment of invention, the significant $r^2$ value can be at least 0.8. Alternatively, linkage disequilibrium as described herein, refers to linkage disequilibrium characterized by values of $r^2$ of at least 0.2, such as 0.3, 0.4, 0.5, 0.6, 0.7, 0.8, 0.85, 0.9, 0.95, 0.96, 0.97, 0.98, 0.99. Thus, linkage disequilibrium represents a correlation between alleles of distinct markers. It is measured by correlation coefficient or |D'| ($r^2$ up to 1.0 and |D'| up to 1.0). Linkage disequilibrium can be determined in a single human population, as defined herein, or it can be determined in a collection of samples comprising individuals from more than one human population. In one embodiment of the invention, LD is determined in a sample from one or more of the HapMap populations. These include samples from the Yoruba people of Ibadan, Nigeria (YRI), samples from individuals from the Tokyo area in Japan (JPT), samples from individuals Beijing, China (CHB), and samples from U.S. residents with northern and western European ancestry (CEU), as described (The International HapMap Consortium, Nature 426:789-796 (2003)). In one such embodiment, LD is determined in the Caucasian CEU population of the HapMap samples. In another embodiment, LD is determined in the African YRI population. In yet another embodiment, LD is determined in samples from the Icelandic population. In certain embodiments, LD is determined in a white population.

If all polymorphisms in the genome were independent at the population level (*i.e.,* no LD between polymorphisms), then every single one of them would need to be investigated in association studies, to assess all different polymorphic states. However, due to linkage disequilibrium between polymorphisms, tightly linked polymorphisms are strongly correlated, which reduces the number of polymorphisms that need to be investigated in an association study to observe a significant association. Another consequence of LD is that many polymorphisms may give an association signal due to the fact that these polymorphisms are strongly correlated.

Genomic LD maps have been generated across the genome, and such LD maps have been proposed to serve as framework for mapping disease-genes (Risch, N. & Merkiangas, K, Science 273:1516-1517 (1996); Maniatis, N., et al., Proc Natl Acad Sci USA 99:2228-2233 (2002); Reich, DE et al, Nature 411:199-204 (2001)).

It is now established that many portions of the human genome can be broken into series of discrete haplotype blocks containing a few common haplotypes; for these blocks, linkage disequilibrium data provides little evidence indicating recombination (see, *e.g.*, Wall., J.D. and Pritchard, J.K., Nature Reviews Genetics 4:587-597 (2003); Daly, M. et al., Nature Genet. 29:229-232 (2001); Gabriel, S.B. et al., Science 296:2225-2229 (2002); Patil, N. et al., Science 294:1719-1723 (2001); Dawson, E. et al., Nature 418:544-548 (2002); Phillips, M.S. et al., Nature Genet. 33:382-387 (2003)).

Haplotype blocks (LD blocks) can be used to map associations between phenotype and haplotype status, using single markers or haplotypes comprising a plurality of markers. The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks.

It has thus become apparent that for any given observed association to a polymorphic marker in the genome, it is likely that additional markers in the genome also show association. This is a natural consequence of the uneven distribution of LD across the genome, as observed by the large variation in recombination rates. The markers used to detect association thus in a sense represent "tags" for a genomic region (i.e., a haplotype block or LD block) that is associating with a given disease or trait, and as such are useful for use in the methods and kits of the invention.

By way of example, the -/AA indel, encoding the truncated form of BRIP1 shown in SEQ ID NO:14 herein, may be detected directly to determine risk of ovarian cancer. Alternatively, any marker in linkage disequilibrium with the -/AA indel may be detected to determine risk. In other embodiments, markers in linkage disequilibrium with any one of the markers rs34289250 (SEQ ID NO:1); rs12938171 (SEQ ID NO:2); an A/T polymorphism at position 55,422,245 in NCBI Build 36 (SEQ ID NO:3); a C/T polymorphism at position 55,217,320 in NCBI Build 36 (SEQ ID NO:4); rs12451939 (SEQ ID NO:5); a G/T polymorphism at position 56,567,990 in NCBI Build 36 (SEQ ID NO:6); an A/C polymorphism at position 56,478,611 in NCBI Build 36 (SEQ ID NO:7); a C/T polymorphism at position 56,505,864 in NCBI Build 36 (SEQ ID NO:8); and rs12937080 (SEQ ID NO:9), may be used to determine risk.

Suitable surrogate markers may be selected using public information, such as from the International HapMap Consortium (http://www.hapmap.org) and the International 1000genomes Consortium (http://www.1000genomes.org). The markers may also be suitably selected from results of whole-genome sequencing. The stronger the linkage disequilibrium (i.e., the higher the correlation) to the anchor marker, the better the surrogate, and thus the mores similar the association detected by the surrogate is expected to be to the association detected by the anchor marker. Markers with values of $r^2$ equal to 1 are perfect surrogates for the at-risk variants, i.e. genotypes for one marker perfectly predicts genotypes for the other. In other words, the surrogate will, by necessity, give exactly the same association data to any particular disease as the anchor marker. Markers with smaller values of $r^2$ than 1 can also be surrogates for the at-risk anchor variant.

The present invention encompasses the assessment of such surrogate markers for the markers as disclosed herein. Such markers are annotated, mapped and listed in public databases, as well known to the skilled person, or can alternatively be readily identified by sequencing the region or a part of the region identified by the markers of the present invention in a group of individuals, and identify polymorphisms in the resulting group of sequences. As a consequence, the person skilled in the art can readily and without undue experimentation identify and select appropriate surrogate markers.

One consequence of LD is that causative variants are not necessarily the variants first used for detecting an association signal. It is for example contemplated that a variant that is in linkage disequilibrium with the -/AA indel may be a functionally relevant variant. Alternatively, one or more variants in linkage disequilibrium with one or more of the markers rs34289250 (SEQ ID NO:1); rs12938171 (SEQ ID NO:2); an A/T polymorphism at position 55,422,245 in NCBI Build 36 (SEQ ID NO:3); a C/T polymorphism at position 55,217,320 in NCBI Build 36 (SEQ ID NO:4); rs12451939 (SEQ ID NO:5); a G/T polymorphism at position 56,567,990 in NCBI Build 36 (SEQ ID NO:6); an A/C polymorphism at position 56,478,611 in NCBI Build 36 (SEQ ID NO:7); a C/T polymorphism at position 56,505,864 in NCBI Build 36 (SEQ ID NO:8); and rs12937080 (SEQ ID NO:9) may be a functionally relevant variant predictive of risk of ovarian cancer.

*Association analysis*

For single marker association to a disease, the Fisher exact test can be used to calculate two-sided p-values for each individual allele. Correcting for relatedness among patients can be done by extending a variance adjustment procedure previously described (Risch, N. & Teng, J. Genome Res., 8:1273-1288 (1998)) for sibships so that it can be applied to general familial relationships. The method of genomic controls (Devlin, B. & Roeder, K. Biometrics 55:997 (1999)) can also be used to adjust for the relatedness of the individuals and possible stratification.

For both single-marker and haplotype analyses, relative risk (RR) and the population attributable risk (PAR) can be calculated assuming a multiplicative model (haplotype relative risk model) (Terwilliger, J.D. & Ott, J., Hum. Hered. 42:337-46 (1992) and Falk, C.T. & Rubinstein, P, Ann. Hum. Genet. 51 (Pt 3):227-33 (1987)), i.e., that the risks of the two alleles/haplotypes a person carries multiply. For example, if RR is the risk of A relative to a, then the risk of a person homozygote AA will be RR times that of a heterozygote Aa and $RR^2$ times that of a homozygote aa. The multiplicative model has a nice property that simplifies analysis and computations - haplotypes are independent, *i.e.,* in Hardy-Weinberg equilibrium, within the affected population as well as within the control population. As a consequence, haplotype counts of the affecteds and controls each have multinomial distributions, but with different haplotype frequencies under the alternative hypothesis. Specifically, for two haplotypes, $h_i$ and $h_j$, risk($h_i$)/risk($h_j$) = $(f_i/p_i)/(f_j/p_j)$, where $f$ and $p$ denote, respectively, frequencies in the affected population and in the control population. While there is some power loss if the true model is not multiplicative, the loss tends to be mild except for extreme cases. Most importantly, p-values are always valid since they are computed with respect to null hypothesis.

*Risk assessment and Diagnostics*

Within any given population, there is an absolute risk of developing a disease or trait, defined as the chance of a person developing the specific disease or trait over a specified time-period. For example, a woman's lifetime absolute risk of breast cancer is one in nine. That is to say, one woman in every nine will develop breast cancer at some point in their lives. Risk is typically measured by looking at very large numbers of people, rather than at a particular individual. Risk is often presented in terms of Absolute Risk (AR) and Relative Risk (RR). Relative Risk is used to compare risks associating with two variants or the risks of two different groups of people. For example, it can be used to compare a group of people with a certain genotype with another group having a different genotype. For a disease, a relative risk of 2 means that one group has twice the chance of developing a disease as the other group. The risk presented is usually the relative risk for a person, or a specific genotype of a person, compared to the population with matched gender and ethnicity. Risks of two individuals of the same gender and ethnicity could be compared in a simple manner. For example, if, compared to the population, the first individual has relative risk 1.5 and the second has relative risk 0.5, then the risk of the first individual compared to the second individual is 1.5/0.5 = 3.

*Risk Calculations*

The creation of a model to calculate the overall genetic risk involves two steps: i) conversion of odds-ratios for a single genetic variant into relative risk and ii) combination of risk from multiple variants in different genetic loci into a single relative risk value.

*Deriving risk from odds-ratios*

Most gene discovery studies for complex diseases that have been published to date in authoritative journals have employed a case-control design because of their retrospective setup. These studies sample and genotype a selected set of cases (people who have the specified disease condition) and control individuals. The interest is in genetic variants (alleles) which frequency in cases and controls differ significantly.

The results are typically reported in odds ratios, that is the ratio between the fraction (probability) with the risk variant (carriers) versus the non-risk variant (non-carriers) in the groups of affected versus the controls, i.e. expressed in terms of probabilities conditional on the affection status:

$$OR = (Pr(c|A)/Pr(nc|A)) / (Pr(c|C)/Pr(nc|C))$$

Sometimes it is however the absolute risk for the disease that we are interested in, i.e. the fraction of those individuals carrying the risk variant who get the disease or in other words the probability of getting the disease. This number is typically not directly measured in case-control studies, in part, because the ratio of cases versus controls is typically not the same as that in the general population. However, under certain assumption, we can estimate the risk from the odds ratio.

It is well known that under the rare disease assumption, the relative risk of a disease can be approximated by the odds ratio. This assumption may however not hold for many common diseases. Still, it turns out that the risk of one genotype variant relative to another can be estimated from the odds ratio expressed above. The calculation is particularly simple under the assumption of random population controls where the controls are random samples from the same population as the cases, including affected people rather than being strictly unaffected individuals. To increase sample size and power, many of the large genome-wide association and replication studies use controls that were neither age-matched with the cases, nor were they carefully scrutinized to ensure that they did not have the disease at the time of the study. Hence, they often approximate a random sample from the general population. It is noted that this assumption is rarely expected to be satisfied exactly, but the risk estimates are usually robust to moderate deviations from this assumption.

Calculations show that for the dominant and the recessive models, where we have a risk variant carrier, "c", and a non-carrier, "nc", the odds ratio of individuals is the same as the risk ratio between these variants:

$$OR = Pr(A|c)/Pr(A|nc) = r$$

And likewise for the multiplicative model, where the risk is the product of the risk associated with the two allele copies, the allelic odds ratio equals the risk factor:

$$OR = Pr(A|aa)/Pr(A|ab) = Pr(A|ab)/Pr(A|bb) = r$$

Here "a" denotes the risk allele and "b" the non-risk allele. The factor "r" is therefore the relative risk between the allele types.

For many of the studies published in the last few years, reporting common variants associated with complex diseases, the multiplicative model has been found to summarize the effect adequately and most often provide a fit to the data superior to alternative models such as the dominant and recessive models.

*Determining risk*

In the present context, an individual who is at an increased susceptibility (i.e., increased risk) for ovarian cancer is an individual who is carrying at least one at-risk variant as described herein.

In certain embodiments, the variant is within the human *BRIP1* gene, or a variant encoded by a variation in the human *BRIP1* gene. In one embodiment, significance associated with a marker is measured by a relative risk (RR). In another embodiment, significance associated with a marker or haplotye is measured by an odds ratio (OR). In a further embodiment, the significance is measured by a percentage. In one embodiment, a significant increased risk is measured as a risk (relative risk and/or odds ratio) of at least 2.0, including but not limited to at least 3.0, at least 3.5, at least 4.0, at least 5.0, at least 6.0, at least 7.0, at least 8.0, at least 9.0, at least 10.0, at least 11.0, at least 12.0, at least 13.0, at least 14.0, at least 15.0, at least 16.0, at least 18.0, at least 20.0, at least 22.0, or at least 24.0. In a particular embodiment, a risk (relative risk and/or odds ratio) of at least 5.0 is significant. In another particular embodiment, a risk of at least 7.0 is significant.

An at-risk variant as described herein is one where at least one allele of at least one marker is more frequently present in an individual at risk for ovarian cancer (affected), or diagnosed with ovarian cancer, compared to the frequency in a comparison group (control), such that the presence of the marker allele is indicative of susceptibility to ovarian cancer. The control group may in one embodiment be a population sample, i.e. a random sample from the general population. In another embodiment, the control group is represented by a group of individuals who are disease-free, *i.e.* individuals who have not been diagnosed with Ovarian cancer.

The person skilled in the art will appreciate that for markers with two alleles present in the population being studied (such as SNPs), and wherein one allele is found in increased frequency in a group of individuals with a trait or disease in the population, compared with controls, the other allele of the marker will be found in decreased frequency in the group of individuals with the trait or disease, compared with controls. In such a case, one allele of the marker (the one found in increased frequency in individuals with the trait or disease) will be the at-risk allele, while the other allele will be a protective allele.

*Database*

Determining susceptibility can alternatively or additionally comprise comparing nucleic acid sequence data and/or protein sequence data (*e.g.*, genotype data) to a database containing correlation data between polymorphic markers and susceptibility to ovarian cancer. The database can be part of a computer-readable medium described herein.

In a specific aspect of the invention, the database comprises at least one measure of susceptibility to ovarian cancer for the polymorphic markers. For example, the database may comprise risk values associated with particular genotypes at such markers. The database may also comprise risk values associated with particular genotype combinations for multiple such markers.

In another specific aspect of the invention, the database comprises a look-up table containing at least one measure of susceptibility to ovarian cancer for the polymorphic markers.

*Further steps*

The methods disclosed herein can comprise additional steps which may occur before, after, or simultaneously with one of the aforementioned steps of the method of the invention. In a specific embodiment of the invention, the method of determining a susceptibility to cancer further comprises reporting the susceptibility to at least one entity selected from the group consisting of the individual, a guardian of the individual, a genetic service provider, a physician, a medical organization, and a medical insurer. The reporting may be accomplished by any of several means. For example, the reporting can comprise sending a written report on physical media or electronically or providing an oral report to at least one entity of the group, which written or oral report comprises the susceptibility. Alternatively, the reporting can comprise providing the at least one entity of the group with a login and password, which provides access to a report comprising the susceptibility posted on a password-protected computer system.

*Study population*

In a general sense, the methods and kits described herein can be utilized from samples containing nucleic acid material (DNA or RNA) or protein material from any source and from any individual, or from genotype or sequence data derived from such samples. In preferred embodiments, the individual is a human individual. The individual can be an adult, child, or fetus. In some embodiments, the individual is a female individual. The nucleic acid or protein source may be any sample comprising nucleic acid or protein material, including biological samples, or a sample comprising nucleic acid or protein material derived therefrom. The present invention also provides for assessing markers in individuals who are members of a target population. Such a target population is in one embodiment a population or group of individuals at risk of developing ovarian cancer, based on other genetic factors, biomarkers, biophysical parameters, or lifestyle factors.

The Icelandic population is a Caucasian population of Northern European ancestry. A large number of studies reporting results of genetic linkage and association in the Icelandic population have been published in the last few years. Many of those studies show replication of variants, originally identified in the Icelandic population as being associating with a particular disease, in other populations (Sulem, P., et al. Nat Genet May 17 2009 (Epub ahead of print); Rafnar, T., et al. Nat Genet 41:221-7 (2009); Gretarsdottir, S., et al. Ann Neurol 64:402-9 (2008); Stacey, S.N., et al. Nat Genet 40:1313-18 (2008); Gudbjartsson, D.F., et al. Nat Genet 40:886-91 (2008); Styrkarsdottir, U., et al. N Engl J Med 358:2355-65 (2008); Thorgeirsson, T., et al. Nature 452:638-42 (2008); Gudmundsson, J., et al. Nat Genet. 40:281-3 (2008); Stacey, S.N., et al., Nat Genet. 39:865-69 (2007); Helgadottir, A., et al., Science 316:1491-93 (2007); Steinthorsdottir, V., et al., Nat Genet. 39:770-75 (2007); Gudmundsson, J., et al., Nat Genet. 39:631-37 (2007); Frayling, TM, Nature Reviews Genet 8:657-662 (2007); Amundadottir, L.T., et al., Nat Genet. 38:652-58 (2006); Grant, S.F., et al., Nat Genet. 38:320-23 (2006)). Thus, genetic findings in the Icelandic population have in general been replicated in other populations, including populations from Africa and Asia.

It is thus believed that the markers described herein to be associated with risk of ovarian cancer will show similar association in other human populations. It is further contemplated that additional variants in the human *BRIP1* gene may be conferring risk of ovarian cancer in other populations. Particular embodiments comprising individual human populations are thus also contemplated and within the scope of the invention. Such embodiments relate to human subjects that are from one or more human population including, but not limited to, white populations, Caucasian populations, European populations, American populations, Eurasian populations, Asian populations, Central/South Asian populations, East Asian populations, and African populations. In certain embodiments, the invention pertains to individuals from Caucasian populations. In certain embodiments, the invention pertains to Icelandic individuals. In certain embodiments, the invention pertains to white individuals.

The population origin of individuals can be determined using methods known in the art. In certain embodiments, the origin of individuals is determined through self-reporting. In such embodiments, individuals describe their population origin themselves. For example, individuals may characterize themselves as belonging to any of the above mentioned populations. This method is routinely used in the art, for example in clinical studies.

Alternatively, the population origin of individuals may be determined at the nucleic acid level using genetic markers, which is a method well known to the skilled person. Using groups of individuals from specific populations/ethnic groups as a reference, it is possible to assign genomic material of unknown origin to particular populations. This may be routinely performed by the skilled person, using genetic markers that are population specific, and thus appropriate for determining genetic origin of nucleic acid samples.

In certain embodiments, the invention relates to markers identified in specific populations, as described in the above. The person skilled in the art will appreciate that measures of linkage disequilibrium (LD) may give different results when applied to different populations. This is due to different population history of different human populations as well as differential selective pressures that may have led to differences in LD in specific genomic regions. It is also well known to the person skilled in the art that certain markers, e.g. SNP markers, have different population frequency in different populations, or are polymorphic in one population but not in another. The person skilled in the art will however apply the methods available and as taught herein to practice the present invention in any given human population. This may include assessment of polymorphic markers in the LD region of the present invention, so as to identify those markers that give strongest association within the specific population. Thus, the at-risk variants of the present invention may reside on different haplotype background and in different frequencies in various human populations. However, utilizing methods known in the art and the markers of the present invention, the invention can be practiced in any given human population.

*Diagnostic Methods*

Polymorphic markers associated with increased susceptibility of cancer, e.g. ovarian cancer, are useful in diagnostic methods. While methods of diagnosing cancer are known in the art, the detection risk markers for ovarian cancer advantageously may be useful for detection of cancer at its early stages and may also reduce the occurrence of misdiagnosis. In this regard, the invention further provides methods of diagnosing cancer comprising obtaining sequence data identifying at least one risk allele as described herein, in conjunction with carrying out one or more clinical diagnostic steps for the identification of cancer. Such diagnostic steps may include transvaginal ultrasound (TVU) and determination of CA-125 levels in the blood. The diagnostic steps may further include assessment of symptoms selected from abdominal pain or discomfort, an abdominal mass, bloating, back pain, urinary urgency, constipation, tiredness, pelvic pain, abnormal vaginal bleeding and involuntary weight loss.

The present invention pertains in some embodiments to methods of clinical applications of diagnosis, *e.g.*, diagnosis performed by a medical professional. In other embodiments, the invention pertains to methods of diagnosis or methods of determination of a susceptibility performed by a layman. The layman can be the customer of a sequencing or genotyping service. The layman may also be a genotype or sequencing service provider, who performs analysis on a DNA sample from an individual, in order to provide service related to genetic risk factors for particular traits or diseases, based on the genotype status of the individual (*i.e.,* the customer). Sequencing methods include for example those discussed in the above, but in general any suitable sequencing method may be used in the methods described and claimed herein. Recent technological advances in genotyping technologies, including high-throughput genotyping of SNP markers, such as Molecular Inversion Probe array technology (*e.g.*, Affymetrix GeneChip), and BeadArray Technologies (*e.g.*, Illumina GoldenGate and Infinium assays) have made it possible for individuals to have their own genome assessed for up to one million SNPs simultaneously, at relatively little cost. The resulting genotype information, which can be made available to the individual, can be compared to information about disease or trait risk associated with various SNPs, including information from public literature and scientific publications.

The application of disease-associated alleles as described herein, can thus for example be performed by the individual, through analysis of his/her genotype data, by a health professional based on results of a clinical test, or by a third party, including the genotype or sequencing service provider. The third party may also be service provider who interprets genotype or sequence information from the customer to provide service related to specific genetic risk factors, including the genetic markers described herein. In other words, the diagnosis or determination of a susceptibility of genetic risk can be made by health professionals, genetic counselors, third parties providing genotyping and/or sequencing service, third parties providing risk assessment service or by the layman (*e.g.*, the individual), based on information about the genotype status of an individual and knowledge about the risk conferred by particular genetic risk factors (*e.g.*, particular SNPs). In the present context, the term "diagnosing", "diagnose a susceptibility" and "determine a susceptibility" is meant to refer to any available method for determining a susceptibility or risk of disease, including those mentioned above.

In certain embodiments, a sample containing genomic DNA or protein from an individual is collected. Such sample can for example be a buccal swab, a saliva sample, a blood sample, or other suitable samples containing genomic DNA or protein, as described further herein. In certain embodiments, the sample is obtained by non-invasive means (*e.g.*, for obtaining a buccal sample, saliva sample, hair sample or skin sample). In certain embodiments, the sample is obtained by non-surgical means, *i.e.* in the absence of a surgical intervention on the individual that puts the individual at substantial health risk. Such embodiments may, in addition to non-invasive means also include obtaining sample by extracting a blood sample (*e.g.*, a venous blood sample). The genomic DNA or protein obtained from the individual is then analyzed using any common technique available to the skilled person, such as high-throughput technologies for genotyping and/or sequencing. Results from such methods are stored in a convenient data storage unit, such as a data carrier, including computer databases, data storage disks, or by other convenient data storage means. In certain embodiments, the computer database is an object database, a relational database or a post-relational database. The genotype data is subsequently analyzed for the presence of certain variants known to be susceptibility variants for a particular human condition, such as the genetic variants described herein associated with risk of ovarian cancer. Genotype and/or sequencing data can be retrieved from the data storage unit using any convenient data query method. Calculating risk conferred by a particular genotype for the individual can be based on comparing the genotype of the individual to previously determined risk (expressed as a relative risk (RR) or and odds ratio (OR), for example) for the genotype, for example for an heterozygous carrier of an at-risk variant. The calculated risk for the individual can be the relative risk for a person, or for a specific genotype of a person, compared to the average population with matched gender and ethnicity. The average population risk can be expressed as a weighted average of the risks of different genotypes, using results from a reference population, and the appropriate calculations to calculate the risk of a genotype group relative to the population can then be performed. Alternatively, the risk for an individual is based on a comparison of particular genotypes, for example heterozygous carriers of an at-risk allele of a marker compared with non-carriers of the at-risk allele. The calculated risk estimated can be made available to the customer via a website, preferably a secure website.

*Methods of selecting individuals for therapy*

Most currently-used cancer treatments aim to introduce damaging lesions into DNA of replicating cells to the extent that the cell cannot repair the damage and will die. The two most common form of damage are DNA double stranded breaks (DSB) and DNA single stranded breaks (SSB) (reviewed in Yap et al, *CA Cancer* 61, 31 (2011)). Several anticancer drugs, as well as ionizing radiation, cause toxic double stranded breaks in DNA while other anticancer drugs produce different kinds of primary DNA lesions, including single stranded breaks.

Cells can employ a diverse range of DNA repair pathways to counteract the damage, depending on the types of lesions and repair required (Yap et al, CA Cancer 61, 31 (2011)). For example, the homologous recombination (HR) and non-homologous end joining pathways may be utilized for the repair of double stranded breaks in DNA. The HR system is highly conserved and error free, and is therefore the favored form of double stranded break repair. Unlike homologous recombination, the non-homologous end joining pathway is error prone and may lead to genomic instability. The major pathways that are used to repair single-stranded breaks in DNA are the nucleotide excision repair, base excision repair, or mismatch repair. Of these, base excision repair, is a key pathway for the repair of single-stranded breaks and encompasses the sensing of the DNA lesion, followed by recruitment of several other repair effectors through the action of Poly(ADP-ribose) polymerase (PARP).

Many tumor cells have specific genetic lesions in pathways that are important for DNA repair. This can be exploited by targeting genetically defective tumor cells with a specific molecular therapy that inhibits the remaining repair machinery, resulting in selective tumor cell killing (Helleday, T., Carcinogenesis 31, 955 (2010)). For example, tumor cells that have mutations in the breast cancer genes *BRCA1* or *BRCA2* have a defective homologous recombination repair pathway, making these cells dependent on the error-prone non-homologous end joining mechanism for repairing double-stranded breaks. When these cells are treated with PARP inhibitors, the PARP enzymes can no longer perform repair of single-stranded breaks. Unrepaired single-stranded breaks in PARP inhibited cells may be converted into toxic double-stranded breaks during replication, which will not be repaired efficiently in the absence of homologous recombination-mediated repair, and results in cell death. The demonstration of single-agent antitumor activity and the wide therapeutic index of PARP inhibitors in *BRCA1* and *BRCA2* mutation carriers with advanced breast or ovarian cancers provide strong evidence for the clinical application of this approach (Fong et al N Engl J Med 361, 123 (2009), Fong et al J Clin Oncol 28, 2512 (2010)).

BRIP1 interacts with the BRCT domain of BRCA1 and has several BRCA1-dependent, as well as independent, functions in preserving the structural and genetic integrity of the genome (reviewed by (Cantor, S.B. and Guillemette, G., FANCJ/BACH1/BRIP1. Future Oncol 7, 253)). It has been shown that *BRIP1* is required for homologous recombination -mediated repair of double-stranded breaks, suggesting that *BRIP1*-deficient tumor cells will have the same sensitivity to PARP inhibitors as has been shown for *BRCA1* and *BRCA2* deficient tumors.

In addition to its role in homologous recombination-mediated DNA repair, *BRIP1* is required for normal progression through S-phase by assisting in the resolution of stalled replication forks. Mutations in *BRIP1* that impair its helicase function, render cells highly sensitive to crosslinking agents such as cisplatin, suggesting that *BRIP1*-mutant ovarian cancer cells may be good candidates for platinum drugs.

Given that individuals who carry truncating germline mutations in *BRIP1* also have LOH over *BRIP1,* suggests that measurement of *BRIP1* in the germline of ovarian cancer patients may help to identify patients who are likely to be responsive to drugs that target DNA repair pathways including PARP inhibitors, and cisplatin-class drugs.

It is therefore contemplated that individuals with ovarian cancer that carry loss-of-function mutations in *BRIP1* are more likely to show a positive response to treatment for ovarian cancer, such as treatment by PARP inhibitors and/or a DNA crosslinking agents, than individuals that do not carry such mutations.

Accordingly, in one aspect of the invention, a method of assessing the responsiveness of a human individual to a therapeutic agent for ovarian cancer is provided, the method comprising determining the presence or absence of a loss-of-function mutation in the human *BRIP1* gene in the genome of the individual, wherein a determination of the presence of the mutation is indicative that the individual is responsive to the therapeutic agent.

Another aspect provides a method of selecting a human subject with ovarian cancer for treatment with an ovarian cancer therapeutic agent, the method comprising determining the presence or absence of at least one allele of at least one polymorphic marker in a nucleic acid sample obtained from the individual, wherein the at least one allele causes a loss of function or loss of expression of *BRIP1,* and selecting for treatment with the therapeutic agent a subject identified as having the at least one allele in the nucleic acid sample. The nucleic acid sample may in certain embodiments be from an ovarian cancer tumor. In certain embodiments, the selecting step comprises selecting for treatment with the therapeutic agent a subject identified as having loss of heterozygosity of *BRIP1,* indicative of a loss-of-function of *BRIP1* in the nucleic acid sample.

Also disclosed is a method of selecting a therapeutic regimen for a human subject with ovarian cancer, the method comprising analyzing data representative of at least one allele of a *BRIP1* gene in a human subject with ovarian cancer to identify the presence or absence of a loss-of-function *BRIP1* mutant allele, and selecting a therapeutic regimen of a therapeutic agent for treating ovarian cancer for a subject identified from the data as having the loss-of-function *BRIP1* mutant allele. The *BRIP1* gene is preferably a gene with sequence as set forth in SEQ ID NO:15 herein.

The analyzing suitably includes screening for presence or absence of mutant alleles in *BRIP1* that are predictive of risk of ovarian cancer. In certain embodiments, such screening comprises screening for (a) a premature truncation or frameshift of an encoded BRIP1 protein, relative to the BRIP1 amino acid sequence set forth in SEQ ID NO: 13, (b) expression of a BRIP1 protein with reduced activity compared to a wild-type BRIP1 protein (SEQ ID NO: 13), or (c) reduced expression of BRIP1 protein, compared to another allele of the variant. The reduced activity is in certain embodiments an activity selected from BRIP1 binding to C-terminal BRCT motifs of wild-type BRCA1 protein, DNA-dependent ATPase activity, and DNA helicase activity. Such reduced activity can be determined using methods known in the art, including those described herein.

In certain embodiments, a tumor sample from an individual with ovarian cancer is analyzed for *BRIP1* expression. Determination of the loss of wild-type BRIP1 protein in a tumor sample is in certain embodiments indicative of loss of heterozygosity (LOH). The selecting can therefore in certain embodiments comprise identifying an individual who also lacks wildtype *BRIP1* in an ovarian cancer tumor sample.

The chemotherapy agent is preferably a PARP inhibitor or a DNA crosslinking agent. In preferred embodiments, the PARP inhibitor is selected from the group consisting of iniparib (4-iodo-3-nitrobenzamide), Olaparib (AZD-2281; 4-[(3-[(4-cyclopropylcarbonyl)piperazin-4-yl]carbonyl)-4-fluorophenyl]methyl(2H)phthalazin-1-one); Veliparib (ABT-888; 2-((R)-2-Methylpyrrolidin-2-yl)-1*H*-benzimidazole-4-carboxamide); Rucaparib (AG 014699; 8-Fluoro-2-{4-[(methylamino)methyl]phenyl}-1,3,4,5-tetrahydro-6*H*-azepino[5,4,3-cd]indol-6-one); 3-aminobenzamide; CEP 9722 (Cephalon); MK 4827 (Merck); KU-0059436 (AZD2281).

The DNA crosslinking agent is preferably selected from the group consisting of alkylating agents, such as Carmustine (1,3-bis (2-chloroethyl)-1-nitrosourea (BCNU)) and Nitrogen mustard (*e.g.*, bis(2-chloroethyl)ethylamine, bis(2-chloroethyl)methylamine and tris(2-chloroethyl)amine), cisplatin ((*SP*-4-2)-diamminedichloridoplatinum) and cisplatin derivatives. In more preferred embodiments, the DNA crosslinking agent is cisplatin or a cisplatin derivative.

In certain embodiments, cisplatin derivatives are selected from the group consisting of Carboplatin (*cis*-diammine(cyclobutane-1,1-dicarboxylate-*O,O'*)platinum(II)) and Oxaliplatin ([(1*R*,2*R*)-cyclohexane-1,2-diamine](ethanedioato-*O,O'*)platinum(II)).

Methods of the invention relating to selecting patients may further include a step of administering the therapeutic to the human individual selected for the therapy. Methods of the invention relating to selecting patients may further include a step of prescribing the therapeutic for the human for self-administration, or administration by a medical professional other than the professional that selects the patient.

*Prognostic methods*

In addition to the utilities described above, the polymorphic markers of the invention are useful in determining a prognosis of a human individual with cancer. The variants described herein are indicative of risk of cancer, including ovarian cancer. Individuals carrying mutant alleles that predispose to cancer are at increased risk of the cancer. Such mutant alleles are predicted to be indicative of prognosis of the cancer.

The prognosis predicted can be any type of prognosis relating to the progression of the cancer, including ovarian cancer, and/or relating to the chance of recovering from the cancer. The prognosis can, for instance, relate to the severity of the cancer, or how the cancer will respond to therapeutic treatment.

Accordingly, the disclosure provides a method of predicting prognosis of an individual experiencing symptoms associated with, or an individual diagnosed with, ovarian cancer. The method comprises analyzing data representative of at least one allele of a *BRIP1* gene in a human subject, wherein different alleles of the human *BRIP1* gene are associated with different susceptibilities to at least one cancer in humans, and determining a prognosis of the human subject from the data. In certain embodiments, the cancer is ovarian cancer. The analyzing may comprise analysis for a mutation in *BRIP1* that leads to loss of function or loss of expression of *BRIP1.* In certain embodiments, the analyzing comprises analyzing for the presence or absence of at least one mutant allele indicative of a *BRIP1* defect selected from the group consisting of premature truncation or frameshift of an encoded BRIP1 protein, relative to the BRIP1 amino acid sequence set forth in SEQ ID NO:13, expression of a BRIP1 protein with reduced activity compared to a wild-type BRIP1 protein (SEQ ID NO:13), and reduced expression of BRIP1 protein, compared to wild-type BRIP1.

With regard to the prognostic methods described herein, the sequence data can be nucleic acid sequence data or amino acid sequence data. For example, in one embodiment, determination of the presence of a frameshift mutation or a nonsense mutation in *BRIP1* is indicative of prognosis of ovarian cancer. The determination of the presence of a mutation in *BRIP1* that leads to loss of function or loss of expression of *BRIP1* is in certain embodiments indicative of a worsened prognosis of ovarian cancer. In other words, the presence of such mutations is in certain embodiments indicative that the individual has a worse prognosis of the cancer than do individuals with ovarian cancer who do not carry such mutations.

In some variations, the prognostic method further includes one or more additional steps, such as a step relating to generating the data by analyzing a biological sample; and/or a step involving selecting or administering a medial protocol to the subject, as described elsewhere herein.

*Methods of treatment*

It may be useful to select individuals for treatment based on the presence of altered forms of BRIP1, including mutations in *BRIP1* that cause premature stop codons, or otherwise result in protein with reduced or no activity. As discussed in the above, it is contemplated that loss-of-function mutations in *BRIP1* result in tumors that are particularly susceptible to therapy using PARP inhibitors or crosslinking agents. Therefore, it is contemplated that it may be beneficial to select individuals for therapy based on whether the individuals are carriers of such mutations.

Accordingly, the disclosure provides in one aspect a method of treatment of ovarian cancer, the method comprising steps of (a) determining the presence or absence of a mutation that causes a loss of function or loss of expression of *BRIP1* in a nucleic acid sample from the human individual; (b) selecting for treatment an individual determined to have such a mutation; and (c) administering to the selected individual a pharmaceutically acceptable amount of a therapeutic agent for ovarian cancer selected from a PARP inhibitor and a DNA crosslinking agent.

In certain embodiments, the therapeutic agent is a PARP inhibitor or a DNA crosslinking agent. The PARP inhibitor may suitably be selected from the group consisting of iniparib (4-iodo-3-nitrobenzamide), Olaparib (AZD-2281; 4-[(3-[(4-cyclopropylcarbonyl)piperazin-4-yl]carbonyl)-4-fluorophenyl]methyl(2H)phthalazin-1-one); Veliparib (ABT-888; 2-((*R*)-2-Methylpyrrolidin-2-yl)-1*H*-benzimidazole-4-carboxamide); Rucaparib (AG 014699; 8-Fluoro-2-{4-[(methylamino)methyl]phenyl}-1,3,4,5-tetrahydro-6*H*-azepino[5,4,3-cd]indol-6-one); 3-aminobenzamide; CEP 9722 (Cephalon); MK 4827 (Merck); KU-0059436 (AZD2281).

The DNA crosslinking agent is preferably selected from the group consisting of alkylating agents, such as Carmustine (1,3-bis(2-chloroethyl)-1-nitrosourea (BCNU)) and Nitrogen mustard (*e.g.*, bis(2-chloroethyl)ethylamine, bis(2-chloroethyl)methylamine and tris(2-chloroethyl)amine), cisplatin ((*SP*-4-2)-diamminedichloridoplatinum) and cisplatin derivatives. In certain more preferred embodiments, the DNA crosslinking agent is cisplatin or a cisplatin derivative.

In certain embodiments, cisplatin derivatives are selected from the group consisting of Carboplatin (*cis*-diammine(cyclobutane-1,1-dicarboxylate-*O,O'*)platinum(II)) and Oxaliplatin ([(1*R*,2*R*)-cyclohexane-1,2-diamine](ethanedioato-*O,O'*)platinum(II)).

*Kits*

Kits useful in the methods of the invention comprise components useful in any of the methods described herein, including for example, primers for nucleic acid amplification, hybridization probes (*e.g.* probes for detecting particular mutant alleles), restriction enzymes (*e.g.*, for RFLP analysis), allele-specific oligonucleotides, antibodies, *e.g.*, antibodies that bind to an altered BRIP1 polypeptide (*e.g.* a missense variant in BRIP1 or a truncated BRIP1 polypeptide) or to a non-altered (native) BRIP1 polypeptide, means for amplification of nucleic acids, means for analyzing the nucleic acid sequence of nucleic acids, means for analyzing the amino acid sequence of polynucleotides, etc. The kits can for example include necessary buffers, nucleic acid primers for amplifying nucleic acids (*e.g.*, a nucleic acid segment comprising one or more of the polymorphic markers as described herein), and reagents for allele-specific detection of the fragments amplified using such primers and necessary enzymes (*e.g.*, DNA polymerase). Additionally, kits can provide reagents for assays to be used in combination with the methods of the present invention, *e.g.*, reagents for use with other diagnostic assays for ovarian cancer or related conditions.

The disclosure pertains to a kit for assaying a sample from a subject to detect a susceptibility to cancer (e.g.,ovarian cancer) in the subject, wherein the kit comprises reagents necessary for selectively detecting at least one at-risk variant for cancer in the individual, wherein the at least one at-risk variant is a polymorphic marker in the human *BRIP1* gene or an amino acid substitution in an encoded BRIP1 protein. In certain embodiments, the markers encodes a BRIP1 protein with a defect selected from (a) premature truncation or frameshift of BRIP1 polypeptide, relative to wild-type BRIP1; (b) expression of BRIP1 protein with reduced activity compared with wild-type BRIP1, wherein the activity is selected from (1) BRIP1 binding to BRCT motif in BRCA1, (2) DNA-dependent ATPase activity, and (3) DNA helicase activity, and (c) reduced expression of BRIP1 protein compared with wild-type BRIP1. In a particular embodiment, the reagents comprise at least one contiguous oligonucleotide that hybridizes to a fragment of the genome of the individual comprising at least one polymorphism of the present invention. In another embodiment, the reagents comprise at least one pair of oligonucleotides that hybridize to opposite strands of a genomic segment obtained from a subject, wherein each oligonucleotide primer pair is designed to selectively amplify a fragment of the genome of the individual that includes at least one polymorphism associated with the condition risk. In one such embodiment, the polymorphism is selected from chr17:57208601 ins+AA and chr17: 57213073 delTT. In yet another embodiment the fragment is at least 20 base pairs in size. Such oligonucleotides or nucleic acids (*e.g.*, oligonucleotide primers) can be designed using portions of the nucleic acid sequence flanking the polymorphism. In another embodiment, the kit comprises one or more labeled nucleic acids capable of allele-specific detection of one or more specific polymorphic markers or haplotypes, and reagents for detection of the label. Suitable labels include, *e.g.*, a radioisotope, a fluorescent label, an enzyme label, an enzyme co-factor label, a magnetic label, a spin label, an epitope label.

In certain embodiments, determination of the presence of a particular marker allele is indicative of a increased susceptibility of cancer. In another embodiment, determination of the presence of a particular marker allele is indicative of prognosis of ovarian cancer, or selection of appropriate therapy for ovarian cancer. In another embodiment, the presence of the marker allele or haplotype is indicative of response to therapy for ovarian cancer. In yet another embodiment, the presence of the marker allele is indicative of progress of treatment of ovarian cancer.

In certain embodiments, the kit comprises reagents for detecting no more than 100 alleles in the genome of the individual. In certain other embodiments, the kit comprises reagents for detecting no more than 20 alleles in the genome of the individual.

In a further aspect of the present invention, a pharmaceutical pack (kit) is provided, the pack comprising a therapeutic agent and a set of instructions for administration of the therapeutic agent to humans diagnostically tested for an at-risk variant for ovarian cancer. The therapeutic agent can be a small molecule drug, an antibody, a peptide, an antisense or RNAi molecule, or other therapeutic molecules. In one embodiment, an individual identified as a carrier of at least one variant of the present invention is instructed to take a prescribed dose of the therapeutic agent. In one such embodiment, an individual identified as a homozygous carrier of at least one variant of the present invention (*e.g.*, an at-risk variant) is instructed to take a prescribed dose of the therapeutic agent. In another embodiment, an individual identified as a non-carrier of at least one variant of the present invention (*e.g.*, an at-risk variant) is instructed to take a prescribed dose of the therapeutic agent.

The kit may additionally or alternatively comprise reagents for detecting an amino acid variation in a human BRIP1 protein (*e.g.*, an amino acid substitution, or a truncated or otherwise altered amino acid sequence of an encoded BRIP1 protein). In one embodiment, the kit comprises at least one antibody for selectively detecting a truncated BRIP1 polypeptide compared with wild-type BRIP1 (SEQ ID NO:13). Other reagents useful for detecting amino acid variations are known to the skilled person and are also contemplated.

In certain embodiments, the kit further comprises a set of instructions for using the reagents comprising the kit. In certain embodiments, the kit further comprises a collection of data comprising correlation data between the at least one at-risk variant and susceptibility to Ovarian cancer.

*Antisense agents*

The nucleic acids and/or variants described herein, or nucleic acids comprising their complementary sequence, may be used as antisense constructs to control gene expression in cells, tissues or organs. The methodology associated with antisense techniques is well known to the skilled artisan, and is for example described and reviewed in AntisenseDrug Technology: Principles, Strategies, and Applications, Crooke, ed., Marcel Dekker Inc., New York (2001). In general, antisense agents (antisense oligonucleotides) are comprised of single stranded oligonucleotides (RNA or DNA) that are capable of binding to a complimentary nucleotide segment. By binding the appropriate target sequence, an RNA-RNA, DNA-DNA or RNA-DNA duplex is formed. The antisense oligonucleotides are complementary to the sense or coding strand of a gene. It is also possible to form a triple helix, where the antisense oligonucleotide binds to duplex DNA.

Several classes of antisense oligonucleotide are known to those skilled in the art, including cleavers and blockers. The former bind to target RNA sites, activate intracellular nucleases (*e.g.*, RnaseH or Rnase L), that cleave the target RNA. Blockers bind to target RNA, inhibit protein translation by steric hindrance of the ribosomes. Examples of blockers include nucleic acids, morpholino compounds, locked nucleic acids and methylphosphonates (Thompson, Drug Discovery Today, 7:912-917 (2002)). Antisense oligonucleotides are useful directly as therapeutic agents, and are also useful for determining and validating gene function, for example by gene knock-out or gene knock-down experiments. Antisense technology is further described in Lavery et al., Curr. Opin. Drug Discov. Devel. 6:561-569 (2003), Stephens et al., Curr. Opin. Mol. Ther. 5:118-122 (2003), Kurreck, Eur. J. Biochem. 270:1628-44 (2003), Dias et al., Mol. Cancer Ter. 1:347-55 (2002), Chen, Methods Mol. Med. 75:621-636 (2003), Wang et al., Curr. Cancer Drug Targets 1:177-96 (2001), and Bennett, Antisense Nucleic Acid Drug. Dev. 12:215-24 (2002).

In certain embodiments, the antisense agent is an oligonucleotide that is capable of binding to a particular nucleotide segment. In certain embodiments, the nucleotide segment comprises the human *BRIP1* gene. In certain other embodiments, the antisense nucleotide is capable of binding to a nucleotide segment of a human *BRIP1* transcript, as set forth in SEQ ID NO:10. In one embodiment, the antisense nucleotide is capable of binding the a nucleotide segment of a human *BRIP1* transcript with sequence as set forth in SEQ ID NO:10 that has a TT insertion between position 2345 and 2346. In another embodiment, the antisense nucleotide is capable of binding the a nucleotide segment of the human *BRIP1* gene that has an AA insertion between position 57,208,601 and 57,208,602 in NCBI Build 36 (SEQ ID NO:12). Antisense nucleotides can be from 5-400 nucleotides in length, including 5-200 nucleotides, 5-100 nucleotides, 10-50 nucleotides, and 10-30 nucleotides. In certain preferred embodiments, the antisense nucleotides is from 14-50 nucleotides in length, including 14-40 nucleotides and 14-30 nucleotides..

The variants described herein can also be used for the selection and design of antisense reagents that are specific for particular variants. Using information about the variants described herein, antisense oligonucleotides or other antisense molecules that specifically target mRNA molecules that contain one or more variants of the invention can be designed. In this manner, expression of mRNA molecules that contain one or more variant of the present invention can be inhibited or blocked. In one embodiment, the antisense molecules are designed to specifically bind a particular allelic form of the target nucleic acid, thereby inhibiting translation of a product originating from this specific allele, but which do not bind other or alternate variants at the specific polymorphic sites of the target nucleic acid molecule. In one embodiment, the antisense molecule is designed to specifically bind to nucleic acids comprising the AA insertion in *BRIP1.* As antisense molecules can be used to inactivate mRNA so as to inhibit gene expression, and thus protein expression, the molecules can be used for disease treatment. The methodology can involve cleavage by means of ribozymes containing nucleotide sequences complementary to one or more regions in the mRNA that attenuate the ability of the mRNA to be translated. Such mRNA regions include, for example, protein-coding regions, in particular protein-coding regions corresponding to catalytic activity, substrate and/or ligand binding sites, or other functional domains of a protein.

The phenomenon of RNA interference (RNAi) has been actively studied for the last decade, since its original discovery in *C. elegans* (Fire et al., Nature 391:806-11 (1998)), and in recent years its potential use in treatment of human disease has been actively pursued (reviewed in Kim & Rossi, Nature Rev. Genet. 8:173-204 (2007)). RNA interference (RNAi), also called gene silencing, is based on using double-stranded RNA molecules (dsRNA) to turn off specific genes. In the cell, cytoplasmic double-stranded RNA molecules (dsRNA) are processed by cellular complexes into small interfering RNA (siRNA). The siRNA guide the targeting of a protein-RNA complex to specific sites on a target mRNA, leading to cleavage of the mRNA (Thompson, Drug Discovery Today, 7:912-917 (2002)). The siRNA molecules are typically about 20, 21, 22 or 23 nucleotides in length. Thus, one aspect of the disclosure relates to isolated nucleic acid molecules, and the use of those molecules for RNA interference, i.e. as small interfering RNA molecules (siRNA). In one embodiment, the isolated nucleic acid molecules are 18-26 nucleotides in length, preferably 19-25 nucleotides in length, more preferably 20-24 nucleotides in length, and more preferably 21, 22 or 23 nucleotides in length.

Another pathway for RNAi-mediated gene silencing originates in endogenously encoded primary microRNA (pri-miRNA) transcripts, which are processed in the cell to generate precursor miRNA (pre-miRNA). These miRNA molecules are exported from the nucleus to the cytoplasm, where they undergo processing to generate mature miRNA molecules (miRNA), which direct translational inhibition by recognizing target sites in the 3' untranslated regions of mRNAs, and subsequent mRNA degradation by processing P-bodies (reviewed in Kim & Rossi, Nature Rev. Genet. 8:173-204 (2007)).

Clinical applications of RNAi include the incorporation of synthetic siRNA duplexes, which preferably are approximately 20-23 nucleotides in size, and preferably have 3' overlaps of 2 nucleotides. Knockdown of gene expression is established by sequence-specific design for the target mRNA. Several commercial sites for optimal design and synthesis of such molecules are known to those skilled in the art.

Other applications provide longer siRNA molecules (typically 25-30 nucleotides in length, preferably about 27 nucleotides), as well as small hairpin RNAs (shRNAs; typically about 29 nucleotides in length). The latter are naturally expressed, as described in Amarzguioui et al. (FEBS Lett. 579:5974-81 (2005)). Chemically synthetic siRNAs and shRNAs are substrates for *in vivo* processing, and in some cases provide more potent gene-silencing than shorter designs (Kim et al., Nature Biotechnol. 23:222-226 (2005); Siolas et al., Nature Biotechnol. 23:227-231 (2005)). In general siRNAs provide for transient silencing of gene expression, because their intracellular concentration is diluted by subsequent cell divisions. By contrast, expressed shRNAs mediate long-term, stable knockdown of target transcripts, for as long as transcription of the shRNA takes place (Marques et al., Nature Biotechnol. 23:559-565 (2006); Brummelkamp et al., Science 296: 550-553 (2002)).

Since RNAi molecules, including siRNA, miRNA and shRNA, act in a sequence-dependent manner, the variants presented herein can be used to design RNAi reagents that recognize specific nucleic acid molecules comprising specific alleles and/or haplotypes (*e.g.*, the alleles and/or haplotypes of the present invention), while not recognizing nucleic acid molecules comprising other alleles or haplotypes. These RNAi reagents can thus recognize and destroy the target nucleic acid molecules. As with antisense reagents, RNAi reagents can be useful as therapeutic agents (i.e., for turning off disease-associated genes or disease-associated gene variants), but may also be useful for characterizing and validating gene function (*e.g.*, by gene knock-out or gene knock-down experiments).

Delivery of RNAi may be performed by a range of methodologies known to those skilled in the art. Methods utilizing non-viral delivery include cholesterol, stable nucleic acid-lipid particle (SNALP), heavy-chain antibody fragment (Fab), aptamers and nanoparticles. Viral delivery methods include use of lentivirus, adenovirus and adeno-associated virus. The siRNA molecules are in some embodiments chemically modified to increase their stability. This can include modifications at the 2' position of the ribose, including 2'-O-methylpurines and 2'-fluoropyrimidines, which provide resistance to Rnase activity. Other chemical modifications are possible and known to those skilled in the art.

The following references provide a further summary of RNAi, and possibilities for targeting specific genes using RNAi: Kim & Rossi, Nat. Rev. Genet. 8:173-184 (2007), Chen & Rajewsky, Nat. Rev. Genet. 8: 93-103 (2007), Reynolds, et al., Nat. Biotechnol. 22:326-330 (2004), Chi et al., Proc. Natl. Acad. Sci. USA 100:6343-6346 (2003), Vickers et al., J. Biol. Chem. 278:7108-7118 (2003), Agami, Curr. Opin. Chem. Biol. 6:829-834 (2002), Lavery, et al., Curr. Opin. Drug Discov. Devel. 6:561-569 (2003), Shi, Trends Genet. 19:9-12 (2003), Shuey et al., Drug Discov. Today 7:1040-46 (2002), McManus et al., Nat. Rev. Genet. 3:737-747 (2002), Xia et al., Nat. Biotechnol. 20:1006-10 (2002), Plasterk et al., curr. Opin. Genet. Dev. 10:562-7 (2000), Bosher et al., Nat. Cell Biol. 2:E31-6 (2000), and Hunter, Curr. Biol. 9:R440-442 (1999).

*Nucleic acids and polypeptides*

The nucleic acids and polypeptides described herein can be used in methods and kits of the present invention. An "isolated" nucleic acid molecule, as used herein, is one that is separated from nucleic acids that normally flank the gene or nucleotide sequence (as in genomic sequences) and/or has been completely or partially purified from other transcribed sequences (e.g., as in an RNA library). For example, an isolated nucleic acid of the invention can be substantially isolated with respect to the complex cellular milieu in which it naturally occurs, or culture medium when produced by recombinant techniques, or chemical precursors or other chemicals when chemically synthesized. In some instances, the isolated material will form part of a composition (for example, a crude extract containing other substances), buffer system or reagent mix. In other circumstances, the material can be purified to essential homogeneity, for example as determined by polyacrylamide gel electrophoresis (PAGE) or column chromatography (e.g., HPLC). An isolated nucleic acid molecule of the invention can comprise at least about 50%, at least about 80% or at least about 90% (on a molar basis) of all macromolecular species present. With regard to genomic DNA, the term "isolated" also can refer to nucleic acid molecules that are separated from the chromosome with which the genomic DNA is naturally associated. For example, the isolated nucleic acid molecule can contain less than about 250 kb, 200 kb, 150 kb, 100 kb, 75 kb, 50 kb, 25 kb, 10 kb, 5 kb, 4 kb, 3 kb, 2 kb, 1 kb, 0.5 kb or 0.1 kb of the nucleotides that flank the nucleic acid molecule in the genomic DNA of the cell from which the nucleic acid molecule is derived.

The disclosure also pertains to nucleic acid molecules that hybridize under high stringency hybridization conditions, such as for selective hybridization, to a nucleotide sequence described herein (*e.g.*, nucleic acid molecules that specifically hybridize to a nucleotide sequence containing a polymorphic site associated with a marker or haplotype described herein). Such nucleic acid molecules can be detected and/or isolated by allele- or sequence-specific hybridization (*e.g.*, under high stringency conditions). Stringency conditions and methods for nucleic acid hybridizations are well known to the skilled person (see, *e.g.*, Current Protocols in Molecular Biology, Ausubel, F. et al, John Wiley & Sons, (1998), and Kraus, M. and Aaronson, S., Methods Enzymol., 200:546-556 (1991), the entire teachings of which are incorporated by reference herein.

The percent identity of two nucleotide or amino acid sequences can be determined by aligning the sequences for optimal comparison purposes (*e.g.*, gaps can be introduced in the sequence of a first sequence). The nucleotides or amino acids at corresponding positions are then compared, and the percent identity between the two sequences is a function of the number of identical positions shared by the sequences (i.e., % identity = # of identical positions/total # of positions x 100). In certain embodiments, the length of a sequence aligned for comparison purposes is at least 30%, at least 40%, at least 50%, at least 60%, at least 70%, at least 80%, at least 90%, or at least 95%, of the length of the reference sequence. The actual comparison of the two sequences can be accomplished by methods well known to the skilled person, for example, using the NBLAST and XBLAST programs, as described in Altschul, S. et al., Nucleic Acids Res., 25:3389-3402 (1997). Another example of an algorithm is BLAT (Kent, W.J. Genome Res. 12:656-64 (2002)).

The present disclosure also provides isolated nucleic acid molecules that contain a fragment or portion that hybridizes under highly stringent conditions to a nucleic acid that comprises, or consists of, the nucleotide sequence of the human *BRIP1* gene as set forth in SEQ ID NO:15, or a nucleotide sequence comprising, or consisting of, the complement of the nucleotide sequence of SEQ ID NO:15. In certain embodiments, the nucleotide sequence comprises at least one polymorphic allele as described herein (*e.g.*, chr17:57208601 ins+AA or chr17: 57213073 delTT). The nucleic acid fragments of the invention may suitably be at least about 15, at least about 18, 20, 23 or 25 nucleotides, and can be up to 30, 40, 50, 100, 200, 300 or 400 nucleotides in length.

The nucleic acid fragments of the disclosure are used as probes or primers in assays such as those described herein. "Probes" or "primers" are oligonucleotides that hybridize in a base-specific manner to a complementary strand of a nucleic acid molecule. In addition to DNA and RNA, such probes and primers include polypeptide nucleic acids (PNA), as described in Nielsen, P. et al., Science 254:1497-1500 (1991). A probe or primer comprises a region of nucleotide sequence that hybridizes to at least about 15, typically about 20-25, and in certain embodiments about 40, 50 or 75, consecutive nucleotides of a nucleic acid molecule. In one embodiment, the probe or primer comprises at least one allele of at least one polymorphic marker or at least one haplotype described herein, or the complement thereof. In particular embodiments, a probe or primer can comprise 100 or fewer nucleotides; for example, in certain embodiments from 6 to 50 nucleotides, or, for example, from 12 to 30 nucleotides. In other embodiments, the probe or primer is at least 70% identical, at least 80% identical, at least 85% identical, at least 90% identical, or at least 95% identical, to the contiguous nucleotide sequence or to the complement of the contiguous nucleotide sequence. In another embodiment, the probe or primer is capable of selectively hybridizing to the contiguous nucleotide sequence or to the complement of the contiguous nucleotide sequence. Often, the probe or primer further comprises a label, *e.g.,* a radioisotope, a fluorescent label, an enzyme label, an enzyme co-factor label, a magnetic label, a spin label, an epitope label.

*Antibodies*

The disclosure also provides antibodies which bind to an epitope comprising either a BRIP1 variant amino acid sequence (e.g., a polypeptide comprising an amino acid substitution or a truncated polypeptide) encoded by a variant allele or the reference amino acid sequence encoded by the corresponding non-variant or wild-type allele of *BRIP1.* The term "antibody" as used herein refers to immunoglobulin molecules and immunologically active portions of immunoglobulin molecules, *i.e*., molecules that contain antigen-binding sites that specifically bind an antigen. A molecule that specifically binds to a polypeptide of the invention is a molecule that binds to that polypeptide or a fragment thereof (*e.g.*, a BRIP1 polypeptide with sequence as set forth in SEQ ID NO:13, or a fragment thereof), but does not substantially bind other molecules in a sample, *e.g.,* a biological sample, which naturally contains the polypeptide. Examples of immunologically active portions of immunoglobulin molecules include F(ab) and $F(ab')_2$ fragments which can be generated by treating the antibody with an enzyme such as pepsin. The disclosure provides polyclonal and monoclonal antibodies that bind to a polypeptide of the invention. The term "monoclonal antibody" or "monoclonal antibody composition", as used herein, refers to a population of antibody molecules that contain only one species of an antigen binding site capable of immunoreacting with a particular epitope of a polypeptide of the invention. A monoclonal antibody composition thus typically displays a single binding affinity for a particular polypeptide of the invention with which it immunoreacts.

Polyclonal antibodies can be prepared as described above by immunizing a suitable subject with a desired immunogen, *e.g.*, polypeptide of the invention or a fragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (*e.g.,* from the blood) and further purified by well-known techniques, such as protein A chromatography to obtain the IgG fraction. At an appropriate time after immunization, *e.g.*, when the antibody titers are highest, antibody-producing cells can be obtained from the subject and used to prepare monoclonal antibodies by standard techniques, such as the hybridoma technique originally described by Kohler and Milstein, Nature 256:495-497 (1975), the human B cell hybridoma technique (Kozbor et al., Immunol. Today 4: 72 (1983)), the EBV-hybridoma technique (Cole et al., Monoclonal Antibodies and Cancer Therapy, Alan R. Liss,1985, Inc., pp. 77-96) or trioma techniques. The technology for producing hybridomas is well known (see generally Current Protocols in Immunology (1994) Coligan et al., (eds.) John Wiley & Sons, Inc., New York, NY). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide of the invention.

Any of the many well known protocols used for fusing lymphocytes and immortalized cell lines can be applied for the purpose of generating a monoclonal antibody to a polypeptide of the invention (see, *e.g., Current Protocols in Immunology, supra;* Galfre et al., Nature 266:55052 (1977); R.H. Kenneth, in Monoclonal Antibodies: A New Dimension In Biological Analyses, Plenum Publishing Corp., New York, New York (1980); and Lerner, Yale J. Biol. Med. 54:387-402 (1981)). Moreover, the ordinarily skilled worker will appreciate that there are many variations of such methods that also would be useful.

Alternative to preparing monoclonal antibody-secreting hybridomas, a monoclonal antibody to a polypeptide of the invention can be identified and isolated by screening a recombinant combinatorial immunoglobulin library (*e.g.*, an antibody phage display library) with the polypeptide to thereby isolate immunoglobulin library members that bind the polypeptide. Kits for generating and screening phage display libraries are commercially available (*e.g.*, the Pharmacia *Recombinant Phage Antibody System,* Catalog No. 27-9400-01; and the Stratagene *Surf*ZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., Bio/Technology 9: 1370-1372 (1991); Hay et al., Hum. Antibod. Hybridomas 3:81-85 (1992); Huse et al., Science 246: 1275-1281 (1989); and Griffiths et al., EMBO J. 12:725-734 (1993).

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art.

In general, antibodies of the invention (*e.g.*, a monoclonal antibody) can be used to isolate a polypeptide as described herein by standard techniques, such as affinity chromatography or immunoprecipitation. A polypeptide-specific antibody can facilitate the purification of natural polypeptide from cells and of recombinantly produced polypeptide expressed in host cells. Moreover, an antibody specific for a polypeptide of the invention can be used to detect the polypeptide (*e.g.*, in a cellular lysate, cell supernatant, or tissue sample) in order to evaluate the abundance and pattern of expression of the polypeptide. Antibodies can be used diagnostically to monitor protein levels in tissue as part of a clinical testing procedure, *e.g.*, to, for example, determine the efficacy of a given treatment regimen. The antibody can be coupled to a detectable substance to facilitate its detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}I$, $^{131}I$, $^{35}S$ or $^{3}H$.

Antibodies can furthermore be useful for assessing expression of proteins, *e.g.* BRIP1 expression. Antibodies specific BRIP1, or variants or truncated forms of BRIP1, may be used to determine the expression levels of BRIP1 in a sample from an individual.

Antibodies can be used in other methods. Thus, antibodies are useful as diagnostic tools for evaluating proteins, such as variant proteins of the invention, in conjunction with analysis by electrophoretic mobility, isoelectric point, tryptic or other protease digest, or for use in other physical assays known to those skilled in the art. Antibodies may also be used in tissue typing. In one such embodiment, a specific variant protein has been correlated with expression in a specific tissue type, and antibodies specific for the variant protein can then be used to identify the specific tissue type. For example, BRIP1 antibodies may be used to determine the expression levels of *BRIP1* in ovarian cancer tumors.

Subcellular localization of proteins, including variant proteins, can also be determined using antibodies, and can be applied to assess aberrant subcellular localization of the protein in cells in various tissues. Such use can be applied in genetic testing, but also in monitoring a particular treatment modality. For example, it may be useful to determine the expression levels of *BRIP1* in tumor samples from an individual. In certain embodiments, expression levels of *BRIP1* in ovarian tumor samples are determined.

Antibodies are further useful for inhibiting variant protein function, for example by blocking the binding of a variant protein to a binding molecule or partner. Such uses can also be applied in a therapeutic context in which treatment involves inhibiting a variant protein's function. An antibody can be for example be used to block or competitively inhibit binding, thereby modulating (i.e., agonizing or antagonizing) the activity of the protein. Antibodies can be prepared against specific protein fragments containing sites required for specific function or against an intact protein that is associated with a cell or cell membrane. For administration *in vivo,* an antibody may be linked with an additional therapeutic payload, such as radionuclide, an enzyme, an immunogenic epitope, or a cytotoxic agent, including bacterial toxins (diphtheria or plant toxins, such as ricin). The *in vivo* half-life of an antibody or a fragment thereof may be increased by pegylation through conjugation to polyethylene glycol.

The present disclosure further relates to kits for using antibodies in the methods described herein. This includes, but is not limited to, kits for detecting the presence or absence of a protein (*e.g.*, BRIP1, or variants or truncated forms thereof) in a test sample. One preferred embodiment comprises antibodies such as a labelled or labelable antibody and a compound or agent for detecting proteins in a biological sample, means for determining the amount or the presence and/or absence of protein (*e.g.*, BRIP1, or variants or truncated forms thereof) in the sample, and means for comparing the amount of variant protein in the sample with a standard, as well as instructions for use of the kit.

*Computer-implemented aspects*

As understood by those of ordinary skill in the art, the methods and information described herein may be implemented, in all or in part, as computer executable instructions on known computer readable media. For example, the methods described herein may be implemented in hardware. Alternatively, the method may be implemented in software stored in, for example, one or more memories or other computer readable medium and implemented on one or more processors. As is known, the processors may be associated with one or more controllers, calculation units and/or other units of a computer system, or implanted in firmware as desired. If implemented in software, the routines may be stored in any computer readable memory such as in RAM, ROM, flash memory, a magnetic disk, a laser disk, or other storage medium, as is also known. Likewise, this software may be delivered to a computing device via any known delivery method including, for example, over a communication channel such as a telephone line, the Internet, a wireless connection, etc., or via a transportable medium, such as a computer readable disk, flash drive, etc.

More generally, and as understood by those of ordinary skill in the art, the various steps described above may be implemented as various blocks, operations, tools, modules and techniques which, in turn, may be implemented in hardware, firmware, software, or any combination of hardware, firmware, and/or software. When implemented in hardware, some or all of the blocks, operations, techniques, etc. may be implemented in, for example, a custom integrated circuit (IC), an application specific integrated circuit (ASIC), a field programmable logic array (FPGA), a programmable logic array (PLA), etc.

When implemented in software, the software may be stored in any known computer readable medium such as on a magnetic disk, an optical disk, or other storage medium, in a RAM or ROM or flash memory of a computer, processor, hard disk drive, optical disk drive, tape drive, etc. Likewise, the software may be delivered to a user or a computing system via any known delivery method including, for example, on a computer readable disk or other transportable computer storage mechanism.

Thus, another aspect of the invention is a system that is capable of carrying out a part or all of a method of the invention, or carrying out a variation of a method of the invention as described herein in greater detail. Exemplary systems include, as one or more components, computing systems, environments, and/or configurations that may be suitable for use with the methods and include, but are not limited to, personal computers, server computers, hand-held or laptop devices, multiprocessor systems, microprocessor-based systems, set top boxes, programmable consumer electronics, network PCs, minicomputers, mainframe computers, distributed computing environments that include any of the above systems or devices, and the like. In some variations, a system of the invention includes one or more machines used for analysis of biological material (e.g., genetic material), as described herein. In some variations, this analysis of the biological material involves a chemical analysis and/or a nucleic acid amplification.

With reference to Fig. 1, an exemplary system of the invention, which may be used to implement one or more steps of methods of the invention, includes a computing device in the form of a computer 110. Components shown in dashed outline are not technically part of the computer 110, but are used to illustrate the exemplary embodiment of Fig. 1. Components of computer 110 may include, but are not limited to, a processor 120, a system memory 130, a memory/graphics interface 121, also known as a Northbridge chip, and an I/O interface 122, also known as a Southbridge chip. The system memory 130 and a graphics processor 190 may be coupled to the memory/graphics interface 121. A monitor 191 or other graphic output device may be coupled to the graphics processor 190.

A series of system busses may couple various system components including a high speed system bus 123 between the processor 120, the memory/graphics interface 121 and the I/O interface 122, a front-side bus 124 between the memory/graphics interface 121 and the system memory 130, and an advanced graphics processing (AGP) bus 125 between the memory/graphics interface 121 and the graphics processor 190. The system bus 123 may be any of several types of bus structures including, by way of example, and not limitation, such architectures include Industry Standard Architecture (ISA) bus, Micro Channel Architecture (MCA) bus and Enhanced ISA (EISA) bus. As system architectures evolve, other bus architectures and chip sets may be used but often generally follow this pattern. For example, companies such as Intel and AMD support the Intel Hub Architecture (IHA) and the Hypertransport™ architecture, respectively.

The computer 110 typically includes a variety of computer-readable media. Computer-readable media can be any available media that can be accessed by computer 110 and includes both volatile and nonvolatile media, removable and non-removable media. By way of example, and not limitation, computer readable media may comprise computer storage media. Computer storage media includes both volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other physical medium which can be used to store the desired information and which can accessed by computer 110.

The system memory 130 includes computer storage media in the form of volatile and/or nonvolatile memory such as read only memory (ROM) 131 and random access memory (RAM) 132. The system ROM 131 may contain permanent system data 143, such as identifying and manufacturing information. In some embodiments, a basic input/output system (BIOS) may also be stored in system ROM 131. RAM 132 typically contains data and/or program modules that are immediately accessible to and/or presently being operated on by processor 120. By way of example, and not limitation, Fig. 1 illustrates operating system 134, application programs 135, other program modules 136, and program data 137.

The I/O interface 122 may couple the system bus 123 with a number of other busses 126, 127 and 128 that couple a variety of internal and external devices to the computer 110. A serial peripheral interface (SPI) bus 126 may connect to a basic input/output system (BIOS) memory 133 containing the basic routines that help to transfer information between elements within computer 110, such as during start-up.

A super input/output chip 160 may be used to connect to a number of 'legacy' peripherals, such as floppy disk 152, keyboard/mouse 162, and printer 196, as examples. The super I/O chip 160 may be connected to the I/O interface 122 with a bus 127, such as a low pin count (LPC) bus, in some embodiments. Various embodiments of the super I/O chip 160 are widely available in the commercial marketplace.

In one embodiment, bus 128 may be a Peripheral Component Interconnect (PCI) bus, or a variation thereof, may be used to connect higher speed peripherals to the I/O interface 122. A PCI bus may also be known as a Mezzanine bus. Variations of the PCI bus include the Peripheral Component Interconnect-Express (PCI-E) and the Peripheral Component Interconnect - Extended (PCI-X) busses, the former having a serial interface and the latter being a backward compatible parallel interface. In other embodiments, bus 128 may be an advanced technology attachment (ATA) bus, in the form of a serial ATA bus (SATA) or parallel ATA (PATA).

The computer 110 may also include other removable/non-removable, volatile/nonvolatile computer storage media. By way of example only, Fig. 1 illustrates a hard disk drive 140 that reads from or writes to non-removable, nonvolatile magnetic media. The hard disk drive 140 may be a conventional hard disk drive.

Removable media, such as a universal serial bus (USB) memory 153, firewire (IEEE 1394), or CD/DVD drive 156 may be connected to the PCI bus 128 directly or through an interface 150. A storage media 154 may couple through interface 150. Other removable/non-removable, volatile/nonvolatile computer storage media that can be used in the exemplary operating environment include, but are not limited to, magnetic tape cassettes, flash memory cards, digital versatile disks, digital video tape, solid state RAM, solid state ROM, and the like.

The drives and their associated computer storage media discussed above and illustrated in Fig. 1, provide storage of computer readable instructions, data structures, program modules and other data for the computer 110. In Fig. 1, for example, hard disk drive 140 is illustrated as storing operating system 144, application programs 145, other program modules 146, and program data 147. Note that these components can either be the same as or different from operating system 134, application programs 135, other program modules 136, and program data 137. Operating system 144, application programs 145, other program modules 146, and program data 147 are given different numbers here to illustrate that, at a minimum, they are different copies. A user may enter commands and information into the computer 20 through input devices such as a mouse/keyboard 162 or other input device combination. Other input devices (not shown) may include a microphone, joystick, game pad, satellite dish, scanner, or the like. These and other input devices are often connected to the processor 120 through one of the I/O interface busses, such as the SPI 126, the LPC 127, or the PCI 128, but other busses may be used. In some embodiments, other devices may be coupled to parallel ports, infrared interfaces, game ports, and the like (not depicted), via the super I/O chip 160.

The computer 110 may operate in a networked environment using logical connections to one or more remote computers, such as a remote computer 180 via a network interface controller (NIC) 170, . The remote computer 180 may be a personal computer, a server, a router, a network PC, a peer device or other common network node, and typically includes many or all of the elements described above relative to the computer 110. The logical connection between the NIC 170 and the remote computer 180 depicted in Fig. 1 may include a local area network (LAN), a wide area network (WAN), or both, but may also include other networks. Such networking environments are commonplace in offices, enterprise-wide computer networks, intranets, and the Internet. The remote computer 180 may also represent a web server supporting interactive sessions with the computer 110, or in the specific case of location-based applications may be a location server or an application server.

In some embodiments, the network interface may use a modem (not depicted) when a broadband connection is not available or is not used. It will be appreciated that the network connection shown is exemplary and other means of establishing a communications link between the computers may be used.

In some variations, the invention is a system for identifying susceptibility to ovarian cancer in a human subject. For example, in one variation, the system includes tools for performing at least one step, preferably two or more steps, and in some aspects all steps of a method of the invention, where the tools are operably linked to each other. Operable linkage describes a linkage through which components can function with each other to perform their purpose.

In some variations, a system of the invention is a system for identifying susceptibility to ovarian cancer in a human subject, and comprises:

(a) at least one processor;

(b) at least one computer-readable medium;

(c) a susceptibility database operatively coupled to a computer-readable medium of the system and containing population information correlating the presence or absence of one or more alleles of the human *BRIP1* gene and susceptibility to ovarian cancer in a population of humans;

(d) a measurement tool that receives an input about the human subject and generates information from the input about the presence or absence of at least one mutant *BRIP1* allele indicative of a frameshift or nonsense *BRIP1* defect in the human subject; and

(e) an analysis tool or routine that:

(i) is operatively coupled to the susceptibility database and the information generated by the measurement tool,

(ii) is stored on a computer-readable medium of the system,

(iii) is adapted to be executed on a processor of the system, to compare the information about the human subject with the population information in the susceptibility database and generate a conclusion with respect to susceptibility to the cancer for the human subject.

Exemplary processors (processing units) include all variety of microprocessors and other processing units used in computing devices. Exemplary computer-readable media are described above. When two or more components of the system involve a processor or a computer-readable medium, the system generally can be created where a single processor and/or computer readable medium is dedicated to a single component of the system; or where two or more functions share a single processor and/or share a single computer readable medium, such that the system contains as few as one processor and/or one computer readable medium. In some variations, it is advantageous to use multiple processors or media, for example, where it is convenient to have components of the system at different locations. For instance, some components of a system may be located at a testing laboratory dedicated to laboratory or data analysis, whereas other components, including components (optional) for supplying input information or obtaining an output communication, may be located at a medical treatment or counseling facility (e.g., doctor's office, health clinic, HMO, pharmacist, geneticist, hospital) and/or at the home or business of the human subject (patient) for whom the testing service is performed.

Referring to Figure 2, an exemplary system includes a susceptibility database **208** that is operatively coupled to a computer-readable medium of the system and that contains population information correlating the presence or absence of one or more alleles of the human *BRIP1* gene and susceptibility to a cancer in a population of humans. For example, the one or more alleles of the *BRIP1* gene include mutant alleles that cause, or are indicative of, a *BRIP1* defect such as reduced or lost function, as described elsewhere herein.

In a simple variation, the susceptibility database contains **208** data relating to the frequency that a particular allele of *BRIP1* has been observed in a population of humans with the cancer and a population of humans free of the cancer. Such data provides an indication as to the relative risk or odds ratio of developing the cancer for a human subject that is identified as having the allele in question. In another variation, the susceptibility database includes similar data with respect to two or more alleles of *BRIP1,* thereby providing a useful reference if the human subject has any of the two or more alleles. In still another variation, the susceptibility database includes additional quantitative personal, medical, or genetic information about the individuals in the database diagnosed with the cancer or free of the cancer. Such information includes, but is not limited to, information about parameters such as age, sex, ethnicity, race, medical history, weight, diabetes status, blood pressure, family history of the cancer, smoking history, and alcohol use in humans and impact of the at least one parameter on susceptibility to the cancer. The information also can include information about other genetic risk factors for the cancer besides *BRIP1.* These more robust susceptibility databases can be used by an analysis routine **210** to calculate a combined score with respect to susceptibility or risk for developing the cancer.

In addition to the susceptibility database **208,** the system further includes a measurement tool **206** programmed to receive an input **204** from or about the human subject and generate an output that contains information about the presence or absence of the at least one *BRIP1* allele of interest. (The input **204** is not part of the system per se but is illustrated in the schematic Figure 2.) Thus, the input **204** will contain a specimen or contain data from which the presence or absence of the at least one *BRIP1* allele can be directly read, or analytically determined. In a simple variation, the input contains annotated information about genotypes or allele counts for BRIP1 in the genome of the human subject, in which case no further processing by the measurement tool **206** is required, except possibly transformation of the relevant information about the presence/absence of the *BRIP1* allele into a format compatible for use by the analysis routine **210** of the system.

In another variation, the input **204** from the human subject contains data that is unannotated or insufficiently annotated with respect to *BRIP1,* requiring analysis by the measurement tool **206.** For example, the input can be genetic sequence of a chromosomal region or chromosome on which *BRIP1* resides, or whole genome sequence information, or unannotated information from a gene chip analysis of a variable loci in the human subject's genome. In such variations of the invention, the measurement tool **206** comprises a tool, preferably stored on a computer-readable medium of the system and adapted to be executed on a processor of the system, to receive a data input about a subject and determine information about the presence or absence of the at least one mutant *BRIP1* allele in a human subject from the data. For example, the measurement tool **206** contains instructions, preferably executable on a processor of the system, for analyzing the unannotated input data and determining the presence or absence of the *BRIP1* allele of interest in the human subject. Where the input data is genomic sequence information, and the measurement tool optionally comprises a sequence analysis tool stored on a computer readable medium of the system and executable by a processor of the system with instructions for determining the presence or absence of the at least one mutant *BRIP1* allele from the genomic sequence information.

In yet another variation, the input **204** from the human subject comprises a biological sample, such as a fluid (e.g., blood) or tissue sample, that contains genetic material that can be analyzed to determine the presence or absence of the *BRIP1* allele of interest. In this variation, an exemplary measurement tool **206** includes laboratory equipment for processing and analyzing the sample to determine the presence or absence (or identity) of the *BRIP1* allele(s) in the human subject. For instance, in one variation, the measurement tool includes: an oligonucleotide microarray (e.g., "gene chip") containing a plurality of oligonucleotide probes attached to a solid support; a detector for measuring interaction between nucleic acid obtained from or amplified from the biological sample and one or more oligonucleotides on the oligonucleotide microarray to generate detection data; and an analysis tool stored on a computer-readable medium of the system and adapted to be executed on a processor of the system, to determine the presence or absence of the at least one *BRIP1* allele of interest based on the detection data.

To provide another example, in some variations the measurement tool **206** includes: a nucleotide sequencer (e.g., an automated DNA sequencer) that is capable of determining nucleotide sequence information from nucleic acid obtained from or amplified from the biological sample; and an analysis tool stored on a computer-readable medium of the system and adapted to be executed on a processor of the system, to determine the presence or absence of the at least one mutant *BRIP1* allele based on the nucleotide sequence information.

In some variations, the measurement tool **206** further includes additional equipment and/or chemical reagents for processing the biological sample to purify and/or amplify nucleic acid of the human subject for further analysis using a sequencer, gene chip, or other analytical equipment.

The exemplary system further includes an analysis tool or routine **210** that: is operatively coupled to the susceptibility database **208** and operatively coupled to the measurement tool **206,** is stored on a computer-readable medium of the system, is adapted to be executed on a processor of the system to compare the information about the human subject with the population information in the susceptibility database **208** and generate a conclusion with respect to susceptibility to the cancer for the human subject. In simple terms, the analysis tool **210** looks at the *BRIP1* alleles identified by the measurement tool **206** for the human subject, and compares this information to the susceptibility database **208**, to determine a susceptibility to the cancer for the subject. The susceptibility can be based on the single parameter (the identity of one or more *BRIP1* alleles), or can involve a calculation based on other genetic and non-genetic data, as described above, that is collected and included as part of the input **204** from the human subject, and that also is stored in the susceptibility database **208** with respect to a population of other humans. Generally speaking, each parameter of interest is weighted to provide a conclusion with respect to susceptibility to the cancer. Such a conclusion is expressed in the conclusion in any statistically useful form, for example, as an odds ratio, a relative risk, or a lifetime risk for subject developing the cancer.

In some variations of the invention, the system as just described further includes a communication tool **212.**

For example, the communication tool is operatively connected to the analysis routine **210** and comprises a routine stored on a computer-readable medium of the system and adapted to be executed on a processor of the system, to: generate a communication containing the conclusion; and to transmit the communication to the human subject **200** or the medical practitioner **202,** and/or enable the subject or medical practitioner to access the communication. (The subject and medical practitioner are depicted in the schematic Fig. 2, but are not part of the system per se, though they may be considered users of the system. The communication tool **212** provides an interface for communicating to the subject, or to a medical practitioner for the subject (e.g., doctor, nurse, genetic counselor), the conclusion generated by the analysis tool **210** with respect to susceptibility to the cancer for the subject. Usually, if the communication is obtained by or delivered to the medical practitioner **202,** the medical practitioner will share the communication with the human subject **200** and/or counsel the human subject about the medical significance of the communication. In some variations, the communication is provided in a tangible form, such as a printed report or report stored on a computer readable medium such as a flash drive or optical disk. In some variations, the communication is provided electronically with an output that is visible on a video display or audio output (e.g., speaker). In some variations, the communication is transmitted to the subject or the medical practitioner, e.g., electronically or through the mail. In some variations, the system is designed to permit the subject or medical practitioner to access the communication, e.g., by telephone or computer. For instance, the system may include software residing on a memory and executed by a processor of a computer used by the human subject or the medical practitioner, with which the subject or practitioner can access the communication, preferably securely, over the internet or other network connection. In some variations of the system, this computer will be located remotely from other components of the system, e.g., at a location of the human subject's or medical practitioner's choosing.

In some variations of the invention, the system as described (including embodiments with or without the communication tool) further includes components that add a treatment or prophylaxis utility to the system. For instance, value is added to a determination of susceptibility to a cancer when a medical practitioner can prescribe or administer a standard of care that can reduce susceptibility to the cancer; and/or delay onset of the cancer; and/or increase the likelihood of detecting the cancer at an early stage, to facilitate early treatment when the cancer has not spread and is most curable. Exemplary lifestyle change protocols include loss of weight, increase in exercise, cessation of unhealthy behaviors such as smoking, and change of diet. Exemplary medicinal and surgical intervention protocols include administration of pharmaceutical agents for prophylaxis; and surgery, including in extreme cases surgery to remove a tissue or organ before it has become cancerous. Exemplary diagnostic protocols include non-invasive and invasive imaging; monitoring metabolic biomarkers; and biopsy screening.

For example, in some variations, the system further includes a medical protocol database **214** operatively connected to a computer-readable medium of the system and containing information correlating the presence or absence of the at least one *BRIP1* allele of interest and medical protocols for human subjects at risk for the cancer. Such medical protocols include any variety of medicines, lifestyle changes, diagnostic tests, increased frequencies of diagnostic tests, and the like that are designed to achieve one of the aforementioned goals. The information correlating a *BRIP1* allele with protocols could include, for example, information about the success with which the cancer is avoided or delayed, or success with which the cancer is detected early and treated, if a subject has a *BRIP1* susceptibility allele and follows a protocol.

The system of this embodiment further includes a medical protocol tool or routine **216,** operatively connected to the medical protocol database **214** and to the analysis tool or routine **210.** The medical protocol tool or routine **216** preferably is stored on a computer-readable medium of the system, and adapted to be executed on a processor of the system, to: (i) compare (or correlate) the conclusion that is obtained from the analysis routine **210** (with respect to susceptibility to cancer for the subject) and the medical protocol database **214,** and (ii) generate a protocol report with respect to the probability that one or more medical protocols in the medical protocol database will achieve one or more of the goals of reducing susceptibility to the cancer; delaying onset of the cancer; and increasing the likelihood of detecting the cancer at an early stage to facilitate early treatment. The probability can be based on empirical evidence collected from a population of humans and expressed either in absolute terms (e.g., compared to making no intervention), or expressed in relative terms, to highlight the comparative or additive benefits of two or more protocols.

Some variations of the system just described include the communication tool **212.** In some examples, the communication tool generates a communication that includes the protocol report in addition to, or instead of, the conclusion with respect to susceptibility.

Information about *BRIP1* allele status not only can provide useful information about identifying or quantifying susceptibility to cancers; it can also provide useful information about possible causative factors for a human subject identified with a cancer, and useful information about therapies for the cancer patient. In some variations, systems of the invention are useful for these purposes.

For instance, in some variations the invention is a system for assessing or selecting a treatment protocol for a subject diagnosed with ovarian cancer. An exemplary system, schematically depicted in Figure 3, comprises:

(a) at least one processor;

(b) at least one computer-readable medium;

(c) a medical treatment database **308** operatively connected to a computer-readable medium of the system and containing information correlating the presence or absence of at least one frameshift or nonsense *BRIP1* allele and efficacy of treatment regimens for the cancer;

(d) a measurement tool **306** to receive an input (**304,** depicted in Fig. 3 but not part of the system per se) about the human subject and generate information from the input **304** about the presence or absence of the at least one *BRIP1* allele indicative of a *BRIP1* defect in a human subject diagnosed with the cancer; and

(e) a medical protocol routine or tool **310** operatively coupled to the medical treatment database **308** and the measurement tool **306,** stored on a computer-readable medium of the system, and adapted to be executed on a processor of the system, to compare the information with respect to presence or absence of the at least one *BRIP1* allele for the subject and the medical treatment database, and generate a conclusion with respect to at least one of:

(i) the probability that one or more medical treatments will be efficacious for treatment of the cancer for the patient; and

(ii) which of two or more medical treatments for the cancer will be more efficacious for the patient.

Preferably, such a system further includes a communication tool **312** operatively connected to the medical protocol tool or routine **310** for communicating the conclusion to the subject **300,** or to a medical practitioner for the subject **302** (both depicted in the schematic of Fig. 3, but not part of the system per se). An exemplary communication tool comprises a routine stored on a computer-readable medium of the system and adapted to be executed on a processor of the system, to generate a communication containing the conclusion; and transmit the communication to the subject or the medical practitioner, or enable the subject or medical practitioner to access the communication.

The present invention will now be exemplified by the following non-limiting examples.

**EXAMPLE 1**

An analysis of sequence variants in the entire genome was performed, the aim being to identify sequence variants that predispose to ovarian cancer. With this purpose, a study of 873 Icelandic women diagnosed with ovarian cancer and over 37,000 Icelandic population controls was performed. All cases had been diagnosed with Invasive Ovarian Cancer, excluding individuals with borderline tumors. The analysis included data for 15,957,390 autosomal SNPs that include those identified through whole genome sequencing of 457 Icelanders and imputed into Icelandic cancer cases and controls.

Genotype data used for analysis contained genotype chip data for genotyped individuals supplemented with genotype information generated by imputation into ungenotyped relatives of genotyped subjects. A total of 68 Icelandic ovarian cancer cases had been chip typed with the Illumina HumanHap300 or CNV370 chips and an additional 572 ovarian cancer cases without chip genotype information were assigned *in silico* genotypes. The genotypes of the cases were compared to the genotypes of controls matched on genotype informativeness, including 41,607 chip typed controls. Logistic regression was used to test for association between SNPs and disease, treating disease status as the response and expected genotype counts from imputation or allele counts from direct genotyping as covariates. Testing was performed using the likelihood ratio statistic.

The association analysis yielded a number of genome-wide significant association ($P < 5 \times 10^{-8}$) signals on chromosome 17q23.2 (**Table 1**)**.** The most significant SNP was found to be rs34289250, which is located in the *BRIP1* gene (OR = 7.95, p-value $5.6x10^{-13}$). The markers identified are all located in a segment that spans about 2Mb, and contains a number of genes, including *TMEM49, TUBD1, RNFT1, DHX40P1, HEATR6, CA4, USP32, C17orf64, APPBP2, PPM1D, BCAS3, TBX2, C17orf82, TBX4, NACA2, BRIP1, INTS2,* and *MED13.*

**Table 1.** Association results for markers in chromosome 17q23.2 that are associated with ovarian cancer. Shown are: marker identity, p-value of association with ovarian cancer, odds ratio (OR), number of individuals whose genotype was imputed, frequency of effect allele in population, information content of imputation, location of marker on chromosome 17 in NCBI Build 36, identity of effect and other allele, and reference to SEQ ID showing flanking sequence and location of that marker.

| SNP | P adj | OR | # indiv | Freq | Info | Location | Effect allele | Other allele | SEQ ID NO: |
|---|---|---|---|---|---|---|---|---|---|
| rs34289250 | 5.65E-13 | 7.95 | 37538 | 0.008944 | 0.91 | 57,235,428 | C | T | 1 |
| rs12938171 | 2.05E-12 | 6.59 | 37540 | 0.011346 | 0.94 | 57,335,137 | A | G | 2 |
| chr17: 55422245 | 5.62E-09 | 10.6 | 37602 | 0.003988 | 0.88 | 55,422,245 | A | T | 3 |
| chr17: 55217320 | 5.71E-09 | 10.61 | 37598 | 0.00404 | 0.87 | 55,217,320 | C | T | 4 |
| rs12451939 | 7.13E-09 | 4.22 | 37540 | 0.983001 | 0.95 | 57,402,424 | G | A | 5 |
| chr17: 56567990 | 7.20E-09 | 10.23 | 37535 | 0.004065 | 0.90 | 56,567,990 | G | T | 6 |
| chr17: 56478611 | 7.66E-09 | 10.19 | 37536 | 0.004077 | 0.90 | 56,478,611 | A | C | 7 |
| chr17: 56505864 | 7.66E-09 | 10.19 | 37536 | 0.004077 | 0.90 | 56,505,864 | C | T | 8 |
| rs12937080 | 8.26E-09 | 4.19 | 37540 | 0.983041 | 0.96 | 57,284,519 | G | A | 9 |

**EXAMPLE 2**

Inspection of the genomic sequence over the *BRIP1* gene in carriers of the at-risk C allele of rs34289250 revealed a two nucleotide insertion in exon 14 of *BRIP1.* This insertion introduces two A nucleotides (AA) between position 57,208,601 and 57,208,602 (chr17:57208601 ins+AA). Thus, a stretch of AAAA at position 57,208,601 - 57,208,604 increases in size to a stretch of AAAAAA. This mutation inserts TT in the mRNA sequence at position 2345, causing a frameshift and a premature termination of protein translation. As shown in Table 2, the insertion leads to a frameshift in the transcribed *BRIP1* sequence, starting at codon 680, resulting in a premature STOP codon at codon 688 (last translated amino acid at codon 687).

**Table 2.** Results of sequencing in the *BRIP1* gene. The upper part (reference) shows the reference wild-type cDNA sequence (middle line) and its translated polynucleotide product (top line; SEQ ID NO:21). The lower part (mutation) shows the mutated sequence. The two-nucleotide TT insertion in the trancribed sequence is shown in bold (position 2345-2346 in the altered transcript). The altered peptide starts with codon 680, and the predicted STOP codon is in codon 688 (SEQ ID NO:14). The bottom line in each case shows the corresponding wild-type sequence in NCBI Build 36 (inverse orientation (3' to 5') compared with the transcript since gene is transcribed on opposite strand). The * in the translated protein sequence of the mutated form indicates a STOP codon, which is predicted to terminate translation of the encoded protein.

reference:

```
     661  C  A  T  F  Q  N  T  E  T  F  E  F  Q  D  E  V  G  A  L  L
    2287  TGTGCTACCTTCCAGAATACTGAAACATTTGAGTTCCAAGATGAAGTGGGAGCACTTTTG
57208660  ACACGATGGAAGGTCTTATGACTTTGTAAACTCAAGGTTCTACTTCACCCTCGTGAAAAC

     681  L  S  V  C  Q  T  V  S  Q  G  I  L  C  F  L  P  S  Y  K
    2347  TTATCTGTGTGCCAGACTGTGAGCCAAGGAATTTTGTGTTTCTTGCCATCTTACAAG
57208600  AATAGACACACGGTCTGACACTCGGTTCCTTAAAACACAAAGAACGGTAGAATGTTC
```

mutation +AA (+TT in transcribed sequence), first stop codon in position 688:

```
     661  C  A  T  F  Q  N  T  E  T  F  E  F  Q  D  E  V  G  A  L  F
    2287  TGTGCTACCTTCCAGAATACTGAAACATTTGAGTTCCAAGATGAAGTGGGAGCACTTTTT
57208660  ACACGATGGAAGGTCTTATGACTTTGTAAACTCAAGGTTCTACTTCACCCTCGTGAAAA-

     681  C  Y  L  C  A  R  L  *  A  K  E  F  C  V  S  C  H  L  T
          TGTTATCTGTGTGCCAGACTGTGAGCCAAGGAATTTTGTGTTTCTTGCCATCTTACAAG
57208600  -CAATAGACACACGGTCTGACACTCGGTTCCTTAAAACACAAAGAACGGTAGAATGTTC
```

**Table 3.** Results of association analysis of the -/AA insertion/deletion polymorphism in *BRIP1* in Iceland. Shown are p-values of association, relative risk, number of affected individuals assessed, allelic frequency of insertion, number of controls assessed and the frequency of the insertion polymorphism in controls.

| p-val | RR | #aff | aff.freq | #con | con.freq |
|---|---|---|---|---|---|
| **8.11E-06** | 7.12 | 291 | 0.0241 | 1159 | 0.00345 |

To investigate this mutation further, direct Sanger sequencing was performed on 291 individuals with Ovarian cancer and 1159 controls over the -/AA insertion in exon 14 of *BRIP1.*

As can be seen in Table 3, the insertion is significantly associated with risk of ovarian cancer. The insertion has an allelic frequency of 0.35% in controls and about 2.4% in cases, which means that the carrier frequency is about 0.7% in controls and about 5% in cases. Given the functional consequence of the AA insertion, it is likely a causative variant for the association to ovarian cancer observed in Icelandic samples.

Further analysis included Sanger sequencing and microsatellite genotyping; in total, we genotyped the insert directly in 11,741 Icelandic cancer cases and 3,913 controls, including 318 ovarian cancer cases. Using these data, the insert was imputed into all available cancer cases and controls, both those who had been chip genotyped and un-genotyped relatives of chip-typed individuals. Combining results from directly genotyped and imputed cancer cases, the association between ovarian cancer and the insert became even more significant (OR=8.13 (95% CI 4.74, 13.95), $P=2.8\times10^{-14}$),(Table 4).

| | **N genotyped** | | | | | **Removing ovarian cancer cases** | |
|---|---|---|---|---|---|---|---|
| **Phenotype** | **Directly** | **Chipped** | ***In silico*** | **OR (95% CI)** | **P** | **OR (95% CI)** | **P** |
| Ovarian Cancer | 318 | 2 | 548 | 8.13 (4.74, 13.95) | $2.8\times10^{-14}$ | - | - |

(continued)

| | N genotyped | | | | | Removing ovarian cancer cases | |
|---|---|---|---|---|---|---|---|
| **Phenotype** | **Directly** | **Chipped** | ***In silico*** | **OR (95% CI)** | **P** | **OR (95% CI)** | **P** |
| Pancreatic Cancer | 158 | 6 | 1,074 | 2.71 (1.31, 5.58) | 0.0069 | 2.58 (1.22, 5.46) | 0.013 |
| Breast Cancer | 2,740 | 173 | 2,543 | 1.28 (0.84, 1.96) | 0.25 | 1.28 (0.84,1.96) | 0.26 |
| All Cancers | 4,457 | 9,874 | 25,280 | 1.50 (1.25, 1.79) | $8.9\times10^{-6}$ | 1.36 (1.14,1.64) | 0.00092 |

**Table 4.** Association of the *BRIP* mutation, chr17:57208601 ins+AA, with cancer in the Icelandic population. For the individual cancers, directly typed cases are compared to 3,913 directly typed controls, in each case excluding the known cases of the cancer being tested from the controls. The remaining cancer cases and controls that have been chip typed or in silico genotyped are compared to over 40,000 controls and then combined using the Mantel-Haenszel model (OR with 95% CI and P value given).

## EXAMPLE 3

Mutations in the genes previously shown to increase risk of ovarian cancer, i.e. *BRCA1, BRCA2* and the MMR genes, also carry an increased risk of other cancer types, mainly breast and pancreatic cancers in the case of *BRCA1* and *BRCA2* and colorectal and endometrial cancers in the case of the MMR genes. Experimentation was performed to determine whether the chr17:57208601 ins+AA insert is associated with an increase in the risk of other cancer types, using information from the nation-wide Icelandic Cancer Registry and direct genotype information from 14,331 directly genotyped and 16,873 *in silico* genotyped cancer cases (representing 23 different cancer types). Analysis of the risk of individual cancer types showed an excess of pancreatic cancer in carriers (OR=2.71, *P*=0.0069).

In contrast to the individual cancers, when all the cancers were analyzed together, carriers of the insert had an increased risk of being diagnosed with cancer in general (OR=1.50, $P=1.5\times10^{-6}$) (Table 5). Given the large effect of the insert on ovarian cancer and the fact that ovarian cancer can co-occur with other cancer types, the analysis was repeated excluding the ovarian cancer cases (Table 6). Even without the ovarian cancer cases, the risk of any cancer remained significant (OR=1.36, $P=9.2\times10^{-4}$). Notably, although no cancer type was individually significantly associated with the insert after correcting for the number of tests, three cancer types had P values <0.05, i.e. cancers of the pancreas, rectum and upper airways. In total, 16 of the 22 cancer types showed an effect in the same direction as ovarian cancer. The lifespan of 8,342 deceased Icelanders who had been directly typed or imputed for the chr17:57208601 ins+AA insert was determined along with 7,604 deceased Icelanders with high quality *in silico* genotypes who were born after 1900 and lived to be at least 50 years old. It was found that the insert reduces lifespan in the Icelandic population by 3.6 years (95% CI 1.5, 5.7 years, $P=7.2\times10^{-4}$).

**Table 5. Association of the BRIP mutation, chr17:57208601 ins+AA, with 23 cancers or having any cancer in the Icelandic population.**

| | N | | | Directly typed | | Imputed | | Combined | | |
|---|---|---|---|---|---|---|---|---|---|---|
| **Phenotype** | **Direct** | **Chipped** | ***In silico*** | **OR** | **P** | **OR** | **P** | **OR (95% CI)** | **P** | **$P_{het}$** |
| Bladder Cancer | 754 | 42 | 1,049 | 0.32 | 0.079 | 0.98 | 0.97 | 0.66 (0.31, 1.40) | 0.28 | 0.17 |
| Brain Cancer Glioma | 121 | 4 | 298 | 0.00 | 0.18 | 1.03 | 0.98 | 0.63 (0.07, 5.62) | 0.68 | 0.20 |
| Brain Cancer Meningioma | 139 | 2 | 79 | 1.76 | 0.48 | 1.02 | 0.99 | 1.57 (0.39, 6.37) | 0.52 | 0.76 |
| Breast Cancer | 2,740 | 173 | 2,543 | 1.60 | 0.097 | 0.94 | 0.86 | 1.28 (0.84, 1.96) | 0.25 | 0.23 |
| Cervix Uteri Cancer | 232 | 19 | 584 | 1.08 | 0.92 | 0.23 | 0.14 | 0.61 (0.18, 2.01) | 0.42 | 0.22 |
| Chronic Lymphocytic Leukemia | 113 | 7 | 283 | 1.08 | 0.94 | 1.48 | 0.71 | 1.28 (0.29, 5.66) | 0.75 | 0.84 |
| Colon Cancer | 291 | 601 | 1,944 | 3.03 | 0.031 | 0.85 | 0.63 | 1.25 (0.72, 2.16) | 0.43 | 0.038 |
| Endometrial Cancer | 428 | 16 | 507 | 0.56 | 0.41 | 2.40 | 0.13 | 1.33 (0.55, 3.19) | 0.53 | 0.11 |
| Esophagus Cancer | 103 | 2 | 576 | 2.38 | 0.32 | 1.12 | 0.88 | 1.57 (0.50, 4.91) | 0.43 | 0.52 |
| Gastric Cancer | 354 | 13 | 2,907 | 2.43 | 0.083 | 1.24 | 0.54 | 1.54 (0.87, 2.70) | 0.14 | 0.28 |
| Kidney Cancer | 519 | 18 | 1,036 | 1.88 | 0.16 | 0.44 | 0.23 | 1.20 (0.57, 2.49) | 0.63 | 0.074 |
| Liver Cancer | 70 | 5 | 374 | 0.00 | 0.31 | 1.37 | 0.72 | 1.15 (0.21, 6.22) | 0.87 | 0.29 |
| Lung Cancer | 273 | 631 | 3,160 | 1.37 | 0.65 | 1.40 | 0.19 | 1.39 (0.87, 2.23) | 0.16 | 0.98 |
| Lymphoma Hodgkin | 79 | 2 | 215 | 4.71 | 0.041 | 1.19 | 0.89 | 3.30 (0.92, 11.85) | 0.067 | 0.36 |

(continued)

**Table 5. Association of the BRIP mutation, chr17:57208601 ins+AA, with 23 cancers or having any cancer in the Icelandic population.**

| | N | | | Directly typed | | Imputed | | Combined | | |
|---|---|---|---|---|---|---|---|---|---|---|
| **Phenotype** | **Direct** | **Chipped** | ***In silico*** | **OR** | **P** | **OR** | **P** | **OR (95% CI)** | **P** | **$P_{het}$** |
| Lymphoma Non Hodgkin | 259 | 22 | 604 | 2.85 | 0.046 | 0.54 | 0.45 | 1.74 (0.74, 4.13) | 0.21 | 0.086 |
| Multiple Myeloma | 115 | 3 | 365 | 1.06 | 0.96 | 2.40 | 0.21 | 1.89 (0.60, 5.98) | 0.28 | 0.53 |
| Ovarian Cancer | 318 | 2 | 548 | 5.86 | 3.7E-06 | 11.60 | 7.10E-10 | 8.13 (4.74, 13.95) | 2.8E-14 | 0.22 |
| Pancreatic Cancer | 161 | 6 | 1,077 | 2.29 | 0.26 | 2.87 | 0.014 | 2.71 (1.31, 5.58) | 0.0069 | 0.79 |
| Prostate Cancer | 2,244 | 235 | 2,567 | 1.12 | 0.73 | 0.78 | 0.46 | 0.94 (0.59, 1.49) | 0.80 | 0.44 |
| Rectal Cancer | 77 | 230 | 781 | 3.29 | 0.18 | 2.09 | 0.062 | 2.25 (1.11, 4.58) | 0.025 | 0.64 |
| Testicular Cancer | 196 | 1 | 108 | 1.25 | 0.77 | 1.24 | 0.9 | 1.25 (0.31, 4.93) | 0.75 | 1.00 |
| Thyroid Cancer | 550 | 12 | 595 | 0.88 | 0.82 | 0.22 | 0.16 | 0.66 (0.25, 1.74) | 0.40 | 0.26 |
| Upper Airway Cancer | 35 | 35 | 381 | 3.52 | 0.33 | 0.02 | 0.044 | 0.76 (0.09, 6.40) | 0.80 | 0.026 |
| All Cancers* | 4,457 | 9, 874 | 25,280 | 1.78 | 0.048 | 1.47 | 5.60E-05 | 1.50 (1.25, 1.79) | 8.90E-06 | 0.53 |
| | | | | | | | | | | |

For the individual cancers, directly typed cases are compared to 3,913 directly typed controls, in each case excluding the known cases of the cancer being tested from the controls (OR and P value given). The remaining cancer cases and controls that have been chip typed or in silico genotyped are also compared (OR and P value given) and then combined using the Mantel-Haenszel model (OR with 95% CI and P value given). A P-value for testing for the difference between the ORs in the two groups is given in the Phet column. *Due to the relatively small number of directly genotyped controls we removed the chip typed cases from the list of directly genotyped cases when testing for association with any cancer type, thus including all chip typed cases in the second group. The P-values and 95%CIs have been adjusted using a genomic control correction factor for testing each phenotype based on the chip typed and in silico genotype data.

**Table 6. Association of the BRIP mutation, chr17:57208601 ins+AA, with 22 cancers or having any cancer in the Icelandic population after removal of all ovarian cancer cases.**

| | N | | | Directly typed | | Imputed | | Combined | | |
|---|---|---|---|---|---|---|---|---|---|---|
| **Phenotype** | **Direct** | **Chipped** | ***In silico*** | **OR** | **P** | **OR** | **P** | **OR (95% CI)** | **P** | **$P_{het}$** |
| Bladder Cancer | 752 | 42 | 1,048 | 0.32 | 0.079 | 1.00 | 1.0 | 0.66 (0.31, 1.42) | 0.29 | 0.16 |
| Brain Cancer Glioma | 121 | 4 | 298 | 0.00 | 0.18 | 1.05 | 0.96 | 0.74 (0.12, 4.65) | 0.75 | 0.19 |
| Brain Cancer Meningioma | 136 | 2 | 79 | 1.80 | 0.47 | 1.04 | 0.98 | 1.61 (0.40, 6.53) | 0.51 | 0.76 |
| Breast Cancer | 2,712 | 173 | 2,516 | 1.57 | 0.12 | 0.97 | 0.93 | 1.28 (0.84, 1.96) | 0.26 | 0.28 |
| Cervix Uteri Cancer | 230 | 19 | 579 | 1.09 | 0.91 | 0.24 | 0.15 | 0.62 (0.19, 2.06) | 0.44 | 0.23 |
| Chronic Lymphocytic Leukemia | 112 | 7 | 282 | 1.09 | 0.94 | 1.54 | 0.68 | 1.30 (0.29, 5.79) | 0.73 | 0.82 |
| Colon Cancer | 286 | 601 | 1,937 | 3.07 | 0.029 | 0.87 | 0.68 | 1.27 (0.73, 2.22) | 0.39 | 0.04 |
| Endometrial Cancer | 421 | 16 | 499 | 0.57 | 0.43 | 2.23 | 0.19 | 1.24 (0.50, 3.05) | 0.64 | 0.14 |
| Esophagus Cancer | 103 | 2 | 576 | 2.38 | 0.32 | 1.15 | 0.86 | 1.59 (0.51, 4.98) | 0.42 | 0.53 |
| Gastric Cancer | 352 | 13 | 2,900 | 2.43 | 0.082 | 1.28 | 0.48 | 1.58 (0.89, 2.78) | 0.12 | 0.30 |
| Kidney Cancer | 518 | 18 | 1,034 | 1.87 | 0.16 | 0.45 | 0.24 | 1.21 (0.58, 2.52) | 0.61 | 0.081 |
| Liver Cancer | 69 | 5 | 374 | 0.00 | 0.32 | 1.39 | 0.70 | 1.18 (0.23, 6.14) | 0.84 | 0.29 |
| Lung Cancer | 267 | 631 | 3,151 | 1.40 | 0.63 | 1.36 | 0.24 | 1.36 (0.85, 2.19) | 0.20 | 0.97 |
| Lymphoma Hodgkin | 79 | 2 | 215 | 4.69 | 0.041 | 1.21 | 0.88 | 3.32 (0.92, 11.94) ; | 0.066 | 0.37 |

(continued)

**Table 6. Association of the BRIP mutation, chr17:57208601 ins+AA, with 22 cancers or having any cancer in the Icelandic population after removal of all ovarian cancer cases.**

| | N | | | Directly typed | | Imputed | | Combined | | |
|---|---|---|---|---|---|---|---|---|---|---|
| **Phenotype** | **Direct** | **Chipped** | ***In silico*** | **OR** | **P** | **OR** | **P** | **OR (95% CI)** | **P** | **$P_{het}$** |
| Lymphoma Non Hodgkin | 258 | 21 | 603 | 2.85 | 0.046 | 0.56 | 0.48 | 1.77 (0.74, 4.19) | 0.20 | 0.093 |
| Multiple Myeloma | 113 | 3 | 364 | 1.08 | 0.95 | 2.46 | 0.20 | 1.93 (0.61, 6.12) | 0.26 | 0.52 |
| Pancreatic Cancer | 158 | 6 | 1,074 | 1.54 | 0.61 | 2.95 | 0.012 | 2.58 (1.22, 5.46) | 0.013 | 0.49 |
| Prostate Cancer | 2,244 | 235 | 2,567 | 1.12 | 0.73 | 0.80 | 0.51 | 0.95 (0.60, 1.51) | 0.83 | 0.48 |
| Rectal Cancer | 75 | 230 | 779 | 3.38 | 0.17 | 2.15 | 0.053 | 2.32 (1.14, 4.73) | 0.020 | 0.65 |
| Testicular Cancer | 196 | 1 | 108 | 1.24 | 0.78 | 1.26 | 0.90 | 1.25 (0.32, 4.93) | 0.75 | 0.99 |
| Thyroid Cancer | 544 | 12 | 589 | 0.89 | 0.83 | 0.23 | 0.17 | 0.67 (0.25, 1.77) | 0.42 | 0.27 |
| Upper Airway Cancer | 33 | 35 | 380 | 0.00 | 0.49 | 0.02 | 0.046 | 0.02 (0.00, 0.76) | 0.035 | 0.92 |
| All Cancers | 4,205 | 9,806 | 24,732 | 1.51 | 0.18 | 1.35 | 0.0023 | 1.36 (1.14, 1.64) | 0.00092 | 0.73 |
| | | | | | | | | | | |

Table legend is the same as for Table 5 except ovarian cancer cases have been removed from both cases and controls.

## EXAMPLE 4

To screen for additional mutations in *BRIP1* that might contribute to ovarian cancer risk in Iceland or other populations, the whole gene (exons, introns and upstream regulatory region) was sequenced in ovarian cancer cases and controls from Iceland, the Netherlands and Spain, using a pooling strategy where samples from ovarian cancer cases and controls were pooled separately, amplified by long-range PCR and sequenced using Solexa technology. Further details of the protocols used for this purpose are provided below under Example 6.

One deletion was detected in the Spanish case pools but not in control pools, chr17: 57213073 delTT (*i.e.,* a two basepair deletion following position 57213073) corresponding to deletion of TT in position 2008-2009 in SEQ ID NO:10). This out-of-frame mutation is located in exon 12 and leads to a termination of protein translation at amino acid 576 (Figure 4). Genotyping of the deletion in cancer cases and controls from Spain showed that the deletion is very rare (allelic frequency 0.03% in controls, N=1,780) but associates with a greatly increased risk of ovarian cancer (OR=24, P=0.017) and a significant risk of breast cancer (OR=11, P=0.009) (Table 7). The deletion was not found in any of the 2,758 Spanish cases with other cancer types, except for a single case of lung cancer. In addition to the Spanish deletion, 11 coding variants identified through the pool sequencing were genotyped (10 missense variants and the known FA variant R798X), as well as three missense variants that have previously been found in breast cancer cases (M299I, Q944E and P1034L), in the three study populations (Table 8). In short, these variants were either found in similar frequencies in cases and controls or they were too rare to be conclusively associated with ovarian cancer. It is therefore contemplated that association with ovarian cancer may be confined to mutations leading to truncated transcripts.

**Table 7. Association of the *BRIP* mutation, chr17: 57213073 delTT, with cancer in a Spanish population.**

| Phenotype | N individuals | | N alleles | Allele freq. | OR | P |
|---|---|---|---|---|---|---|
| | Carriers | Non-carriers | Mut/Wt | | | |
| Controls | 1 | 1779 | 1/3559 | 0.03% | 1.00 | |
| Ovarian cancer | 2 | 142 | 2/286 | 0.70% | 25.00 | 0.016 |
| Breast cancer | 6 | 921 | 6/1848 | 0.32% | 11.17 | 0.0079 |
| Lung cancer | 1 | 514 | 1/1029 | 0.10% | 3.34 | |
| Colorectal cancer | 0 | 497 | 0/994 | 0.00% | 0.00 | |
| Endometrial cancer | 0 | 130 | 0/260 | 0.00% | 0.00 | |
| Bladder cancer | 0 | 238 | 0/476 | 0.00% | 0.00 | |
| Prostate cancer | 0 | 699 | 0/1398 | 0.00% | 0.00 | |
| Basal cell carcinoma | 0 | 222 | 0/444 | 0.00% | 0.00 | |
| Cutaneous melanoma | 0 | 278 | 0/556 | 0.00% | 0.00 | |
| Thyroid cancer | 0 | 90 | 0/180 | 0.00% | 0.00 | |
| Kidney cancer | 0 | 89 | 0/178 | 0.00% | 0.00 | |

**Table 8. Allele counts of coding variants in *BRIP1* in ovarian cancer cases and controls from Iceland, The Netherlands and Spain**

| Variant | Iceland # alleles | | | | The Netherlands # alleles | | | | Spain # alleles | | | |
|---|---|---|---|---|---|---|---|---|---|---|---|---|
| | Cases | | Controls | | Cases | | Controls | | Cases | | Controls | |
| | Variant | Wild type | Variant | Wild type | Variant | Wild type | Variant | Wild type | Variant | Wild type | Variant | Wild type |
| P47A | 0 | 370 | 0 | 482 | 1 | 533 | 2 | 1188 | 0 | 284 | * | * |
| R173C | 2 | 434 | 23 | 2713 | 1 | 549 | 7 | 1439 | 0 | 288 | * | * |
| V193I | 4 | 524 | 13 | 2721 | 4 | 546 | 7 | 1437 | 0 | 288 | * | * |
| L195P | 0 | 436 | 2 | 2554 | 1 | 549 | 1 | 1445 | 0 | 286 | * | * |
| K209R | 0 | 436 | 0 | 2554 | 1 | 549 | 0 | 1446 | 0 | 284 | * | * |
| C214S | 0 | 436 | 0 | 2538 | 1 | 549 | 0 | 1366 | 0 | 274 | 0 | 848 |
| E262G | 0 | 438 | 0 | 2542 | 0 | 552 | 0 | 1394 | 0 | 272 | 0 | 858 |
| M299I | 0 | 438 | 0 | 2544 | 0 | 552 | 0 | 1376 | 0 | 274 | 0 | 856 |
| K297R | 1 | 437 | 0 | 2550 | 1 | 551 | 3 | 1373 | 0 | 274 | 0 | 856 |
| R419Q | 0 | 378 | 0 | 496 | 1 | 547 | 0 | 1280 | 0 | 288 | * | * |
| Q741H | 1 | 435 | 3 | 2181 | 0 | 540 | * | * | 0 | 286 | 5 | 813 |
| D745D | 0 | 436 | 1 | 2177 | 0 | 540 | * | * | 1 | 285 | 1 | 821 |
| R798X | 1 | 435 | 7 | 3051 | 0 | 544 | * | * | 0 | 284 | * | * |
| Q944E | 0 | 378 | 0 | 2854 | 0 | 538 | * | * | 0 | 282 | * | * |
| P1034L | 0 | 436 | 0 | 898 | 0 | 540 | * | * | 0 | 282 | * | * |

Variants were genotyped by Sanger sequencing of the relevant exons. Shown are the number of mutant and wild type alleles among cases and controls in each population.
*Indicates that genotyping was not done in control groups since no variant alleles were found in the cases.

**EXAMPLE 5**

It was tested whether *BRIP1* conforms to the classical paradigm of a tumor suppressor gene where the wild-type allele is lost in tumors of heterozygous carriers. Ovarian tumor samples from 10 carriers of the Icelandic mutation were obtained (2 fresh-frozen and 8 paraffin-embedded) and PCR and Sanger sequencing of genomic DNA was used to test for LOH. Eight of the 10 tumors showed loss of the wild-type allele (Figure 5). Whole-genome sequencing of tumor DNA from the two fresh-frozen tumors further confirmed the LOH at the *BRIP1* locus (Table 9). Finally, the ratio of wild-type to mutant mRNA was assessed using Sanger sequencing of cDNA. Both tumors showed a greatly reduced mRNA expression from the wild-type allele compared to the mutant allele supporting loss of the wild type allele in most of the cells within the tumor sample (Figure 6).

**Table 9. Whole-genome sequencing. Number of mutant and wild-type sequence reads in the area of chr17: 57208601 ins+AA**

| | Germ-line DNA | | | | Tumor DNA | | |
|---|---|---|---|---|---|---|---|
| | Cove rage | # WT alleles | # Mut alleles | | Coverage | #WT alleles | # Mut alleles |
| Case 6 | 44 | 28 | 29 | | 23 | 5 | 23 |
| Case 11 | 15 | 16 | 14 | | 27 | 7 | 37 |

Results from whole genome sequencing of germ-line DNA and tumor DNA from two heterozygous carriers of chr17: 57208601 ins+AA. Shown is the sequence coverage for each of the samples, the number of sequence reads for each allele, wild-type (WT)and mutant (Mut). Pathology review of the tumor samples estimated the tumor cell percentage to be 50-60% for case 6 and 60-70% for case 11

**EXAMPLE 6**

**Methods**

*Study populations:*

**Iceland.** Cancer cases were identified in the nation-wide Icelandic Cancer Registry (ICR; www.krabbameinsskra.is) which includes information on the age, month and year of diagnosis, month and year of death, SNOMED code and ICD-10 classification. A total of 868 cases of invasive ovarian cancer were diagnosed in Iceland from 1955-2009. Blood samples were collected from all prevalent cases available at the start of 2001, and all incident cases since then or a total of 224 cases. In addition, paraffin-embedded tissues samples from additional 116 ovarian cancer cases was obtained from the Biobank of Landspitali hospital. Thus, DNA from a total of 340 cases was available for genotyping. Written informed consent was obtained from all live cases. All projects at deCODE Genetics have been approved by the National Bioethics Committee and the Data Protection Authority of Iceland. The Icelandic controls used in this study consisted of individuals from other ongoing genome-wide association studies at deCODE. In addition to the directly genotyped cases, the ovarian cancer case group was augmented by *in-silico* genotyping of un-genotyped cases by imputation as described below.

**Spain.** Ovarian cancer cases were recruited from the Oncology Department of Zaragoza Hospital between September 2007 and February 2008. The Spanish control samples were from individuals who attended the University Hospital in Zaragoza for diseases other than cancer. Controls were questioned to rule out prior cancers before the blood sample was collected. Study protocols were approved by the Institutional Review Board of Zaragoza University Hospital and all subjects gave written informed consent.

**The Netherlands.** Ovarian cancer cases, diagnosed between 1989 and 2006 and still alive in 2008, were identified from the population-based cancer registry of the Comprehensive Cancer Center The Netherlands, Location Nijmegen, in the Mid-Eastern part of the Netherlands (8). Dutch control individuals were recruited within the Nijmegen Biomedical Study (NBS) (25).

**Finland.** Paraffin-embedded samples from Finnish ovarian cancer cases were used in the study. Informed consent was obtained from consecutive cases of epithelial ovarian cancer patients during 2008-2009 while they were visiting the Tampere University Hospital gynecological oncology clinic for adjuvant chemotherapy or follow-up, during 2008-2009. The 55 cases used in the study were patients who also had a matching blood sample. The 1,000 Finnish control samples were blood derived genomic DNA from anonymous Finnish blood donors.

*Illumina genome-wide genotyping:*

The Icelandic chip-typed samples were assayed with the Illumina Human Hap300, Hap CNV370, Hap 610, 1M or Omni-1 Quad bead chips at deCODE genetics. Only the 317,503 SNPs from the Human Hap300 chip were used in the long range phasing and the subsequent SNP imputations. SNPs were excluded if they had (i) yield lower than 95%, (ii) minor allele frequency less than 1% in the population or (iii) significant deviation from Hardy-Weinberg equilibrium in the controls ($P < 0.001$), (iv) if they produced an excessive inheritance error rate (over 0.001), (v) if there was substantial difference in allele frequency between chip types (from just a single chip if that resolved all differences, but from all chips otherwise). All samples with a call rate below 97% were excluded from the analysis. The final set of SNPs used for long range phasing was composed of 297,835 autosomal SNPs.

*Whole-genome sequencing and SNP imputations:*

SNPs were imputed based on unpublished data from the Icelandic whole genomic sequencing project (457 Icelandic individuals) selected for various neoplastic, cardiovascular and psychiatric conditions. All of the individuals were sequenced to a depth of at least 10X. Sixteen million SNPs were imputed based on this set of individuals.

1. *Sample preparation.* Paired-end libraries for sequencing were prepared according to the manufacturer's instructions (Illumina). In short, approximately 5 μg of genomic DNA, isolated from frozen blood samples, were fragmented to a mean target size of 300 bp using a Covaris E210 instrument. The resulting fragmented DNA was end repaired using T4 and Klenow polymerases and T4 polynucleotide kinase with 10 mM dNTP followed by addition of an 'A' base at the ends using Klenow exo fragment (3' to 5'-exo minus) and dATP (1 mM). Sequencing adaptors containing 'T' overhangs were ligated to the DNA products followed by agarose (2%) gel electrophoresis. Fragments of about 400 bp were isolated from the gels (QIAGEN Gel Extraction Kit), and the adaptor-modified DNA fragments were PCR enriched for ten cycles using Phusion DNA polymerase (Finnzymes Oy) and PCR primers PE 1.0 and PE 2.0 (Illumina). Enriched libraries were further purified using agarose (2%) gel electrophoresis as described above. The quality and concentration of the libraries were assessed with the Agilent 2100 Bioanalyzer using the DNA 1000 LabChip (Agilent). Barcoded libraries were stored at -20 °C. All steps in the workflow were monitored using an in-house laboratory information management system with barcode tracking of all samples and reagents.
2. *DNA sequencing.* Template DNA fragments were hybridized to the surface of flow cells (Illumina PE flowcell, v4) and amplified to form clusters using the Illumina cBot. In brief, DNA (3-10 pM) was denatured, followed by hybridization to grafted adaptors on the flowcell. Isothermal bridge amplification using Phusion polymerase was then followed by linearization of the bridged DNA, denaturation, blocking of 3' ends and hybridization of the sequencing primer. Sequencing-by-synthesis was performed on Illumina GAIIx instruments equipped with paired-end modules. Paired-end libraries for whole genome sequencing were sequenced using either $2\times101$ or $2\times120$ cycles of incorporation and imaging with Illumina sequencing kits, v4 or v5 (TruSeq). Each library or sample was initially run on a single lane for validation followed by further sequencing of $\geq4$ lanes with targeted raw cluster densities of 500-700 k/mm2, depending on the version of the data imaging and analysis packages. Imaging and analysis of the data was performed using SCS2.6 /RTA1.6, SCS2.8/RTA1.8 or SCS2.9&RTA1.9 software packages from Illumina, respectively. Real-time analysis involved conversion of image data to base-calling in real-time.
3. *Alignment.* For each lane in the DNA sequencing output, the resulting qseq files were converted into fastq files using an in-house script. All output from sequencing was converted, and the Illumina quality filtering flag was retained in the output. The fastq files were then aligned against Build 36 of the human reference sequence using bwa version 0.5.7 (26).
4. *BAM file generation.* SAM file output from the alignment was converted into BAM format using samtools version 0.1.8 (*27*), and an in-house script was used to carry the Illumina quality filter flag over to the BAM file. The BAM files for each sample were then merged into a single BAM file using samtools. Finally, Picard version 1.17 (see http://picard.sourceforge.net/) was used to mark duplicates in the resulting sample BAM files.
5. *SNP calling and genotyping in whole-genome sequencing.* A two-step approach was applied. The first step was to detect SNPs by identifying sequence positions where at least one individual could be determined to be different from the reference sequence with confidence (quality threshold of 20) based on the SNP calling feature of the pileup tool samtools (27). SNPs that always differed heterozygous or homozygous from the reference were removed. The second step was to use the pileup tool to genotype the SNPs at the positions that were flagged as polymorphic. Because sequencing depth varies and hence the certainty of genotype calls also varies, genotype likelihoods rather than deterministic calls were calculated (see below). Of the 2.5 million SNPs reported in the HapMap2 CEU samples, 96.3% were observed in the whole-genome sequencing data. Of the 6.9 million SNPs reported in the 1000 Genomes Project data, 89.4% were observed in the whole-genome sequencing data.

*Long range phasing:*

Long range phasing of all chip-genotyped individuals was performed with methods described previously (*10, 28*). In brief, phasing was achieved using an iterative algorithm which phases a single proband at a time given the available phasing information about everyone else that shares a long haplotype identically by state with the proband. Given the large fraction of the Icelandic population that has been chip-typed, accurate long range phasing is available genome-wide for all chip-typed Icelanders.

*Genotype imputation:*

The SNPs identified and genotyped through sequencing were imputed into all Icelanders who had been phased with long range phasing using the same model as used by IMPUTE (*29*). The genotype data from sequencing can be ambiguous due to low sequencing coverage. In order to phase the sequencing genotypes, an iterative algorithm was applied for each SNP with alleles 0 and 1. With $H$ representing the long range phased haplotypes of the sequenced individuals, the following algorithm was applied:

1. For each haplotype $h$ in $H$, use the Hidden Markov Model of IMPUTE to calculate for every other $k$ in $H$, the likelihood, denoted $Y_{h,k}$, of $h$ having the same ancestral source as $k$ at the SNP.

2. For every $h$ in $H$, initialize the parameter $\theta_h$, which specifies how likely the one allele of the SNP is to occur on the background of $h$ from the genotype likelihoods obtained from sequencing. The genotype likelihood $L_g$ is the probability of the observed sequencing data at the SNP for a given individual assuming $g$ is the true genotype at the SNP. If $L_0$, $L_1$ and $L_2$ are the likelihoods of the genotypes 0, 1 and 2 in the individual who carries $h$, then set $\theta_h = \frac{L_2+\frac{1}{2}L_1}{L_2+L_1+L_0}$.

3. For every pair of haplotypes $h$ and $k$ in $H$ that are carried by the same individual, use the other haplotypes in $H$ to predict the genotype of the SNP on the backgrounds of $h$ and $k$: $\tau_h = \sum_{l\in H\backslash\{h\}}\gamma_{h,l}\theta_l$ and $\tau_k = \sum_{l\in H\backslash\{k\}}\gamma_{k,l}\theta_l$. Combining these predictions with the genotype likelihoods from sequencing gives un-normalized updated phased genotype probabilities: $P_{00} = (1 - \tau_h)(1 - \tau_k)L_0$, $P_{10} = \tau_h(1-\tau_k)\frac{1}{2}L_1$, $P_{01} = (1-\tau_h)\tau_k\frac{1}{2}L_1$ and $P_{11} = \tau_h\tau_k L_2$. Now use these values to update $\theta_h$ and $\theta_k$ to $\theta_h = \frac{P_{10}+P_{11}}{P_{00}+P_{01}+P_{10}+P_{11}}$ and $\theta_k = \frac{P_{01}+P_{11}}{P_{00}+P_{01}+P_{10}+P_{11}}$.

4. Repeat step 3 when the maximum difference between iterations is greater than a convergence threshold $\varepsilon$. We used $\varepsilon$=$10^{-7}$.

Given the long range phased haplotypes and $\theta$, the allele of the SNP on a new haplotype $h$ not in $H$, is imputed as $\sum_{l\in H}\gamma_{h,l}\theta_l$.

The above algorithm can easily be extended to handle simple family structures such as parent-offspring pairs and triads by letting the $P$ distribution run over all founder haplotypes in the family structure. The algorithm also extends easily to the X-chromosome. If source genotype data are only ambiguous in phase, such as chip genotype data, then the algorithm is still applied, but all but one of the $L$s will be 0. In some instances, the reference set was intentionally enriched for carriers of the minor allele of a rare SNP in order to improve imputation accuracy. In this case, expected allele counts is biased toward the minor allele of the SNP. Call the enrichment of the minor allele $E$ and let $\theta'$ be the expected minor allele count calculated from the naive imputation method, and let $\theta$ be the unbiased expected allele count, then $\theta' = \frac{E\theta}{1-\theta+E\theta}$ and hence $\theta = \frac{\theta'}{E+(1-E)\theta'}$.

This adjustment was applied to all imputations based on enriched imputations sets. We note that if $\theta'$ is 0 or 1, then $\theta$ will also be 0 or 1, respectively.

*In silico (genealogy-based) genotyping:*

In addition to imputing sequence variants from the whole genome sequencing effort into chip genotyped individuals, a second imputation step where genotypes were imputed into relatives of chip genotyped individuals was also performed, creating *in silico* genotypes. The inputs into the second imputation step are the fully phased (in particular every allele has been assigned a parent of origin) imputed and chip type genotypes of the available chip typed individuals. The algorithm used to perform the second imputation step consists of:

1. For each ungenotyped individual (the proband), find all chip genotyped individuals within two meiosis of the individual. The six possible types of two meiosis relatives of the proband are (ignoring more complicated relationships due to pedigree loops): Parents, full and half siblings, grandparents, children and grandchildren. If all pedigree paths from the proband to a genotyped relative go through other genotyped relatives, then that relative is excluded. E.g. if a parent of the proband is genotyped, then the proband's grandparents through that parent are excluded. If the number of meiosis in the pedigree around the proband exceeds a threshold (we used 12), then relatives are removed from the pedigree until the number of meiosis falls below 12, in order to reduce computational complexity.
2. At every point in the genome, calculate the probability for each genotyped relative sharing with the proband based on the autosomal SNPs used for phasing. A multipoint algorithm based on the hidden Markov model Lander-Green multipoint linkage algorithm using fast Fourier transforms is used to calculate these sharing probabilities (*30, 31*). First single point sharing probabilities are calculated by dividing the genome into 0.5cM bins and using the haplotypes over these bins as alleles. Haplotypes that are the same, except at most at a single SNP, are treated as identical. When the haplotypes in the pedigree are incompatible over a bin, then a uniform probability distribution was used for that bin. The most common causes for such incompatibilities are recombinations within the pedigree, phasing errors and genotyping errors. Note that since the input genotypes are fully phased, the single point information is substantially more informative than for unphased genotyped, in particular one haplotype of the parent of a genotyped child is always known. The single point distributions are then convolved using the multipoint algorithm to obtain multipoint sharing probabilities at the center of each bin. Genetic distances were obtained from the most recent version of the deCODE genetic map (*32*).
3. Based on the sharing probabilities at the center of each bin, all the SNPs from the whole genome sequencing are imputed into the proband. To impute the genotype of the paternal allele of a SNP located at *x,* flanked by bins with centers at $x_{left}$ and $x_{right}$. Starting with the left bin, going through all possible sharing patterns *v*, let $I_v$ be the set of haplotypes of genotyped individuals that share identically by descent within the pedigree with the proband's paternal haplotype given the sharing pattern *v* and $P(v)$ be the probability of *v* at the left bin - this is the output from step 2 above - and let $e_i$ be the expected allele count of the SNP for haplotype *i*. Then $e_v = \frac{\sum_{i \in I_v} e_i}{\sum_{i \in I_v} 1}$ is the expected allele count of the paternal haplotype of the proband given *v* and an overall estimate of the allele count given the sharing distribution at the left bin is obtained from $e_{left} = \sum_v P(v) e_v$. If $I_v$ is empty then no relative shares with the proband's paternal haplotype given *v* and thus there is no information about the allele count. The probability that some genotyped relative shared the proband's paternal haplotype is therefore stored, $O_{left} = \sum_{v,I_v} = \emptyset P(V)$ and an expected allele count, conditional on the proband's paternal haplotype being shared by at least one genotyped relative: $c_{left} = \frac{\sum_{v,I_{v \neq \emptyset}} P(v) e_v}{\sum_{v,I_{v \neq \emptyset}} P(v)}$. In the same way calculate $O_{right}$ and $c_{right}$. Linear interpolation is then used to get an estimates at the SNP from the two flanking bins:

$$O = O_{left} + \frac{x - x_{left}}{x_{right} - x_{left}} \left( O_{right} - O_{left} \right),$$

$$c = c_{left} + \frac{x - x_{left}}{x_{right} - x_{left}} \left( c_{right} - c_{left} \right).$$

If $\theta$ is an estimate of the population frequency of the SNP then $Oc + (1 - O)\theta$ is an estimate of the allele count for the proband's paternal haplotype. Similarly, an expected allele count can be obtained for the proband's maternal haplotype.

*Case-control association testing:*

Logistic regression was used to test for association between SNPs and disease, treating disease status as the response and expected genotype counts from imputation or allele counts from direct genotyping as covariates. Testing was performed using the likelihood ratio statistic. When testing for association based on the *in silico* genotypes, controls were matched to cases based on the informativeness of the imputed genotypes, such that for each case *c* controls of matching informativeness where chosen. Failing to match cases and controls will lead to a highly inflated genomic control factor, and in some cases may lead to spurious false positive findings. The informativeness of each of the imputation of each one of an individual's haplotypes was estimated by taking the average of

$$a(e,\theta) = \begin{cases} \dfrac{e-\theta}{1-\theta}, & e \geq \theta \\ \dfrac{\theta - e}{\theta}, & e < \theta \end{cases}$$

over all SNPs imputed for the individual, where e is the expected allele count for the haplotype at the SNP and $\theta$ is the population frequency of the SNP. Note that $a(\theta,\theta) = 0$ and $a(0,\theta) = a(1,\theta) = 1$. The mean informativeness values cluster into groups corresponding to the most common pedigree configurations used in the imputation, such as imputing from parent into child or from child into parent. Based on this clustering of imputation informativeness, the haplotypes of individuals were divided into seven groups of varying informativeness which created 27 groups of individuals of similar imputation informativeness; 7 groups of individuals with both haplotypes having similar informativeness, 21 groups of individuals with the two haplotypes having different informativeness, minus the one group of individuals with neither haplotype being imputed well. Within each group the ratio of the number of controls and the number of cases is calculated, and the largest integer c that was less than this ratio in all the groups is chosen. For example, if in one group there are 10.3 times as many controls as cases and if in all other groups this ratio was greater, then *c* = 10 would be set and within each group there would be a random selection of ten times as many controls as there are cases.

*Inflation factor adjustment:*

In order to account for the relatedness and stratification within our case and control sample sets the method of genomic control is applied based on chip markers. For the ovarian cancer genome-wide association study the correction factors based on the genomic control was 1.12.

*Sequencing of BRIP1 in pooled DNA:*

*1. Target enrichment of BRIP1 in pooled DNA samples.* Pooled DNA samples were enriched for *BRIP1* (chr17:57,110,449-57,302,085, Build36/Hg18) using long range PCR (L-PCR). Primers were designed for overlapping (300-500bp) fragments of approximately 6 kb in size. Primer design was done using the following restrictions: Tm, 62 °C; length 23bp; GC content, 40-60% and by avoiding repeats and known SNP's or indels. Primer pairs which initially failed to generate products were re-designed to generate fragments of 3-3.5 kb in size. The sequences of the L-PCR primers are shown in Supplementary table 7. The Expand Long-Range dNTP pack from Roche Applied Science was used for L-PCR following the manufacturer's instructions. Each PCR reaction was done in a volume of 50 μl with an input of 80 ng of DNA per reaction. All PCR reactions were performed on MJR PTC-225 thermocyclers with 96-well blocks. PCR products were seperated using agarose (1.2%) gel electrophoresis and detected with BlueView (Sigma) staining. Bands of correct size were cut out and the DNA was isolated using Ultrafree DA DNA spin columns (Millipore). The concentration of each L-PCR product was measured using PicoGreen fluorescence measurements and the products (50 ng each) were combined into a single enriched sample for each pool. Final concentration and size distribution of the combined samples were assessed using the Agilent Bioanalyzer 2100 with a DNA 12000 LabChip kit.

*2. Preparation of target enriched samples for indexed sequencing.* Indexed paired-end libraries for sequencing were prepared using the TruSeq™ sample preparation kit according to the manufacturer's instructions (Illumina) and as described for the tumor samples above. Appropriate TruSeq sequencing adaptors for multiplexing (index #1-3) were employed

*3. DNA sequencing of target enriched samples:* Libraries with index #1, #2 and #3 were mixed together in equal quantities to generate indexed stocks of 2 nM. Samples were clustered as described above for whole genome sequencing using a template concentration of 3 pm. Sequencing was performed on Illumina GAIIx instruments equipped with paired-end modules. Paired-end samples (three indexed samples per lane) were sequenced using 2 × 50 cycles of incorporation and imaging with TruSeq Illumina sequencing kits, v5. Imaging and analysis of the data was performed using the SCS2.9/RTA1.9 software packages from Illumina, respectively.

*Analysis of pool sequencing data:*

A simple likelihood ratio test was used to test for the presence of a SNP at every exonic position. Since the number of individuals in each pool is less than 50, implying a minimal pool frequency of 1/100, only SNPs reaching an estimated pool frequency of at least 0.005 were considered. Indel calling within exons was done manually. Potential inserts and deletions falling within exons were identified using the samtools alignment results and all the resulting candidates were then inspected by eye. Only the Icelandic two-base insert and the Spanish two-base deletion were deemed to be likely to be real polymorphisms.

*Microsatellite genotyping:*

The PCR amplifications were set up and pooled using Zymark SciClone ALH 500 robots. The sequences of the primers used for genotyping are listed in Supplementary table 8. The reaction volume was 5 μl, and, for each PCR, 20 ng of genomic DNA was amplified in the presence of 2 pmol of each primer, 0.14 U AmpliTaq Gold, 0.33 mmol/liter dNTPs, and 3.3 mmol/liter MgCl2. The PCR conditions were 95°C for 10 minutes, then stepdown 4 cycles of 15 s at 94°C, 30 s at 63°C -2,5°C per cycle, and 30 s at 72°C, 11 cycles of 15 s at 94°C, 30 s at 55°C, and 1 min at 72°C, at last 22 cycles of 15 s at 89°C, 30 s at 55°C, and 1 min at 72°C. The PCR products were supplemented with an internal size standard, and the fragments were separated and detected on an Applied Biosystems model 3730 sequencer, using Genescan (v. 3.0) peak-calling software. Alleles were called using an internal allele-calling program (*33*).

*Association analysis*

For association analysis of the replication datasets, a standard likelihood ratio statistic was used, implemented in the NEMO software (*34*) to calculate two-sided *P*-values for each individual allele, assuming a multiplicative model for ovarian cancer risk, i.e. the ovarian cancer risk multiplies by the number of risk alleles a person carries. Results from multiple case-control groups were combined using a Mantel-Haenszel model in which the groups were allowed to have different population frequencies for alleles and genotypes but were assumed to have common odds ratios.

*Isolation of nucleic acids from fresh-frozen and paraffin-embedded tissue samples:*

DNA was extracted from sectioned frozen and paraffin-embedded tissue using MasterPure reagents (Epicentre Biotechnologies). For paraffin-embedded tissue, paraffin was removed prior to extraction by heating to 95°C in Tissue and Cell Lysis buffer (TCLS), cooling on ice and transferring the lysate to clean tubes. Reagent volumes were adjusted according to the amount of tissue. Tissue sections were lysed at room temperature whilst rotating until visibly digested. The manufacturer's protocol was followed for the remainder of the protocol. RNA was extracted from sectioned frozen tissue using RNAzol® RT (Molecular Research Centre). Tissue sections were homogenized using a rotor-stator homogenizer. The manufacturer's protocol for isolation of RNA containing fraction >200bp was followed.

*Test for BRIP1 expression and LOH in ovarian tumor samples:*

RNA samples from fresh-frozen ovarian tumor samples were converted to cDNA using the High Capacity cDNA Reverse Transcriptase kit (Applied Biosystems Inc.). The region around the 2 bp deletion in exon 12 was amplified from cDNA from frozen tumors and genomic DNA from frozen and paraffin-embedded tumors using conventional PCR (primer sequences are listed in Supplementary table 9). The PCR fragments were sequenced using the same primers and the Big-Dye R terminator v3.1 chemistry, followed by loading onto a 3730 DNA Analyzer (Applied Biosystems Inc.).

*Whole-genome sequencing of tumor DNA:*

The TruSeq™ sample preparation kit (Illumina) was employed for the preparation of sequencing libraries for genomic DNA from tumor samples and matched germline/blood samples. The method was the essentially the same as described above for germ-line DNA above, except the DNA input was 1 μg and the supplied TruSeq adaptors and PCR primer cocktail were used. Purification of samples following PCR amplification was also done using AMPure XP beads instead of gel electrophoresis.

## EXAMPLE 7

*BRIP1 binding to C-terminal BRCT motifs of wild-type human BRCA1 protein*

The BRCA1-interacting fraction of BRIP1 extends from amino acid residues 979-1006 and phosphorylation of Ser990 is important for the binding. Missense mutations within this region may affect the binding of BRIP1 to BRCA1. In addition, mutations in BRIP1 that are located outside of the BRCA1-binding region might also affect the interaction between the two proteins if the mutation affect the three-dimensional structure of the protein.

In order to test if a particular mutation affects the binding of BRIP1 to BRCA, the following assay could be applied as described by Cantor et al Cell 105:149-160 (2001).

A recombinant protein "bait", composed of the BRCT region of BRCA1 (amino acids 1529-1863) fused to glutathione S-transferase (GST), is immobilized on glutathione-coated sepharose beads. The mutant *BRIP1* cDNA is cloned into an appropriate plasmid vector, labeled ($^{35}$S) BRIP1 protein is produced by *in vitro* translation and incubated with the BRCT-GST fusion protein. After washing of the beads, binding between BRCT-GST and BRIP1 is assessed by SDS gel electrophoresis and autoradiography. Wild-type BRIP1 protein, cloned and produced in the same manner is used as positive control and empty vector as a negative control.

## EXAMPLE 8

*Assay for determining DNA-dependent ATPase activity*

BRIP1 contains both DNA-dependent ATPase and helicase activities and the helicase activity is dependent on the ATPase activity. To test the ATPase activity of BRIP1, the mutant *BRIP1* cDNA to be tested can be cloned into a FLAG-epitope containing baculovirus vector, transfected into insect cells and the recombinant protein purified using an anti-FLAG antibody. Wild-type *BRIP1* cDNA is also expressed in the same manner for use as positive control. The ATPase activity of the BRIP1 proteins is then assessed by measuring the release of free phosphate during ATP hydrolysis in the presence of different types of DNA (calf thymus DNA, circular single-stranded M13 DNA, and supercoiled pCDNA3.0) as cofactors, as described (Cantor et al. PNAS 101:2357-2362 (2004)).

## EXAMPLE 9

*Determination of DNA helicase activity*

To test for the helicase activity of mutant BRIP1 protein, the helicase assays described by Cantor et al. PNAS 101:2357-2362 (2004) may be used. Briefly, the mutant *BRIP1* cDNA is cloned into a FLAG-epitope containing baculovirus vector, transfected into insect cells and the recombinant protein is purified using anti-FLAG antibody. Wild-type *BRIP1* cDNA is also expressed in the same manner and used as positive control.

Various types of DNA and RNA oligonucleotides are used to construct the substrates for helicase assays and all DNA oligonucleotides are designed to be complementary to a segment of M13mp18 single-stranded DNA (M13). Oligonucleotides are used to generate partially doublestranded DNA and DNA:RNA duplexes by annealing to M13 DNA. After annealing, the annealed primer is extended by one nucleotide with DNA polymerase I (Klenow fragment) by using [$\alpha$-32P]GTP. After a purification step, helicase activity is measured by detecting the displacement of labeled DNA or RNA oligonucleotide from the partially duplexed substrate. The reaction is initiated by addition of immunoprecipitated, recombinant *BRIP1* and incubated at 30°C for 30 min. The reaction is stopped and the reaction products are resolved by electrophoresis in an 8% native TBE polyacrylamide gel containing 15% glycerol.

## EXAMPLE 10

*Determination of expression levels of of BRIP1 protein*

Missense mutations, although they may not alter the function of the protein, may result in a less stable protein product. Insufficient levels of the BRIP1 protein will have adverse effect on genomic integrity and increase the risk of tumor formation.

In order to test the stability of mutant BRIP1 proteins, a cycloheximide-chase analysis can be applied as described in Cantor et al Cell 105:149-160 (2001). Mutant and wild-type *BRIP1* cDNAs are cloned into an expression vector containing an appropriate tag, such as the Myc epitope. A eukaryotic cell line is transfected with the vectors, lysates are collected at fixed time points and the quantity of recombinant protein is assessed by western blotting using an anti-Myc antibody. To assess the half-life of the recombinant proteins, cycloheximide (an inhibitor of protein biosynthesis) is added to the cultures at a set time after transfection and the level of wild-type and mutant BRIP1 protein is assessed by western blotting at serial timepoints.

## Claims

1. A method of determining whether an individual is at increased risk of developing ovarian cancer, the method comprising steps of
determining, in a biological sample obtained from an individual, nucleic acid sequence information about the *BRIP1* gene; and
comparing the sequence information to the wild-type sequence of *BRIP1* (SEQ ID NO:10);
wherein an identification of a nonsense mutation or a frameshift mutation in *BRIP1* in the individual is indicative that the individual is at increased risk of developing ovarian cancer.

2. The method of claim 1, wherein the mutation results in a *BRIP1* defect selected from the group consisting of:

(a) premature truncation or frameshift of an encoded BRIP1 protein, relative to the BRIP1 amino acid sequence set forth in SEQ ID NO:13;
(b) expression of a BRIP1 protein with reduced activity compared to a wild-type BRIP1 protein (SEQ ID NO:13), wherein the activity is at least one BRIP1 activity selected from:

(i) BRIP1 binding to C-terminal BRCT motifs of wildtype human BRCA1 protein;
(ii) DNA-dependent ATPase activity; and
(iii) DNA helicase activity;

(c) reduced expression of BRIP1 protein, compared to wild-type BRIP1,

wherein mutant alleles indicative of the defect are associated with increased susceptibility to ovarian cancer.

3. The method of claim 1 or claim 2, wherein the mutation is selected from the group consisting of chr17:57208601 ins+AA in NCBI Build 36, and chr17: 57213073 delTT in NCBI Build 36.

4. A method of determining whether a human subject is at increased risk of developing ovarian cancer, the method comprising analyzing amino acid sequence data about a BRIP1 polypeptide from the subject, wherein a determination of the presence of a truncated BRIP1 polypeptide compared with a wild-type BRIP1 polypeptide with sequence as set forth in SEQ ID NO:13 is indicative that the subject is at increased risk of developing ovarian cancer.

5. The method of claim 4, wherein the amino acid sequence data is obtained from a biological sample from the human subject comprising human BRIP1 polypeptide, using a method that comprises at least one procedure selected from:

(i) an antibody assay; and
(ii) protein sequencing.

6. A method of selecting a human subject with ovarian cancer for treatment with an ovarian cancer therapeutic agent, the method comprising:

determining the presence or absence of at least one allele of at least one polymorphic marker in a nucleic acid sample obtained from the individual, wherein the at least one allele causes a loss of function or loss of expression of a *BRIP1,* and
selecting for treatment with the therapeutic agent a subject identified as having the at least one allele in the nucleic acid sample.

**7.** The method of claim 6, wherein the nucleic acid sample is from a cell of the ovarian cancer, and wherein the determining step comprises analyzing for loss of heterozygosity of *BRIP1* gene in the nucleic acid sample, and wherein the selecting step comprises selecting for treatment with the therapeutic agent a subject identified as having loss of heterozygosity of *BRIP1,* indicative of a loss-of-function of *BRIP1* in the nucleic acid sample.

**8.** The method of any claim 6 or claim 7, wherein the therapeutic agent for treating ovarian cancer is selected from the group consisting of PARP inhibitors and DNA crosslinking agents, wherein the PARP inhibitor is preferably selected from the group consisting of Iniparib, Olaparib, Veliparib, Rucaparib, CEP 9722, MK 4827 and KU-0059436, and wherein the DNA crosslinking agent is preferably selected from alkylating agents, cisplatin and cisplatin derivatives.

**9.** A system for identifying susceptibility to ovarian cancer in a human subject, the system comprising:

at least one processor;
at least one computer-readable medium;
a susceptibility database operatively coupled to a computer-readable medium of the system and containing population information correlating the presence or absence of one or more alleles of the human *BRIP1* gene and susceptibility to ovarian cancer in a population of humans;
a measurement tool that receives an input about the human subject and generates information from the input about the presence or absence of at least one mutant *BRIP1* allele indicative of a *BRIP1* defect in the human subject;
a communication tool operatively coupled to the analysis tool, stored on a computer-readable medium of the system and adapted to be executed on a processor of the system to communicate to the subject, or to a medical practitioner for the subject, the conclusion with respect to susceptibility to the cancer for the subject; and
an analysis tool that:

is operatively coupled to the susceptibility database and the the measurement tool,
is stored on a computer-readable medium of the system,
is adapted to be executed on a processor of the system, to compare the information about the human subject with the population information in the susceptibility database and generate a conclusion with respect to susceptibility to ovarian cancer for the human subject,

and wherein the mutant allele is a frameshift mutation or a nonsense mutation in *BRIP1.*

**10.** The system of claim 9, wherein the mutation is selected from the group consisting of chr17:57208601 ins+AA in NCBI Build 36 and chr17: 57213073 delTT in NCBI Build 36.

**11.** The system according to claim 9 or claim 10, wherein the at least one mutant *BRIP1* allele is indicative of a *BRIP1* defect selected from the group consisting of:

(a) premature truncation of an encoded BRIP1 protein, relative to the BRIP1 amino acid sequence set forth in SEQ ID NO: 13;
(b) expression of a BRIP1 protein with reduced activity compared to a wild-type BRIP1 protein (SEQ ID NO: 13), wherein the activity is at least one BRIP1 activity selected from:

(i) BRIP1 binding to C-terminal BRCT motifs of wildtype human BRCA1 protein;
(ii) DNA-dependent ATPase activity; and
(iii) DNA helicase activity; and

(c) reduced expression of BRIP1 protein, compared to wildtype expression,

wherein mutant alleles indicative of the defect are associated with increased susceptibility to ovarian cancer.

**12.** The system according to any one of claims 9-11, wherein the measurement tool comprises a tool stored on a computer-readable medium of the system and adapted to be executed by a processor of the system to receive a data input about a subject and determine information about the presence or absence of the at least one mutant *BRIP1* allele in a human subject from the data,
wherein the data is genomic sequence information, and the measurement tool comprises a sequence analysis tool stored on a computer readable medium of the system and adapted to be executed by a processor of the system to determine the presence or absence of the at least one mutant *BRIP1* allele from the genomic sequence information.

**13.** The system according to any one of claims 9-12, wherein the input about the human subject is a biological sample from the human subject, and wherein the measurement tool comprises a tool to identify the presence or absence of the at least one mutant *BRIP1* allele in the biological sample, thereby generating information about the presence or absence of the at least one mutant *BRIP1* allele in a human subject,
wherein the measurement tool optionally further includes:

a. an oligonucleotide microarray containing a plurality of oligonucleotide probes attached to a solid support;
a detector for measuring interaction between nucleic acid obtained from or amplified from the biological sample and one or more oligonucleotides on the oligonucleotide microarray to generate detection data; and
an analysis tool stored on a computer-readable medium of the system and adapted to be executed on a processor of the system, to determine the presence or absence of the at least one mutant *BRIP1* allele based on the detection data, or

wherein the measurement tool optionally includes:

b. a nucleotide sequencer capable of determining nucleotide sequence information from nucleic acid obtained from or amplified from the biological sample; and
an analysis tool stored on a computer-readable medium of the system and adapted to be executed on a processor of the system, to determine the presence or absence of the at least one mutant *BRIP1* allele based on the nucleotide sequence information.

**14.** The system according to any one of claims 9-13, further comprising:

a medical protocol database operatively connected to a computer-readable medium of the system and containing information correlating the presence or absence of the at least one mutant *BRIP1* allele and medical protocols for human subjects at risk for ovarian cancer; and
a medical protocol routine, operatively connected to the medical protocol database and the analysis routine, stored on a computer-readable medium of the system, and adapted to be executed on a processor of the system, to compare the conclusion from the analysis routine with respect to susceptibility to cancer for the subject and the medical protocol database, and generate a protocol report with respect to the probability that one or more medical protocols in the database will:

reduce susceptibility to ovarian cancer; or
delay onset of ovarian cancer; or
increase the likelihood of detecting ovarian cancer at an early stage to facilitate early treatment.

**15.** The system according to any one of claims 9-14, wherein the communication tool is operatively connected to the analysis routine and comprises a routine stored on a computer-readable medium of the system and adapted to be executed on a processor of the system, to:

generate a communication containing the conclusion; and
transmit the communication to the subject or the medical practitioner, or enable the subject or medical practitioner to access the communication.

## Patentansprüche

**1.** Verfahren zur Bestimmung, ob eine Person ein erhöhtes Risiko aufweist, an Eierstockkrebs zu erkranken, wobei das Verfahren die folgenden Schritte umfasst:

Bestimmen von Nukleinsäuresequenzinformationen über das *BRIP1*-Gen in einer von einer Person erhaltenen biologischen Probe und
Vergleichen der Sequenzinformationen mit der Wildtypsequenz von *BRIP1* (SEQ ID-Nr. 10);
wobei eine Ermittlung einer Nonsensmutation oder einer Rasterverschiebungsmutation in *BRIP1* bei der Person ein Indikator dafür ist, dass die Person ein erhöhtes Risiko aufweist, an Eierstockkrebs zu erkranken.

**2.** Verfahren nach Anspruch 1, wobei die Mutation zu einem *BRIP1*-Defekt führt, ausgewählt aus der Gruppe bestehend aus:

(a) vorzeitiger Verkürzung oder Rasterverschiebung eines kodierten BRIP1-Proteins im Verhältnis zur in SEQ ID-Nr. 13 dargestellten BRIP1-Aminosäuresequenz;
(b) Expression eines BRIP1-Proteins mit verminderter Aktivität im Vergleich zu einem Wildtyp-BRIP1-Protein (SEQ ID-Nr. 13), wobei es sich bei der Aktivität um wenigstens eine BRIP1-Aktivität handelt, ausgewählt aus:

(i) BRIP1-Bindung an C-terminale BRCT-Motive von menschlichem Wildtyp-BRCA1-Protein;
(ii) DNA-abhängiger ATPase-Aktivität und
(iii)DNA-Helikase-Aktivität;

(c) reduzierter Expression von BRIP1-Protein im Vergleich zu Wildtyp-BRIP1,

wobei auf den Defekt hinweisende mutierte Allele mit erhöhter Anfälligkeit für Eierstockkrebs in Verbindung gebracht werden.

**3.** Verfahren nach Anspruch 1 oder Anspruch 2, wobei die Mutation ausgewählt ist aus der Gruppe bestehend aus chr17:57208601 ins+AA in NCBI Build 36 und chr17: 57213073 delTT in NCBI Build 36.

**4.** Verfahren zur Bestimmung, ob eine Person ein erhöhtes Risiko aufweist, an Eierstockkrebs zu erkranken, wobei das Verfahren das Analysieren von Aminosäuresequenzdaten über ein BRIP1-Polypeptid von der Person umfasst, wobei eine Bestimmung des Vorliegens eines verkürzten BRIP1-Pölypeptids im Vergleich zu einem Wildtyp-BRIP1-Polypeptid mit einer wie in SEQ ID-Nr. 13 dargestellten Sequenz ein Indikator dafür ist, dass die Person ein erhöhtes Risiko aufweist, an Eierstockkrebs zu erkranken.

**5.** Verfahren nach Anspruch 4, wobei die Aminosäuresequenzdaten aus einer biologischen Probe von der Person erhalten werden, umfassend menschliches BRIP1-Polypeptid, unter Anwendung eines Verfahrens, das wenigstens eine Verfahrensweise umfasst, die ausgewählt ist aus:

(i) einem Antikörpertest und
(ii) Proteinsequenzierung.

**6.** Verfahren der Auswahl einer Person mit Eierstockkrebs für die Behandlung mit einem therapeutischen Mittel gegen Eierstockkrebs, wobei das Verfahren Folgendes umfasst:

Bestimmen des Vorhandenseins oder Fehlens wenigstens eines Allels wenigstens eines polymorphen Markers in einer von der Person erhaltenen Nukleinsäureprobe, wobei das wenigstens eine Allel einen Funktionsverlust oder einen Expressionsverlust eines *BRIP1* verursacht, und
Auswahl einer Person, die als das wenigstens eine Allel in der Nukleinsäureprobe aufweisend identifiziert wurde, für die Behandlung mit dem therapeutischen Mittel.

**7.** Verfahren nach Anspruch 6, wobei die Nukleinsäureprobe aus einer Eierstockkrebszelle stammt und wobei der Bestimmungsschritt Analysieren auf Verlust der Heterozygotie des *BRIP1*-Gens in der Nukleinsäureprobe umfasst und
wobei der Auswahlschritt das Auswählen einer Person, die als Verlust der Heterozygotie von *BRIP1* aufweisend identifiziert wurde, was auf einen Funktionsverlust von *BRIP1* in der Nukleinsäureprobe hinweist, für die Behandlung mit dem therapeutischen Mittel umfasst.

**8.** Verfahren nach Anspruch 6 oder Anspruch 7, wobei das therapeutische Mittel zur Behandlung von Eierstockkrebs ausgewählt ist aus der Gruppe bestehend aus PARP-Inhibitoren und DNA-Vernetzungsmitteln, wobei der PARP-Inhibitor vorzugsweise ausgewählt ist aus der Gruppe bestehend aus Iniparib, Olaparib, Veliparib, Rucaparib, CEP 9722, MK 4827 und KU-0059436, und wobei das DNA-Vernetzungsmittel vorzugsweise aus Alkylierungsmitteln, Cisplatin und Cisplatin-Derivaten ausgewählt ist.

**9.** System zur Identifizierung von Anfälligkeit für Eierstockkrebs bei einer Person, wobei das System Folgendes umfasst:

wenigstens einen Prozessor;
wenigstens ein computerlesbares Medium;
eine funktionsmäßig mit einem computerlesbaren Medium des Systems verbundene Datenbank zur Anfälligkeit, die Informationen über die Bevölkerung enthält, die das Vorhandensein oder Fehlen eines oder mehrerer Allele des menschlichen *BRIP1*-Gens und Anfälligkeit für Eierstockkrebs in einer menschlichen Population zueinander in Beziehung setzen;
ein Messinstrument, das eine Eingabe über die Person erhält und aus der Eingabe Informationen über das Vorhandensein oder Fehlen wenigstens eines mutierten *BRIP1*-Allels erzeugt, die ein Indikator für einen *BRIP1*-Defekt bei der Person sind;
ein funktionsmäßig mit dem Analyseinstrument verbundenes Kommunikationsinstrument, das auf einem computerlesbaren Medium des Systems gespeichert ist und auf einem Prozessor des Systems ausgeführt werden kann, um der Person oder deren Arzt/Ärztin das Ergebnis hinsichtlich der Anfälligkeit der Person für Krebs mitzuteilen, und
ein Analyseinstrument, das:

funktionsmäßig mit der Datenbank zur Anfälligkeit und dem Messinstrument verbunden ist,
auf einem computerlesbaren Medium des Systems gespeichert ist,
auf einem Prozessor des Systems ausgeführt werden kann, um die Informationen über die Person mit den Informationen über die Bevölkerung in der Datenbank zur Anfälligkeit abzugleichen und für die Person ein Ergebnis hinsichtlich der Anfälligkeit für Eierstockkrebs zu erzeugen,

und wobei es sich bei dem mutierten Allel um eine Rasterverschiebungsmutation oder eine Nonsensmutation in *BRIP1* handelt.

**10.** System nach Anspruch 9, wobei die Mutation ausgewählt ist aus der Gruppe bestehend aus chr17:57208601 ins+AA in NCBI Build 36 und chr17: 57213073 delTT in NCBI Build 36.

**11.** System nach Anspruch 9 oder Anspruch 10, wobei das wenigstens eine mutierte *BRIP1*-Allel ein Indikator für einen *BRIP1*-Defekt ist, ausgewählt aus der Gruppe bestehend aus:

(a) vorzeitiger Verkürzung eines kodierten BRIP1-Proteins im Verhältnis zur in SEQ ID-Nr. 13 dargestellten BRIP1-Aminosäuresequenz ;
(b) Expression eines BRIP1-Proteins mit verminderter Aktivität im Vergleich zu einem Wildtyp-BRIP1-Protein (SEQ ID-Nr. 13), wobei es sich bei der Aktivität um wenigstens eine BRIP1-Aktivität handelt, ausgewählt aus:

(i) BRIP1-Bindung an C-terminale BRCT-Motive von menschlichem Wildtyp-BRCA1-Protein;
(ii) DNA-abhängiger ATPase-Aktivität und
(iii)DNA-Helikase-Aktivität und

(c)reduzierter Expression von BRIP1-Protein im Vergleich zu Wildtyp-Expression,

wobei auf den Defekt hinweisende mutierte Allele mit erhöhter Anfälligkeit für Eierstockkrebs in Verbindung gebracht werden.

**12.** System nach einem der Ansprüche 9-11, wobei das Messinstrument ein auf einem computerlesbaren Medium des Systems gespeichertes Instrument umfasst, das durch einen Prozessor des Systems ausgeführt werden kann, um eine Dateneingabe über eine Person zu erhalten und Informationen über das Vorhandensein oder Fehlen des wenigstens einen mutierten *BRIP1*-Allels bei einer Person aus den Daten zu ermitteln,
wobei es sich bei den Daten um genomische Sequenzinformationen handelt, und das Messinstrument umfasst ein auf einem computerlesbaren Medium des Systems gespeichertes Sequenz-Analyseinstrument, das durch einen Prozessor des Systems ausgeführt werden kann, um das Vorhandensein oder Fehlen des wenigstens einen mutierten *BRIP1*-Allels aus den genomischen Sequenzinformationen zu ermitteln.

13. System nach einem der Ansprüche 9-12, wobei es sich bei der Eingabe über die Person um eine biologische Probe von der Person handelt und wobei das Messinstrument ein Instrument zum Ermitteln des Vorhandenseins oder Fehlens des wenigstens einen mutierten *BRIP1*-Allels in der biologischen Probe umfasst, wodurch Informationen über das Vorhandensein oder Fehlen des wenigstens einen mutierten *BRIP1*-Allels bei einer Person erzeugt werden, wobei das Messinstrument darüber hinaus optional Folgendes umfasst:

    a. ein Oligonukleotid-Mikroarray, das mehrere an einem festen Träger befestigte Oligonukleotid-Sonden enthält; einen Detektor zum Messen der Wechselwirkung zwischen aus der biologischen Probe erhaltener oder amplifizierter Nukleinsäure und einem oder mehreren Oligonukleotiden auf dem Oligonukleotid-Mikroarray, um Detektionsdaten zu erzeugen, und
    ein auf einem computerlesbaren Medium des Systems gespeichertes Analyseinstrument, das auf einem Prozessor des Systems ausgeführt werden kann, um das Vorhandensein oder Fehlen des wenigstens einen mutierten *BRIP1*-Allels aufgrund der Detektionsdaten zu ermitteln, oder

    wobei das Messinstrument optional Folgendes umfasst:

    b. einen Nukleotidsequenzer, mit dem Informationen über die Nukleotidsequenz von aus der biologischen Probe erhaltener oder amplifizierter Nukleinsäure ermittelt werden können, und
    ein auf einem computerlesbaren Medium des Systems gespeichertes Analyseinstrument, das auf einem Prozessor des Systems ausgeführt werden kann, um das Vorhandensein oder Fehlen des wenigstens einen mutierten *BRIP1*-Allels aufgrund der Informationen über die Nukleotidsequenz zu ermitteln.

14. System nach einem der Ansprüche 9-13, ferner umfassend:

    eine funktionsmäßig mit einem computerlesbaren Medium des Systems verbundene Datenbank für medizinische Protokolle, die Informationen enthält, die das Vorhandensein oder Fehlen des wenigstens einen mutierten BRIP1-Allels und medizinische Protokolle für Personen mit Risiko, an Eierstockkrebs zu erkranken, zueinander in Beziehung setzen, und
    eine Medizinprotokoll-Routine, die funktionsmäßig mit der Datenbank für medizinische Protokolle und der Analyseroutine verbunden ist, auf einem computerlesbaren Medium des Systems gespeichert ist und auf einem Prozessor des Systems ausgeführt werden kann, um das Ergebnis aus der Analyseroutine hinsichtlich der Anfälligkeit der Person für Krebs und der Datenbank für medizinische Protokolle zu vergleichen und einen Protokollbericht im Hinblick auf die Wahrscheinlichkeit zu erzeugen, dass durch ein oder mehrere medizinische Protokolle in der Datenbank:

        die Anfälligkeit für Eierstockkrebs reduziert wird oder
        das Auftreten von Eierstockkrebs verzögert wird oder
        die Wahrscheinlichkeit erhöht wird, den Eierstockkrebs in einem frühen Stadium nachzuweisen, um eine frühzeitige Behandlung zu ermöglichen.

15. System nach einem der Ansprüche 9-14, wobei das Kommunikationsinstrument funktionsmäßig mit der Analyseroutine verbunden ist und eine auf einem computerlesbaren Medium des Systems gespeicherte Routine umfasst und auf einem Prozessor des Systems ausgeführt werden kann, um:

    eine Mitteilung zu erstellen, welche das Ergebnis enthält, und
    die Mitteilung an die Person oder deren Arzt/Ärztin zu übermitteln bzw. es der Person oder deren Arzt/Ärztin zu ermöglichen, auf die Mitteilung zuzugreifen.

## Revendications

1. Procédé pour déterminer si un individu présente un risque accru de développer un cancer de l'ovaire, le procédé comprenant les étapes de
détermination, dans un échantillon biologique obtenu à partir d'un individu, d'informations de séquence d'acide nucléique concernant le gène *BRIP1* ; et
comparaison des informations de séquence à la séquence de type sauvage de *BRIP1* (SEQ ID NO: 10) ;

dans lequel une identification d'un mutation non-sens ou une mutation de décalage de cadre de lecture dans *BRIP1* chez l'individu est indicative du fait que l'individu présente un risque accru de développer un cancer de l'ovaire.

**2.** Procédé de la revendication 1, dans lequel la mutation conduit à un défaut de *BRIP1* choisi dans le groupe constitué de :

(a) une troncature prématurée ou un décalage de cadre de lecture d'une protéine BRIP1 codée, par rapport à la séquence d'acides aminés de BRIP1 décrite dans SEQ ID NO: 13 ;
(b) l'expression d'une protéine BRIP1 ayant une activité réduite par rapport à une protéine BRIP1 de type sauvage (SEQ ID NO: 13), où l'activité est au moins une activité de BRIP1 choisie parmi :

(i) la liaison de BRIP1 à des motifs BRCT C-terminaux de protéine BRCA1 humaine de type sauvage ;
(ii) une activité ATPase ADN-dépendante ; et
(iii) une activité ADN hélicase ;

(c) l'expression réduite de protéine BRIP1, par rapport à BRIP1 de type sauvage,

dans lequel des allèles mutants indicatifs du défaut sont associés à une susceptibilité accrue de cancer de l'ovaire.

**3.** Procédé de la revendication 1 ou la revendication 2, dans lequel la mutation est choisie dans le groupe constitué de chr17:57208601 ins+AA dans NCBI Build 36, et chr17:57213073 delTT dans NCBI Build 36.

**4.** Procédé pour déterminer si un sujet humain présente un risque accru de développer un cancer de l'ovaire, le procédé comprenant l'analyse de données de séquence d'acides aminés concernant un polypeptide BRIP1 provenant du sujet, dans lequel une détermination de la présence d'un polypeptide BRIP1 tronqué par rapport à un polypeptide BRIP1 de type sauvage ayant la séquence décrite dans SEQ ID NO: 13 est indicative du fait que le sujet présente un risque accru de développer un cancer de l'ovaire.

**5.** Procédé de la revendication 4, dans lequel les données de séquence d'acides aminés sont obtenues à partir d'un échantillon biologique du sujet humain comprenant un polypeptide BRIP1 humain, en utilisant un procédé qui comprend au moins une procédure choisie parmi :

(i) un dosage d'anticorps ; et
(ii) le séquençage de protéine.

**6.** Procédé de sélection d'un sujet humain ayant un cancer de l'ovaire pour traitement avec un agent thérapeutique contre le cancer de l'ovaire, le procédé comprenant :

la détermination de la présence ou l'absence d'au moins un allèle d'au moins un marqueur polymorphe dans un échantillon d'acide nucléique obtenu à partir de l'individu, où l'au moins un allèle cause une perte de fonction ou une perte d'expression d'un BRIP1, et
la sélection pour traitement avec l'agent thérapeutique d'un sujet identifié comme ayant l'au moins un allèle dans l'échantillon d'acide nucléique.

**7.** Procédé de la revendication 6, dans lequel l'échantillon d'acide nucléique provient d'une cellule du cancer de l'ovaire, et dans lequel l'étape de détermination comprend l'analyse pour la perte d'hétérozygotie du gène *BRIP1* dans l'échantillon d'acide nucléique, et
dans lequel l'étape de sélection comprend la sélection pour traitement avec l'agent thérapeutique d'un sujet identifié comme présentant une perte d'hétérozygotie de *BRIP1,* indicative d'une perte de fonction de *BRIP1* dans l'échantillon d'acide nucléique.

**8.** Procédé de l'une quelconque de la revendication 6 ou la revendication 7, dans lequel l'agent thérapeutique pour traiter le cancer de l'ovaire est choisi dans le groupe constitué d'inhibiteurs de PARP et d'agents de réticulation d'ADN, dans lequel l'inhibiteur de PARP est, de préférence, choisi dans le groupe constitué de l'iniparib, l'olaparib, le véliparib, le rucaparib, CEP 9722, MK 4827 et KU-0059436, et dans lequel l'agent de réticulation d'ADN est, de préférence, choisi parmi des agents d'alkylation, le cisplatine et des dérivés de cisplatine.

**9.** Système d'identification de la susceptibilité de cancer de l'ovaire chez un sujet humain, le système comprenant :

au moins un processeur ;
au moins un support lisible par ordinateur ;
une base de données de susceptibilités fonctionnellement couplée à un support lisible par ordinateur du système et contenant des informations de population corrélant la présence ou l'absence d'un ou plusieurs allèles du gène BRIP1 humain et la susceptibilité de cancer de l'ovaire dans une population d'humains ;
un outil de mesure qui reçoit une entrée concernant le sujet humain et génère des informations à partir de l'entrée concernant la présence ou l'absence d'au moins un allèle de *BRIP1* mutant indicative d'un défaut de *BRIP1* chez le sujet humain ;
un outil de communication fonctionnellement relié à l'outil d'analyse, stocké sur un support lisible par ordinateur du système et adapté pour être exécuté sur un processeur du système pour communiquer au sujet, ou à un praticien médical pour le sujet, la conclusion en ce qui concerne la susceptibilité de cancer pour le sujet ; et
un outil d'analyse qui :

est fonctionnellement relié à la base de données de susceptibilités et à l'outil de mesure,
est stocké sur un support lisible par ordinateur du système,
est adapté pour être exécuté sur un processeur du système, pour comparer les informations concernant le sujet humain aux informations de population dans la base de données de susceptibilités et générer une conclusion en ce qui concerne la susceptibilité de cancer de l'ovaire pour le sujet humain,
et dans lequel l'allèle mutant est une mutation de décalage de cadre de lecture ou une mutation non-sens dans *BRIP1.*

**10.** Système de la revendication 9, dans lequel la mutation est choisie dans le groupe constitué de chr17:57208601 ins+AA dans NCBI Build 36 et chr17:57213073 delTT dans NCBI Build 36.

**11.** Système selon la revendication 9 ou la revendication 10, dans lequel l'au moins un allèle de *BRIP1* mutant est indicatif d'un défaut de *BRIP1* choisi dans le groupe constitué de :

(a) une troncature prématurée ou un déphasage d'une protéine BRIP1 codée, par rapport à la séquence d'acides aminés de BRIP1 décrite dans SEQ ID NO: 13 ;
(b) l'expression d'une protéine BRIP1 ayant une activité réduite par rapport à une protéine BRIP1 de type sauvage (SEQ ID NO: 13), où l'activité est au moins une activité de BRIP1 choisie parmi :

(i) la liaison de BRIP1 à des motifs BRCT C-terminaux de protéine BRCA1 humaine de type sauvage ;
(ii) une activité ATPase ADN-dépendante ; et
(iii) une activité ADN hélicase ;

(c) l'expression réduite de protéine BRIP1, par rapport à BRIP1 de type sauvage,

dans lequel des allèles mutants indicatifs du défaut sont associés à une susceptibilité accrue de cancer de l'ovaire.

**12.** Système selon l'une quelconque des revendications 9 à 11, dans lequel l'outil de mesure comprend un outil stocké sur un support lisible par ordinateur du système et adapté pour être exécuté par un processeur du système pour recevoir une entrée de données concernant un sujet et déterminer des informations concernant la présence ou l'absence de l'au moins un allèle de *BRIP1* mutant chez un sujet humain à partir des données,
dans lequel les données sont des informations de séquence génomique, et l'outil de mesure comprend un outil d'analyse de séquence stocké sur un support lisible par ordinateur du système et adapté pour être exécuté par un processeur du système pour déterminer la présence ou l'absence de l'au moins un allèle de *BRIP1* mutant à partir des informations de séquence génomique.

**13.** Système selon l'une quelconque des revendications 9 à 12, dans lequel l'entrée concernant le sujet humain est un échantillon biologique provenant du sujet humain, et dans lequel l'outil de mesure comprend un outil pour identifier la présence ou l'absence de l'au moins un allèle de *BRIP1* mutant dans l'échantillon biologique, de façon à générer des informations concernant la présence ou l'absence de l'au moins un allèle de *BRIP1* mutant chez un sujet humain, dans lequel l'outil de mesure comprend facultativement en outre :

a. un microréseau d'oligonucléotides contenant une pluralité de sondes oligonucléotidiques liées à un support solide ;
un détecteur pour mesurer l'interaction entre un acide nucléique obtenu à partir de, ou amplifié à partir de l'échantillon biologique et un ou plusieurs oligonucléotides sur le microréseau d'oligonucléotides pour générer des données de détection ; et
un outil d'analyse stocké sur un support lisible par ordinateur du système et adapté pour être exécuté sur un processeur du système, pour déterminer la présence ou l'absence de l'au moins un allèle de *BRIP1* mutant sur la base des données de détection, ou

dans lequel l'outil de mesure comprend facultativement :

b. un séquenceur de nucléotides capable de déterminer des informations de séquence nucléotidique à partir d'un acide nucléique obtenu à partir de, ou amplifié à partir de l'échantillon biologique ; et
un outil d'analyse stocké sur un support lisible par ordinateur du système et adapté pour être exécuté sur un processeur du système, pour déterminer la présence ou l'absence de l'au moins un allèle de BRIP1 mutant sur la base des informations de séquence nucléotidique.

**14.** Système selon l'une quelconque des revendications 9 à 13, comprenant en outre :

une base de données de protocoles médicaux fonctionnellement connectée à un support lisible par ordinateur du système et contenant des informations corrélant la présence ou l'absence de l'au moins un allèle de *BRIP1* mutant et des protocoles médicaux pour des sujets humains à risque de cancer de l'ovaire ; et un sous-programme de protocole médical, fonctionnellement connecté à la base de données de protocoles médicaux et au sous-programme d'analyse, stocké sur un support lisible par ordinateur du système, et adapté pour être exécuté sur un processeur du système, pour comparer la conclusion du sous-programme d'analyse en ce qui concerne la susceptibilité de cancer pour le sujet et la base de données de protocoles médicaux, et générer un rapport de protocole en ce qui concerne la probabilité qu'un ou plusieurs protocoles médicaux dans la base de données :

réduiront la susceptibilité de cancer de l'ovaire ; ou
retarderont l'apparition de cancer de l'ovaire ; ou
augmenteront la probabilité de détection d'un cancer de l'ovaire à un stade précoce afin de permettre un traitement précoce.

**15.** Système selon l'une quelconque des revendications 9 à 14, dans lequel l'outil de communication est fonctionnellement connecté au sous-programme d'analyse et comprend un sous-programme stocké sur un support lisible par ordinateur du système et adapté pour être exécuté sur un processeur du système, pour :

générer une communication contenant la conclusion ; et
transmettre la communication au sujet ou au praticien médical, ou permettre au sujet ou praticien médical d'accéder à la communication.

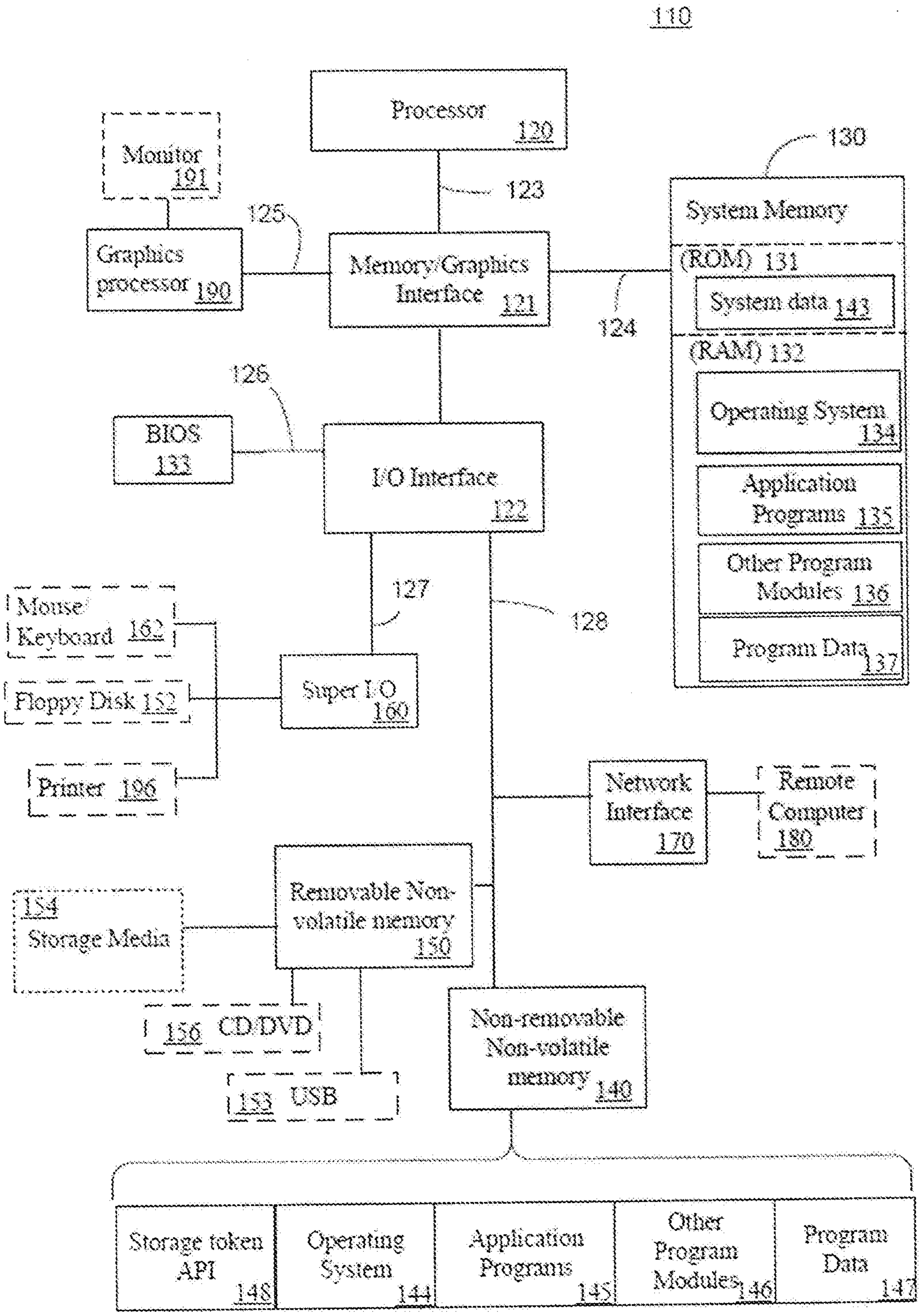
110
Processor 120
123
Monitor 191
Graphics processor 190
125
Memory/Graphics Interface 121
124
130
System Memory
(ROM) 131
System data 143
(RAM) 132
Operating System 134
Application Programs 135
Other Program Modules 136
Program Data 137
126
BIOS 133
I/O Interface 122
127
128
Mouse/ Keyboard 162
Floppy Disk 152
Printer 196
Super I/O 160
Network Interface 170
Remote Computer 180
154 Storage Media
Removable Non-volatile memory 150
156 CD/DVD
153 USB
Non-removable Non-volatile memory 140
Storage token API 148
Operating System 144
Application Programs 145
Other Program Modules 146
Program Data 147

FIG. 1

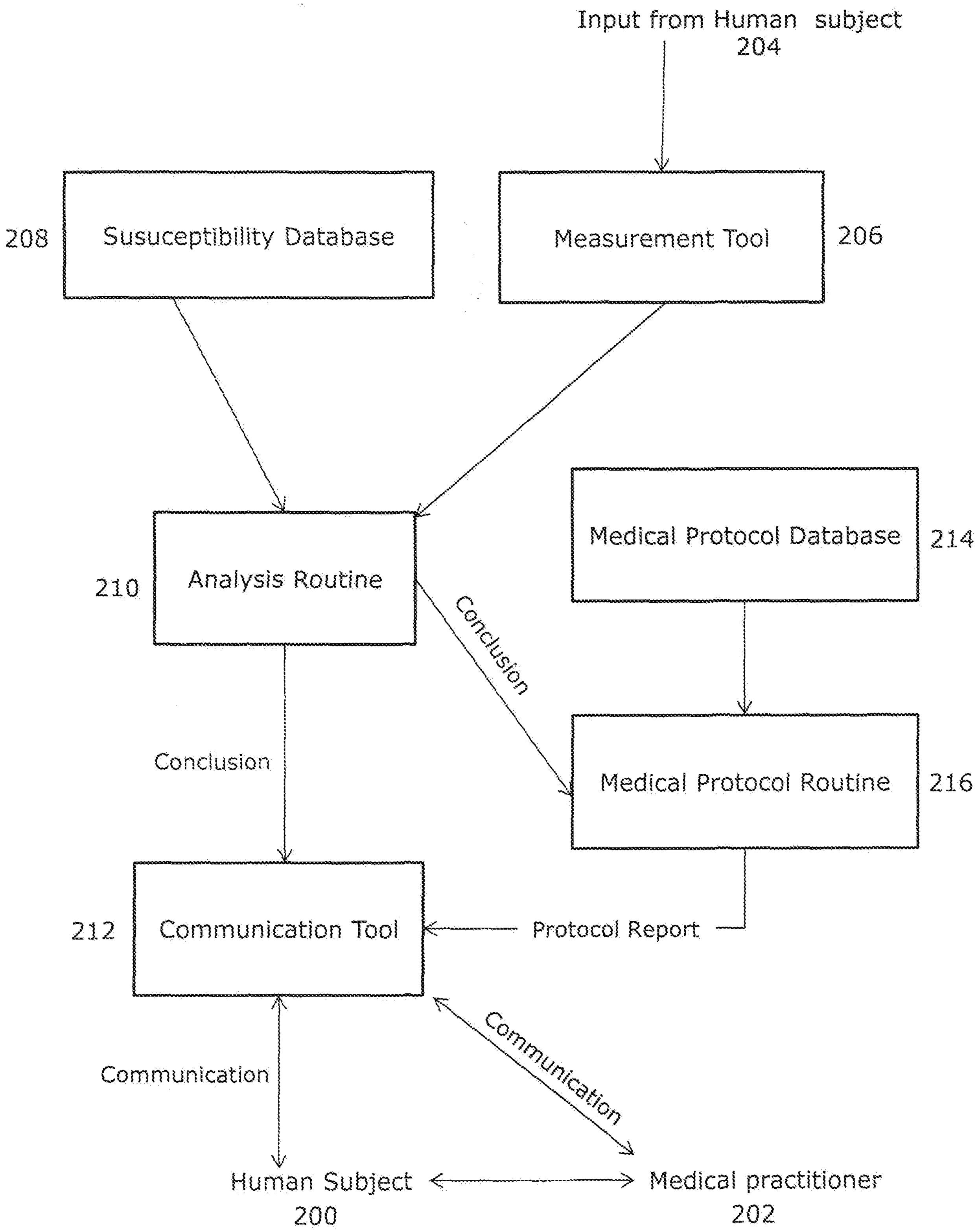
Input from Human subject
204
Susuceptibility Database
208
Measurement Tool
206
Analysis Routine
210
Conclusion
Medical Protocol Database
214
Conclusion
Medical Protocol Routine
216
Communication Tool
212
Protocol Report
Communication
Communication
Human Subject
200
Medical practitioner
202

FIG.2

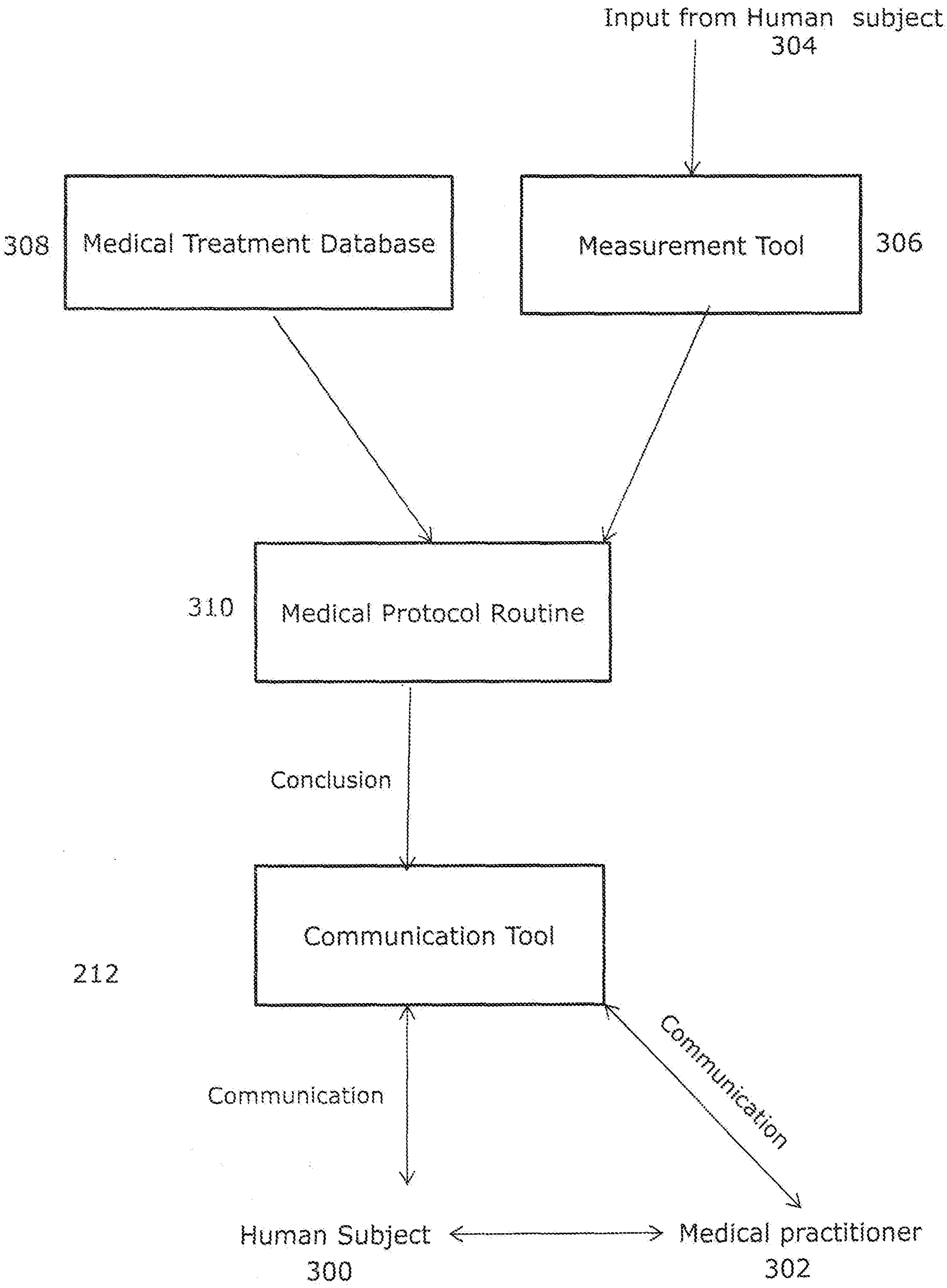
Input from Human subject
304
308
Medical Treatment Database
Measurement Tool
306
310
Medical Protocol Routine
Conclusion
Communication Tool
212
Communication
Communication
Human Subject
300
Medical practitioner
302

FIG. 3

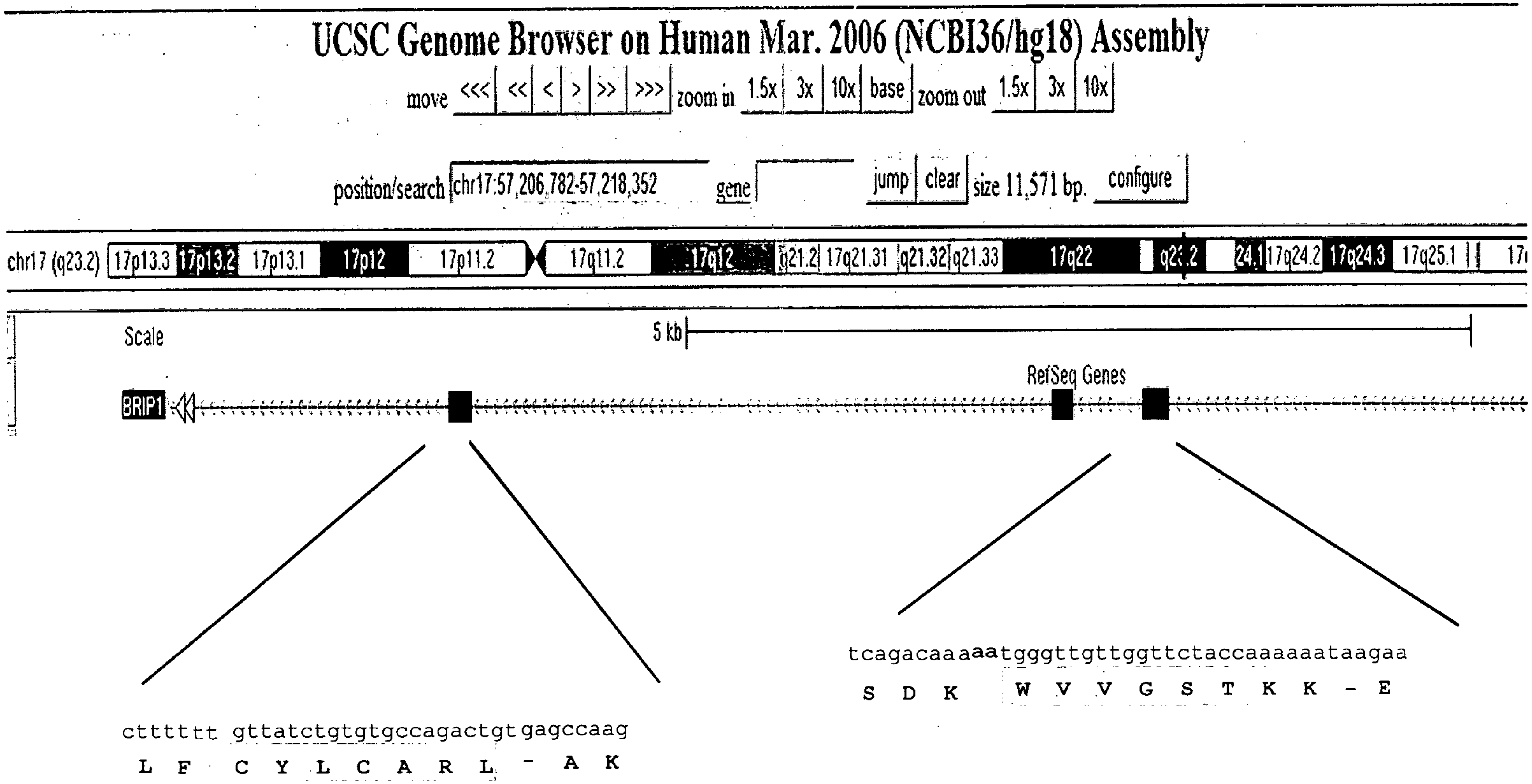


Chr17:57208601ins+AA

Termination of protein translation

at codon 688

Chr17: 57213073 del TT

Termination of protein translation

at codon 576

FIG. 4

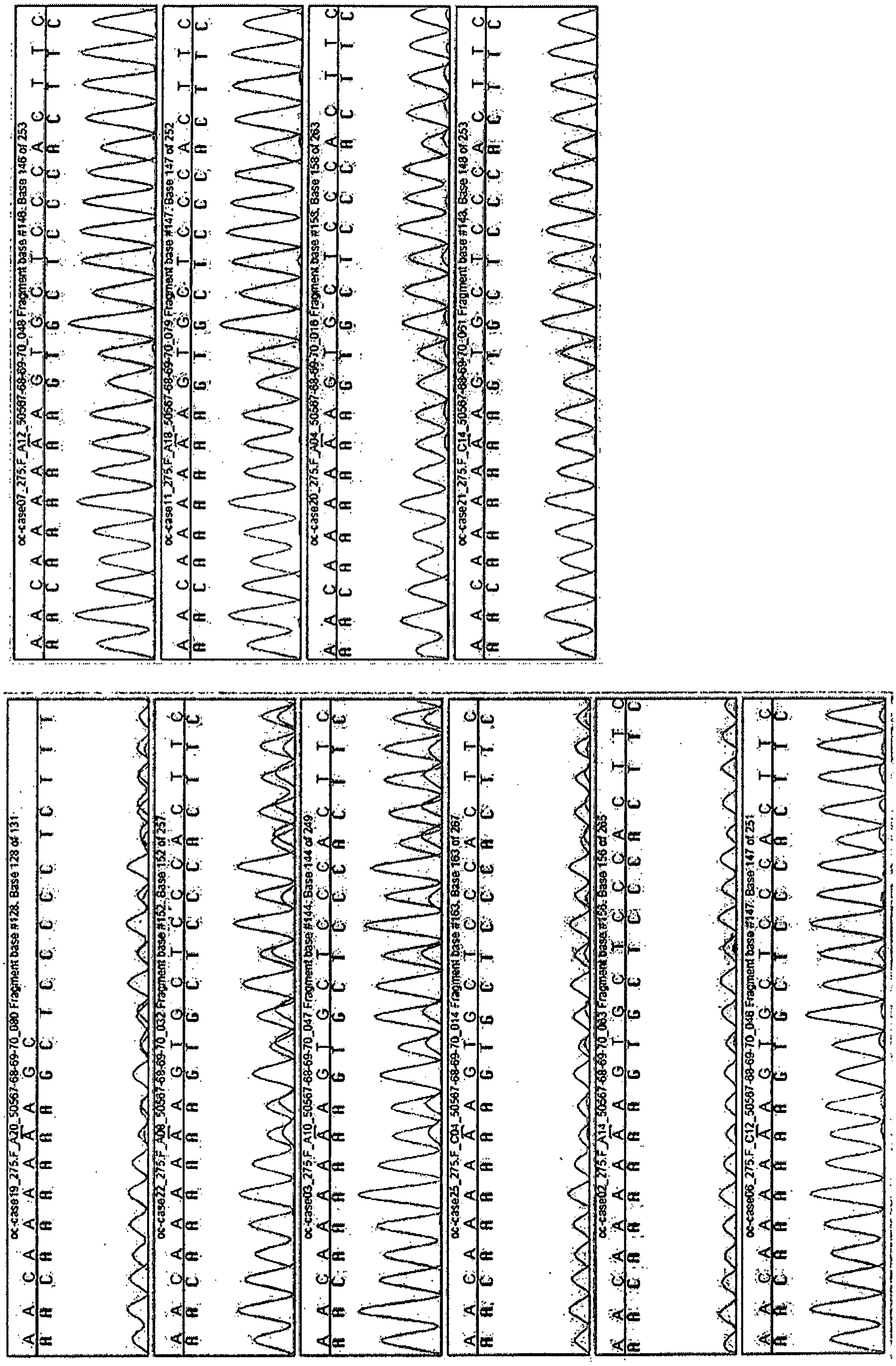

FIG. 5

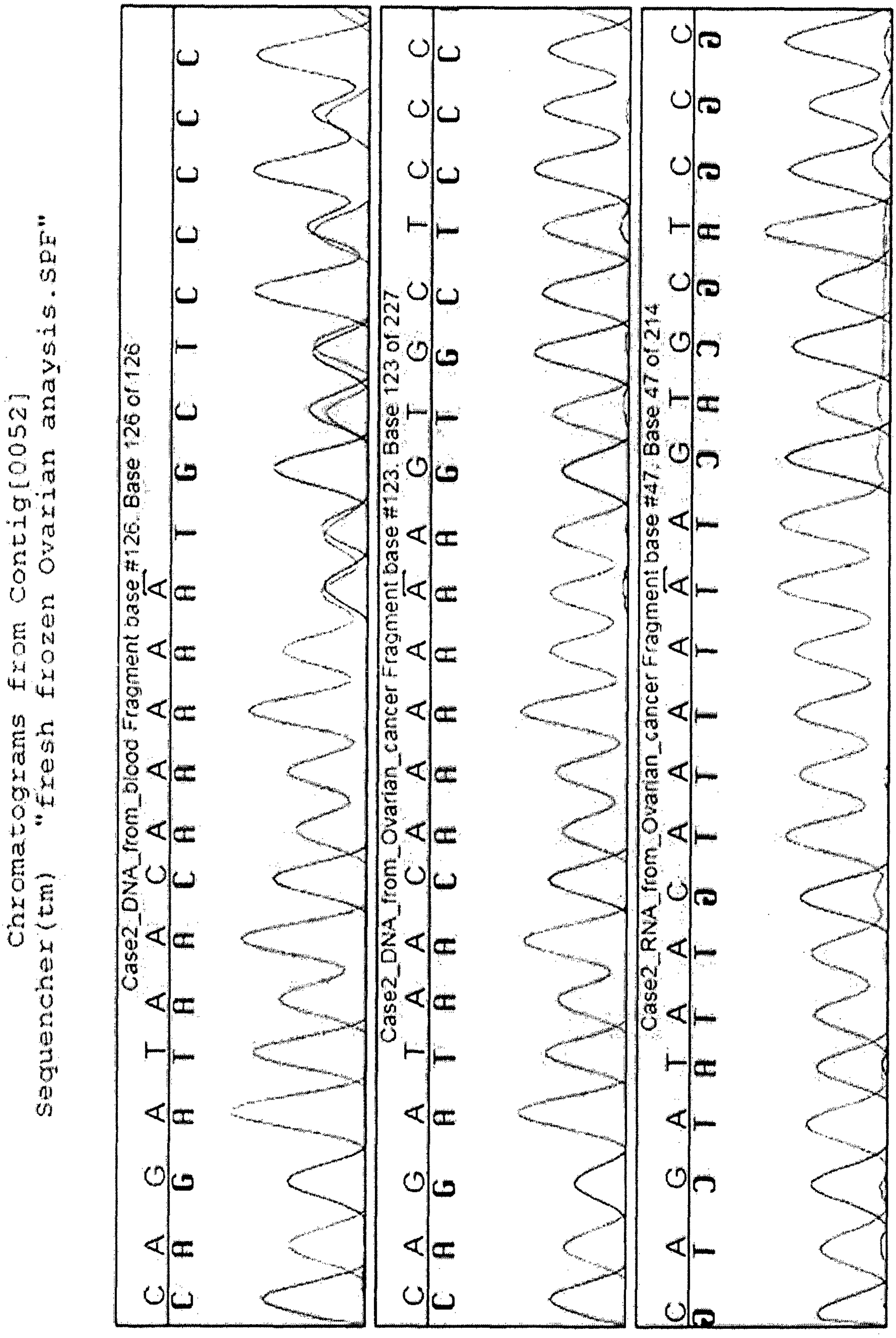
Chromatograms from Contig[0052]
Sequencher(tm) "fresh frozen Ovarian anaysis.SPF"
Case2_DNA_from_blood Fragment base #126. Base 126 of 126
C A G A T A A C A A A A A T G C T C C C C C
Case2_DNA_from_Ovarian_cancer Fragment base #123. Base 123 of 227
C A G A T A A C A A A A A A G T G C T C C C
Case2_RNA_from_Ovarian_cancer Fragment base #47. Base 47 of 214
C A G A T A A C A A A A A A G T G C T C C C

FIG. 6

```
H.sapiens          MSSMWSEYTIGGVKIYFPYKAYPSQLAMMNSILRGLNSKQHCLLESPTGSGKSLALLCSA 60
P.troglodytes      MSSMWSEYTIGGVKIYFPYKAYPSQLAMMNSILRGLNSKQHCLLESPTGSGKSLALLCSA 60
C.familiaris       MSSLWSEYTIGGVKITFPYKAYPSQLAMMNSIVRGLNSKQHCLLESPTGSGKSLALLCST 60
M.musculus         MSSVLSDYTIGGVKIHFPCRAYPAQLAMMNSIVRGLNSSQHCLLESPTGSGKSLALLCSA 60
R.norvegicus       MSSVLSEYTIGGVKIHFPCRAYPAQLAMMNSIVRGLNSSQHCLLESPTGSGKSLALLCSA 60
G.gallus           MSSDVSQYTIGGVKIMFPCKAYPSQLAMMNAIVKGLNNRQHCLLESPTGSGKSLALLCSA 60
                   ***  *:******** ** :***:******:*::***. *******************:

H.sapiens          LAWQQSLSGK----PADEGVSEKAEVQLSCCCACHSKDFTNNDMN--QGTSRHFNYPSTP 114
P.troglodytes      LAWQQSLSGK----PADEGVSEKAEVQLSCCCACHSKDFTNNDMN--QGTSRHFNYPSTP 114
C.familiaris       LAWQQSVSGK----LVDESLS-KTEVSSSCCCACHSKNFTNIDLN--QGTSHHFNSPSTP 113
M.musculus         LAWQQSLSEK----PVDEGLNKKPEAPPSCSCACHSKNFTYSDTN--LDTSPHFNSPSKP 114
R.norvegicus       LAWQQSLTGK----PVDEGLNKKPEAPSSCSCSCHSKNFTYSDTN--LDTSPHFSSPSKP 114
G.gallus           LSWQQSLYEKSLLKSSCEKEDREPAASLPCRCVCHSRSESSEATAGASHGAACSNNYETG 120
                   *:****:  *          *  . :. .  .* * ***:. :         :   .  ..

H.sapiens          PSERNGTSSTCQDSPEKTTLAAKLSAKKQASIYRDENDDFQVEKKRIRPLETTQQIRKRH 174
P.troglodytes      PSERNGTSSTCQDSPEKTTLAAKLSAKKQASIYRDENDDFQVEKKRIRPLETTQQIRKRH 174
C.familiaris       PSERYDTSSTCQDSPEKTTLAAKLSAKKQASIKRDENDDFQVEKKRIRPLETTQQIRKRH 173
M.musculus         SSGRNGVSTPCQDSPEKNTLAAKLSAKKQASIHRDEDDDFQVEKKRIRPLETTQQIRKRH 174
R.norvegicus       SSERNAVSSPCRDSPERNSLAAKLSAKKHASIH--EDDDFQVEKKRIRPLETTQQIRKRH 172
G.gallus           GSVKHGDQLSDTECKENNTLASKLSAKKRASACGNECDDFQVERKRIRPLETEQQVRKRH 180
                    * :    . .  :. *..:**:******:**       * ******:******** **:****

H.sapiens          CFGTEVHNLDAKVDSGKTVKLNSPLEKINS---FSPQKPPGHCSRCCCSTKQGNSQESSN 231
P.troglodytes      CFGTEVHNLDAKVDSGKTVKLNSPLEKINS---FSPQKPPGHCSRCCCSTKQGNSQESSN 231
C.familiaris       CFEKKVHHVDAKVASGKTTKLNSPLEKINS---FSPQNPPGHCSRCCCSTKQGSNQDSSN 230
M.musculus         CLEKDVHHVDARLASEKRVKPESPIGKSFSDRKDSFQNVDGLCSRCCCSAKQGNNQEPAN 234
R.norvegicus       CLEKDVHHLDARLASEKRVKPESPIRKTSS----SFQNPDGLCSRCCCSANQGINKESAN 228
G.gallus           CFSKEVQLVDALEVYNQRKNGELIVHSEKSVKNTSPQTLFSSCTECSCSSGKETRKDSGN 240
                   *: ..*: :**       :  :  :  : .  *      * *.  . *:.*.**: :    ::..*

H.sapiens          TIKKDHTGKSKIPKIYFGTRTHKQIAQITRELRRTAYSGVPMTILSSRDHTCVHPEVVG- 290
P.troglodytes      TIKKDHTGKSKIPKIYFGTRTHKQIAQITRELRRTAYSGVPMTILSSRDHTCVHPEVVG- 290
C.familiaris       TTKKDHGGKSKIPKIYFGTRTHKQIAQITRELRRTAYSGVPMTILSSRDHTCVHPEVVG- 289
M.musculus         TVKKDHGGQCKRPKIYFGTRTHKQIAQITRELRKTAYSGVPMTILSSRDHSCVHPEVVG- 293
R.norvegicus       TVKKDNGDQSKRPKIYFGTRTHKQIAQITRELRKTAYSGVPMTILSSRDHTCVHPEVVG- 287
G.gallus           TKKKANGDQTFIPKIFFGTRTHKQIAQITRELKRTAYSGVPMTILSSRDYTCIHPVVSSS 300
                   * ** : .:     ***:*****************::***************::*:** *  .

H.sapiens          NFNRNEKCMELLDGKNGKSCYFYHGVHKISDQHTLQTFQGMCKAWDIEELVSLGKKLKAC 350
P.troglodytes      NFNRNEKCMELLDGKNGKSCYFYHGVHKISDQHTLQTFQGMCKAWDIEELVSLGKKLKSC 350
C.familiaris       NFNRNEKCMELLDGKNGKSCYFYHGVHKISNQHTLQTLQGMCKAWDIEELVSLGKKLKAC 349
M.musculus         NFNRKEKCMELLDGKHGKSCYFYHGVHKISNQQTLQHLQGMSRAWDIEELVSLGRKLKAC 353
R.norvegicus       NFNRNEKCMELLDGKHGKSCYFYHGVHRISNQQTLQFLHGISKAWDIEELVSLGRKLKAC 347
G.gallus           NSNRNELCVELLEGKHGKSCLYYHGVHKLSEHYALQSAHNTYQAWDIEDLVSLGKKLRAC 360
                   * **:* *:***:**:**** :*****::*::  :**  :.   :*****:*****:**::*

H.sapiens          PYYTARELIQDADIIFCPYNYLLDAQIRESMDLNLKEQVVILDEAHNIEDCARESASYSV 410
P.troglodytes      PYYTARELIQDADIIFCPYNYLLDAQIRESMDLNLKEQVVILDEAHNIEDCARESASYSV 410
C.familiaris       PYYTARELIEDAHIIFCPYNYLLDAQIRESMDINLKEQVVILDEAHNIEDCARESASYSV 409
M.musculus         PYYTARELIEDADIVFCPYNYLLDSQIRETMDIKLKGQVVILDEAHNIEDCARESASYSV 413
R.norvegicus       PYYTARELIDSADIIFCPYNYLLDSQIRESMDIKLKEQVVILDEAHNIEECARETASYSV 407
G.gallus           PYFAARELMVGADIVFCPYNYLLDPQIRESMEINLKGQVVILDEAHNIEDSAREAVSYSV 420
                   **::****:  .*.*:*********.****:*:::** ***********:.***:.****

H.sapiens          TEVQLRFARDELDSMVNNNIRKKDHEPLRAVCCSLINWLEANAEYLVERDYESACKIWSG 470
P.troglodytes      TEVQLRFARDELDSMVNNNIRKKDHEPLRAVCCSLINWLEANAEYLVERDYESACKIWSG 470
C.familiaris       TEVQLRFARDELDSMVNNNIRKKNHEPLRAVCYSLINWLEANSEHLVERDYESSCKIWSG 469
M.musculus         TEVQLRFARDELDSLINGNIRKKSHEPLRDVCYNLINWLETNSKHLVERGYESSCKIWSG 473
R.norvegicus       TEVQLRFARDELDSLINSNIRKKSHEPLRDVCYNLINWLETNSKNLVERDYESSCKIWSG 467
G.gallus           TESQLNAAREELDFMVNNNIRQKDHEQLRAMCCSLTNWLRESSSQLVETGYETSCKVWSG 480
                   ** **. **:*** ::*.***:*.** ** :* .* ***. .:. *** .**::**:***
```

FIG. 7 (A)

```
H.sapiens        NEMLLTLHKMGITTATFPILQGHFSAVLQKEEKISPIYGKEEAREVPVISASTQIMLKGL 530
P.troglodytes    NEMLLTLHKMGITTATFPILQGHFSAVLQKEEKISPIYGKEEAREVPVISASTQIMLKGL 530
C.familiaris     SEMVLNLHKMGITTATFPILQGHFSAVLQKEEKVLPIHGKEETREVPIISASTQIMLKGL 529
M.musculus       NEMLLNLYRMGITTATFPVLQRHLSAVLQKEEKVTPIHGKEEAIQIPIISASTQVVLKGL 533
R.norvegicus     NEMLLNLHRMGITAASFPVLQKHLSAVLQKEEKVTSTHGKEEAIQIPIISASTQIMLKGL 527
G.gallus         KEMLNHFHDMGITNISFPILQKHLSAVLEKEEKIS-MFGKEELVEIPIVSSATQIVLKGL 539
                 .**:   :: ****  :**:** *:****:****:    .****  ::*::*::**::****

H.sapiens        FMVLDYLFRQNSRFADDYKIAIQQTYSWTN--QIDISDKNGLLVLPKNKKRSRQKTAVHV 588
P.troglodytes    FMVLDYLFRQNSRFADDYKIAIQQTYSWTN--QIDISDKNGLLVLPKNKKRSRQKTAVHV 588
C.familiaris     FMVFDYLFRQNSRFADDYKVAIQQTYSWIN--QTDTSDKNGFFAPAKNKKHLRQKTAVHV 587
M.musculus       FMVLDYLFRENSRFADDYKVAIQQTYSWTN--QIAIFDKTGVLAVPKNKKHSRQKIGVNA 591
R.norvegicus     FMVLDYLFRKNSRFADDYKVAIQQTYSWTN--QIAIFDKNALLPVPKNKKHSRQKIGVNV 585
G.gallus         FMVLLYLFKDNSRFADDYRVALQQTYAWTNDNQPDVSDTSAFFTKTKHKRNLRHKTVVHM 599
                 ***: ***:.********::*:****:* *    *....:  .*:*:. *:*  *:

H.sapiens        LNFWCLNPAVAFSDINGKVQTIVLTSGTLSPMKSFSSELGVTFTIQLEANHIIKNSQVWV 648
P.troglodytes    LNFWCLNPAVAFSDINGKVQTIVLTSGTLSPMKSFSSELGVTFTIQLEANHIIKNSQVWV 648
C.familiaris     LNFWCLNPAVAFSDINGKVLTIVLTSGTLSPMKSFSSELGVTFTIQLEANHVINNSQVWV 647
M.musculus       LNFWCLNPAVAFSDINDKVRTIVLTSGTLSPLKSFSSELGVTFSIQLEANHVISNSQVWV 651
R.norvegicus     LNFWCLNPAVAFSDINDKVRTIVLTSGTLSPLKSFSSELGVTFNIQLEANHVISNSQVWV 645
G.gallus         LNFWCLNPAVAFSDLN-DVRTVVLTSGTLSPMDSFSSELGVKFSIQLEANHVIRNSQVWV 658
                 **************:*  .* *:*********:.********.*.*******:* ******

H.sapiens        GTIGSGPKGRNLCATFQNTETFEFQDEVGALLLSVCQTVSQGILCFLPSYKLLEKLKERW 708
P.troglodytes    GTIGSGPKGRNLCATFQNTETFEFQDEVGALLLSVCQTVSQGILCFLPSYKLLEKLKERW 708
C.familiaris     GTIGSGPKGRNLCATFQHTETFEFQDEVGALLLSVCQTVNQGILCFLPSYKLLEKLKERW 707
M.musculus       GTVGSGPKGRNLCATFQHTETFEFQDEVGMLLLSVCQTVSQGILCFLPSYKLLEKLRERW 711
R.norvegicus     GTVGSGPKGRNLCATFQHTETFEFQDEVGMLLLSVCQTVSQGILCFLPSYKLLEKLRERW 705
G.gallus         GTIGTGPNGRKLCATFQHTETFEFQDEVGALLLSVCQKVGQGILCFLPSYKLLDKLKDRW 718
                 **:*:**:**:******:*********** *******.*.*************:**:.**

H.sapiens        LSTGLWHNLELVKTVIVEPQGGEKTNFDELLQVYYDAIKYKGEKDGALLVAVCRGKVSEG 768
P.troglodytes    LSTGLWHNLELVKTVIVEPQGGEKTNFDELLQVYYDAIKYKGEKDGALLVAVCRGKVSEG 768
C.familiaris     LYTGLWHNLELVKTVIVEPQGGEKTDFNELLQVYYDAIKYKGEKDGALLVAVCRGKVSEG 767
M.musculus       IFTGLWHSLESVKTVIAEPQGGEKTDFDELLQVYYDAIKFKGEKDGALLIAVCRGKVSEG 771
R.norvegicus     IFTGLWHSLESVKTVIAEPQGGEKTDFDELLQVYYDAIKFKGEKDGALLIAVCRGKVSEG 765
G.gallus         IHTGLWRNLELVKTVIAEPQGGAKSDFDELLKIYYDAIKFKGEKDGALLIAVCRGKVSEG 778
                 : ****:.** *****.***** *::*:***::******:*********:**********

H.sapiens        LDFSDDNARAVITIGIPFPNVKDLQVELKRQYNDHHSKLRGLLPGRQWYEIQAYRALNQA 828
P.troglodytes    LDFSDDNARAVITIGIPFPNVKDLQVELKRQYNDHHSKLRGLLPGRQWYEIQAYRALNQA 828
C.familiaris     LDFSDDNARAVITIGIPFPNVKDLQVELKRQYNDQHSKLRGLLPGRQWYEIQAYRALNQA 827
M.musculus       LDFSDDNARAVITVGIPFPNVKDLQVELKRQYNDHHSKSRGLLPGRQWYEIQAYRALNQA 831
R.norvegicus     LDFSDDNARAVITIGIPFPNVKDLQVELKRQYNDHHSKLRGLLPGRQWYEIQAYRALNQA 825
G.gallus         LDFCDENARAVITIGIPFPNVKDLQVELKRKYNDQHKTTRGLLPGSQWYEIQAYRALNQA 838
                 ***.*:*******:****************:***:*:.. ****** *************

H.sapiens        LGRCIRHRNDWGALILVDDRFRNNPSRYISGLSKWVRQQIQHHSTFESALESLAEFSKKH 888
P.troglodytes    LGRCIRHRNDWGALILVDDRFRNNPSRYISGLSKWVRQQIQHHSTFESALESLAEFSKKH 888
C.familiaris     LGRCIRHKNDWGALILVDDRFRSNPSRYISGLSKWIRQQIQHHSTFESALESLSDFSRKH 887
M.musculus       LGRCIRHKNDWGALILVDDRFNNNPNRYISGLSKWVRQQIQHHSSFASALESLTEFSRRH 891
R.norvegicus     LGRCIRHKNDWGALILVDDRFNNNPDRYISGLSKWVRQQIQHHSTFASALESLTEFSKRH 885
G.gallus         LGRCIRHRSDWGALILVDDRFRNNPNKYITGLSKWIRQQVQHHENFGSALESLHAFAERN 898
                 *******:.************..**.:**:*****:***:***..* ******  *:.::

H.sapiens        QKVLNVSIKDRTNIQDNESTLEVTSLKYSTSPYLLEAASHLSPENFVEDEAKICVQELQC 948
P.troglodytes    QKVLNVSIKDRTNIQDNESTLEMTSLKYSTPPYLLEAASHLSPENFVEDEAKICVQELQC 948
C.familiaris     QKVINVSKEDRKFTQDSESILEVTCLKDNTLTY-LEAASPLIPENPRKGEAKIRVQEQQC 946
M.musculus       QKVTNRSKKDEKCTKDNEPTLEVACLEDSTFTS-VSESSHQSPENS-TEEAEVCVQELQC 949
R.norvegicus     QKVTNRSKKEEKCTKENGSTLDVACLEGSTLTS-VSEASHQTPENSLEEEAKVCVQEQRY 944
G.gallus         QKGIDFSSQCS------NEVFHVPLNSKEPSSASQQEATIHLSPDVPVKSEEQSFVPETHL 953
                 **  : * :           .          : : .   . :     *:     * :  * * :
```

FIG. 7 (B)

```
H.sapiens         PKIITKNSPLPSSIISRKEKNDPVFLEEAG---KAEKIVISRSTSPTFNKQTKRVSWSSF 1005
P.troglodytes     PKIITKNSPLPSSIISRKEKNDPVFLEEAG---KAEKIVISRSTSPTFNKQTKRVSWSSF 1005
C.familiaris      -LTITESPPLPRGIISKKEKDDPVLSEEFAQAVKAEKNVISRSTSPIFNKQTKPVSWSNF 1005
M.musculus        PQVATKSPSVASHGVSRRKKSDPGLRGESLQTMKTEKNEISRSSSPTFGKQTEPVNWPIF 1009
R.norvegicus      PQMAAENPSGPSHGVSRRKESDPGLREKSVQTMKTEKSEISRSSSPTFGKQTEPVNWPIF 1004
G.gallus          TTTINSINPGPSNQPGGQKVDVESCSHNGIQ----RRKHMDSTPRRPANKTEKKSDRTNS 1009
                      .  . .    . :: .      :      .:  :. :.     .*  :  . .

H.sapiens         NSLGQYFTGKIPKATPELGSSENSASSPPRFKTEKMESKTVLPFTDKCESSNLTVNTSFG 1065
P.troglodytes     NSLGQYFTGKIPKATPELRSSENSASSPPRFKTEKMESKTVLPFTDKCESSNLTVNTSFG 1065
C.familiaris      NSLERYFTGEILTAGPKFRSPEDCASSISTLETEEGY-KTVLPLTDKCESSSPMLN---A 1061
M.musculus        NSLRRHFNSKVKNCTPVLKSSKNRAPGSSTFN------KTALPLTGNCVPSNETADTSLG 1063
R.norvegicus      NSLKRHFNSKVKNRTPVLKSSKNHASASSAFN------KTALPLTGKCVSSSAPANTPLA 1058
G.gallus          DFMKEHCCFKPLTSTPLPVATNCVSTASSKQRKNVNSASELIGGVNQCQSSFTLEHKPSI 1069
                  : : .:    :  .  *    :.:  :.. .  .       .  :   ..:* .*    .

H.sapiens         SCPQSETIISSLKIDATLTRKNHSEHPLCSEEALDPDIELSLVSEEDKQSTSNRDFETEA 1125
P.troglodytes     SCPQSETIISSLKIDATLTRKNHSEHPLCSEEALDPDIELSLVSEEDKQSTSNRDFETEA 1125
C.familiaris      SCSQSE-VISSVKIDSTLLKRNCSKQLFCCEEAVDPDIELSLLGEEAKSSTSHRASETEA 1120
M.musculus        PCLQSEVIISPVKIEATPATN-YSKQVFCCEKDLLPDTELSPGTEEAKCPSSNKAAETEV 1122
R.norvegicus      PCPQSEVIIPSVKADTTPAKT-HCKQ---NEKDPSPDAELSPVTEEAKGSSSNPAVGTEV 1114
G.gallus          PESHLETTNFSVKSTEAPVAEEHLDE---QKLQIEPCSELPSVG--GRPELSVLEISAED 1124
                  .  : *    .:*   :       ..     :     *  **.       :    *    :*

H.sapiens         EDESIYFTPELYDPEDTDEEKNDLAETDRGNRLANNSDCILAKDLFEIRTIKEVDSAREV 1185
P.troglodytes     EDESIYFTPELYDPEDTDEEKNDLAETDRGNRLANNSDCILAKDLFEIRTIKEVDSAREV 1185
C.familiaris      EDESIYFSPELYDPADTNEEKNELVESDRDNRFVDHSNCILVEDLFEIRTIKGVDSVQEM 1180
M.musculus        DDDSECFTPELFDPVDTNEENGELVETDRSS---HSSDCFSAEELFETATGFGQK----- 1174
R.norvegicus      DDDSVCFTPELFDPVSTDEENSELVETDRSS---NNSDCLSAEELFETVTGFGQK----- 1166
G.gallus          EDESLYFTPELYDDAESEEQEMRPLDPDENQ--IECGKPTVADDLFVISTSKTLSEPKEM 1182
                  :*:*  *:***:*  .::*::    :.*...    .  ..    ..:**    *     .

H.sapiens         KAEDCIDTKLNGILHIEESKIDDIDGNVKTTWINELELG-KTHEIEIKNFKPSPSKNKGM 1244
P.troglodytes     KAEDCIDTKLNGILHIEESKIDDIDGNVKTTWIKELELG-KTHEIEIKNFKPSPSKNKGM 1244
C.familiaris      KAEDCTVTTLNRLMHIKESKIDNIDSNMKKNSYK-------------------------- 1214
M.musculus        ------------------------------------------------------------
R.norvegicus      ------------------------------------------------------------
G.gallus          INDDGRNTSLHGTMLSDISKNSTVNIEKMTNGEEAEQVESQEVDTKKRKISLSRSRNKGV 1242

H.sapiens         FPGFK----- 1249
P.troglodytes     FPGFK----- 1249
C.familiaris      ----------
M.musculus        ----------
R.norvegicus      ----------
G.gallus          SPFLLDSTST 1252
```

**FIG. 7 (C)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5288644 A, Beavis
- WO 9322456 A
- US 6858394 B
- US 6429027 B
- US 5445934 A
- US 5700637 A
- US 5744305 A
- US 5945334 A
- US 6054270 A
- US 6300063 B
- US 6733977 B
- US 7364858 B
- EP 619321 A
- EP 373203 A
- US 5223409 A
- WO 9218619 A
- WO 9117271 A
- WO 9220791 A
- WO 9215679 A
- WO 9301288 A
- WO 9201047 A
- WO 9209690 A
- WO 9002809 A


### Non-patent literature cited in the description


- **REBBECK et al.** Modification of Ovarian Cancer Risk by BRCA1/2-Interacting Genes in a Multicenter Cohort of BRCA1/2 Mutation Carriers. *CANCER RESEARCH,* vol. 69 (14), 5801-5810
- **CANTOR ; SUILLEMETTE.** *Future Oncol,* 2011, vol. 7, 253-261
- **LITMAN, R. et al.** *Cancer Cell,* 2005, vol. 8, 255
- **YU,X. et al.** *Science,* 2003, vol. 302, 639
- **KUMARASWAMY, E. et al.** *Mol Cell Biol,* 2007, vol. 27, 6733
- **BRIDGE, W.L. et al.** *Nat Genet,* 2005, vol. 37, 953
- **GODWIN, A.K. et al.** *Am J Hum Genet,* 1994, vol. 55, 666
- **CANTOR et al.** *Cell,* 2001, vol. 105, 149-160
- **CANTOR et al.** *PNAS,* 2004, vol. 101, 2357-2362
- **CANTOR et al.** *Future Oncol.,* 2011, vol. 7, 253-261
- **LI et al.** *Nucleic Acids Research,* 2000, vol. 28 (2), e1
- **LIU et al.** *Biochem Cell Bio,* 2000, vol. 80, 17-22
- **BURCZAK et al.** *Polymorphism Detection and Analysis,* 2000
- **SHEFFIELD et al.** *Proc. Natl. Acad. Sci. USA,* 1989, vol. 86, 232-236
- **ORITA et al.** *Proc. Natl. Acad. Sci. USA,* 1989, vol. 86, 2766-2770
- **FLAVELL et al.** *Cell,* 1978, vol. 15, 25-41
- **GEEVER et al.** *Proc. Natl. Acad. Sci. USA,* 1981, vol. 78, 5081-5085
- **COTTON et al.** *Proc. Natl. Acad. Sci. USA,* 1985, vol. 85, 4397-4401
- **MYERS et al.** *Science,* 1985, vol. 230, 1242-1246
- **CHURCH ; GILBERT.** *Proc. Natl. Acad. Sci. USA,* 1988, vol. 81, 1991-1995
- **SANGER et al.** *Proc. Natl. Acad. Sci. USA,* 1977, vol. 74, 5463-5467
- **NYREN, P. et al.** *Anal Biochem,* 1993, vol. 208, 171-75, http://www.454.com
- **BENTLEY, D.R.** *Curr Opin Genet Dev,* 2006, vol. 16, 545-52, http://www.illumina.com
- **STRAUSBERG, R.L. et al.** *Drug Disc Today,* 2008, vol. 13, 569-77, http://www.appliedbiosystems.com
- Current Protocols in Molecular Biology. John Wiley & Sons
- **NIELSEN et al.** *Bioconjug. Chem.,* 1994, vol. 5, 3-7
- **GIBBS et al.** *Nucleic Acids Res.,* 1989, vol. 17, 2437-2448
- **BIER et al.** *Adv Biochem Eng Biotechnol,* 2008, vol. 109, 433-53
- **HOHEISEL.** *Nat Rev Genet,* 2006, vol. 7, 200-10
- **FAN et al.** *Methods Enzymol,* 2006, vol. 410, 57-73
- **RAQOUSSIS ; ELVIDGE.** *Expert Rev Mol Diagn,* 2006, vol. 6, 145-52
- **MOCKLER et al.** *Genomics,* 2005, vol. 85, 1-15

- **CHEN et al.** *Genome Res.,* 1999, vol. 9 (5), 492-98
- **KUTYAVIN et al.** *Nucleic Acid Res.,* 2006, vol. 34, e128
- **SAMBROOK et al.** Molecular Cloning: A Laboratory Manual. Cold Spring Harbor Laboratory Press, 2001
- **DEVLIN, B. ; RISCH, N.** *Genomics,* 1995, vol. 29, 311-22
- **LEWONTIN, R.** *Genetics,* 1964, vol. 49, 49-67
- **HILL, W.G. ; ROBERTSON, A.** *Theor. Appl. Genet.,* 1968, vol. 22, 226-231
- The International HapMap Consortium. *Nature,* 2003, vol. 426, 789-796
- **RISCH, N. ; MERKIANGAS, K.** *Science,* 1996, vol. 273, 1516-1517
- **MANIATIS, N. et al.** *Proc Natl Acad Sci USA,* 2002, vol. 99, 2228-2233
- **REICH, DE et al.** *Nature,* 2001, vol. 411, 199-204
- **WALL., J.D. ; PRITCHARD, J.K.** *Nature Reviews Genetics,* 2003, vol. 4, 587-597
- **DALY, M. et al.** *Nature Genet.,* 2001, vol. 29, 229-232
- **GABRIEL, S.B. et al.** *Science,* 2002, vol. 296, 2225-2229
- **PATIL, N. et al.** *Science,* 2001, vol. 294, 1719-1723
- **DAWSON, E. et al.** *Nature,* 2002, vol. 418, 544-548
- **PHILLIPS, M.S. et al.** *Nature Genet.,* 2003, vol. 33, 382-387
- **RISCH, N. ; TENG, J.** *Genome Res.,* 1998, vol. 8, 1273-1288
- **DEVLIN, B. ; ROEDER, K.** *Biometrics,* 1999, vol. 55, 997
- **TERWILLIGER, J.D. ; OTT, J.** *Hum. Hered.,* 1992, vol. 42, 337-46
- **FALK, C.T. ; RUBINSTEIN, P.** *Ann. Hum. Genet.,* 1987, vol. 51, 227-33
- **SULEM, P. et al.** *Nat Genet,* 17 May 2009
- **RAFNAR, T. et al.** *Nat Genet,* 2009, vol. 41, 221-7
- **GRETARSDOTTIR, S. et al.** *Ann Neurol,* 2008, vol. 64, 402-9
- **STACEY, S.N. et al.** *Nat Genet,* 2008, vol. 40, 1313-18
- **GUDBJARTSSON, D.F. et al.** *Nat Genet,* 2008, vol. 40, 886-91
- **STYRKARSDOTTIR, U. et al.** *N Engl J Med,* 2008, vol. 358, 2355-65
- **THORGEIRSSON, T. et al.** *Nature,* 2008, vol. 452, 638-42
- **GUDMUNDSSON, J. et al.** *Nat Genet.,* 2008, vol. 40, 281-3
- **STACEY, S.N. et al.** *Nat Genet.,* 2007, vol. 39, 865-69
- **HELGADOTTIR, A. et al.** *Science,* 2007, vol. 316, 1491-93
- **STEINTHORSDOTTIR, V. et al.** *Nat Genet.,* 2007, vol. 39, 770-75
- **GUDMUNDSSON, J. et al.** *Nat Genet.,* 2007, vol. 39, 631-37
- **FRAYLING, TM.** *Nature Reviews Genet,* 2007, vol. 8, 657-662
- **AMUNDADOTTIR, L.T. et al.** *Nat Genet.,* 2006, vol. 38, 652-58
- **GRANT, S.F. et al.** *Nat Genet.,* 2006, vol. 38, 320-23
- **YAP et al.** *CA Cancer,* 2011, vol. 61, 31
- **HELLEDAY, T.** *Carcinogenesis,* 2010, vol. 31, 955
- **FONG et al.** *N Engl J Med,* 2009, vol. 361, 123
- **FONG et al.** *J Clin Oncol,* 2010, vol. 28, 2512
- **CANTOR, S.B. ; GUILLEMETTE, G.** FAN-CJ/BACH1/BRIP1. *Future Oncol,* vol. 7, 253
- AntisenseDrug Technology: Principles, Strategies, and Applications. Marcel Dekker Inc, 2001
- **THOMPSON.** *Drug Discovery Today,* 2002, vol. 7, 912-917
- **LAVERY et al.** *Curr. Opin. Drug Discov. Devel.,* 2003, vol. 6, 561-569
- **STEPHENS et al.** *Curr. Opin. Mol. Ther.,* 2003, vol. 5, 118-122
- **KURRECK.** *Eur. J. Biochem.,* 2003, vol. 270, 1628-44
- **DIAS et al.** *Mol. Cancer Ter.,* 2002, vol. 1, 347-55
- **CHEN.** *Methods Mol. Med.,* 2003, vol. 75, 621-636
- **WANG et al.** *Curr. Cancer Drug Targets,* 2001, vol. 1, 177-96
- **BENNETT.** *Antisense Nucleic Acid Drug. Dev.,* 2002, vol. 12, 215-24
- **FIRE et al.** *Nature,* 1998, vol. 391, 806-11
- **KIM ; ROSSI.** *Nature Rev. Genet.,* 2007, vol. 8, 173-204
- **AMARZGUIOUI et al.** *FEBS Lett.,* 2005, vol. 579, 5974-81
- **KIM et al.** *Nature Biotechnol.,* 2005, vol. 23, 222-226
- **SIOLAS et al.** *Nature Biotechnol.,* 2005, vol. 23, 227-231
- **MARQUES et al.** *Nature Biotechnol.,* 2006, vol. 23, 559-565
- **BRUMMELKAMP et al.** *Science,* 2002, vol. 296, 550-553
- **KIM ; ROSSI.** *Nat. Rev. Genet.,* 2007, vol. 8, 173-184
- **CHEN ; RAJEWSKY.** *Nat. Rev. Genet.,* 2007, vol. 8, 93-103
- **REYNOLDS et al.** *Nat. Biotechnol.,* 2004, vol. 22, 326-330
- **CHI et al.** *Proc. Natl. Acad. Sci. USA,* 2003, vol. 100, 6343-6346

- **VICKERS et al.** *J. Biol. Chem.,* 2003, vol. 278, 7108-7118
- **AGAMI.** *Curr. Opin. Chem. Biol.,* 2002, vol. 6, 829-834
- **SHI.** *Trends Genet.,* 2003, vol. 19, 9-12
- **SHUEY et al.** *Drug Discov. Today,* 2002, vol. 7, 1040-46
- **MCMANUS et al.** *Nat. Rev. Genet.,* 2002, vol. 3, 737-747
- **XIA et al.** *Nat. Biotechnol.,* 2002, vol. 20, 1006-10
- **PLASTERK et al.** *curr. Opin. Genet. Dev.,* 2000, vol. 10, 562-7
- **BOSHER et al.** *Nat. Cell Biol.,* 2000, vol. 2, E31-6
- **HUNTER.** *Curr. Biol.,* 1999, vol. 9, R440-442
- **AUSUBEL, F. et al.** Current Protocols in Molecular Biology. John Wiley & Sons, 1998
- **KRAUS, M. ; AARONSON, S.** *Methods Enzymol.,* 1991, vol. 200, 546-556
- **ALTSCHUL, S. et al.** *Nucleic Acids Res.,* 1997, vol. 25, 3389-3402
- **KENT, W.J.** *Genome Res.,* 2002, vol. 12, 656-64
- **NIELSEN, P. et al.** *Science,* 1991, vol. 254, 1497-1500
- **KOHLER ; MILSTEIN.** *Nature,* 1975, vol. 256, 495-497
- **KOZBOR et al.** *Immunol. Today,* 1983, vol. 4, 72
- **COLE et al.** Monoclonal Antibodies and Cancer Therapy. Alan R. Liss,1985, Inc, 1985, 77-96
- Current Protocols in Immunology. John Wiley & Sons, Inc, 1994
- **GALFRE et al.** *Nature,* 1977, vol. 266, 55052
- **R.H. KENNETH.** Monoclonal Antibodies: A New Dimension In Biological Analyses. Plenum Publishing Corp, 1980
- **LERNER.** *Yale J. Biol. Med.,* 1981, vol. 54, 387-402
- **FUCHS et al.** *Bio/Technology,* 1991, vol. 9, 1370-1372
- **HAY et al.** *Hum. Antibod. Hybridomas,* 1992, vol. 3, 81-85
- **HUSE et al.** *Science,* 1989, vol. 246, 1275-1281
- **GRIFFITHS et al.** *EMBO J.,* 1993, vol. 12, 725-734

SEQUENCE LISTING

<110> deCODE Genetics ehf

<120> RISK VARIANTS FOR CANCER

<130> 2598-PC00

<160> 22

<210> 1
<211> 401
<212> DNA
<213> Homo sapiens

<400> 1

```
gccattctat taccttttt tttttcagag atgggagtct cactctgtca cctggggtgc     60
agtggcacga tccaatcttg gttcactgca acctccgcct cccaggttca agcaattctt    120
gtgcctcagc caccaagtag ctggaaccac aagtgcatca ccacatatgg ctcctttttt    180
gtagagacag ggttttgtca ygttgctcag gctggtcttg aactcctgaa ctcaagcgat    240
ccgcctgcct cggcctccca aagtgctggg gattacaggc atgagccact gcactccacc    300
ccattctatc actggaaata aaaaaatttc attacaggct gggcacagtg gctcatgcct    360
ataatcccaa cattttgaga ggccaaggcc ggaggactgc a                        401
```

<210> 2
<211> 401
<212> DNA
<213> Homo sapiens

<400> 2

```
caggagttca agattacagt gagctatgat tgtgccatag cactacagcc tgggtgaaag     60
agcttaaaaa aaaaaaaaaa aaaaaaacag aggctgggca cggtggctca cgcctgtaat    120
cccagcactt tgggaggccg aggcgggtgg atcgcgaggt caggagatcg agaccatcgt    180
ggctaacatg gtgaaacccc rtctctacta aaaatacaaa aattagccgg gcgtggtggc    240
aggctcctgt agtcccagct actcgggagg ctgaggcagg agaatggcgt gaacctggga    300
ggtggagctt gcagtgagcc aagatatcgc accactgcac tccagcctgg gcgacaaagc    360
tagactccgt ctcaaaaaag gaaaaaaaaa aaaaaaaaaa g                        401
```

<210> 3
<211> 401
<212> DNA
<213> Homo sapiens

<400> 3

agctgggact acaggcacat gccaccacgc ctagctaatt ttttgtattt tagtagagac 60
agggtttcac catgttgccc aggctggtct tgaactcctg tgcttaagca atccgcccac 120
ctcggcctcc caaagtgcta ggattacagg cgtgagccac cgcacccagc ctcaaaatgc 180
ttcttaaatt caatttaatt wttttttttt tttgagacgg agtctcactc tgctgcccag 240
gttggagtgc agtgtggcaa tctcggctca ctacaacctc cgtctcctgg gttcaagtga 300
ttctcctgcc tcagcctgct gagtagctgg gattacaggc acctgccacc atgcccagat 360
aatttttgta tttttagtag aggcggggtt tcaccatatt g 401

<210> 4
<211> 401
<212> DNA
<213> Homo sapiens

<400> 4

tgtttattct ataagctgta taaccaaaaa gaaaaattaa agatacttta ggaataaaat 60
aatttgaaaa ataaaagaac ttatttgcat gatgtatcct gaaaaattag ggcagccagt 120
attactgaaa taatggagga gtccgggagt ggttgtggta tttttgtatt taaagcttac 180
acaatagagt agaaacgttg yggtacatat ttacttacaa gaatatgttt tgaataagtt 240

tactttccta aatgtcatct gttttaatga cctgctactt ggattctttc tcacagtgtt 300
aggttgctga ctatgtgaaa tttcctactt cttattattg ctatcagaat aggaaattta 360
aaattaaatc agaaatgtaa aagtcttaaa acttcacctt t 401

<210> 5
<211> 401
<212> DNA
<213> Homo sapiens

<400> 5

taggtactgt tcaaagctct ttgcatgtac taactcattt aatcctcaca aaaaacataa 60
ggcagaaact attattaact tcattttaga gatgagaaaa cataaatgag gaagttaagc 120
aatttctcta gcatttcaca agaagtagca gagtaggaat ttaaacccaa gaaatctatc 180
cccacagtct gtactattta rtctctcaaa ctataatggg aaataaccat ttttcatcca 240
ctgaatcagc atcctactag gcaaggcttt gggaaaacag gcactttaac tggtgggaat 300
tcaaactgat attacagggg ctgggcatgg tggctcacgc ctgtaatctc agcactttgg 360
gaggcctagg taggtggatc acttgaggca agcagttcaa g 401

<210> 6
<211> 401
<212> DNA
<213> Homo sapiens

<400> 6

cgggctagaa cagttctcac atttttaaat gggaggaaaa acatcaaaag aagattttat 60
gaaacctgaa aatcacatga aattcacatg tcaggctcat aaataagttt taatgcaaca 120
gagtcatgtc tgttcactta tgtattatct atggctgctc tccctatgat ggcagagttg 180
agcagctgta acagagaccg kgcggcctaa agatacttac tgtcgggctg gttacagaaa 240
atgttttcca aacagaagct gtattccaaa ttctttccta atgtggcata caaagccttt 300
aggatctggc tcctgtgtgc ctctcctgcc gctgctgctg ctgctgctgc tcctgtttcc 360
gcttctgcca tggccaccgc cactgccgcc gcctcctcct t 401

<210> 7
<211> 401
<212> DNA

<213> Homo sapiens

<400> 7

```
tttaaaacac aaatatatca tacctggtgc cagaaagttt tttgttttcc ttaactatat     60
tggaaatcac ctatttccca acttgaagac tttaataaat attattctca aagaattttc    120
aaatatagtc attccttggt gtcatgggaa attggttcca gaacctccct cagataccaa    180
aatccacaga tgctcaagtc mctgatgctg gtactatatt tgcatatgac ccacgcacat    240
ccccctgtat gcttcaagtc atctttagat tacttataat acctaataca atgtaagtgc    300
tatataaata gttgttaaca ctgtattgtt tgggaatcat gacaagaaaa aagtctagac    360
atgttcggta gagacgcaac catccttttt tttttcacag a                        401
```

<210> 8
<211> 401
<212> DNA
<213> Homo sapiens

<400> 8

```
ttcactgctt gggtaagtgt ataactcttt ctgggaacat atattaagaa atttatatat     60
ttacatcctc tgatttcata tgtaggaatc taactcaagg aaatttttgt atgttgatgt    120
gtatatataa atgtcttttg gtggtagaaa ttatgagggt tttcagcttc tcattctttt    180
ctatttcttc cacacttctt yaatgatcat ctcttacttt cttacttttc tttctactta    240
cctcccctct ttttctctgt tcctcccagt acttcattgc ttgatttctc tgagtattaa    300
aatggtaggc caggcgtggt ggttcatgcc tgtaatccca gcactctgag agccaaagcc    360
agaggatcac ttgaggccag gagtttgaca ccagcctggt c                        401
```

<210> 9
<211> 401
<212> DNA
<213> Homo sapiens

<400> 9

```
agggtaaccc caattcataa aaataaaaat catattatca tcacatacaa aaaggcttat     60
cttcctggtc ttgatagcaa gggagtttga aggaagttca tcaaaggggt aaaatcttta    120
agatacaaag ttaaaaagaa agaagatatc tcccgattat gttttttttaa aatcttcaag    180
aaacctaaga aatacccaat rgaaaaatgg aggctgggca cggtggctca cacttgtaat    240
tcaagcactt tgggaggctg aggcgagtga tcatttgagg tcaggagtta gagatcagcc    300
tggccaacat ggtgaaaccc catttctact aaaaatacaa aaatcagccg ggcgtggtgg    360
caggtgcctg taatcccagc tactcaggag gttgaagcag g                        401
```

<210> 10
<211> 8166
<212> DNA
<213> Homo sapiens

<400> 10

gctattgggc gctgggagtc gagggggcgg gaggcgggaa ttcgtctcgg gttgtgtggt 60
tgagggtct ggtgggtcga ggaaaggtaa cggcggcccc agtcctgcac acaaggccgg 120
ggaagtagca gcaccccag gaagagggag gaggaagggc tcgtgccctt tcttctcttc 180
cagggctccg ctttatttgc tctcagaagt cggtttcctt tccttttctt cagtgaatcg 240
gagctcagag cgttgcttcg gtttccctcc agacagttag gaatctgaaa taaacaggaa 300
agcactatgt cttcaatgtg gtctgaatat acaattggtg gggtgaagat ttactttcct 360
tataaagctt acccgtcaca gcttgctatg atgaattcta ttctcagagg attaaacagc 420
aagcaacatt gtttgttgga gagtcccaca ggaagtggaa aaagcttagc cttactttgt 480
tctgctttag catggcaaca atctcttagt gggaaaccag cagatgaggg cgtaagtgaa 540
aaagctgaag tacaattgtc atgttgttgt gcatgccatt caaaggattt tacaaacaat 600
gacatgaacc aaggaacttc acgtcatttc aactatccaa gcacaccacc ttctgaaaga 660
aatggcactt catcaacttg tcaagactcc cctgaaaaaa ccactctggc tgcaaagtta 720
tctgctaaga aacaggcatc catatacaga gatgaaaatg atgattttca agtagagaag 780
aaaagaattc gacccttaga aactacacag cagattagaa aacgtcattg ctttggaaca 840
gaagtacaca atttggatgc aaaagttgat tcaggaaaga ctgtaaaact caactctcca 900
ctggaaaaga taaactcctt ttcgccacag aaacccctg gccactgttc taggtgctgt 960
tgttctacta aacaaggaaa cagtcaagag tcatcgaata ccattaagaa ggatcataca 1020
gggaaatcca agatacccaa aatatatttt gggacacgca cacacaagca gattgctcag 1080
attactagag agctccggag gacggcatat tcaggggttc caatgactat tctttccagc 1140
agggatcata cttgtgtcca tcctgaggta gtcggtaact tcaacagaaa tgagaagtgc 1200
atggaattgc tagatgggaa aaacggaaaa tcctgctatt tttatcatgg agttcataaa 1260
attagtgatc agcacacatt acagactttc caagggatgt gcaaagcctg ggatatagaa 1320
gaacttgtca gcctggggaa gaaactaaag gcctgtccat attacacagc ccgagaacta 1380
atacaagatg ctgacatcat attttgtccc tacaactatc ttctagatgc acaaataagg 1440
gaaagtatgg atttaaatct gaaagaacag gttgtcattt tagatgaagc tcataacatc 1500
gaggactgtg ctcgggaatc agcaagttac agtgtaacag aagttcagct tcggtttgct 1560
cgggatgaac tagatagtat ggtcaacaat aatataagga agaaagatca tgaaccccta 1620
cgagctgtgt gctgtagcct cattaattgg ttagaagcaa acgctgaata tcttgtagaa 1680
agagattatg aatcagcttg taaaatatgg agtggaaatg aaatgctctt aactttacac 1740
aaaatgggta tcaccactgc tacttttccc attttgcagg gacattttc tgctgttctt 1800
caaaaagagg aaaaaatctc accaatttat ggtaaagagg aggcaagaga agtacctgtt 1860
attagtgcat caactcaaat aatgcttaaa ggacttttta tggtacttga ctatcttttt 1920
aggcaaaata gcagatttgc agatgattat aaaattgcga ttcaacagac ttactcctgg 1980
acaaatcaga ttgatatttc agacaaaaat gggttgttgg ttctaccaaa aaataagaaa 2040
cgttcacgac agaaaactgc agttcatgtg ctaaactttt ggtgcttaaa tccagctgtg 2100
gccttttcag atattaatgg caaagttcag accattgttt tgacatctgg tacattatca 2160
ccaatgaaat ccttttcgtc agaacttggt gttacattta ctatccagct ggaggctaat 2220
catatcatta aaaattcaca ggtttgggtt ggtaccattg ggtcaggccc caagggtcgg 2280
aatctctgtg ctaccttcca gaatactgaa acatttgagt tccaagatga agtgggagca 2340
cttttgttat ctgtgtgcca gactgtgagc caaggaattt tgtgtttctt gccatcttac 2400
aagttattag aaaaattaaa agaacgttgg ctctctactg gtttatggca taatctggag 2460
ttggtgaaga cagtcattgt agaaccacag ggaggagaaa aaacaaattt tgatgaatta 2520
ctgcaggtgt actatgacgc aatcaaatac aaaggagaga aagatggagc tctcctggta 2580
gcagtttgtc gtggtaaagt gagtgagggt ctggatttct cagatgacaa tgcccgtgct 2640
gtcataacaa taggaattcc ttttccaaat gtgaaagatc tacaggttga actaaaacga 2700

caatacaatg accaccattc aaaattgaga ggtcttctac ctggccgtca gtggtatgaa 2760
attcaagcat acagggcctt aaaccaggcc cttggtagat gtattagaca cagaaatgat 2820
tggggagctc ttattctagt ggatgatcgc tttaggaata acccaagtcg ctatatatct 2880
ggactttcta aatgggtacg gcagcagatt cagcaccatt caacctttga aagtgcactg 2940
gaatccttgg ctgaattttc caaaaagcat caaaaagttc ttaatgtatc cataaaggac 3000
agaaccaata tacaggacaa tgagtctaca cttgaagtga cctctttaaa gtacagtacc 3060
tcaccttatt tactggaagc agcaagtcat ctatcaccag aaaattttgt ggaagatgaa 3120
gcaaagatat gtgtccagga actacagtgt cctaaaatta ttaccaaaaa ttcacctcta 3180
ccaagtagca ttatctccag aaaggagaaa aatgatccag tattcctgga agaagcaggg 3240
aaagcagaaa aaattgtgat ttccagatcc acaagcccaa ctttcaacaa acaaacaaag 3300
agagttagct ggtcaagctt taattctttg ggacagtatt ttactggtaa aataccgaag 3360
gcaacacctg agctcgggtc atcagagaat agtgcctcta gtcctccccg tttcaaaaca 3420
gagaagatgg aaagtaaaac tgttttgccc ttcactgata aatgtgaatc ctcaaatctg 3480
acagtaaaca catcgtttgg atcatgccct caatcagaaa ccattatttc atcattaaag 3540
attgatgcca cccttactag aaaaaatcat tctgaacatc cgctctgttc tgaagaagcc 3600
ctggatccag acattgaatt gtctctagta agtgaagaag ataaacagtc cacttcaaat 3660
agagattttg aaacagaagc agaagatgaa tctatctatt ttacacctga actttatgat 3720
cctgaagata cagatgaaga aaaaaatgac ctagctgaaa ctgatagagg aaatagattg 3780
gctaacaatt cagattgcat tttagctaaa gaccttttttg aaattagaac tataaaagaa 3840
gtagattcag ccagagaagt gaaagctgag gattgcatag atacaaagtt gaatggaatt 3900
ctgcatattg aagaaagtaa aattgatgac attgatggta atgtaaaaac aacttggata 3960
aatgaactgg aactgggaaa aactcatgaa atagaaataa agaactttaa accatctcct 4020
tccaaaaata aaggcatgtt tcctggtttt aagtaataat acttaactct caagctaagt 4080
aaaaatatgt catcatgctt atgttaaact ctgttgtaag taataatttg taaattgaat 4140
aagtggcata ctttttaaaa aactatttta tgttcagaaa tgtaaatgtt attattcttg 4200
agtttttggg ttttttttttt tgagacagag tcttggtctg ttgcccaggc tggaatgcag 4260
tggtgtgctc tgggctcact gcaaccttca cctccaggtt caagtgattc tcctgcctca 4320
gccttctgag tagctgggac tacaggtgtg caccaccatg cccagctagt tttgtatttt 4380
ttagtagaga tggggtttca ccatgttggc caggctggtc tcgaactcct ggcctcgtga 4440
tctgcccttc tctgcctccc taagttgctg ggattacagg tgtgagccac agtgcctggc 4500
ccattcttga gttttgataa agtaattcat acaaagtact gtcctcaaat aagtcttcct 4560
tagctaaatg caatttaaaa ttattcaaag atcctagggc acttctagtt tcacgtaaat 4620
attcatatta ggtggttctc ttcatccatt tgttttcaca ctgatacata aaaattaaca 4680
gcagtctaat ctagtgacac ctcagtcata tttcgctata gattttacct caaatcagtc 4740
caagactttt tcagagatca ccatttgtct tgaaaggttt atttcgttat taaactgcct 4800
acttataagt aattaagaga aattaagaaa gtagtatgca tttttaattg aaattgtttt 4860
acattctttg tataataaac ctaaaaccaa acatgtcata aacaaattga cgtaaagata 4920
taaaatgcca aatgaagtat tccaaatttt ctattctaat tatttagctt caccatcatt 4980
gtggaaaaaa atactagatc ctgcttagta ttatatattt ttcctagtgg atcagtgagt 5040
aataagtacc aaacactaga ctagaaggta atttctacat tgtttagaaa gggtgaaaca 5100
atttatcccc tctggtattg ttctagcata agctttagtt atacaatgat taagatagaa 5160
aacttcatat ataaatttga taagcaaacc cacatttata gctgcagcta aaatatgttt 5220
ccttagggca cagtaatcct ttctgtgaat tttgaccttg tttgtgtttt tgtgaatgaa 5280
gctatatgtc taatcaaaaa tgattataaa agaggctcat ctctgacatc attccaaaaa 5340
tacattcatt gatctctttt taagaaacat ctgttattca ctgggcattg ggactttttg 5400
tgagtaattt gaattgaaat tttatgagct atccaagaat tctgtatggt ctattatttt 5460
caagtcaaaa tttccagtaa ggatttactt tacatttcat ttggataaat gaatcattat 5520
ataggtatgt ctttgcttcc attttgagac atttagattt ttacagcctg tttctatagc 5580
atttgatgtt acaactctaa gcgtagttca aagacattta aattgacaag ttaccagtta 5640
aagaatttag aatatattag atcccatcta gtattatata ttttttctag ttgatcattg 5700
agcagtaaat accaaatact cgattagaag gtaattttta cattgttttg aaagggtgaa 5760
acaatttatc tcctctggta ttattcttaa accacagata gggatagtag ggtagtgaaa 5820
cgaataaata cctggtagaa gacaagagac ttgggctcta cacctggctc tgccactgat 5880
ttgctaagtc atattggcaa tcaccacacc cttcagggaa ttagtttcat ctgtaaaatg 5940
cagcggttag tactataaaa tcatacaaat ttctttgtgc tttgagaatc tataaaggaa 6000
tgtctgttga tattctgagt cgattttcat ttgcttttgt tccagaacgg ttaaaataaa 6060
gcatattatt tcatttaaaa agtaaagtgg ctcttactca tttacattta gggaacgatg 6120
gtgacgtggt ttggctatgt ccccagccag atctcatctt gaattgtagt tcccataatc 6180
ccgacgtgtt gtgggaagga cccagtggga ggtaattgaa tcgtgggggc ggttaccctc 6240
atgcggttct cgtgatactg agttcttatg agacctgatg attttataag gagcttttcc 6300
ctgcttcact ctcattcttc tctcctgctg ccctgtgaaa agttgccttc cgccatgatt 6360
gtaagtttct tgaggcctcc tcagtcatgc agaactgtga gtcaattaaa cctctttcct 6420
ttataaatta cccagtcttg ggtatgactt tattagcagc atgagaacag actgacacag 6480

```
atggtatatg tgcataataa cttggaaagc tagatattta ttttcgcaat gctacaatta 6540
aaacattttg aggactttta aaattacctt tagggccagg cgcggtggct cacgcctgta 6600
atcccagcac tttgggaggc cgaggcgggc ggatcacgag gtcaggagat cgagaccatc 6660
ctggttaaca cggtgaaacc ccgtctctac taaaaataca aaaaattagc caggcgtggt 6720
ggcgggcgcc tgtagtccca gctactcagg aggctgaggc aggagaatgg cgtgaacccg 6780
ggaggtggag cttgcagtga gccgagattg cgccactgca ctccagcctg ggcgacagag 6840
cgggactccg tctcaaaaaa aaaaaaaaaa agaaattacc tttagaattg gtaaactaca 6900
tgagaaaatt aagcatatta ttcatatctt agtgttatta cccaacttca tctccaatac 6960
tttccccttt ccctagctag atatgttttt gtttgtttgt tttagacagt cttgctctgt 7020
cgctcaggct ggagtgcaaa ggcacgatct tggctcactg caacctctgc ctcgcaggtt 7080
aaagcgattc tcatgtctca gcctcctgcg tagctgggat tacaggtgta caccactgca 7140
cccagctaat tttttttttt tttttttttt aagtatcgac agggtttcac catgttggcc 7200
aggctggtct caaactcctg acctcaagtg atctgcccac ctcggcctcc caaagtgttg 7260
ggattacagg cgtgagccac catgcctggc ctagatattt tttattcttt ccaaaattta 7320
attctccctg aagttaaaaa ttttcattac tgagaatgta catagagatg tgtcacaacc 7380
ctttagtaat tccaaaaggt gtttcaaaaa ttttatacaa tgataatcac tgttgaaaca 7440
ggtgtatatt ctcctcagat catgactgac caagatgata ttccaagaag taaactactg 7500
gcctttatag agtaggatgt aggccatttt cattactgat aacatacttt taaaagaaat 7560
gtttacagat ttaaataagt taaaacatct aaatgctttt aaaagagcac ctggcacatt 7620
gcaagttatt cattattaat tagtagatga atcatatatc tggagtgcac ccttgctcta 7680
tatgaaagct tccctaacta tagatatata ggtgatagac tgacaataaa gatttgagga 7740
aagaaaaatt atttggctgg cttatttttt aagcttttga gatgtataag gtagagattg 7800
tttatcaaat aatttatgtg acacattcaa tgcatatgaa ataattatct aatactgaat 7860
ttattcaagg actgaaagta tatagagcac acaggctgca aaccaggaaa actaccagtt 7920
agagacaggg ctttattctt ggaataaagg tacaatgtaa agagtagatg tttcatgact 7980
tgattaaatt atttaaaact gtctagaatt gtgttataaa actattacta tgtttctatg 8040
cacttagtta ttacatgggt ttaaatttgg cactgtttct aagtttctat aaggctttgt 8100
tgttaagatc tttctattca aaacattaat tttaacaaaa agcttttccc cgttattttt 8160
ctgcaa                                                            8166
```

<210> 11
<211> 117
<212> DNA
<213> Homo sapiens

<400> 11

```
cttgtaagat ggcaagaaac acaaaattcc ttggctcaca gtctggcaca cagataacaa     60
aagtgctccc acttcatctt ggaactcaaa tgtttcagta ttctggaagg tagcaca      117
```

<210> 12
<211> 118
<212> DNA
<213> Homo sapiens

<220>
<221> variant
<222> 59
<223> aa(insertion,ins)or -(deletion, del)

<400> 12

```
cttgtaagat ggcaagaaac acaaaattcc ttggctcaca gtctggcaca cagataacna     60
aaagtgctcc cacttcatct tggaactcaa atgtttcagt attctggaag gtagcaca     118
```

<210> 13
<211> 1249

<212> PRT
<213> Homo sapiens

<400> 13

```
Met Ser Ser Met Trp Ser Glu Tyr Thr Ile Gly Gly Val Lys Ile Tyr
 1               5                  10                  15
Phe Pro Tyr Lys Ala Tyr Pro Ser Gln Leu Ala Met Met Asn Ser Ile
            20                  25                  30
Leu Arg Gly Leu Asn Ser Lys Gln His Cys Leu Leu Glu Ser Pro Thr
```

```
        35                  40                  45
Gly Ser Gly Lys Ser Leu Ala Leu Leu Cys Ser Ala Leu Ala Trp Gln
    50                  55                  60
Gln Ser Leu Ser Gly Lys Pro Ala Asp Glu Gly Val Ser Glu Lys Ala
65                  70                  75                  80
Glu Val Gln Leu Ser Cys Cys Cys Ala Cys His Ser Lys Asp Phe Thr
                85                  90                  95
Asn Asn Asp Met Asn Gln Gly Thr Ser Arg His Phe Asn Tyr Pro Ser
            100                 105                 110
Thr Pro Pro Ser Glu Arg Asn Gly Thr Ser Ser Thr Cys Gln Asp Ser
        115                 120                 125
Pro Glu Lys Thr Thr Leu Ala Ala Lys Leu Ser Ala Lys Lys Gln Ala
    130                 135                 140
Ser Ile Tyr Arg Asp Glu Asn Asp Asp Phe Gln Val Glu Lys Lys Arg
145                 150                 155                 160
Ile Arg Pro Leu Glu Thr Thr Gln Gln Ile Arg Lys Arg His Cys Phe
                165                 170                 175
Gly Thr Glu Val His Asn Leu Asp Ala Lys Val Asp Ser Gly Lys Thr
            180                 185                 190
Val Lys Leu Asn Ser Pro Leu Glu Lys Ile Asn Ser Phe Ser Pro Gln
        195                 200                 205
Lys Pro Pro Gly His Cys Ser Arg Cys Cys Cys Ser Thr Lys Gln Gly
    210                 215                 220
Asn Ser Gln Glu Ser Ser Asn Thr Ile Lys Lys Asp His Thr Gly Lys
225                 230                 235                 240
Ser Lys Ile Pro Lys Ile Tyr Phe Gly Thr Arg Thr His Lys Gln Ile
                245                 250                 255
Ala Gln Ile Thr Arg Glu Leu Arg Arg Thr Ala Tyr Ser Gly Val Pro
            260                 265                 270
Met Thr Ile Leu Ser Ser Arg Asp His Thr Cys Val His Pro Glu Val
        275                 280                 285
Val Gly Asn Phe Asn Arg Asn Glu Lys Cys Met Glu Leu Leu Asp Gly
    290                 295                 300
Lys Asn Gly Lys Ser Cys Tyr Phe Tyr His Gly Val His Lys Ile Ser
305                 310                 315                 320
Asp Gln His Thr Leu Gln Thr Phe Gln Gly Met Cys Lys Ala Trp Asp
                325                 330                 335
Ile Glu Glu Leu Val Ser Leu Gly Lys Lys Leu Lys Ala Cys Pro Tyr
            340                 345                 350
Tyr Thr Ala Arg Glu Leu Ile Gln Asp Ala Asp Ile Ile Phe Cys Pro
        355                 360                 365
Tyr Asn Tyr Leu Leu Asp Ala Gln Ile Arg Glu Ser Met Asp Leu Asn
    370                 375                 380
Leu Lys Glu Gln Val Val Ile Leu Asp Glu Ala His Asn Ile Glu Asp
385                 390                 395                 400
Cys Ala Arg Glu Ser Ala Ser Tyr Ser Val Thr Glu Val Gln Leu Arg
                405                 410                 415
Phe Ala Arg Asp Glu Leu Asp Ser Met Val Asn Asn Asn Ile Arg Lys
            420                 425                 430
Lys Asp His Glu Pro Leu Arg Ala Val Cys Cys Ser Leu Ile Asn Trp
        435                 440                 445
Leu Glu Ala Asn Ala Glu Tyr Leu Val Glu Arg Asp Tyr Glu Ser Ala
    450                 455                 460
Cys Lys Ile Trp Ser Gly Asn Glu Met Leu Leu Thr Leu His Lys Met
465                 470                 475                 480
Gly Ile Thr Thr Ala Thr Phe Pro Ile Leu Gln Gly His Phe Ser Ala
                485                 490                 495
Val Leu Gln Lys Glu Glu Lys Ile Ser Pro Ile Tyr Gly Lys Glu Glu
            500                 505                 510
Ala Arg Glu Val Pro Val Ile Ser Ala Ser Thr Gln Ile Met Leu Lys
        515                 520                 525
Gly Leu Phe Met Val Leu Asp Tyr Leu Phe Arg Gln Asn Ser Arg Phe
    530                 535                 540
```

```
Ala Asp Asp Tyr Lys Ile Ala Ile Gln Gln Thr Tyr Ser Trp Thr Asn
545                 550                 555                 560
Gln Ile Asp Ile Ser Asp Lys Asn Gly Leu Leu Val Leu Pro Lys Asn
                565                 570                 575
Lys Lys Arg Ser Arg Gln Lys Thr Ala Val His Val Leu Asn Phe Trp
            580                 585                 590
Cys Leu Asn Pro Ala Val Ala Phe Ser Asp Ile Asn Gly Lys Val Gln
        595                 600                 605
Thr Ile Val Leu Thr Ser Gly Thr Leu Ser Pro Met Lys Ser Phe Ser
    610                 615                 620
Ser Glu Leu Gly Val Thr Phe Thr Ile Gln Leu Glu Ala Asn His Ile
625                 630                 635                 640
Ile Lys Asn Ser Gln Val Trp Val Gly Thr Ile Gly Ser Gly Pro Lys
                645                 650                 655
Gly Arg Asn Leu Cys Ala Thr Phe Gln Asn Thr Glu Thr Phe Glu Phe
            660                 665                 670
Gln Asp Glu Val Gly Ala Leu Leu Leu Ser Val Cys Gln Thr Val Ser
        675                 680                 685
Gln Gly Ile Leu Cys Phe Leu Pro Ser Tyr Lys Leu Leu Glu Lys Leu
    690                 695                 700
Lys Glu Arg Trp Leu Ser Thr Gly Leu Trp His Asn Leu Glu Leu Val
705                 710                 715                 720
Lys Thr Val Ile Val Glu Pro Gln Gly Gly Glu Lys Thr Asn Phe Asp
                725                 730                 735
Glu Leu Leu Gln Val Tyr Tyr Asp Ala Ile Lys Tyr Lys Gly Glu Lys
            740                 745                 750
Asp Gly Ala Leu Leu Val Ala Val Cys Arg Gly Lys Val Ser Glu Gly
        755                 760                 765
Leu Asp Phe Ser Asp Asp Asn Ala Arg Ala Val Ile Thr Ile Gly Ile
    770                 775                 780
Pro Phe Pro Asn Val Lys Asp Leu Gln Val Glu Leu Lys Arg Gln Tyr
785                 790                 795                 800
Asn Asp His His Ser Lys Leu Arg Gly Leu Leu Pro Gly Arg Gln Trp
                805                 810                 815
Tyr Glu Ile Gln Ala Tyr Arg Ala Leu Asn Gln Ala Leu Gly Arg Cys
            820                 825                 830
Ile Arg His Arg Asn Asp Trp Gly Ala Leu Ile Leu Val Asp Asp Arg
        835                 840                 845
Phe Arg Asn Asn Pro Ser Arg Tyr Ile Ser Gly Leu Ser Lys Trp Val
    850                 855                 860
Arg Gln Gln Ile Gln His His Ser Thr Phe Glu Ser Ala Leu Glu Ser
865                 870                 875                 880
Leu Ala Glu Phe Ser Lys Lys His Gln Lys Val Leu Asn Val Ser Ile
                885                 890                 895
Lys Asp Arg Thr Asn Ile Gln Asp Asn Glu Ser Thr Leu Glu Val Thr
            900                 905                 910
Ser Leu Lys Tyr Ser Thr Pro Pro Tyr Leu Leu Glu Ala Ala Ser His
        915                 920                 925
Leu Ser Pro Glu Asn Phe Val Glu Asp Glu Ala Lys Ile Cys Val Gln
    930                 935                 940
Glu Leu Gln Cys Pro Lys Ile Ile Thr Lys Asn Ser Pro Leu Pro Ser
945                 950                 955                 960
Ser Ile Ile Ser Arg Lys Glu Lys Asn Asp Pro Val Phe Leu Glu Glu
                965                 970                 975
Ala Gly Lys Ala Glu Lys Ile Val Ile Ser Arg Ser Thr Ser Pro Thr
            980                 985                 990
Phe Asn Lys Gln Thr Lys Arg Val Ser Trp Ser Ser Phe Asn Ser Leu
        995                 1000                1005
Gly Gln Tyr Phe Thr Gly Lys Ile Pro Lys Ala Thr Pro Glu Leu Gly
   1010                1015                1020
Ser Ser Glu Asn Ser Ala Ser Ser Pro Pro Arg Phe Lys Thr Glu Lys
1025               1030                1035                1040
Met Glu Ser Lys Thr Val Leu Pro Phe Thr Asp Lys Cys Glu Ser Ser
```

```
                1045                1050                1055
Asn Leu Thr Val Asn Thr Ser Phe Gly Ser Cys Pro Gln Ser Glu Thr
            1060                1065                1070
Ile Ile Ser Ser Leu Lys Ile Asp Ala Thr Leu Thr Arg Lys Asn His
        1075                1080                1085
Ser Glu His Pro Leu Cys Ser Glu Glu Ala Leu Asp Pro Asp Ile Glu
    1090                1095                1100
Leu Ser Leu Val Ser Glu Glu Asp Lys Gln Ser Thr Ser Asn Arg Asp
1105                1110                1115                1120
Phe Glu Thr Glu Ala Glu Asp Glu Ser Ile Tyr Phe Thr Pro Glu Leu
                1125                1130                1135
Tyr Asp Pro Glu Asp Thr Asp Glu Glu Lys Asn Asp Leu Ala Glu Thr
            1140                1145                1150
Asp Arg Gly Asn Arg Leu Ala Asn Asn Ser Asp Cys Ile Leu Ala Lys
        1155                1160                1165
Asp Leu Phe Glu Ile Arg Thr Ile Lys Glu Val Asp Ser Ala Arg Glu
    1170                1175                1180
Val Lys Ala Glu Asp Cys Ile Asp Thr Lys Leu Asn Gly Ile Leu His
1185                1190                1195                1200
Ile Glu Glu Ser Lys Ile Asp Asp Ile Asp Gly Asn Val Lys Thr Thr
                1205                1210                1215
Trp Ile Asn Glu Leu Glu Leu Gly Lys Thr His Glu Ile Glu Ile Lys
            1220                1225                1230
Asn Phe Lys Pro Ser Pro Ser Lys Asn Lys Gly Met Phe Pro Gly Phe
        1235                1240                1245
Lys
```

<210> 14
<211> 27
<212> PRT
<213> Homo sapiens

<400> 14

```
Cys Ala Thr Phe Gln Asn Thr Glu Thr Phe Glu Phe Gln Asp Glu Val
1               5                   10                  15
Gly Ala Leu Phe Cys Tyr Leu Cys Ala Arg Leu
            20                  25
```

<210> 15
<211> 182771
<212> DNA
<213> Homo sapiens

<400> 15

```
tctaatcgag tatttggtat ttactgctca atgatcaact agaaaaaata tataatacta      60
gatgggatct aatatattct aaattcttta actggtaact tgtcaattta aatgtctttg     120
aactacgctt agagttgtaa catcaaatgc tatagaaaca ggctgtaaaa atctaaatgt     180
ctcaaaatgg aagcaaagac atacctatat aatgattcat ttatccaaat gaaatgtaaa     240
gtaaatcctt actggaaatt ttgacttgaa aataatagac catacagaat tcttggatag     300
ctcataaaat ttcaattcaa attactcaca aaaagtccca atgcccagtg aataacagat     360
gtttcttaaa aagagatcaa tgaatgtatt tttggaatga tgtcagagat gagcctcttt     420
tataatcatt tttgattaga catatagctt cattcacaaa aacacaaaca aggtcaaaat     480
tcacagaaag gattactgtg ccctaaggaa acatatttta gctgcagcta taaatgtggg     540
tttgcttatc aaatttatat atgaagtttt ctatcttaat cattgtataa ctaaagctta     600
tgctagaaca ataccagagg ggataaattg tttcaccctt tctaaacaat gtagaaatta     660
ccttctagtc tagtgtttgg tacttattac tcactgatcc actaggaaaa atatataata     720
ctaagcagga tctagtattt ttttccacaa tgatggtgaa gctaaataat tagaatagaa     780
aatttggaat acttcatttg gcattttata tctttacgtc aatttgttta tgacatgttt     840
ggttttaggt ttattataca aagaatgtaa aacaatttca attaaaaatg catactactt     900
tcttaatttc tcttaattac ttataagtag gcagtttaat aacgaaataa acctttcaag     960
acaaatggtg atctctgaaa aagtcttgga ctgatttgag gtaaaatcta tagcgaaata    1020
tgactgaggt gtcactagat tagactgctg ttaattttta tgtatcagtg tgaaaacaaa    1080
```

tggatgaaga gaaccaccta atatgaatat ttacgtgaaa ctagaagtgc cctaggatct 1140
ttgaataatt ttaaattgca tttagctaag gaagacttat ttgaggacag tactttgtat 1200
gaattacttt atcaaaactc aagaatgggc caggcactgt ggctcacacc tgtaatccca 1260
gcaacttagg gaggcagaga agggcagatc acgaggccag gagttcgaga ccagcctggc 1320
caacatggtg aaacccccatc tctactaaaa atacaaaaac tagctgggca tggtggtgca 1380
cacctgtagt cccagctact cagaaggctg aggcaggaga atcacttgaa cctggaggtg 1440
aaggttgcag tgagcccaga gcacaccact gcattccagc ctgggcaaca gaccaagact 1500
ctgtctcaaa aaaaaaaacc caaaaactca agaataataa catttacatt tctgaacata 1560
aaatagtttt ttaaaaagta tgccacttat tcaatttaca aattattact tacaacagag 1620
tttaacataa gcatgatgac atatttttac ttagcttgag agttaagtat tattacttaa 1680
aaccaggaaa catgccttta tttttggaag gagatggttt aaagttcttt atttctattt 1740
catgagtttt tcccagttcc agttcattta tccaagttgt ttttacatta ccatcaatgt 1800
catcaatttt actttcttca atatgcagaa ttccattcaa ctttgtatct atgcaatcct 1860
cagctttcac ttctctggct gaatctactt cttttatagt tctaatttca aaaaggtctt 1920
tagctaaaat gcaatctgaa ttgttagcca atctatttcc tctatcagtt tcagctaggt 1980
cattttttttc ttcatctgta tcttcaggat cataaagttc aggtgtaaaa tagatagatt 2040
catcttctgc ttctgtttca aaatctctat ttgaagtgga ctgtttatct tcttcactta 2100
ctagagacaa ttcaatgtct ggatccaggg cttcttcaga acagagcgga tgttcagaat 2160
gattttttct agtaagggtg gcatcaatct ttaatgatga aataatggtt tctgattgag 2220
ggcatgatcc aaacgatgtg tttactgtca gatttgagga ttcacattta tcagtgaagg 2280
gcaaaacagt tttactttcc atcttctctg ttttgaaacg gggaggacta gaggcactat 2340
tctctgatga cccgagctca ggtgttgcct tcggtatttt accagtaaaa tactgtccca 2400
aagaattaaa gcttgaccag ctaactctct ttgtttgttt gttgaaagtt gggcttgtgg 2460
atctggaaat cacaattttt tctgctttcc ctgcttcttc caggaatact ggatcatcta 2520
agaatacaag aatttaagag atttaacttt ctgctcctag ctaacataat tgctaggtta 2580
aaataattat ttattagaaa tacctaaata actgtatagg atacataact tagagccccc 2640
aacgaatact agtggatttt catcttggaa cagaatatta actctgaaag aaatcttagg 2700
tctctaatgt ttcctagccc aagttataat gctgtctggt aaactatcaa attccacagc 2760
agggagctca agaatgaatg aggaaagaag gggtaaaagg gaagaggaag ggagctgagg 2820
aaaggaggga acaggctgta aacatttgcc ttgaaaatcc actaaccagt gactgagaaa 2880
aacttaccta gactaattct tttatttaat aaataaggaa agtgacataa ggctaagtta 2940
tttgctcaaa gttataaaac tggttagtaa gaagaccaag actagaaact aggtttcctg 3000
attaccaagt taatcggctt gaattcacta tttatagtga attttgtatt actattttgt 3060
agtatttata tacaaaattg tattactatt ttgtagtact tatatactac aaaatacatg 3120
tttaaaaatt gatgtttttc tttttttttag tccgagtttt gcttttgttg cccaggctgg 3180
agtgcaatgg cgcaatcctg gctcactgca acctccacct cccagttcaa gcaattctcc 3240
tgcctcagcc tcctgagtag ctgggattac agacagctgc ctccacaccc agctaatttt 3300
tgtatttttt tagtagagac ggtgtttcac cacactggtc aggctggtct cggactcctg 3360
acctcggatg atccacccgc cttggatata tgaattaaaa caactgaaat tatatccaat 3420
ggaatagacc agagtttggc aaacctttttc tgtcataggc cagatagtaa tatttaggct 3480
ttgtggagca taatgtatct gttgcgacta ctcaattatg ctgttctagt gcaatacaca 3540
caaatactca actctgctgt tgtttctaaa atgtaaataa atgggcatga ctacatttca 3600
ttaaacttta tttacaaaaa caggcagcag gccaaatttg gcccatgggc cacagtttgc 3660
caatctgata cagatgatta tttcaaaata ttttaaaata ttatttttca tcacttaaaa 3720
taaatgtctc tcacctccta aaaataagtt cataagaact atctatatag gaaaagtagt 3780
tcatatagaa gtatctacat aaaaattagg ccaaattttt atatagcctt tagaataaca 3840
tctaccactt acatggtact ttacaatgct gtgatattta taattttgac ctttacaacc 3900
caacctgaag ggatagctag gcaggcattt tttttttcct attacgactc aaaaaggaca 3960
aatgacttgc tcatgatcac aaagctagta agtggcaaag ccagttagtg gaaaaccaga 4020
aaatatgctc tggtctcctg acaaaagttt gttcccatta cagtacacca tactgtttca 4080
ctattttatg ataacacctt atattacaaa ccaccatatt taaggaatta atctataccc 4140
aaataaatat catttcacta aatataatga aagacttctc tatcaaaggt aaatgggaag 4200
aacttttcat acttttctcc tttctggaga taatgctact tggtagaggt gaatttttgg 4260
taataatttt aggacactgt agttcctgga cacatatctt tgcttcatct tccacaaaat 4320
tttctggtga tagatgactt gctgcttcca gtaaataagg tgaggtactg tactttaaag 4380
aggtcacttc aagtgtagac tcattgtcct gtatattggt tctgtccttt atggatacat 4440
taagaacttt ttgatgcttt ttggaaaatt cagccaagga ttccagtgca ctttcaaagg 4500
ttgaatggtg ctgaatctgc tgccgtaccc atttagaaag tcctaaagaa aaaggtaaac 4560
ccagggaaaa tttggttact tagttattaa aatattacat gctaaggtaa tacacttgct 4620
ttttctagtg aagtaaccta atttgtattt tgaatataca gaatggttct ccatatgttt 4680
tttctgcaat tcagcaattt tactacatat gtatcagtaa catatgagta acattctctt 4740
tatatacagt aattgtcaaa tggagagagc aaagaggcag tagaagtagg gcagagatgg 4800
gaaaacatat tctctggtta agaataactg gtgaacatgg caaatttaat ttcatcataa 4860

ttccccacaa aaattgcctt tttttttgt agggaaacta gaagttgtaa acaatacaac 4920
tttccattct aatattctaa attttactcc ttttgcaaaa ataatttcaa gattctagtc 4980
tcaagttttt ttgttccctt cctccaccaa ttccaaatat atcttaaatt aaaattattc 5040
tggaggtgaa ataaataaca ctgtcttatg ggtaaatcac atcccttctg aatttttatg 5100
atataattat ttttaatata taatcaagtt ataaaggata tgatttgacc tgtgacataa 5160
aaactgcaca aaaatgaaac ctaagagttt aaggtgtttt ctagtatggt gtgcttccaa 5220
atgaaatgca aaatcaggta tttggacttt tacaaaataa atattccctg ctaacactac 5280
taaaactagc atcatctttt ggaggaatta gttttaaaag tcactatagg gtaaaaattt 5340
aagttctaga ttttaaaact aaaaaaattt taaataagcc atttcccttg aagtttttta 5400
ttttgttcat ttggggaata tgattagacc ataaaaatat gtgaaattct ctaaaattga 5460
aaatttttag gctgagtgtg gtggctcaca tgtataatcc cagcatttgg ggaggctgag 5520
gtgggaagat tgcttgaggc caggagttca aggtttcagt gaactatgat cacgtcactg 5580
cactccagcc tgggcaacag agtaagacct gtctctaaaa aagaaataag taaataaaaa 5640
taagaatttt agtactatat ttacctcttc atttcaacct ttttataatc aaagaaagcc 5700
ttttcaatgt aaaatatcat tgtctgcttt tgaaaacttg aaagaatctg agctccacct 5760
gctgactaga caccagcact tagcattttt gcctttagtt actgtttgac ctatgctact 5820
gcacacttta cgtggaaagg gaagataaaa caaataaagc aatattttct gctttatgtt 5880
ttaatatcaa accaaataca gtcattaagc tctgaaattg aaaaggtaga gtctacaccc 5940
acacatggct aatctaaaac aaaatttaac atctctttat tataataaaa tgtatttttt 6000
ttttagctaa aaggtaataa taaagataca catgtatccc atagctcttt agtaatcact 6060
cctggtattg actctgaaaa ctgtttactg ttggatgaaa ccatcgagtg agaaaaaaat 6120
gatactactg cctgcatcaa agcggtagga aaatttgtgg caccctcttg tcatgatccc 6180
cacttcagaa tatacctggc tgcttcttcc tggggagaaa agagaaaact ggaccttatg 6240
tctttttctg ggaggctgcc ccagggactg gcttctatct tacctgtact gaagagctga 6300
catgagctgg catacccctac atgcccaggg gccactaaga acaaaagcgc tgtgcagcaa 6360
gtttctgctc cacaggaacc acagtgcaac agaggcccat atagtttgat ggccactccc 6420
tcaggggac agaaaagcct ggagtgtgca tccaacatct tggcttttca aggagctgtc 6480
tagggactcg tttctgtctt gcttgtcata gagtactgac tggacctagc atactctgta 6540
tgcctggaat ctactaagaa caaaagagag tcaggcgtga tggctcatgc ctgtaatccc 6600
agcacttagg gaggccgagg caggcagatg acttgagccc agagcccagg agtttgagac 6660
cagcgtggac aacatggtga aaccccatct ctacaaaaaa tacaagaatt aactgggtat 6720
agtggtgtac acctgtggtc ccagttactt gggagactga ggtgggagaa tcccttgaac 6780
ccaggaagcc gaggctgcaa tgagctgaga ttgtaccact gcactccaac atgggcgaca 6840
gagtgagacc ttgaccaaaa caaaaaaaaa ccaagcaaat atttctaata aggaatttac 6900
agagaagtcc aaagaagaca gatccacgga aaaggtttga gaggctccca gaatgcctac 6960
cagggctgat gagtgaaggt ctttccctgt acaaaaccag ttagtaaaca ctgtgagagg 7020
tggctgtttt ttcaaatgtg tggataccat acaaggttac aagaaacata aagaaacagg 7080
aaaaaataac cctgtcaaag gaacaaaata agtctccaga aaccaaccct taaaaaaaaa 7140
aaaaagctat atgaattacc tggcaaggaa tttaaaataa ctgtcaacat gatgctcaca 7200
agttcaagaa aataatatag aaaaaaagga gaatttcaac aatgataaag aatacaaaaa 7260
agaactaaag agattttgga gctaaaaaat gcaataactg gactgaaaaa ttcactacag 7320
gggttcatca gcagacttga tcaagcagaa ggaagaatca ggaaatttat tttattttac 7380
tttattttat atattttata ttctgttatg ttatgttatg ttatgttatg ttattttttt 7440
gagacagtct cgctctgtcg cccaggctgg agtgcagtgg cgccatctcg gctcactgaa 7500
acctccgcct ccctgattca agcgattctc ctgcctcggc ctctcaagta gctgggacta 7560
caggcatgta ccaccacacc cggctaattg tcgtattttt agtagaggtg gggtttcacc 7620
acgttggcca ggctggtctt gaactcctga cctcaagtga tccaccttcc tcagcctccc 7680
aaagtgctaa gaatcaggaa atttgaaggc aagtcgtttg aaatcatcca gtcagggaag 7740
caaaaagtaa aaaacaaaat ggaaaaaaaa aagtgaaggc ttatggcttc atttatgact 7800
tgacttatgg ggcaccgtca agctgaccaa tatacacatt ctgggagtcc tagaaagaga 7860
aagagagaaa ggagcagaaa gcttatttat agaaataatg accgaaaact taataaacct 7920
gggggggagga aaatgacatc caaatacatg aagcccagtg aacctgaaca ggatgaaccc 7980
aaaagtctgt accaaaacac tttataatca aagtgtcaaa agtcaaagaa aacaatagaa 8040
atttgaaaac agcaagataa aagcaacttg tcatatacaa atgaagtccc ataagacagt 8100
cactaggccg ggcatgaagg ctcatgccta taatcccaac actttgggag gcagaggtag 8160
gagaattgct tgagccgagg agtttgagat tagcctgggc aacatagtga gaccccatct 8220
ctacaaaaaa aattaaaaat tagccaggtc tggtggtgca tgcctgtagt cccagctgag 8280
gctgaggtgg gaggatcact tgagcctggg aggctgaggc tgcagtgagt catgatcatg 8340
ccactggact cctgcctggg tggcagagta acatcctgtc tccaaaaaga gaaagatttc 8400
tcaggcgaca tcttgcatgc tgtaaggaag tgggatgaca tgtttgaagt gctgaaagaa 8460
aaaaacctgc cagtgaagga tattatatct ggtaaagcca tctatcaaaa tgaataggag 8520
gtaaaaactt ttccagataa gcaaaagctg agggacttca ccactaaacg tgccttacaa 8580
gatatgctaa aggaagcctg ctaaacagca acacaaaagc ataaagtttg ctcgtgaagt 8640

taaaagtgta cacaaataca gaatacttta atataacaac agtgatacat aaataatgtt 8700
taattctata cagaagttaa aagacaaagt atgaaaataa ctataatagt atgttagtgg 8760
atacactata taaaagggta taatttgtga ctttaataat aaaatgtgat gggaaaagta 8820
aaagtgtaga gtttatacaa ctgaaatgaa tttgttagct gaaaatattg ttataactat 8880
gtttcatgta agacccatgt aaccacaaag gaaatatcta tagaaggtat acaaaagaaa 8940
aatgagaaag gaatcaaagc atttttcttc aaaaaattaa aaaatgaaaa gcttttaaga 9000
ccagcaacaa agcaaggatg ttcattctca ctacttctat ttagcttagt actaagaatc 9060
ctagccagac tattaggcaa gaaaatgaaa taaaaggaat ccaaattgaa aaagaataaa 9120
gtgaaatgat ctctctttgc agatgacatg atcttttatg tggaaaaccc taaagattga 9180
aaaaaaacct gttaggacta ataaatccag taaagttgca gaatacaaaa atcaatgtac 9240
aacaatcact tgcaattcta tacactaaaa acaaccaaaa gaggaatgta agaaaacaat 9300
cccatttata atagtatgaa tgaagctgga aaccatcatt ctcagcacac taccgcaagg 9360
acagaaaacc aaacatggca tgttctcact cataggtgag aactgaacaa tgagaacact 9420
tggacacagg aaggggggaca tcacacaccg ggcctgttg tggggtgggg ggagaggggg 9480
gagggatagc attaggagat atacctaatg taaacgacaa gttaatgggt gcagcacact 9540
aacatgggac atgtatacat atgtaacaaa cctgcacgtt gtgcacatgt accctagaac 9600
ttaaagtata ataataaaaa taaaaattaa aataaaaaaa ctcaaaccag ggaaaaaaaa 9660
tagcatcaaa aataataaaa tacctaggaa taaacaacta aggaggcaaa aaacctgtac 9720
actggaaatt ataaaacatt gctgaaaaac agtaaagata acacaaataa atggaaagat 9780
atcccatgtt cacagattgg aacacctaat attgtcagaa tgtccatact actgaaagca 9840
atcttttttt gagacagaat cttgctctgt tgcccaggct agagtgcagt ggcgcggtct 9900
tggctcactg caacctccgc ctcccaggtt caagccattc tcctgcctca gcctccagaa 9960
tagttgagat tacaggcacc cgccaccgca cccggctaat ttttgtattt ttaatagaga 10020
tggggtttca ccatgttggc caggctggtc tcaaactcct gacctcgtga tccacctgcc 10080
ttggcctccc aaaacactgg gattacaggc gtgagccacc atgcccggct gcaatctaaa 10140
ttcatatgga accacaaagg accacaaata ggcaaaagaa ccttgagaaa aatgataaaa 10200
gctgcaggca tcatacctcc cgattataaa atacattaca cagctgtagt agtcaaaaaa 10260
gtatggtact ggcataaaga gaaccatata agccaatgga acataataga gagctcagaa 10320
gcagatccac acatatatgg tcagcggatc ttcaacaggg tgtcattggg gattgatatg 10380
ctttggttat ttgtttcctc caaagctcat gttgaaatgt gttcccccat gtcataggtg 10440
gggcctagtg ggaggtatgt gggtcatggg gacagaaccc tcatgaaagc cttggtgcta 10500
tgattgtaga ctttctgagg tcctcattag aagcagatgc tgatgccatg attcttgtgc 10560
agcctgcaga accataagcc aaataaatct cttatcttta taaattatcc agtcttggta 10620
ttggcaatga ttgtttggat atgacaccaa aaacacaggc aataaaagca aaattagaca 10680
aggaagacca tgttaaacta aaaaacttct gtgcagcaaa gtaaataatc agcagagtca 10740
aaaggcagcc tatagaatgg gagaaaataa tttgcaacgc atatatctga taaagagtta 10800
atccccaaaa tatataagaa actcctatat ctcaatagaa aaaacaaaca aaaaaacaca 10860
ccaaaaacca aaccaaaaaa acagatatgc aaagaaatcc aaataaccca attaaaaaat 10920
gagcaaatga aaagatgttt ctccaaagaa gacatatgat ttggccaaca ggtatagaaa 10980
agatgcttaa catccctatt catcagagaa atgcaaatca aatccacact gtgctatcac 11040
attatacctg ctaggagagc cattatgaaa ttttaaaaag aaaaagaaga aaaaaggaaa 11100
taagcattag ggaggatatg gagaaactgg aacccttgtg tgctattggt gggaatgtta 11160
aatggtgcag ttgctatgaa gaacagtatc aaatcaaaaa attaaactta gaactatgat 11220
gattcgccaa atcctacgct tggtatttgt tcaaaaaaat tgaaatcagc atcttgaaga 11280
gatatctgca ttcccatgtc cattgcagca ttgttcccaa tatccatgag gggaaacaa 11340
cctaaatgtc tactgacaga tgaatggata aagaaaatat ggtataatgt atacataaaa 11400
taaaatatta agcagtcatt gaaaagaagg aaatcctgca acatagatga accttgagga 11460
cattatgcta actgaaataa gccagttaca gaaggacaaa tactgcataa ttcaatttat 11520
atgaggtatc caaaacagtc aaactaatag aagcagaaag tatagcggtg gttgtcagtg 11580
gacagggggg agagggacat ggggcattgc tgttcagttg gtgttaagtt tcagttatgt 11640
aagatttaaa agttctagat atctgctgtg aaatactgtg cttatagtta acaatattgt 11700
actgtgcact taataagata gtagagctca tgttatgtgt ttttcaccac aataaaaata 11760
tgaagattgt tactagtttt tactctaagc ccagctgaga tcttaccaga tatatagcga 11820
cttgggttat tcctaaagcg atcatccact agaataagag ctccccaatc atttctgtgt 11880
ctaatacatc tagaaaaaat agggaaaaag tcaaataatt ataacatcgg aaataaatcc 11940
agttttccag tgggacagac agaaaattgg aaaaaaatca attttataga ttcctttaaa 12000
caatttgtta ttatcagaaa agttacagaa gctatccaac acaatgtaac aaactagatg 12060
tattaaaaat tccctacttt ttccaaatac agataacttg catccaaaat gttgtatttt 12120
attttgcact caagaaaatt atagaaaata tattctaaga tctttcttag ccacatagac 12180
tgttttcttt tttttacctt aagtattata aataattgta aaggtccttt ttactagttt 12240
gatgagagtt ttaaatcatg aataaacatt taattttgtc aaaaatattc taaaattcaa 12300
ttgacataac atgtagtttt tcttcttttg actgttaata tggtagatta cactggttca 12360
ttttcaaaca ctgaatcatc cttgcattcc tagtcatggt gtattattct ttttatagat 12420

tgttggattc aatttgctag tttaaaaaaa cacattaaaa ataaaaacat actttttaat 12480
aattattaaa caattttaaa gtgcacaatg caatggtttg tggtatattt acaaggctgt 12540
ataacctata ccacttatct atcccagaac attttcacca ccccaaaaag aaaccctgtg 12600
cccactaatt gttactccaa attatcccaa ttattcccca ttacctctgg caaccactaa 12660
tctttctgtc cttagagagt tgccaattct ggacatttca tttaacggga atcagacaat 12720
atgtagcatt ttgtatctgg catatttcac tgaacatgat gttttcaaga ttcactcata 12780
ttgtatcatt tctcagtact tcatttataa gttaatgttt ccacttttg gctagtatga 12840
ataatgctgc tattaaacat ttgggtacaa gtttttatat gaatacatgt tttttattct 12900
cttgggcata cacctagaag tggaattggg gagtcatata gtaattctat gtttaacttt 12960
ttcaaacact gccatactgt tttccaaagc agctgcacat tttacattcc caccacctac 13020
gtacaagggt tccaaaatct ccacatcctc accatcattt gttattgtat acccttctaa 13080
aaatatatct accctagaaa gtgtgaaata atatttcatt atggctttga tttgcatttc 13140
cctaacaacc aatgatgttg aacatcattt tttacgtgct tactggccat ttgtgtatcc 13200
tttttttggag aagtctgttc aaattattgg tccattttta atggcccatt ttgtcttttt 13260
attattaagt tgtaagagtt ctttatatat tctggatact agatcctgct acacatgatt 13320
tacaaatatt ttctccatat gtgagttgtc ttttccattt cttgatactg tcctttgaag 13380
aataaaaatt tgaactgtaa caaagtccaa tttatctgct ttttctttgg ttgcttgtgc 13440
ttctgatagc agatctaaga aactattgcc taatacaagg tcaagaaaat ttatacctct 13500
gtttttgcct ttttctgtct ttgatccact ttgagttaac ttttgtatat ggtgtgaggt 13560
aggagtctaa tttcattatt tgcacagata tccagttgtc ccaacaccat ctgttgaaaa 13620
gactattctt tctttgagtt gtcttggcac tcttgtcaaa aaccagttca ctgtagatgt 13680
atgaatttgt ttatggaccc tcaattctac ccattgatct atacatctgt ctttatgcca 13740
gtacaacata gtcttgatta ttgtagcttt gtagtaagtt tgaaatagaa aactgtttgt 13800
tctcctagta tgttcttctt tttcaagatt gttttgacta ttctgggttc cttgcttttc 13860
cataataatt ttagaatcag cttctcaatt cctgccagaa agtcagctgg gattttgatt 13920
agtattgtat ttaacctcta tatcaatata ggggagtgtt gatatcctaa tgataagtct 13980
tctaatccat gaacgtggga ttctttccat ttatttagaa ttttctttca ataatgtctt 14040
gcagttttca gtatacagat ctttcatctc cttagttaag tatatttcta agtattttct 14100
tctttctgat gttattttaa atggtatttt ttaagttcat ttttagattt ttggctgcta 14160
gtgaataaaa atgtcattga ttttttaata ttggtcttgt aacctgaaac cttgctgaac 14220
ctatcagttc tgttatttta aaaatggatt tcttagaatt ttcttagaat gtaatatatc 14280
atttgcaaat aaaaatagtt ctgcttcctc ctttccaatc tgtatacctt tctttcttct 14340
tcttgtctca ctgccctggt tagaacctcc aatacaatat tgaatagaag tggtaagagt 14400
gaaaatcact gtcgtgatct tgaacttagg aaaaacattt cagtctttta agattaagta 14460
tgatgttagc tgtgagcttt taatagatga cctcattaaa gctaagtaag taaccttata 14520
ttcccacttt gttgagtatt ttcatcatga aaatgtgttg aatactgttt tatgcttttt 14580
tttttttttt gcatcttgag aagatcgtgt ggttttagtc ttcttttaat atggtgtatt 14640
acattaattg attttataaa gcatgttgaa ccagtcttgg attgctggga tcaatcttac 14700
ttggtcatgg tgtataatcc ttttagtatg ttgctgattc tgtcaatggc ttttatttct 14760
atagtcatat gaaatattgc tgtacaatct tcttttctcg taatgtattt tacttgtttt 14820
agtatcaggt aatactggcc acatagaatg agttggtaaa tatttctctc ccattttttg 14880
ctagagttaa tgaaagatta atgcaaattc ttctgtaaat ttttggtaga attcaccagt 14940
gaagccattt gatcatgagg ctgaggcaga agaatcactt gaacctggga ggtagaggtt 15000
gcagtgagct gagatcacgc cactgcattc cagcctgagt gacagagtga gactttgtct 15060
cacaaaaaaa aaaaaaaaaa ggccaggcgt ggtggctcat gcctgtaatc ccagcacttt 15120
gggaggccaa ggtgggcaga tcacgagatc aggagatcga taccatcctg gctaacacgg 15180
tgaaaccctg tctctactaa atatacaaaa aattagccgg gtgtggtggc gggcacctgt 15240
agtccccagc tactcgggag gctgaggcag gagaatggcg tgaacctggg aggtggagct 15300
tgcagtaagc tgagattgcg ccgctgtact ccagcctggg caacagatag agactctgtc 15360
tcaaaaaaaa aaaaaaaaaa gggggggga agtgttttga tgacaaattc tatctttact 15420
tgttatacat ctattcagat attctatttc agctggtcgc agtggctcac acctgtaatc 15480
ccagcagttt gggaggtgga tcacgaggtc aggagttcaa gatcagcctg gccaacatag 15540
tgaaaccccg tctcttctaa aaatgcagcc gggcatggtg gcaggcacct gtaatcctag 15600
ctactcagga ggctgagaca ggagaattgc ttgaacctgg gaggcagagg ttgcagtgag 15660
ccgagatcat gccattgcac tccagcccag gcaacagtgt gagactctgt ctcaaaaaaa 15720
aaaaaaaaaa aaaaaaaaaa aaaaaaaaga tattctattt cttcttgata tattctcagt 15780
attttgtgtc tatctatgaa tttgtccatt taatctaagt tatttgtcat cacacaattt 15840
ttgaaggtat tctcttataa tcctttgtat ttctgtaagg tagtaacatc ccctctttca 15900
tttctaattt tagtgaatta gaacttctct gtattttctt tggtcagtct tggtacaggt 15960
ctgtcaattt tgttgatctt ttcaacaaat caacttttgt taccttggtt attctctact 16020
gttctttgaa ttctctattt catttatttc ctttttcatt tctagtattt cctttaattg 16080
cctcgctttg ggtttagttt gctctttttt ttttcctaat tccttaagga ggaaggttag 16140
attattgatt ccagatgttt attcttttaa aatacagatg tttacagtat tttttttttt 16200

tagacagagt ctcgctctgt cagccaggat ggagtgcagt ggcatgatct cagctcactg 16260
aaacctccgc cccctgggct caagcagttc tcctgcctca gcctcccaag tagctgggat 16320
tacaggcatg tactaccaca cctggctaat ttttgtattt ttagtagaga cggggtttca 16380
ccatgttggc caggctggtc tcgaactcct gacctcaggt aatctgcccg tgttggcctc 16440
ccaaagtgct aggattacac acgtgagcca ccgtgcctgg cctagatgtt tacagctttt 16500
ttaagtttcc ctgggagtag tactttcact gtgtccccaa aattttggga cattgtttcc 16560
attttcattt atcacaatgt atttttcaat ttttcttctg atttcttctt tgacccactg 16620
gttgtttaaa agtatatcat tgaatttcca tgtatttgtg agttttccat ttttcctccc 16680
attattgatt tctggtttat ttcattgtag tcagaaataa tacttgtaat gatttcaacc 16740
tcttaacatt tatggaacct tatattttat ggatgaacgg atggcctatc ctgaagaaca 16800
tgccatgttc acttgagcag aatgcgtatt ttcctgttgt tgggtgaagt gtgctacaga 16860
tgtctgttag gtttgtttgt agttgttggt ttgtagtttt gttcaaggct tttattttct 16920
gttgattttc cttctagttg ttctacccat tattgagcag ggtatggaag tctccaagta 16980
ttactaaatt attgatttct gatgcttttt gctagatgtg ttttggggcc tttttgttaa 17040
ctttgtatgt ttataattgc atatttagac ttgttatttt tttttatttt ttatttttat 17100
ttttttttga gacagggtct tactccagtt gcccaggctg gagtgaagta gtgaggtcat 17160
ggcttactgc agcctcaact tcctgggctc aggtgattct cccacatcag tctcctgaat 17220
agctgggatt ataggcacat gccactacac ctggctaatt tttttgtact tttagtagag 17280
acagggtttc atcatgttac ctaggctagt cttgaactcc taagctcaag cgatctgccc 17340
acctcagcct cccaaagtgc tgggattgca ggcagaatgg cacttttatc attataaaat 17400
gttaattgtt tctattaact tttttttgtc ttaagggtct attttatctg atattagtat 17460
aaccaaacca gctctctttt gtttactatt tgcatggtat gtcttttccc atcattttat 17520
tttcaaccta tttgtgtttt ttaaactagt gtttctcttg cagacagcat atatcacatt 17580
gttttcaatc cattcagcca aactttgcct ttagattgga gtatttaatc catttacatt 17640
taatgtaatt actgataagg taggatttac tcagctattt tgtaaaatac tcactttgct 17700
atttgctttc tatatgcctt acgtattttg ttcttctgtc tttccattac tttccatttt 17760
ctccaataga cactttccag tatataattt taattctctt gtcatttaca ttactatatt 17820
ttcttatttt tattagtggt tgaagagtat aataaacata tcaaccagta ataaactagt 17880
caggaaaatt gccaacttaa tttcaatagt acaaaaaaat ggtccttctg ttccctcatc 17940
tgtcaccttt gggttgttat tgtcatataa attatatctt taaacattgt atgctggtgc 18000
agaagaatta acattgtagg ttggattgtt taccccctaga aaggtctgtt taaaggtgtt 18060
cctgggctga tgaatgaaaa cttagattca ggagggtacc tgtgattgac tgataagagt 18120
gtctcactgt gcctgaactg tttgagtaaa caatatatta atgctgaata ctcattctcc 18180
ttttgagagt ctagtcttct ggaaactgct aggcagaggc tgcctatatg accagcccca 18240
gtaaaatccc tgggtgctaa tgagttcctc tgattggaaa tatttgacac atgttgccac 18300
aactcattgc tcggggaagt gtgtcctgtg taactgcact gggagaggac acttggaagc 18360
ttaagactgg ctccctagac tttgtcccat gcacatgttt tgttctcttt gctgactgtg 18420
cttggtgtcg tttcactgta ataaatccta gccatgtata cagttatatc ctgaggctat 18480
ctaactgttc caagtgaatt gctgaacctg aaggtggtct tggcaaccca caagacaatc 18540
accacagatt tacaattatt gttttatgca gttgctcttt taaaaaatca agagtaaaag 18600
aattataagc aagaagtacc tttatattaa cttttacatt tacgtatgtc gttaactttg 18660
tgggtattct ttatttcatc gtgtgcatct gacttacttc ctaatatcct ttcatttcag 18720
cctgaaagac tccttttagt atttctcgta ggacaagttt gctacagagt aattctcttg 18780
gtttttgttt atttgggaat gtcttaattt ttaccttatt tctgaaggac agttttgtta 18840
gatatagtat tcttggttga cagtcatttt ctttcagcac tttgaacatg tcaattaact 18900
gcttcctggt ctggaggatg ttttgtatat gatgagttat ttctctcttg ccgctttcta 18960
gattctgtct tctggctgtt tgatttcaac catctagata ggaatctccc tgagttcatc 19020
ctacttggag ttcgcttatc ttcttggatg tataaattaa cgtttttcat caaattcgga 19080
acgttttcag ccataatttc tttaaatatt ctttctattc ttttttctct cctctccttc 19140
tgggactcca aatatgcata tgttggtacg cttgatgagg ttttacaggt ctctgagatt 19200
atgctagttg ttcttcattc tttttctttc tgttcttcag agtaaataat ctcaattgat 19260
gtttaagtaa atcaattctt tcttctgcct tcttaaatct gctgttgggc ccctgtaatg 19320
aatatttcat tttagctatt atactttcca actccagaat ttccattagg tttttgttaa 19380
gcaaatttta tctctttatt gatattccct atttagtgag gtgagacatc attctcacag 19440
tttttgacac agtttgctta gttattgaat gtatttgaac tagctttctc tcctaaggca 19500
tagctttgtc tactaagaca aatgtctggg tttcctcagg gagagtttct attatttttt 19560
tctcttagtt atactttctt tgttatgtgt catgattttt gttgaaaaca gtacatttta 19620
aataatataa tgtggcaaat atagaaatcg gattattctc cctcttcaag gtttttttgtt 19680
cttgtaattt ttctaaaata atcttgtaaa gtctgtattg tcatgtgtgg tcattgaaga 19740
ctgctcgatt aacttagtag tcagctaata attagacaga gatttcctta aatgtctaga 19800
accatcatgt ctcccattct ttgccaagag tctctgcata tattggggca taatttcaat 19860
cctcagcact gtaggtaata actctgatct tgttttcact tcctgcttca gcagagcacc 19920
aaggtgatag cttagggcct tttcaggtct tttcttagca tgtccacatc actggtcaca 19980

tgcactctcc tacacatatg catggcccac taaaatccca ggaatatgtc agaactttaa 20040
aagctctcta tggacattgc attcctcagc ttttctcttt acatttttg gctagcctct 20100
cacttgcccc aaatgtcatt cattgtctca ggcaactgtg aagttaaaca gctggctggg 20160
tgagctctga gtcaggtcaa ataaagatag ccttgtgagt ggggtcttcc aggaaactcc 20220
caggtaggtc acataattct ctggaaatgt actccagacc tgttttgctc cctccaatga 20280
ctgtcaggtt gttggctttc ctcacgattt caggctattg gtttttaagg ctattgtgaa 20340
cttggagaga ggtgagaaca gagcaagtta aaataccaca aagcttactg ttcttactgc 20400
aattcagcta tttttaagga gtaaattctt cctagaatgc tgtaactttt ggttaatacc 20460
cagagttttg ataaagttgc ttctaactta tttttgccta ttttctcatt gcttttagaa 20520
atttcagagg tctttgttct gccattttg aggaccttac ccaatttgct ggcactctgt 20580
tgaggacatc ttcatctatg tttcagaggg atattgatct atagttgttg ttgttgttgt 20640
tgttgtttta acttttattt tagtttcagg aatacatgtg caggtttgtt atagaggcag 20700
attgcatgtc atggtgattt gttgtgacaa attaggaccg attattttgt cacccaggta 20760
aaaagcagca tagtatccaa caggtagttt tccttgaatg gtagctcaac tcttagttca 20820
aacagcttaa ttttaagctc tccaccctca agtaggccct ggtgtctgtt gttcccttct 20880
ctgtccacat atactcaatg tttagctccc gtgtataagt gagaacatgt ggtatttggt 20940
tttctgttcc tccgttagtt catttagaat aatggcttcc agctccatcc atgttgctgc 21000
aaaggacatg atcttactct tttttatggt tacatagtat tccattgtgt atatgtacca 21060
cattttctta atccagtcta ctatcaatag gcatttaggt tgattccacg tctttgctac 21120
tgtgaatagt gctgtgataa acatacagat acatatgtct ttatgggaga atgactcaca 21180
ttcctttggg tatacaccca ataatggaat tgttgggttg aacagtactt ctgctttaac 21240
ttctttcaga aatcaacaaa ctgctttcca cagtggctga actaatttac attcccacca 21300
gcagtacata tgtggtcctt tttctctgta accttgcgag catctgttct tttttttttt 21360
tttttttga ctttttaacg atagccattc tgactagtgc gagatgttat ctcattgtgt 21420
atgtatgtgt ttttttgttt ttgtttgttt gtttgttttg agacagagtc tcactctatt 21480
gcccaggctg aagtgcagtg gcacaatccc aactcactgc aaactccacc tcccgggttc 21540
aaacgattct cctgcttagc ctcccaagta gctggaatta caggtgtgcg ccacaacacc 21600
cacctaattt ttgtattttt agtagagatg gggttttgcc atgttgggca gagtggtctt 21660
caactcctga cctcaagtga cctgcccgcc ttgacctccc agtgctgaga ttataggcgt 21720
gagccaccac gcccagccac attgtggttt tgatttgcat ttctctaatg atagtgtgtt 21780
gaacattttt tcatatgctt cttggccaca tgcatgtctc cttttgagaa atgtctgttc 21840
atgtcctttg gccacttttt aatggggttg tttttttgctt gtcaatttaa gttccttatg 21900
gattctggat attagacgtt tgtaagatgc atagtttgca aacattttct cgcattctgt 21960
ggtttgtctt tactctgctg atagtttctt ttgctgtgca gaagctctta agtttaatta 22020
agtcccattc gtcgatttttg tttttgttgc aattgctttt ggtgtcttca tgatgaaatc 22080
tttgctagag cctatgtcca gaatgatatt tcctaggttt tcttcaaggg tttttagttt 22140
aaggttttac atttaagtct ttaattcacc ctgaggtggt tgttgtatat agtgtaagga 22200
agggggtcca gtttcaatct tctgcttacg gctagccagt taggtcccac ttgtcaattt 22260
ttgttttgtt gcaattgctt ttgaggactt agtcacaaat tctttgccaa atctgatgtt 22320
tagaacagta tttcctaggt tttcttctag gatttttata gtttgaggtc ttacacttaa 22380
atctttaatc catcttgagt taattttaat ccatcttgag ttgattttg tatatggtaa 22440
aaggtagggg gtgcagtttc attcttttgc atacagcaag ccagttactc cagcaccatt 22500
tattgaacaa ggagtccttt ccccattgtt tatttttgct aattttgtca aagatcagat 22560
ggttgtaggt gtgcagtttt atttttaggc tcttatagtt ttcttttttc cccctttttcc 22620
ttttgttttt gtagtcttca tctggtttgg ttgctggtta attctggtct cataaaataa 22680
attgggaagg attgcatctt ctgtttttta gaagagatta tgtaaaattt gtgttatttc 22740
ttctttaaat gtttggtaat atttgctagt gaacacagct gggtgaattt tttagaaggc 22800
ttttaactat taattctatt tctttgctag atagctatgc gtgttatctg tttcatcttg 22860
gcctcataaa ataaattgag aaggattaca tcttctattt tttagaagag atcttgtaaa 22920
atttatgtta tttcttcttt aaatgtttgg taatatttgt taatgaacac atctgggtgg 22980
agaatttttt agaaggcttt taactatgaa ttctatttat ttgctagata gctattcatg 23040
ttatctattt catcttgggt gaattttggt attttgtggg ttttgtggaa ttaatctact 23100
tcatctaagt tatcaaatgt atatgtatag agtttttcgt agtattctca tattattctt 23160
ttaatgcctg tggagtctgt agttatacta ttaatacctc ttttattcct gatattagta 23220
actgtggctt ctcttttttt tctttgttaa ttttgctaga ggtttatcaa ttttattgat 23280
cttttcaaag agtctatttt tttctgtttt tcaactttta ttttatttta gattccagga 23340
gtacatgtgc aggtttgtta caaaggtata ttgcatgatg ttgagtttgg agtacaattg 23400
aacctgttac ccagaaagca agcatagaac acaataggta gtttttcaac ccttgcttcc 23460
ctccttctct gcctccactt acattcattc cctagtgtct atgttcccat ctttaagtcc 23520
atgtgtaccc attgtttatc tcccatttat aagtgggaac atatggtatt tggttttctg 23580
tttctgtgtt agtttgctta ggataatggc ctccagttgc atccatgttg cggcaaggac 23640
atgatttcat tcttttttaa tggctgtgca gtattccacg gtgtatatgt accacatttt 23700
ctttatctag tccaccgttg ataggtattt gagttgcttc tatgcctttg cttttgtgaa 23760

ttgtgctgtg atgaacacac agctgcatgt gtctttttgg cagaatgatt tattttcctt 23820
tgggcatata accagtaatg ggattgctga gttgaatggt agctcaactc ttagttcaag 23880
cagcttaatt ttcatttcac tgatttcatt atttttgtta caattcatca atttatactt 23940
gtttttatta ctcctttttt tcctgtttgc ctcacattta tttagctctt ctttttctag 24000
cttcataagg tagaaactaa gatcaatgat ttgaggatgt tttttcctaa aataagcatt 24060
taatgacata aatatccatc tgagcactgc tttagctgca gtccttacat tttgatatgc 24120
tgtattttca tttttgatca gttcaaaata ttttctaatt ttacttgaaa cttgctgttt 24180
tgcctgtgga ttatttagaa atatgctgct taatttccaa gtatttggac atttttctgt 24240
tattgatttc tagtttaaac tagattacta gtttaattcc attacagtca gagaacatac 24300
tggacttgag ttggctaata atgttgctca agtcatctat atctttatta gtttgtctac 24360
tagttctgtt attgagagga attttgaagt ttccaagtat aattgtgtat ttttctattt 24420
ttctcttcag atctgtcagt ttttgcttca tgtattttga aaccctgttt tggggtgcat 24480
acacatttat gattcttatg tcttcttggt gaattcaccc ttttatcatt atgtaatgtt 24540
ctcttttatc cctaattttg tttgcttgga attctacttt atctggtatt atacagttac 24600
ttgagctaga tcattggtat tttgaattct ggtctccttt cttaatctac ctgctagtgt 24660
tgacttttca gagttctcaa acagctactc cacatattct gtctaggttt tttagactac 24720
taaatatgct tacttcatct tttctaggac tgaaacgccc aaactatttt ttaaagttaa 24780
ttttagcttt gtaaatttta acttttatca aagaaacacg aacacatagt tttatgtttt 24840
caatgaaaaa cataactgtc ctttccctac ccacctcctg tttacaattc ccattcctca 24900
gaaacaatta ctttcaatat tttaggtaga tcttctggtt atttatctcc atatttctaa 24960
ggaacattga tcctactact acttcttggt ttttgaactt tagaacttac gtactgattc 25020
tctcctgtgg aagatgttta tttctcttat accactgata cttcaccatc ccttcctggg 25080
aatattaaca actgagtcac atagtgcact ataattagat ttcctttatt gtacaagact 25140
ttttgccttc cctggagtta ataactgcct cttctttttg tggattaatt tttaatgttt 25200
tctgtcatta attaatcccc aaaccatgcc atagctgtaa aaattcttcc aagtatggtc 25260
aaaccttcta tcctctgaac tcaatttaga ctacttgctg cactgttatc attccagagt 25320
tttccttcct ggttgtttta ggaattatct ttgcctctat tctggattgg aaccctagtt 25380
ccctgcatcc ttgatattcc tttttaaatt tttttttttt attttgatgg agcacatctt 25440
ccagtaacat cctgagaaag tgtccatttg aagcaaatgt tttaggtcct tgtatgttga 25500
aggctttatt ttgtcttata gtttattgat agtttggctc atgtagaact taagttacaa 25560
atcaaaaatt tccctaaaat atttgaagac aatttcttct atttttttgg tttcagtgtt 25620
gcttttcaga agtctgttat gattctgatt ctctatcttt tgtaagatag agacctgtct 25680
ttttgtcttt ggaagatttt aagacttttt ctttatcccc ggtacttgga aagggcacag 25740
tgaagtgctt tgtagctttt cctcctttac ctgtgcctga gtgagcccct tcaaccaaga 25800
aatcgtgtta ctcaattctg agaaatttct ttgagttatt tctttgagat gtttctttcc 25860
tttatttttt ctgttctcat gatctgagcc ttctatgtat cagaagttgg ttattgcata 25920
ctgatcctct aattttctag ttcttctttc ctgctttctc tttttgatct ggaaaatttc 25980
cattttatct tccaagccct attatgcatt taaaaattgt tggtatcatg ttaatttcta 26040
agcacttttt cttattcttt tgttcttgtt tcatggaggc aatatggtac atctctatgg 26100
atattaagat tttttttga agatttctcc tatctggggg ttggcaaact atagcctata 26160
gtgcctacaa aaatagccca tgtcttgttt ttgtacagcc tccaatgtgt aggaatggtc 26220
ttcacatttt tttttttttt tgagaaggag tctcattctg tcgcccaggc tgaagtgcag 26280
tggtgcaatc tcggctcact gcaacctccg cctcccaggt tcaagtgatt ctcctgcctc 26340
agcctcctga gtagctggga ttacaggcgt gcgccaccat acctggctaa tttttgtatt 26400
tttagtagag atggggtttc accatgttgg ccaggctggt ctcaaactgc tgacctcaag 26460
tgatcatttt taagaggatt aaggaaaaaa caaaaaatat gtgacagaga ctgtatgtag 26520
cctgcaaagc ctaaaatata cacaatagtc ccccacagcc acaatcttgc ctatggtcaa 26580
agttactcac ggtcaaatgt agtccaaaaa tattaaatgg aaaattccag aaataattca 26640
tacatttaaa aatgcatgcc cttctgagtt gcatgataaa aatctcatgc tgtcccactc 26700
aggatgttaa tcatcccttt gtccaccatg tccatgctgt atactctttt tgcccattag 26760
tcacttagta gccatcttgg ttatcagata gaaaaaacat agtatacgta gagtttggta 26820
ctatccatgg tttcaggcat ttagtggagg ttgtggaatc tatcttctgt ggataaggag 26880
agactactgt actgtctggc cctttataga aaacactggc caatccttgt tctagtctgt 26940
tgtgttgttt ctattttctc agagttcttc tgctgtgttt ttgtttgctt tgttggactt 27000
gttttgttgt tgttgttttg gtctgttttt catgttacag actttctttg acaatctggt 27060
gaagactctg cacttactta agagtaaggc actaaaacac tgattggcag ctctgaatgt 27120
atgggtagag cttataggct gggaagtttg ctatagggta atttggcagg aactgggcca 27180
atttgtagat attttctcat ggggtggtca gtttccctaa aaaggaattc tacacgcttg 27240
gtagatgtaa gactgattgt cagcctactc agagctgaaa agaaaagggg ggtggagttc 27300
tcacattcag tatgtaaaat ccccgtatat aatattaacc tccatcttta atacgtcctc 27360
agctgtgcct ggtgttgaag aatacagagt ctctgttgtt caacctttcc agaagtaaac 27420
ctgggtgagg gaggaggcta attgtttctt atactaattt gcaaccaagc cacctgtttt 27480
taaaccttac tttcattgac attttagatt gctccaaatc ctaggctttt tcatgcataa 27540

atctgaaagt gcttgttgac tatcatcaat gtagatttaa atttcagctt tctcttgcct 27600
gctaagtcat tcagtcattc ggctgttttc cagtgttcaa aatgtgttga tatcgcttgt 27660
ctgtcactgt ctcctctctc tctttgtaat attgtaggtt tgacattttc atttctttat 27720
tatcatagga ttttgagggg gtgaagagta acataaatgt tcaatttttc tacaaagaca 27780
aaaaaaatct aaattttata gggcattatt ttttgatatt attttaaagt aacctatagg 27840
ctccaaggca acagaccata acactgggaa ggataggaag aagattaata ttgtcaatac 27900
tacagaagag tattgaaagt caatggaaag tcagtgtgcc actgtatttt ggttccagtt 27960
tgatttatgt ttaaagcatt ctatggtatg gtagaaaggt atttctgtta atgaaacaat 28020
tcaaagaaaa aagatttcac cttttctttt taccattgtt ttcaaactgc cttgcttaaa 28080
tgaattatgc cggcttgaga caaagattat actttcagta aagcaacaaa gactagttag 28140
aaaactcata gtgcagttgt aacctggttg caaaaccatc ttgtatgttt ccaaaggctg 28200
ttcttgtatt tcatagactt atattacttc aaatactcta acagtcccat gtgtgcttaa 28260
aaaaattaac attaaaagga atccattggt ttctgcttac tttagatctt ctgtatccct 28320
tacaagaaga gtgcctcaga gtataagcac cttttctgtc aatcaatata cattatgagt 28380
tttattaaat aacataaaac atttgcttgg tctttatttt tttaatttaa aaaattttac 28440
ttattgattg gttttaaagg gtaagttcct ttgtaacaaa ttattaatta ttgacattat 28500
tccctgaaaa tcagtaaaat gatagctggg ctctccattt aaaaaaatta ataaaattct 28560
aaaagtacag tattatatat caatactata aatacatgat ttttatgata ttccaggaaa 28620
gcatgttttg aagctctaaa agctctctgg gcaatgttgt atggcaggaa aaagcactga 28680
acctcacatt tttcactaat tcaacaagca tttgagactt agttggttgc tgagaatgta 28740
acaacgtaac acatgttcaa ggaagaaaac agataggcta ttacaataca atgagatatg 28800
tgctataata cagatagcaa ccaaaatgct ataggtttac taaggtataa ataactctag 28860
ttggagaagt tttaaaggtc ctcataaagg acatgacttt caatgaggtt ttgattgcta 28920
aatttgccaa acgaattgta tgataactta taagccaagg caaagcataa tgaaagaata 28980
tagcatgttt gatagtttaa tactgacata cttatgttta atagtgaaaa attcggtggc 29040
tagaggtaga ggataaggat gaaaaagcag gtattacagt tttagaaatg gatgggaaca 29100
agatgtaaag ggccatttag tagccatgtg gccttagaaa aataatttaa tttctcacaa 29160
ccagtttaaa aacaattcag gctgaggcag gtggatcatc tgaggtcagg agtttgagac 29220
cagcctgtcc atcatggtga aaccccgtct ctactaaaaa tataaaaatt agctgggcat 29280
ggtagtgcgt gcctgtaatc tcagctactc aagaggttga ggcaggagaa tcgcttgaac 29340
ttgggtggag gaagatgcag tgagccaaga ctgcaccact gcactgggcg acagagcgag 29400
actccatctc aaaaaaaaaa aaaaaacaaa cccaaaaaaa cccaaacaaa caaacaaaaa 29460
caaagcaaat caaataaaac atcaagtagc aacatgcatt gctactggaa gtgattggta 29520
gaagaatcaa atgagataat atatgtggag aaggtttgta aacgagaaaa ccacatacaa 29580
atatgacttc tgtttcacat ggtttcttga aattcaaggc attgcttata ctaaaaggaa 29640
gaaaaaaatc aactgagcta agaatactta ttttccgcaa attgagtata atgactaaaa 29700
gtcaacaatt tcaaaaatat attcaagcgc ccctagaaac tattagctga atattaaaat 29760
tcaaaaaaaa aatgtcaatc agcagggaga cacaaagtaa gcagatgtga tgcttcaata 29820
accttcagtg caacttgatt ccaagtaaaa tgggatttct agggaaaaaa ggggagcagg 29880
atctctgtag cttaaccata gacctgaatc actcagtttg aagaaaaaca catgcagaat 29940
accattctaa aatgatgggg agtggctggg tgtggtggct cacgcctgta atcttaacac 30000
tttgggagcc tgaagcagga ggatcacttg aggccaggag ttcaagacca gcctggccaa 30060
catagcgaaa cccgtctcta ctaaaaatac aaaaattagc caggcgtggt ggcatgtgcc 30120
tgtaatccca gctactcagg aggttgaggt aggagaatcg cttgaacctg ggaggtagag 30180
gttgcagtga actgagatcg caccactgct ctccagcctg ggaaacagag caagactctc 30240
tcaaaaaaaa aataataata ataataaaaa caaaaataaa aaataaaatg atggggagta 30300
cagtgggtga catgttctag ggcttaaatc tagttgttgt agtcttatgt ctaagctata 30360
cacaaataca catagcttat gtatttgtat atatatacat atagctatgt ctaagcgata 30420
cacaaatctg aacccacatg ccatctggtt tacaaatggg ctaatgtaaa tacacaagac 30480
accagaaaaa taaaaatttt ttatttgact ggttaagatt atgattagtt gtgggacttc 30540
tggttgaagt gcaaatatgt caactgatca tgttttcatg attttactct taaccaattt 30600
ctttttttta gatggagtct ccctctgttg cccaggctgg agtgcagtgg cgcaatctta 30660
gctcaacgca acctccacct accaggttca agcaattctc ctgcctcagc ctccccagta 30720
gctgggacta cagacacaca ccaccatgcc cagctaattt ttgtattttt agtagagacg 30780
gggtttcacc acgttgacca ggctgatctc aaactcctga cctcaggtga tccacccgcc 30840
tccacctacc aaagggctgg gattacaggc atgagccacc tcacctggcc caacctgttt 30900
ctatgaatta tccaaatgcc ttacagcttc actaggaaat aaataattac atggtgaagt 30960
taattaaaat ttcatagaga atataaaata ttaatttgtg ggccgaatgt ggtggctcac 31020
gcctgtaatt ccagcacttt gggaggccaa ggcaggcgga tcacaaggtc aagaaatcga 31080
gaccatcctg gccaacatgg tgaaacccca tctctactaa gaatacaaaa attagccagg 31140
cgtggtggtg cgagcctgta gtgccagcta cttgggaggc tgaggcagga gaattgcttg 31200
aacgcaggag gcagaggttg cagtgagctg agattgtgcc actgcactcc agcctggcta 31260
cagagcgaga cttcatctca aaaaaaaaaa aaaaaattta atttgcacaa aacctaatta 31320

```
tagcatttac aaattagatg ttgatgccct acaatatgaa attaagggat agtaatgcac      31380
cacataatga tgtttcagtc agtgtgaagt gcatatatga tagtggtccc ataagattat      31440
aatggagctg aaaaattcct atcacctagt gatgtcacca ttgtaacatt atagcacaat      31500
gcattacaag tgcttgtggc gatgctggtg taaacaaacc tactgtgctg ctagtcttat      31560
aaaagtatag cacatacaat tatgtatggt atataatact tgataatgat aataactgtg      31620
ttactggttt gtgtatttac tatactgtac ttcttatcac tacagtgtac tcctttcact      31680
tacattaaaa aaaaagttaa ctataaaaca gcttcaggca gatccctcag gaagaattcc      31740
agaagaaggc attgttatca caggagatga cagctccatg catgttattt attggtcctg      31800
aagaccttcc agtgattcaa gacatggagg tagaagacag ggatattaat gctcctgatc      31860
ctgtgtaggc ctaggatact acatgagtgt ttatgtcttc attttttttt ttttttgaga      31920
tggagtctcg ctctgtcgcc caggctggag tgcagtgggg tgatctcggc tcactgcagc      31980
ctccacctcc cgggttcaag taattctcct gccccagcct cccaagtagc tgggattata      32040
ggcacctgcc accacatcca gctaattttt gtagttttag tagagatggg gtttcaccac      32100
gttggccagg ttgctatcga actcctgacc ttaagtgatc tgcccacctt ggcctcccaa      32160
agtgctggat tacaggcttc agctattgcg ctccccatgt cttcattttt aacagaaaag      32220
tttaaaaagt aaaaaaataa aaaataaaaa tttcaaaaat agaaaaagct tatagaataa      32280
ggatataaaa attattttg tacagctgta taatttgtgt tttaagctaa gtgttgtaaa      32340
agagtcaaaa aggtaaaaaa aaagataaaa agataaaaag tttataaagt aaaaaagtta      32400
cagcaagctg ggcacagtgg cgtgtgcctg gagttccagc tacttgggag gctaaggtga      32460
gaggactgct tgaacccagg tgttcgagac cagcctgggc aacatagcca tacctcatct      32520
ctcaaaaaga aaaagtcaga ataagctcag ggtaatttat tattgaaaaa ataaaaataa      32580
ataaatttag tatagcctaa gtgtacagtg tttataaagc ctacagttag tgtacagtaa      32640
tgtcctaggc cttcacattc actcactact tgatcgactc actcagagca acttatagtc      32700
ctgtaagctc tattcgtggt aagtgccata tacatttgta ccatttttaa tatactgtat      32760
ttctatacca tatctttact gtactttttc tacacttata tactcaatta cttatccttg      32820
tgttacaatt gtctatagta cagtaacatg ctgtataggt ttgtgatcta ggaacaatag      32880
gctatatcat atagcctata tgtgtagtag actatactat ctagatttgt gtaagtacat      32940
tttataatgt tcccacaaca atacaattgc ctaacaactc acttctcaga atatatccgt      33000
tattaagtga tgcatgactg tataaaataa tgatttaaaa atgagtctca caaagcacaa      33060
atcttaatgg gcaagaatat ttagaagtcg gagtatcatg attaaatgtt tagttggtgt      33120
taattcttta ggatcacgat tatagtttgt tttaaccctt cttcaatgat ttgctaattt      33180
tttttttttt ttttttgaga catggtctgg ctctgttgcc caggctggaa tgcagtggca      33240
tgattttggc tcactgcaac ccccacctcc caggctcaag cgatcctcct acctcagcct      33300
cccgagtagc tcacatctgt aatcccagca ctttgggaag ctgacgtgag tggatcactt      33360
gaggccagga gttccagacc agcctggcca acatagtgaa accccatctc tactaaaaat      33420
acaaaaatta gctgggcatg gtggcgcacg cctgtaatcc cagctactca ggaggctgag      33480
gcaggagaat tgcttgaacc tgggcggtgg aggatgcagt gagccgatat cacgccactg      33540
cactccagcc tgggcaacac agtgaggctg tctaaaacaa taataataat aataataatt      33600
aattatttta ttttttcttt aattattaaa tatccttcag tttcaatgtt atccttcaac      33660
attatcacta tacataaaag gcacaagaaa atatagattg taatcacatt gttatctaaa      33720
gcttacaaat aacattaaaa tgaaaatgat tttttttttct gtcagatgtc ttctttacat      33780
agaagcagaa tgaaaaattg tgcaactaca gaagctcact gggcagtaaa atagatttg       33840
tttctatttg atgatatttt aacagataca cagattccgt actgaaagta cactttcaaa      33900
tagaatatct cctcacactg gctggtatat caattttctt ccctgacatt ttgggggagg      33960
tgaggagaga ggtctgttta tctataaaat tgttatctga gctttgatgt tcacaagaca      34020
aaactttttt gatttgataa gctttcttta atatttactg agcatctttg tgtgctattc      34080
attgttagat caatcacaaa atttctttaa actttagcaa attgtgagtt ttgttttggg      34140
aataacttat tcttcttact cttttcaaat tctacttgtg acagctatca ttgaatacat      34200
accagttcct atggttccag ttaaataaaa ttttaccaag tttattataa agagtaaagt      34260
agcaagacta gatttatata tatagccctg tcacagataa tattatatta aatttcactc      34320
cacttaccta ccaagggcct ggtttaaggc cctgtatgct tgaatttcat accactgacg      34380
gccaggtaga agacctctca attttgaatg gtggtcattg tattgtcgtt ttagttcaac      34440
ctaataattt taaaatatat ttaaaaaatt agtagataat taaagctcat tttaaacatc      34500
atattaactt ctaacagttt gaatatacat atttttaggg tagagatttt attttgtttt      34560
tcagtactga tttttttcgt ttcatttaaa atagttttat ttagcttcac attccttaaa      34620
ttacctaatg catataacta atcagctgtg tgatctcaag cagttttatt gaacttacat      34680
tcattaaatc cacacaattt tcagtgtaca agtaggtgag ttttgacata tatagtaatg      34740
taaccaccat cataatatag aacattttca tcaccccccaa aagttccctc ttatcccttt      34800
atagtaaatt tctttcttcc actgctggcc ctggcaacca ctgatctgct ttctgttttg      34860
ccctttatag aatttcatat aaatattatc atatactatg taatgacttg tgacattttt      34920
catttaatat acttttagaa ttcattcatg ttgaaaggtt tatgagtagt tcattctttt      34980
acgtttatcc agtctggtaa tctctgcttt ttaattgcaa tgtttagacc atttacattt      35040
aatgtagtta ttgttatgat tgggtttaag actaccacct tgcttccccc ctaccccccc      35100
```

aatctgtacc attttgtttt tatcttttgg atcaatcaag ttcccccctc cccccatatt 35160
atttccacta ctggaaataa tatgcttcca tattatttcc actactggat tattagctat 35220
gtctttgtgt gtgtgtgact cctctagggt ttatgatata catccttatt agtctacctt 35280
aaaataatat tttaccactt cacatatgat gtaaaaacaa taccacaata tacttccatt 35340
tccttttatt gtcctttatg ctattgctgc catacatttt agttttatac atgctataaa 35400
cccataaaac attttgatta tgctttaaac agtacattat cttttaattt taaataagtt 35460
tataattttg aaataagttt agatttacag aaaagttcca aagatagtac agagattcct 35520
tataccettt acccaatttc ccctaatgtt aacatcttat attactatag ctcatgtcaa 35580
attaagaaat taacattagt acattagtat taaataatct atgtatttta ttcagatttc 35640
tgttttgtcc caaaagatta tcttttaaaa tataccgtat ttacccacag atttaccatt 35700
tctggcactc ttcattttct cacgtagatt taaatttcca tctggtatca ttttccttct 35760
acctgaagaa ctttctttaa tattagtagt gcacgtttgc tagcaatgag ttctttcaac 35820
ttttcttttt ctgaaaagac ttcactatct tcatttttaa gagattgtcc cacttgatca 35880
agaattctaa attgacaggt tttttctttt gttaaagacg gcttttcatt gttttctgat 35940
ttgcatagtt ctgaagaaaa aatatgctat tgtcctatct ctgtaatgtt cccctttctt 36000
ttagcgactt tggattggat tacagtgtgc tgttgtgtgt attttgcttg cgtttttatt 36060
aagcttcttg gacctgtggg cttatagttt ctatcaaact tgaaaaattt tcagccatta 36120
tttctctaaa tatatatatt attctgcctc gaaactcttc ttcttagact ccaattacaa 36180
ctatattaga ctactttata ttactccaca tgtcactgac cctctttttt ttcactctac 36240
acttcatttt ggataatttt tatttataag atttcaagtt caccaaattt tccttctgca 36300
gtgtctaatc catccagtgt atttttttgtt tcatatagag agatttcaca taggtattta 36360
aaaaatagct tctgtttctc ttttcagtat gcttatgttt tctgctatct tctttgaact 36420
tacagagcat aaaacataag atattttaaa aatccttgtc tgctagccag ttctaacatc 36480
tctgtcattt ctggttttgt ttctattgat tgattttcct ctgtattatg ggttaaattc 36540
tcttctttgc atgcctgcta atttttgatt ggatactaga cattgtgggc attaagtcag 36600
tggttactgg attttgctgt gaatgcaatt agggtacttg gaaccagttc attctttggc 36660
agctttgtta gggaaagtca agagcagcct ttagtctagg gctaatttat cccccataca 36720
aaggtgaaac tctactgatg attgtttcaa gtaccccatg tgttatgaag tttttctctt 36780
ctggctgctg ggaactcaaa ctattcccag actttgtaag cactaggaag ttttctgcct 36840
actcatttcc ctggccttat gtagcttcct ctcatgtata tgccaatcag tacttgacca 36900
aaaacttaag ggacgcccta tgcagatacc tggagctttc tctctatgta gctccatcct 36960
ctctagtact ctaccttgca aattctagct gccacggact ttccctaaac tttatctctt 37020
taactcagtg tgaccactga gctctgtttg agtttattct ccctgaactg tggcctagta 37080
attgtctcca ggtaataatc tggtgcaatt gtagtattcc aggacaagta tatcaaatgg 37140
tacagttgtc ccttggtatc catgggtgac tggtttcaga acttccctca gataccaaaa 37200
tccatggatg ctcaagtact tgatacaaaa tgacttagta tttgcataac ctatgcatat 37260
cctcctttat actttaaata atctctggat tactttaata gctaatatga tgtaaagcta 37320
tgcaaatagt tgttacattg tagtgtttag ggaataatga caagaaaaaa atctgtacat 37380
gttcagtaca gaagcaattt tttttcaaat atttttgatt cacacttagg tgaatccaca 37440
gatgcagaac ccataaatac agagggccag ttgtgtattt ttgtagtaga agcctaaata 37500
aagatatgtg gagttacttt cttgaaggaa atcattatat tgaagagaca ttgccccccc 37560
cccatgttta ttgcagcact attctcaata accaagatat ggaatcaacc taggtatcca 37620
acaacagata agtggataaa gaaaatgtgg tatatataca caatggaata ctaaacagtc 37680
ataaaaatga atgaaatcct gttattcacg gcaacatgga tggaactgag gacactacct 37740
taagtgaaat aaataaaaaa cataaagtta aacaccacat gttcttgctc atatgtggaa 37800
gcttaaaaat gttaatctcg gccaggcacg gtggctcagg cctgtaatcc cagcactttg 37860
ggaagctgag gcgggtggat cacaaggtca ggagatcaag accatcctgg ctaacacggt 37920
gaaatcccgt ctttactaaa aatacaaaaa aattagccag gtgtggtggc aggcgcctgt 37980
agtcccagct actcgggagg ctgaggcaga agaatggcat gaacccagga ggcggaggtt 38040
gcagtgagcc aagatcgcac cactgcactc cagcctgggt gacagagtga gactctgtct 38100
cggaaaaaaa aaaaaaggtt aatctcataa aaataaaaag tagaacagag gatactagca 38160
gatgggaagg ggagaaggaa gggagggatg gggagatatt tgttttttgt cttgtttttt 38220
tgagagacag ggcctcagtc tgtcccccag actggacggc agtggtgcga tcatggctta 38280
cttcagcctt gacctcctgg gctcaagtga ttctcccacc tcagcctccc aagtaggttg 38340
gactacaggt gtgtgctgcc atgcctggcc aattttttttc tttttttaatt tttatagaga 38400
tggattcttg ctatgttgcc tatgatggtc tccaactcat aagctcaaga aaacctccca 38460
ccttgacctc ccaaagtgct aggaatacag gcatgagcca ctgcgtctgg ctgagatttg 38520
ttaaaggata taaagttaca gctagataga aggaataagt tctaatcttt tataccacag 38580
taggatgact atagttaaca atattacatc atttcaaata actagaagga ggagattaca 38640
tgttccatac acaaagaaat gataaatgtt tgagatgaca gatacgctaa ttaccctgat 38700
ctaatcaata aacatcatat gtatcaaaac atcactatgt accctatgaa tatgtacaat 38760
aattatctgt caactaaaaa ataaaataaa gagtaaactt cttggaaaca ttttaggctt 38820
tcaagatata ttaacaaaac ttatacagca gttccctctt atctgcagtg gtttaagtta 38880

actgcggtca actgtggtcc aaaaatatta aataaaaaat tccagaaata atttatgttt 38940
taaattgccc actgttctga gtagcatgat gacatctcat gctatatcct gctccatccc 39000
actcagatgt gaatcatccc tttgtccagt atatccatgc tgtacatact acccaccagc 39060
agttagtagt tacttagtag ctgtctaggt ctaatcgagt gtcatggtat tgcagtgctt 39120
aggttcaagt gacctctatt ttacttaata atactttact aaattgtaaa cctactcaag 39180
aaaaaacaaa gtattttccg tgtactctat aacactgtta acttttttctt ttttttttga 39240
gacagagtct cgctccattg cccaggctgg agtgcagtga catgatcttg gctcactgca 39300
acctccgcct cccaggttca agcagttctc ctctcagcct cccaagtagc tgggactaca 39360
ggcggccgcc accatgccta gctaattttt gtatttttag tagagttggg atttcacatt 39420
gttggtcagg ctggtctcaa acgcctgacc tcaggtgatc cacctgcatt ggcctcccaa 39480
aatgctggga ttacaggcgt gagccactgt gcccagccac tattaacctt tttaaataaa 39540
agaacatgct ttaggaaaga aaacagaaca gtacacttct gaaaagcagc tttcaaattg 39600
ggaggtcaaa aaattttatc ttactgtaaa aataaaatct aagctttttt tttttttttt 39660
tttttaacac ggagtcttgc tctgtcacca ggccggagtg cagtggcgcg atcttggctc 39720
actgcaacct ctgcctccca agttcaagag attctcctgc ctcagcctcc cgagtagctg 39780
agactacagg cgtgtgccac cacgcccagc taattttttg tatttttagt agagacaagg 39840
tttcaccatg ttgaccagga tggtctcgat ctcttgacct cgtgatccac ccgcctcggc 39900
ctcccaaagt gctgggatta cgggcgtgag ccactgtgcc tggccaaaat ctaaaaaccc 39960
aaaaccccac cctttttatt ttacattccc tcaaaagaat ctgtcaaatt tctaaaatga 40020
aactttacaa ctagaatata aaggacatat aaaggacata gactttgcct tttttttttt 40080
tttttttttt tttggacaga gtaccgctca gtcacccagg ctgaagtgca gtggtgcgat 40140
ctcagctcac tgcaacctcc acctcctggg ttgaagtgat tctcctgctt cagcctccca 40200
cgtagctggg attacaggtg cacgccacca tgcccagcta atttttgtg tatatatata 40260
tttttttgttt gtttgtttgt tttttagtaa aaacagggtt tcaccatgtt ggctaggctg 40320
gtctcgaact cctgatctca ggtgatctgc ccacctcaac ctcccaaagt gctgggatta 40380
caggtgtgag ccaccacgcc tagccaactt tgcttttttt ttttttcttt tttttgaga 40440
cggagtcttg ctcaatcacc caggctggag tacggtggca tgatctcggc tcactgcaac 40500
ttctgcctct tgggttcaag tgattctcct gcctcagcct cctgagtagc tgggactaca 40560
ggtgcatgcc accatgccca gctattcttt tgtattttta gtagagaggg gggtttcact 40620
gtgttagcca ggtgatcttg atctccagac ctggtgatcc ctctgcctcg gcttcccaaa 40680
gggctgagat tacaggcatg gagccactgc cccccagccca actttgcttt ttagggcaac 40740
ataagtagta aatgataaca taacaccata gtaaatatta gatgatatgt taataccctc 40800
cctggaaatt cagcaataaa gcacatgata attaagacta taagaaaaag tacttttaaa 40860
attctgtcca gctgggaaaa atatatgtat ttcctaccta tctctcaaaa atgtaactaa 40920
agcagcttct cttctttaac aatattttcc tcaaagaaag ttgacatcta ctttgtcaga 40980
cataatagct tatgtgtttt caaaatttcc tacaccatgg acagactgat acattatagt 41040
ggtcactgtt ccgaagacaa cagttgctct aggacgtatt agttataaat ggaatattta 41100
gagtgaatgt agcaactatt agattaaata aaattatgac tccttggtct tctatttttc 41160
attaacataa ttaaaatttt ctgtatggct acattgtttt aatttattgc aaattttcaa 41220
acacctttta cactccaaat ggcctttaaa atactgccaa tattttaccc aggggaattt 41280
attttagata aaaagtttaa aaacctacaa aagaacaaaa aactcccaaa caaaaaacaa 41340
aaccctgaaa tgaactgtat ataaaggtta actttgaaat agttcttatt acataaacag 41400
atttttaaaa ttaatcaata ataaaaatta ctttgtgaga tcacagttaa ttggaaaaag 41460
caaaactaag ggttaagaaa aggtggaaag tgagtacacc tcattttaat gacatcagaa 41520
tctatttcat tgtacactta gggattatca agcatgaaaa tcaatgacca atttaaaatg 41580
acatacagca tatgtgaaaa agccagtttc tcctgccagc aaatagccta aatgttaagt 41640
aaaccaaagg ctggaaggga ttagttgaaa tgtcaaaaga caacctttta cttttacctg 41700
ctcatcctta taaacacatt cttatctctc aggcatgctt tccaaacaaa tagtttcaat 41760
gtgcaccacc agcaaaaggc ccaaggcaag ctctgccatc ttgtaactgt caaagagatt 41820
agtctttgat ctatttggga ggtcatgccc acattcacat taccttataa tctaacaaca 41880
ttatacctgt tgaccaatgc ttccttaatg cctaatgaga cagacatttt acattatgga 41940
agttaactgt gaatgttttt tctaagtttt gacagaaagt aagcaccata aactgagctc 42000
aagtgtggtt tgggaagatg atcttttaga tcagttaaca ttacttttca aatacatttt 42060
acaagccttt cccaaaatag aaagctatac ttaattgtac tttttggaaa cagttaaact 42120
acttgcttcc catccccaat aaattagtgt atgtgtaatt ttggaggggt gtgaagagca 42180
ggaaaaagac aaactatttt attaacagtt gcaaactgaa ggcccatagt tgtgttttgt 42240
gtggccctta ctgtggctta aaataattaa aatttatttg ctaccatcta aaactttggc 42300
tatctcacca ctgtcctcaa ctaacccact catttaaata acctgtttaa ttcatgtagc 42360
tgcttgagtt tgcaacccat tggttcagat gctgcataga ttcacaaaaa tttatcattt 42420
cataattgaa ggtcaagttt gtaccaaatg taaagctccc ttactatgat agtttttaga 42480
caaataaata tatatctgca tcaaaatcta acctaactga cataagtaga aataagatcc 42540
tcaatatcaa aggcattgat aattaagact gaggcggttt agaattgggt aaaaataccc 42600
aatagacaga tggcattcaa gcagaagtta tatgattttg gattctgatt tttaccatta 42660

tatcataatg tacttgagat tgaacagaaa gtttcttaag ttctccatgc cttggtttta 42720
ttggaaacag tataagtagc agagcactta tagcagcaat agacaatatg aaataatcaa 42780
aaggattata ctgcatatgt caacgagaaa ttaaggtcac aattattaac attagacttt 42840
ttcaactttc agtcaaactc tttgcacaaa agtagccttt ttaaagttta tcagtagctt 42900
ctgaaaaaat aaatggtgtg ataaatatga gctagaacta taaatgcatc tcttcatttt 42960
actgctgctt cacatgaaag atggtggatt ttatcccaac tcataccaat acatattatg 43020
atttcagtaa taaatgcctt cctgaaagca atcaatagaa gaatataatt tttttcttgt 43080
atgtaagctt gaagacaaaa gttcaagcct ccaaatgaga ctaaggacaa ataatcagct 43140
aaactacata attataaaaa tattaagaaa actctgcttc agaaaaataa acacatttgt 43200
ctagcaaaca cttgagtaga acagttttat ctttagccaa ggtgcagggc ctaaagccta 43260
aacatttaca gcaaaccaaa tctggaagac agagactgtg cattagagac atttgctgct 43320
ccgtatcatc atagtgtaaa cacttctatc agggtatcac aatataaatt ccaatggtta 43380
gaattataaa ctcagcataa gatcttgtat gtgatataat ttagctagat taaaaagaat 43440
atagctggtt ttatttcatc tggcatattt tcaagatttc tgacacagtt aaccaaatag 43500
tgtgtgtgtg tgtgtgtgtg tgtatataca cttttttaa atggggaaaa tgagttagtt 43560
tctgccaaga gttaagaagc caggctccct tttaaataag aataataatg ataaaagcac 43620
attagaaatg gtttttagtt gttacttact ttccctgtta cgttacaact tgaccacaat 43680
aatctatcaa gacatagtaa cagcaatata tttcacatat gctttaagac tgttttggat 43740
aaggtgcttt ttttggtaga gaaatctata ttaaacttag tatcgctaat tgtcagtgtt 43800
gactaaaata acctggatat ttcaaattta agaaatagaa caaaatatat ccctctgaaa 43860
tcaggtatct cctcttctca acatattcag cctcaactgg ttataaaaat ataaccagtt 43920
tttcctatta aaaagttaat actctaagac atgcaagaaa tcttagcatg cttattctgt 43980
aaagggctaa gttaaagttt tcatttttat tttttagaga caaggtcttg ctctgtcacc 44040
cagaatggag tgcagtggca tggtcatagg tcactgcaac ctccaactcc tgggttcaag 44100
caatcattct gcttcagcct cctcagtagc taggacttac agatacgtgc cccaacacca 44160
ggctaattaa aaaaaaaaat tattttagag acgcagtctc actacattgc ccaggctggt 44220
cttgaactcc tggcctcaaa caatcctcct gtcttggcct cccaaagctg tgggattaag 44280
gtgttatcca ccatgcctgg cctaaaaaat tctttttaag agtaatgacc tttgtgtcaa 44340
tttaggtagg ttatccatgt taaaatatta ctcaaacaaa cctgcaaatg tttaaattat 44400
catctctaga acatgccacc attcgtgtta tagtttattt tgaggaagaa ggtataatgg 44460
aaaaaaagaa aaagcacacg gcttggaatc aaggccttga gacttaggtt gtttgacctt 44520
ggacaagtca cagtagccct ttataatttc ctttctcaat ttcttcgtct gtaagaaggg 44580
gcttagaatt agaagtctct tagctttatt gaagttggag actgtgtgcc aaccttacct 44640
tagagaattg gttgaagaaa taaatgacaa tatgcatgaa acagtaaagc atcagatact 44700
agaggtaagt ggacgagaaa gtcattactt accccatatt tctgaaaccc agtgtaggtt 44760
tcagtggact ttaaatgctg tcgtaatata ttgctgcatg gtatgtgaaa taagccataa 44820
ggctgtggag gccatatagt cattacctta gtcagttcac cccaaatcat tttaccacct 44880
ggaccgattc cttctgaaag agctctgact acattattca atgtttggca cataacttta 44940
ggtgacttaa aaagtaagtt tcttgggttc ttggagtggc tactttttca tcaagtgtca 45000
atgaaaatat atacattggc agaatacata cagaacagtg ttcaaacagt tttgaaacaa 45060
atattttaaa aaataacttc tacaattgat cttaaatttt ctaagaaatt ttagctaaca 45120
ttagcactct ttaatatcct ccataatact ttttggtcac ttttcaactt atgaagtgtt 45180
tttgaaagca taactatata aaatatgcta gtggtgtaat ctatgagaat gagtgcatga 45240
ttggtaccat attagtctaa atattatttt attcaaatat cttacaatct agagtttttc 45300
ttgttaaagt cacatatgag agcccgataa ctgtgaataa acttttgcta gcaataacaa 45360
ctaacaaaat aaaatattat cttatgtaag aaagcagtaa atagaatcag aggttcttaa 45420
aggaatgagt caatattaag aggatttaat aaggggactc agtaatggtg cattcagcag 45480
gtgacagaga cctacttcta ccttatataa cactggaaaa aggcaagatt gctcatggaa 45540
gaggcttttg gatgccagaa gatgtaatat gacgcataat ggcactcagg gaattatata 45600
aaggttcctg gcaatagtag cataaaaagt aaaagttaat cccagtgaga ggaggctcag 45660
actcagcttt catatgatta aaccctaaca tctaaaaaca aacctaagca agagcgagca 45720
tgtacataag aaaaaagatt aaatgagtac tggaaaatgc tgaaaataca cttaaaactt 45780
tattaccacc acagtccaaa agatgaagta taacactttt gctatctaga tttttattat 45840
tacatccaaa ttatatttca gggccaggtg tggtggctca cgtttataat cccagcactt 45900
tgggaggatg aggcaggagg atcacttgag gccaggagtt taggaccagc cagggcaaca 45960
tagcaagacc ctgtctctac aaaataaaaa ttagctgggc atggtggtag gagtctgtag 46020
tcccagctac ttaggaggct aaggcaggag gatcatttga gcccaggagt ttgaggctgc 46080
agtgagccat gatctcacca ctgcatttca ttgtgggcaa cagagactct gtctgtctgt 46140
ctgtctgtct ctctctctct ctctctctat atatatatat ataatctttc aaacatgagc 46200
aaaggtttga tttttttttt ttttgagaca cagtcttgct gtgttgtcca ggctagagta 46260
cagtggcatg atcttggcca ctgcaacctc tgctccctca gttcaagcaa ttcttgtgcc 46320
tcagcctcca cagtagctgg gattacaggt gtgtgctatg acgcccggct aatttttgta 46380
tttttagtag acacggggtt tcgccatgtt ggtcaggctg gtctcaaact cctgacctca 46440

agtgatccac ccgcctcagc ctctgaaagt gctgggatta caggcgtgag ccaccgcgcc 46500
tggccacaaa ggtttgattt ttaaaatata tgatcacttt ctacctttga aatattattg 46560
ccaagaatat ttgattcaga gcttttcaaa tgtgaatgtg agttttggaa taagacatat 46620
ggaatcatag cactttgata actcatgctt tatcttagaa agtaatgcag acttctgatt 46680
tcaataagtc aaaaaaaaaa aaaaaacccc agacatttct ttttccctca cctgtcagag 46740
aaactgcctt taaattaaaa gacaaatagg aaacagaaac acagtctcca acttcagtaa 46800
agctaagaga catctgattc caagccccaa tatatgaaca taaaaggtag atggacaaat 46860
gttaaatgac taagcagaaa agagaaagtt caaaccaacg tatccacaga gctactgctg 46920
ggacataagc cagtttgacc tgcaggagtt cagaaagtct caggaaatgg aggtataagg 46980
aaactgttgg gaggatgtgt accgcaaaga tgaaacagta agcaagaaac aagaggaaga 47040
tctgagaccc aggaaactgg caatccatca cagaagacag acaaatcaaa ttcctaggat 47100
gatagcagtt attggatgat agcaggatat tggcagttat ctgggcttga tgagcaattt 47160
agccctgacc gaagtagaag atcaggaggc tccagaaatg atatctccag gagaaaaaag 47220
gaactggcct gttatctgac aggtctgata gaaaaactgt attgagaggc atggcacaca 47280
actattggaa ggtgacacat ttatttagat aaagatcttt taaaaagaga aaaaggaagt 47340
ggaagagagg agggttctat caaccctaaa gttcaaaaca gaaaggagga cgggcggctg 47400
taaccccagc actttgggag gctgaggcgg gcggatcttc tgaggtcggg agttcgagac 47460
cagcctgacc aacatagaga caccccatct ctactaaaaa tacaaattag ctgggcgtgg 47520
tggcgcatgc ctgtaatccc agctactcgg gaggctgagg caggaggatc gcttgagcct 47580
gggaggtgga ggttgtggtg agccaagatc acgccattgc actccagcct gggaaacaaa 47640
agcaaactcc gtctcaaaaa caaaacaaaa caaaacaaac aaacaaacaa aaaagaaagg 47700
aaatcatttg attacagtga acagtatttt catagtcatg ataatgtaca taataactat 47760
agatttaatt aaaaattgta atgtaactat cttgggaggc tataagaagg aaaaagcaga 47820
gtaagtgagg taaaatcctg tctcaaatag gaatttaata gttcatgtct aaaactgatt 47880
aaataaagat aaaatatgga ggtaaatacc agataaaata gctaaagaag tgaaagtcac 47940
tgcctgtcag aagcaggggc tagaagtggg gtgcaggcca aaagactgtt ataagaaaaa 48000
gctatctact gtatttagtg tatattagac tcttggtaag tgctttattg gacgtttttc 48060
aacttgataa atcataattt aaaaacaaat gcagattatt aattttaatt tattgtatac 48120
atttgtatgc ctcactatgt aaaacagtgg tgctcaaccg agagcaattt tgccaaccag 48180
aagataatgg caatgtctag agatattttg ggttatcaca actggtggca gggtgaccgt 48240
ggggggtgtc tagatcccac taccggcatc tagtgggtaa atgctagaga tgctgttaaa 48300
catcctggaa tgcacaggac actcctcgga acaaagaatt acccagtcca aaatgtcaat 48360
agtgccaagt ttgagaaaca cagcttgaaa gtaatttcaa gtcacaaatc tggtatactt 48420
gatgcagtag gaaaatacac tgagttattt gtatagctta ctatagtcac tgacgcactg 48480
gggtgatgtg tcctacagtt acaaagtata aactgaactc attcccaagc tttcaagcct 48540
ttaatcctag ggaaaaacag gaaagagtga tgttgtaaac atggaacagc aatatctact 48600
acagtttatg ttatttgaga ccttaaagct ttacagttag aaaaaaaagt gacatagtca 48660
cctgaagccc ctctacatgt ctacaatctg taggcattga gagaatttga ctgaataaac 48720
tgattaaaat tcacaactaa aattctcctc aaaagaaaaa aattagttat tttcagagaa 48780
gctaaaaaac aatcccacca ctgcccttaa ggtagagctg ctgattatgc tccatgccaa 48840
cataagtagg taagtgtcaa tcaaccttac aagtcctcag ggtgataatt tcacttttaa 48900
atcctccaac ctagtttgag agcaaaatta atatataaag gtcaaagttc cttttctcac 48960
caaaaataag atgaacacaa gaaaataact tcagaattgt tcaaaaatca aaaccaaaag 49020
tttttattta ttaattatcc agagaaactt ccagcaatat tttcaggcat ggctttatgt 49080
atttatttat ttattttttg ctaaggaaat acaatgacat gagtattagg agaaaaaggt 49140
tatcaaccga acttgcttaa aattttggga ttttaaatat ttccatcctt gatcccaaaa 49200
tatcttttaa aaatatggag aatttaaaaa ttaacctcag caattcatcc atttgttttg 49260
tgttttttttg tttgttcatt tgtttttcag aagggatgga aacagagggg tttcattaac 49320
tcatttttttg ctggccaagg taaatataca atttattaac ctagacagct tacttagatt 49380
taaactaggt tttaattttg gaatttcaag atatacttag aaactgcttt caaaatactt 49440
ttggccaaac tatcttaaaa aaaaaaaaaa gctggggaaa aaaatactct gaggaggaaa 49500
aaaaaggttt aagtaattga ggatatactc actctagact tttagaggtg acaaggtttt 49560
ttcttctata tctgtgatgg aacctaagta tttccaaaaa ttccaagagc aagttgctga 49620
acttgtaaat gttaaagagt taagtattac agcactttaa gtttatattc acatccattt 49680
cccctgggta accatgtatt attatcagag ctatatccag gtgggtctct gtatcagatg 49740
caacactata accagaagtc cactccactc tgacctcagg ctgtattcct ggatctggtt 49800
tcccataact tagaatgtcc caaatcctga aaatggcata cagagtccta agtgatttgg 49860
ctcctaaata cctctcccag cttctattca catgccattc tctcagactc acaacttcag 49920
cttctttcag ttcttggaaa ctgctatcct ctttcaacct tagaacctta acacatgctg 49980
ttctctctgt tgagaatact cttatccatt ctgtacacat tcataactcc tagttttctt 50040
tccagtgtca ggttaaatat cacttcctca ggaagccttt cttgatcccc aaagacctgc 50100
tataacattc ccagggttcc tgtgactttt tcttatagcc tttatcacaa ctgtgattgt 50160
ttcttcatta cattaatgtc aatattcctt gctagactat aagcaacatg agacaagggg 50220

ctataaactg tgttgtgtat cactctgtcc cctgagtcta ttataatact tggtgctcta 50280
gccagatatt aaaaaaaaaa aaggttgctt tattattctg aatagttgct ttttttcttt 50340
tctttctttc tttctttctt tttttttttt tttttttttt gagacagagt ctctgtcacc 50400
caggctggag tgcagtggag caatctcggc tcactgcaac ctcccccctcc caagttcaag 50460
caactctagt gtctagtgtc tcagcctccc aaatagctgg gattgtgcca tcacacccag 50520
ctaatttgtg tatttttagt agagagagag agtttcatta tgttggccag gttgttctcc 50580
aactcctgac ctcaagtgat ccacccacct cggcctccaa gtgatggaat tacaggtgtg 50640
agccactgca cccggcctat tctgaatagt tttataatct gaatagtttt gttgtaatct 50700
aacctctgat gaggccattc cactttatga aatgtttgta tatttgaatc tcactgtaga 50760
gaagattcaa atctcatctc tctggctcct ccatcccttt taggcaagtt agtcaaagta 50820
aattggtatc tgcaagggag gcgaaaggtg ttggtcaact tcagctttcc taagcatctg 50880
agcacctggt ttctattcca ccaaagcatc ttgttatatg ttcctgttct tccataaatt 50940
attgacatta ctagtgtaga gacagcatct tgttcatttt tacacctact gcattttgta 51000
caaattaagc tcttaaaaaa agtttgttaa atgaatgaat aagtgcatga caaaacattc 51060
aatatcatta acattttttt tttttgaga cagtgtcttg ctctgtcgcc cagactggag 51120
tgcagtggcg caatttcagc tcactgcaac ctttgcccct taggtacaag tgattctcct 51180
gcctcagact cccaagtagc tggaattaca gacgtgtgcc accacaccgg ggctaatttt 51240
ttgtgttttt agtagagact gggtttcacc atgttggcca ggctggtctc gaactcctga 51300
cctcaaatga tctgcccacc ttgggctccc aaagtgctgg gataacaggc ataagccact 51360
gcacacggcc gtcattaaca tagttttgat agaagagtaa ttcaaaaggg atttataaat 51420
ctattcaaaa ggaacagatg aatctaccta aaaggtttct gaagtcaagt tcctcttgat 51480
atactacagt cctttgcatt cctgccctca ttcattttct cgtgaatatt catattgccg 51540
tcaaagcaaa aggaataaca atggaacaat atcattctcc ttaaaaagaa atctgttaga 51600
tttaagaatt ctaatacagc caggtgcaat tggcgcgagt ctgtaatccc agctgctcgg 51660
gagctgaggc aggaggatca tcacttgagc ccaggaccag cctgggcaat atagtgagac 51720
tctgtctcaa aaaaaatttt tttttaattc caatactacc atcatctgat aatattttga 51780
cagttgaaag cactggttta acatggttta atgtgcttta ttactttatt aatgctgtta 51840
tcctttaggg aaagcaccct ttttctgtgg tttataaaat gccaacaatg catggaagcc 51900
tatcaatttt tttgtgtgtg tttgttataa agtcgataag taactaatat aactaaaata 51960
gatgtctgaa tttttcaatc ataaatgtaa ttttaaaaaa ttgattaaaa taatatatac 52020
ccaaatagtt tggtacattg agagctaaga taagtttcct aaacaaaaaa aggaactcat 52080
aatggttatt gtatgctaat agtaagatag acaattcaga tcactatgcg aattttaaat 52140
agtttccaaa taaaatctag tttggtaact caacatacta tattaccaaa aaaacccaca 52200
aatataaaat ttattagcat gataaattat tttaatcatt gtaagggcac ttctgacaat 52260
gaaaagtacc atcaaatttt attaaaccaa ttataaaaat ctaaacacac cacttcaaca 52320
ttttaaaaat ataaacattt ttagccatat gccatggttt tatacatttt tcttcagaga 52380
agtaaaggaa caatacctac gaaggagaac ttttctatat actattatac tatactatgc 52440
taggcaatac aaataaaatg caattttaaa acctaaatat atttgtacct tatcatatct 52500
cacaaactat gtccatgtca ccaattggag aggtggatgc ctcaattata aagctgaata 52560
ctcctgtggt tgttagatca cagtaattgg ccagaggatt tgctttagat atccattaaa 52620
aaggcagcct tgttatactg tgatttaatt acatgaaaaa gaaaacaact tttattttag 52680
ttatactttt ccatcgacat ctagtcatat aaaagccttg gttcattctc atgcccctca 52740
aatgctttgg tttagtgcac agaaagtttt ctttttaact attatacgat taagtatatc 52800
cttatgaatg atcagaagca tctgggaaag gtggaagtga aaaaaatcct cactgggaaa 52860
gttagcttaa tgcttacctt cagagtagcc cattatcaac atttcatagt gggtttcttt 52920
attttcaggt ccctcaggta tgtgcaataa attatgactt gaactaaaca agtcacactt 52980
ggctccaaaa tgaaattttc ctacctctac tttctagtcc ctaaggtggg aagaggaaat 53040
cctggtcaca gccaaaggag tcatattttt ggctctaaca tacattttcc ttcttctcaa 53100
aaagccaaac cctgctattg agactgcaaa gtcttatgtt agtattttaa actgtatttt 53160
aacttttact tccataatct ttctcaaatt taagaaaaaa ttttagtatc ataaaattaa 53220
tgtcactgcc ttctttttcc cactcaaatc acttttctga ctcaatgatc ttaacctttt 53280
taaatatgaa gtcaaatgaa atagacaaaa taatgcagaa ataggttgtt tcgtttctat 53340
aaggtagaac atttaaattt ttatttacta aataacttct acttattaga ctattaatgt 53400
aaaagaaaac tataaagttc taatttaagg tcctataatt tgaagcatag ttttagtttt 53460
ggaagagcca tctatgtagg tcatttagat catcatctct tttcataggg aataggagta 53520
tggcagacac accctaaggt gatatacaga gagtcacaac tttgtaatgc tctcccctta 53580
agtgagggca gaaccagtga tttgcttcta gccaacagaa tatggaaagg gttctgggat 53640
agtcactctt aattaggtta tattatatag caaaaatgat gggaagttat tcccatgatt 53700
ttttaatttt atttttttcc tcacaatttt attaagttgt gtaagactct gtattggctg 53760
actggggcta gagagcttcc tattggcctc tggaagtata ctgtcatgat acgagggggc 53820
ttgtgaaaag gccacatggc aagaaactgt gaatagcttc ttgaagccaa gagtacacct 53880
tagggctagg cacggtggct cacacttgta atcccagcac tttgggaggc tgacgcgggt 53940
ggattacttg agctcaggag ttcgagacca gcctgggcaa catagcaaga tcctgcctct 54000

acaaaaaaaa aaaaaccacg tttaaaaatt agctgggtgt ggtggcgtgt ggctgtagtg 54060
ccagctaccc aggaggctga ggtgggagga tcacctgagc ccgagaggcc aaggctgcag 54120
tgacccatga ccgcaccact gcactctagc ctgggcaatg gagtgagacc ctgttttcaa 54180
aaaaatttaa aaaagactgc ctcttggatg gattaggaag aacctcagtc ctacaaccac 54240
aaacggaccc caagtgccag ataacaacac aacctggcca acactctgat tgcagcagtt 54300
ttgtggaacc ctgacccagc taagccatgc ccacacttgt gaccaatgga aactgagata 54360
ataaatgtgt gttaagccac tatgtgataa tttgttacac agcaataaaa aactaataca 54420
ggctgggcat gatggctcac acctataatc ccagcacttt gggaggctga ggtctgagca 54480
tcccttaagg ccaggagttc aaggccagct tgtacaacat agcaagacct cgcctctcca 54540
aaaaaaaaaa aaagtttagc caggtgtggt ggcacatgcc tacagtccta gctacccagg 54600
agactgaggc aggaggatca attgagccca tgagctcaag gctgcagtga gctatgattg 54660
tgccactgca ctccaacctg ggcgacagaa caagaccttg tctataaaat aaaataaaaa 54720
tatttaaata aataaataat aaaagaggga tagctaatat agtcaatata agatactccc 54780
tccacttatt atgttctact atgtattaaa taaagtgtaa gatgctgttg aggattgagc 54840
attcccttct acatatcctc taatgattta ctactataac taccctgtat tgcctcctta 54900
tgagtcatat cactgtttac taccatcctg ttcattatta tctaatgatt actatcattt 54960
ccatccttaa agtctccagg aatggagagt ggttgctgtt gtttttaaaa acaaataaca 55020
aagtcaaata caattttaac aacttctact aaaaccccat tcaatcatta actaaaacat 55080
ttattaaaca ctgtgttttt aggtaatatc ttagttgtag agacacaaag atgctgaaga 55140
aattatccct atcctcaagg tacaccatga aacagattat aaaacatctt tataaacact 55200
aaatatgtgc tttaggaaat aataaaggac actggtaaag gtaactgcag gtgaatacta 55260
caatatgctt ttgaattgca attcctctgt tttccctata ttatttaaaa ggcaaatgca 55320
taaaaacaat aattaaaaac taacagccac acaaagtata aagatggaat ctgtgacaat 55380
aacaatataa aaagggagga acagagattc agatgatagt tataatcccc aaggtaaata 55440
ctaaaaacca aaaaatacat ataaaaggaa agaagggaat caaaatagta tactacaaat 55500
caactaaata caaaaaagga aatgatgaag gattgaggaa caaaaagcac atgacacaag 55560
aaaacaaata gctacatagc aaaagtaagt cctttcctat cagtaattac tttaaatgta 55620
aatggtttaa actctccaat tgaaaggcag agattggcca aatggatttt taaaaacctg 55680
atccaactat acgctgtaaa taaaagattc acttagatat aaggaaattg gacctcacca 55740
tatatcacat agaaaaattt actcaagagg aatcaaatat ctaaattaaa gagttacaac 55800
tataaaactc ttcaaggaaa acattagggg caaatatgca tgatgttgga tttgccagta 55860
ttttcctaaa tatgacacca aagcacaggc aacaaacaaa caaaaaaaac agataaactg 55920
gactgcatta gaattttaaa cttaactttt atattccata gaggcataca aatattttca 55980
aatacttgtt taaccattta taaaaatgtt aagaaatcac ccataatgat gctctgacat 56040
atttcaagta ttttaggtat tgactgctat attctgggca tttccaaata ataaaaagcc 56100
ttcttaatca ttataaaaac tgcatgcgta tactcttaaa gcttgtaaca caccagtctt 56160
ataaatggat aactaacaaa taccttttaa ttatgaaaaa atgcgatagt tgaggacttc 56220
tttttgaagt catgattcta agtcctttgc catgtgcggt agaaattatc ccttttctcc 56280
ccttccttga ctttgagatt gtgcatggac gtgttttggg caacaggagg ttagcagatg 56340
tgatgtaaat aggtatttga cttaactgct tacagttaag cctgccctct tgtactactg 56400
ccattgcaat aataacatgc cttggctagt ctgttggtcc acaaagaatg aaatacaggt 56460
ggaacagagc tgactcagtt gacttcaaca cagatcagcc aactagggca gagtagctga 56520
gcccagctaa aatcgagaga gccaaccaac caagcccaag cctagatcat ctgtcaccca 56580
atcactccag agatgtatga cctgtaataa atgattgctg ttttaagtca ctaagttttg 56640
gagttgttac agaagaagaa ctgatccaga catatggcaa taagaaatac acttcccagt 56700
acatctagta gtgctggtaa ataatttcac tttttctatc ccttgtccct gcagcttgaa 56760
ctattcttgt gtccactcta cttgtacctt ttgggaacca ctagggataa ccctttagaa 56820
ccaccttcct cccctctacc cagaacccat tgatgcttct gttattttgc tgttttatgc 56880
acataagcag aactatgaca aataggatca tgactgagga atgtcagagt aggccatcag 56940
gtgtcttttt tgcatgacag actgagatat tgtctaggaa gaatttatcc tgaggaaaaa 57000
aagcattaag aaccccagat tggtataata acaatgttca aaatcatagc tgttgaggca 57060
aaggtggaga aaagagaaat ggaattttct cacaaatgta tatttttaat tttaagaatt 57120
tttggaaaat aatgaagaat aacataataa atacctgcat tctcatattt tcttgcagct 57180
tattttcctt tactaaaaat tatggaaaaa gttaaaattc ttttgataac tactctgaat 57240
atcattccct tctcttctcc tcatatgtaa tcagaattat aatcttgatg atcctttcag 57300
gccttgttac tttgtacgta taatatttag tacaacctag tattagccat aaagtttttc 57360
ttcttttcca ggtctcaaga actcatatgt agggtccaag gaagagagag gacataggca 57420
aaaatggaaa taattagttt tagggaacaa aattgtattt tattaagata ttggaagaag 57480
atgtggtcta aagaagggaa aaggattctt tccactttaa caaagcagcc ttagtgcaat 57540
aatgtagagc tagagtaaaa tgataagaca ttaaaaggag acaataccag taccaacagc 57600
agtgccatca gtagcactct acttcagtgg aatgcagcac caatgctgtt gatggtgaca 57660
gtagatttct tgggtagatg tcaactgttt catcagtagc tgtagtcaag gcagcatcag 57720
ccattttaga ataggagacc cagctttggg catagccatt agtgccctca gcagtagcac 57780

```
tggttaactg gagaaaggta gtagaaacca ccaaagttac tggaggctga aaaagtatta 57840
gactctgaat catcaagtga gatacccccct gaggactttc agaaattttg aagaggtcca 57900
gaggttacgg aagagtaagg gctaccaaaa gaaatactgc ttattactgt taatgttacc 57960
taaagactgc tacggactag aaaaataaaa gaaaaattat ctaattcaca gtacctagta 58020
aagtattaac aattaaaaag ataactattc taatgaaaga actcctgctt tggtatgaga 58080
ccctaactga ctgtatccta gggggaggga tataggtcat aatcaataag tcagctcagt 58140
ggtcagaaaa tctcaaattt tctcagattg ccagcacttt caaatgcctg taggaagtaa 58200
aagaaaatcc tccttgatga acaataagtt caacttaggc tcaaattatt actatagttt 58260
ttcaaatata ttgtttagga aacagtcagt gataatgaga tacaactgtt tctggccaag 58320
attgagtaac tggtttaaga atatttcttc cattgtaagt aatcgcataa ccgggcaaaa 58380
tggagcaatt gttttcaggt agtagacacc aaacagcaca agacagtaat tctggagcta 58440
agtggaattc attaggtgaa ctccattatc ccagttctct gcttgggtat aatttcctga 58500
ccacagcaca gagcattaaa gtacaagcag agcgcagtgg tcctgttgag ctgaggtgga 58560
agagatcaga atatggagtg actaaatcac aagatctgca aggcaggaca ctggaaagaa 58620
gcgagccaga tggacaaaag accccaggat tccccggagg aacccatgag tctgtggtta 58680
aaagactgat aaaacacaaa ttgctgggct tcacttccac agtttctgat ttggcaggtc 58740
tggatgagcc tgaaagtctt tatttctatt aagttcccag gtgatacgaa tgcacctggc 58800
ccaggaccag actttgaaaa taactcctgt tgaccaaaga cagtataagt tgaaaataaa 58860
ataaaatctg tatctactaa atttttctca ctcattcaga ttcaccaaag atttaaaaat 58920
gtctaaggat acaggaaaac atatacaggc atccctgaga gacattgcaa gtttggttcc 58980
agaccacccc aataaggaga atatcataac aaagtaagtc acatgaattt tttggtttcc 59040
caacacatat aaaagctatg tttacactat actgcagtct attaagtggg caacagcatt 59100
atgtctcaaa agccaatgta tataccttaa cttgaaaata ttttattgct aaaaaaggtg 59160
aatgatcatc tgagccttca gtgagtcact ctctttgctg gtagagggtc ttgcctcaat 59220
gatgactgct gactgatcag ggtggtggtt gctaacgttg gagtggctgt ggcaatttct 59280
taaaataaga cgaccatgaa gtttgctcta tcatgtgact cttcctttca caatagattt 59340
ctctgtggca taagatgctc tttgatagca ttttacccaa cataaaactt ctttcaaaat 59400
tggggtcaag cctctcaaac tctgccgctg cttcatgcac taagtttatt taatattcta 59460
actcctttgt tgtcatttca acaatgttca cagcatcttt actaagaata gttcctatct 59520
caagaaacca ctttctttgc tcatccataa aatgcaactc ctcatctgtt caagtgtcct 59580
catgagattg cagcaattta gtcacgtctt aagctccact tctaatttag ttctcttatt 59640
atttccacca cagctacagt tacttcctcc actgaactct taactccctc agagtcatcc 59700
aatgagggat ggaatcaatg acttccaaac tcctgtaagt gttgatattc tgacctcctc 59760
ccatgaatca tgaatgttct taatggcatc tagaacggtg aatcctttcc agaaggtttt 59820
taatcgactg tgtccagatg catcagagga ctcgctatct atagcagtta tagcctcgtg 59880
aaatgtactt cttaagtaat gagacttgga agtagaaatt actcctttat tcattggctg 59940
cagaatggat gttttgttag caagcatgaa aacaacatta atctccttgt acatctccat 60000
agagttcttg agtgaccaca tgtgtggtta atgagcagta atattttgaa aggaactgat 60060
ttttctgagg agtagatctc aataatggac ttaaaatatt cagtaaacca tgctgtaaac 60120
agatgtgctg tcgtgcaggc tttgttgttc catctataca gcacaggcag agtagattta 60180
gtataattct taaaggctct aggattttta aaatgttaaa tgagcattgg cttcaactta 60240
aagtcaccag ctgcactggc ccatcacaag agagtcagcc tgttctctga agctttgagg 60300
tcaggcattg acttctcctc tctagctatg aatgtcctag gtggcatctt ctttcaatag 60360
aaggctgatt cttctacact gaaaatctgt tgttttgcgt agccaccttc atcaattatc 60420
ttggccagat cttctggata acttgctgta gcttctccat cagcacctgc tgcattaact 60480
tgcactttta agttatggag atagcttctt tccttaaacc tcatgaaaca acctctgcta 60540
gcttcaaact tttctgcagc ctcctcacct ctctcagctt tcacagaatt gaagagagtt 60600
agggccttgc tctgaattaa gttttggctt aagagaatgt tacggacagt ttgatcttct 60660
ttccagacac taaacctttc tctatatcag taataagact gtttgcttat tatttgtaca 60720
ttcactggag taacactttt aattcctttg aagaactttt cctttgcatt cacaatatgg 60780
ctaattggtt ggtgcaagag gcctagcttt tgtcttgtct cagctttcaa tatgccttgc 60840
tcactaagct taatcatgtc tcgcctttga tttaaagtag gaagcatgca acacttcctt 60900
tcacttgaac actctggggc cattgtaggg ttattaactg gcctaatttc agtattgttg 60960
tgtctcaggg aatggagagt cccaaggaga gagaaatggg ggaagggtag tcagtggagc 61020
agttagaata catacatcac ttatgaatta cgtttgctct cttatgtggg tacagtatga 61080
gatgccccaa aacaattaca atagtaacat caaagatcac ttatcacaga ttaccctaac 61140
agtataataa taatgaaaag gtttgaaata tcgtgagaat taacccaaat gtgaaacaga 61200
aacaagtgac cacatgctgt tggaaaaatg gtgctgaaag acttgcacaa tgcagggtta 61260
ccataaacct tcaatttgta aaaaagcact ataaaagcaa agcgcaataa aatgagggat 61320
ccctgcaatt aactttatac aaaaccaatg actcctgtaa taataaaact taaggttttg 61380
atggcctacc tgtagatctt tcacatttgg aaaaggaatt cctattgtta tgacagcacg 61440
ggcattgtca tctgagaaat ccagaccctc actcacttta ccacgacaaa ctgctaccag 61500
gagagctcca tcttaaacaa cagaaaaaag catatccaaa attctcagaa attgcttatt 61560
```

cttgtcattt tgaaaatacc tgatttataa ttatagtaat tacagtagca aatttaaaat 61620
aatcaaaata aaacactatt atgagataaa gttttaaaag ttcaatgtct ttaagtaaat 61680
aaggggaaca aaattacatt tatatgcaaa agggtagcaa accaatttac aaactctgca 61740
tcttccagat aacttataca gtaaaaacat gtcagaatag caacttgcat atgaactgtg 61800
tgattataaa caggtttatt taataaaaat tgaagttgca ttattacctt taccacctga 61860
aagtaaaatt ctcagaataa ttgaaataat tgtaattgat aatacatatc atatggaatc 61920
aatatattta aaagtattga aaacagacac agagaacacc ataatatata caccataaaa 61980
agatactgat atgttatatt acctctttct tgcattgttc ataaactact actatccctt 62040
cacatcaaac tttttatttt tattttatta ttttttgtgt gtgagacagg gtctcactct 62100
gtcagccagg ctggagtgca gtggcacaat cacagctcac tgcagccttg acctcctggg 62160
ctcaggtaat cctcccacct cggcctccta agtagttgga actataggca catgcaccac 62220
accctgctaa tgtttgtact ttttgtagag acaggatttc accatgttgc ccaggctggt 62280
ctcgaactcc tgggctcaag tgatccaccc gcctcagcct cccaaagcgc taggattaca 62340
ggcaagagcc accacgcccg gcctcacatc aaactcttca tggatatgct ctaacttgga 62400
attgagtttt cagttatgtt ttgaaaattc aagtttacct tatgtaatag aatacaagca 62460
ccaagtgtac taaatgcatc agaatatagg tctcatttgg ctagtaagga atatatttat 62520
atatatccta actgcctttt tgcacaaact atttcaggta gtgcactgaa gactgtgaga 62580
tgatggcatc gtttttctta aagttgaatc agcatactca agtgaaaata ttcataggag 62640
aacaagtaca attaaaagaa tttctttacc caatttattt tcttttcact caggattata 62700
atttttctct taagtgtaat tcatctaaaa atataaaatt ataattgtac cttcttactt 62760
tgtaataaaa aatatttttt caccgaccat gaaataattt ccagttacct ttctctcctt 62820
tgtatttgat tgcgtcatag tacacctgca gtaattcatc aaaatttgtt ttttctcctc 62880
cctgtggttc tacaatgact gtcttcacca actccagatt atgccataaa ccagtagaga 62940
gccaacgttc ttttaatttt tctaataact aaagagggga aagaaaaaaa tgattttttg 63000
tgtgtctagc taaacaaact taacttcatt tgtttaagcc aatgtgacta cggcaagtat 63060
attaatctct ctgtgtcttt tctcatttat aaaatgagga aaacaatata tctcatagag 63120
ctgttatggg gttaaattta ttaacagatt tagagagatc tgaatggtgc tggcacagtt 63180
agctgctgct gctgctacta ctactattat cttggcaatt tctaccacta ctactgctac 63240
tactactatt actactacta cttctactat tatcttggca atttctacca ccaccaccac 63300
tactactact actactacta tcttggcaat ttcaaaagca aaagcaaaaa acaaaaaacc 63360
tctgtcaaag ttaacaaact ttaagttctc tctttaaacc taaggtgaag tttcttaata 63420
ttcttaaata gtcttcaaat gaagatgatt taagatcatt ttactaagtg tataggcaga 63480
taagctcatt atttgaatat ttgatcatca ttaataacct gcattgaaaa aagatagaaa 63540
taaagactca gccaagaagt agaaaatata tgggcacaaa tggaacattt acagccatgg 63600
gctagattac aaaaggacat tcaacaaatt ttaagcaatc cttatcatat tgatcacata 63660
tttcaacagt tatgccatgg tagattaaaa gtcaataata taaagtcatg gtagattaaa 63720
agtcaagccc aaaacaaaaa ctcatcttga ttgattgatt gattttagag acagggtctc 63780
tgtcacccag gctggagtac agtggcaaga tcatagctca gtgcagcctc gaactactag 63840
gctcaggcaa tccctccacc ttagcctcct gagtagctgg gactacaggt gcatgccact 63900
acacccagct aatttttata ttttgtgtag agatgagatc ctgctttgtt gcccaggcta 63960
gtctcaaact tctggtctca agggatcctc cagactcagc cttccacagt gctgaaatta 64020
caggtgtgaa ccaacacacg tggctgtaaa actcatattc ttaaaaacca tatgagttaa 64080
atagaaaaaa cacagtataa ctcatgaaat ctttagttat gaataagaaa agcactcaca 64140
attcgaaaca tgtggcatac agcaatggca atacatggaa atacatttat agctttaaat 64200
atatttattt ttaaaatgtc aaaataaaag aatcaagtat tctttccaag aagctagaaa 64260
atgaaatata gcaaatgcaa agaaagaagt taataataaa gctgaataaa tgaaaaagac 64320
aaaagctaat attagagatt attatttaaa ttgaactgtt ttatccactt gttatatact 64380
atgagttcta cacaatttgg acaagttatt ataagaaacc acccaaaatt ctctggaaaa 64440
cagcaactga tcaattttta taagatagtt tatttcctca attttacctt taactatttt 64500
tagaaataaa cttcttaata agactataaa acaaaacatt agtaaaatag ttaactgctg 64560
tacaatatgc tttaattaaa taggaagaac tactttaatt ccataaacac agctaagaag 64620
ttattatatt actttttatt ctccagaaag acatcaaggg aggaatagag gaacaaaaaa 64680
gctgtagtta gacagaaaac aattattgaa atgataataa tgtctttccc tatcggtcat 64740
tactttaaat gtaaatggat taaattttct aatcaaaaga tataaagtag ctggatagat 64800
aaaaacgctg tctgcaagtg actcacttta aatttaagga tacccatagg ctgaaagtga 64860
agggatagaa aaagatatta cttataaatg gtaaccaaaa gacagcagag gtggcaattc 64920
taagatacta aatagattct aagtcaaaaa ttgtcacaag agacaaaaaa aaaggacatt 64980
atataatgat aaatgggtca attcactggg aatatttaac aactataaat atacacacaa 65040
ccaacatcag agcacccaaa tatagcaagc aaatagtgat tgtactgaag agagaaataa 65100
acagcaatat aagaggagta ggaaagtcat atatctaacc ttcagtaatg aataaaacat 65160
ccaaacagaa gatcaacaag gaaacggaac ttgaacaata ctagagacca gatggacctg 65220
acatatatag aacattttac ccaacagcag caaaatacat attcttcaca actgtacatg 65280
gaacattctc taggacagat catgtgttag gtaacaaagc aagtcttaac aaatgtaaga 65340

taaaaattaa taccaggtat cttttgcaac cacaacagaa tcaaactaaa attcagtatc 65400
agaaagaaaa ccagcaaatt tacaaaaata tgaaaattaa cacacttttg aacaaccaat 65460
gaatgggtca aaaaagaaat caaaaggaaa gttatgaaat tatcttgaga ccagtggaaa 65520
aaaaaccaca acataccaca agtaagaaat acaccaaaag cagtactaag aggaaagttt 65580
atagcggtaa tgccaacatt aaaaaagaaa gatctcaagt aacccaattc tacacttcag 65640
ggaactagaa aaagaacaaa gtaaatacaa agtttgcaga aggaaagaaa tattaagatt 65700
agagcagaaa taaaataggg aacagaaaaa caatttaaaa agttggtttt tgaaaagatc 65760
aacagaattg acaagtcttt acctagattg gttaaggaaa aaaaaaagag aaaatcagaa 65820
atgaaagaag acacactgat gccaccgata caataaaaga ctactctgaa aaattatatg 65880
ccaataaatt ggataaaata gatgaaatgg acaaattcct agtaatatac aacctactaa 65940
gactgaataa tgaagaaaca gaaaatctga acagactagt aaggagactg aatcagtaac 66000
aaaaagtctc ccatcaaaga aaagctaagg acctaaatgg cctcaatacc gattctacaa 66060
aacattttta tttatttaat tttattaatt ttttattttc atttctttgc taattaatta 66120
atttattttt gagatacact cttgctctgt cacccaggct ggagtgtagt aatgtgatca 66180
tagttcactg taacctcaaa ctcctgggct caagcaatct tcctacttca gcctcccaag 66240
tagctaggac tagaggcatg agccaccatg ccctggtaat tttttttttt tttttgtagg 66300
tatggagtct cactatattg cccaggttgg tcttgaactc ctggcctcaa gtgctcctcc 66360
tgccttggcc tcccaaagtg ctgggattac aggtgtgagc cactatgcac agtcctctat 66420
gaaacattta aagcaggggt ccccaacccc caggccacag aatactggta gaatccaggc 66480
tgcacagcag gaggtgagtg gcaggtgagt gagcattact gcctgagctc agtctcctgt 66540
cagatcagca gcagcattat attctcatag ggtacaaac actattgtga actgtgcttg 66600
caagggatct aggttatatg ctccttatga gaatctaatg cctgatgatc tgaggtggaa 66660
cagtttcatc ccgaaaccat cacccccacc cccaacccag gtctgtggaa aagctgtctt 66720
ccacaaaacc agtccctggt gctaagaagg ttggggacca ttgattttaa agactaatac 66780
aaatgtagat tcaatgtaac tcctatcaaa atcccaatga catttttttac agaaacagaa 66840
aaaaatacaa aaattaatat gcaaccacaa aggacttgga ataatcaaaa caatcttgag 66900
aaagaacaac aaagctagag gcatcgtgct tcatgacact tacatcttac aaacccacca 66960
taatcaaaat agtaattgca ttgccataaa gacagacata tagaccattg gaagagaata 67020
gagagcctgg aaataaatcc acacatgtat gatcaactga tcttcagcaa gggtgccaaa 67080
aatacataat ggggaaagga cagtctcttc aacaaatagt tcagggcaaa caatatccac 67140
atgcaaaaga ataaaactgg acctttatct tacacaatac acaaaagttc actcaaaatg 67200
gattagactt aaatgtaaga tttcaaactg ttggctgggc gcagtggctc atgcctgtaa 67260
tcccagcact ttgggaggcc aaggcgggca gatcacgagg tcaggagatt gagaccatcc 67320
tggctaacac ggtgaaaccc catctctact aaaaatacaa aaaattagcc gggcatggtg 67380
gcgcacgcct gtagtcccag ctactcggga ggctgaggca agagaatcgc ttgaacctgg 67440
gaggcggagg ttgcagtgag ccaagatcac gccactacac tccagcctgg cgacagagcg 67500
agactctgtc taaaaaaaaa aaagacttga aactgcaaaa ctcttagaaa aaaacacagg 67560
agaaaatttt tgtaacattg gatttaacaa tgattttatg catatgacat aaaagcacag 67620
gcaataaaag taaaacctac gattgggacc acaacaaact aaaaggtttc tgcaaagtaa 67680
agggaaaaaa aagagtggaa aggcaaccta aaggatgaga aaaaatatct gcaaaccata 67740
tatctgataa ggggttaata tccaaaatat attaagaaac ccaatattaa aaaaaaattg 67800
aaaaacaggc aaaggacatg aatagacagt tctccaaaga agacatacaa atggttaaca 67860
ggtatatgga aagatgctca acatcagtaa ttatcaggga aatgcaaatc aaaaccacga 67920
tgagatattg gttcacagct gttcggaagg ccattattta aaaaagtaaa taaaataact 67980
attagtgaag atgttgtgat attggaatcc ttgtgtattg ctggtgggaa tataaaacag 68040
tatagccact gtggaaaaca gaaaaggaaa taaaaatcaa aataaaacag tatatataga 68100
aatgttttat gggtttcaat atacaagtct tttacctcct taagtttatt cctaagtgtt 68160
ctatttgttt tgatactact gcaaatggaa atgttttcct aatttgcttt tcagacagtt 68220
tgttgtcagt gtatagaaat acaactcatt tttgtatgtt aattttgcat attgcaactt 68280
tactcaattt attagtccta acaggttttt taaaaataag atctttagga aagatcacgt 68340
ctgtaaataa ggacaatttt actttttcct ttcccatttg gaagactttt attccttttt 68400
cttgcctaac tgctctggct aggaattcta gtactacgtt atacagaaat ggcaagagtg 68460
ggcatccttg ccctgtttct gatcttagag gaaaaccttt cagtttgcac cactgagtat 68520
gctacgagct tcttacatat gacctttatt atattaaatt tccttctatt cctaccttgt 68580
tgagaatttg tatcatgaaa gtgtgctgca ttttgtcaaa tgcttttttg cattattgaa 68640
acaatgcatg aagattcttt tcagcagatg gtgttgagaa aactggatca ccacatgtaa 68700
aaagaatgaa gtaacacctt gcctacataa taaaaattaa ctcaacattg gtcaaagaca 68760
ttactgtaag agctaaaaaa tataaaactc taaaagaaaa aaatagaagg ctttatgacc 68820
ttggatttgg caatgatttg ttggctgtgg catcaaaagc ataagtaact aaagaaaaat 68880
acagataaac tcggatttca taaaaaatta taaaactttt ttggatactg tcaagaaagt 68940
taaaatgata gtacacagaa tgcaaagcat atggctgata aaagattaat atccagaaga 69000
tataaagaac tacaactcaa caacgaaaaa accaaacaga ttcaaatata ggcaaaggac 69060
tttaatagac atttcttgaa tgattaaaaa aaaaaaaaag gcccaaagga catgaaaaga 69120

tgctcaacat ccttagtcat taggaaactg caagtcaaaa ccacaatgaa gtcccacttc 69180
acttagtata actgctgtcc aaataagtaa gtgttgcaag gacacgcaga aactgtaact 69240
gtcacacatt gctagtggca atgtaaaatg gtacaactgc tgaggaaaac agtctggcag 69300
ttcctcaaaa gttcataggc atagaattac catatgatcc agcaattcca ctcctaagta 69360
tataacccca aatatctgaa agtactagga tccaaacaaa cagctgtatg ctgatgttta 69420
tagaagcatt attcccaata accaaatagt ggaaacaact caagtgtcaa tcaacagatg 69480
aataaacaaa atgtatgacc tacgtgcaat ggaaaattat tcaactataa aggaatgaaa 69540
ttctgacaaa tgcttgaata tggataaacc catagtaagt gaaaaaagcc actgtatttt 69600
tgtacacaaa gtacaaatat tatatgattt cacttatatg aggtacctag aatagacaaa 69660
ttcttagaga tgggaagtag aacagaggtt accagggcct gagggaaggg gctaaacagg 69720
agtcattgtt ccatcagtac agtttctgac tgggatgatt gaaaagtttt ggaaagggat 69780
actggtgatg gttgtacaac atcatgaatg tacttgatgg cactaagttg tacacttaaa 69840
aatgcttaaa aggtaaattt tatgtcatgt gtattttacc acaattttaa aaattgaaaa 69900
gaacacacaa atgaatacta tttagtaaca taaataaatg aactagtttc tcacatatta 69960
accaggatga atcataaact tctagatgtt gagtaaaaaa tgcaaataca gatagataac 70020
aaatatgatt tcatttatat aaagttcaaa aacaatatgt tctttgagaa aaatatatat 70080
aactttaaaa aactcatgaa actaatacct ttagctttag ggggatacaa ttgggaagga 70140
acaaatggag gatttttttt tttgagacag agtctcgctc tgtcacccag gctggagtac 70200
agtggtgcga tctcggctca ctgaaatctc cgcctcctga gttcaagcaa ttctcctgtc 70260
ccagcctcct gagtagctgg gactacaggc ggacaccacc acacctgact aatttttgta 70320
tttttagtag agacggggtt tcaccatatt ggtcaggctg gtctcgaact cctgacctca 70380
ggtgatccac ccctcagcct cccaaagtgc cgggatcaca ggcatgaccc actgcgcctg 70440
gccagatgga agcttctaat gtagaaataa tgttctattt cttaagctgg gtgatggata 70500
ctcagatctc cattttatta ttatttaaaa tgtacatatt agttttgtag acttatatac 70560
acacatattt cacaaatata aaatataaaa attaaacaac attagtctga ttattaatat 70620
gatgagttag aggaggggtt ataacaggta catttaggct actaatatag taatccagag 70680
atggtggtag cttgaattag aaagagttaa gaaaaaaaat gcaaaccttg agaataagat 70740
aaaaaagaaa cagtacaaac tttccatttc aaatggaggc aaaggcccac tagaagatta 70800
tctagggaaa tacactttgt tgaactcact gttcactatt aattaaggtt ccagacttag 70860
caaaattata tttcaactta gatatttttg tctaatacaa atacaaatca tttctgttca 70920
ggagaaaaga taaccctaaa agttactatt tattgccctg tagtacatca accagttttc 70980
ttatagtcta ttcctttatt aaattgccta caagtacgtg aatccttaaa tgctctctga 71040
ataggccaat catcatactt tttcttcatt aatcagtgag tttaataaaa tctgtgatag 71100
gtgttaactt tatatgtgag ctatgacttt atctttttga aaattaactg tagagaaaga 71160
aaagtgaaca gattataaaa atacttaaaa ttaaaatgag taagatatgg tgttggatgg 71220
gacatgacag ggaagggaga aaaaggttca caggtgctct gtaggtatat gattagccca 71280
aatatgatta cagtacctgc tatgttttga acatttgtcc tctccaaaat gttgaaattt 71340
aatctctaat gtggcagcat tgagaggtgg gggattttaa gaggtgacag ggtaacaaga 71400
gtgctgccct catgaatgga tttgtccagt catgtattaa tggcttatca tgggagtggg 71460
actagtggct ttatacaaaa aggaatagag actcgagctt gtatactcag cctccttgcc 71520
ctgtgatact ctgagccacc ctgggactca gcagagagtt cccaccagca agaaggcccg 71580
caccagatgc agcctctcag tcttgaactt ctcagcctct gtaactgcaa gaaataaatt 71640
ccttttaaaa aataaatcac ccagtttcag gtagtctgtt acaagcaaca gaaaatgaac 71700
taagacagta ccattcattc atacaggcaa cactggcaaa ggaccaagac tggaaggaaa 71760
gattaaaagt ttgttgcctt taagatatcc aaatggaaat gttgatatgg cagttgggta 71820
tatgggcata gtacttaagg aagaagtcag aatttaatta tatattagga aagcatgaca 71880
aaagataaaa ctaaaaaaga atgtatagag atgagattgc ctagtgggag agtacagatt 71940
taaaaggggg gaccagcaag aaaatcttat ttcctctcca tttttttttt tttttagaga 72000
tgggatctca ctctgttgct caggctgaag tgcagtggtg tgatcacagc tcattgtagc 72060
ctcacattcc tgggctcaag cgatcctccc acctcaacct ctcaagtagc tgggactaca 72120
ggtatgcact gccatgtctg gcttgaaaag aacaacaaca acaacaacaa caaaaaacca 72180
gagagggtct tgctgtgttg ccaaggctgg tctcaaactc ctggactcaa gcactcctcc 72240
accttggctt cccaaaggga ttatatgcat gagccaccac acccagcccc tcatctctac 72300
tactgctcat ccaattcatc agcaaatcct gccagcacta cttttaaatt tatctagact 72360
ctaaacattt ctcactatct ttactgctac cacagtggtc caagccacca ccatctatca 72420
tctagattac agcagactcc ttaacagtca cccttgcttc agcctatgct ctctacagtc 72480
cattttccat acagcagcca acataatcct tttaaaacta agtttaaacc ctccaatggc 72540
ttcctactgc atttagaaca aaatctaaat tcttactgtg gtgtgatttt acccagctat 72600
ttctttggcc gtatctcctt caattcccca tacttcagtc ctactctgct acaatggctt 72660
ccttttgctc aaactaggta ctctcctgtc tcaagattcc cactatctga acattcttcc 72720
ccctaaatca gaatggtttg cttcctctct tcctttacac ttttactcac tgtcaccttc 72780
tcagataggc cttcccaaac catccaatta aattctaaat gagcaccctc cagccccccac 72840
ccaaaaaaat tccaacccca ggatcctctt ttccccttt ccttgcttta gttttctaca 72900

tagcacttat caccatctgt attagtcccc tcaggctacc acacaaaaat atcagactga 72960
atggcttaaa caaaagaaat ttatttctta cagttattag tttaagccac agattgagtg 73020
gcttaaacaa gagaaattta tttcttactt cttggctatg aagtccaaga taaaggtgcc 73080
tgttaacttg atttctggtt agggctctct tcttggcttg cagacagcca ccttctgtgt 73140
cctcacatgg cctttcctct ctattcagag aggaacaggg agagagagca atgaagtttt 73200
ctggtgtctc ttcttataag aacactaatc ctatcagata aggaaggccc ttacagcctc 73260
atttaaccgc aattacttcc ttagaggccc catctccaaa tataaccaca ctggggggtta 73320
gggcttcaac ataaaaaatt tggtggtata caaacattca gtccataata tcatctaaca 73380
tattagtagc tttctgtgtc tctcatttat tttctgtctc tcaccctgga atacaaaagg 73440
acaatgattt ttgtctgttt tacttactgc tgattgccca gtgcatagaa cagtacctta 73500
catatagaaa gtggtcagta aatacttttt gaatgaattt tataaatgaa tgaaaaatga 73560
aggtccaaaa caatggaagg tcagggagga gagtagggag gggtaaaaca taactgtaaa 73620
accagatgcc caagtcctta ataaaagaag agttggctgg gtgcagtggc ccacacctgt 73680
aatcccagca ctttgagatg ctgaggctgg aggatcactt gagcccagga gttcaagacc 73740
agcctgggca acttggcaaa accctgtatc tacaaaagta caaaaattag ctgggtgtgg 73800
tggtgtgtgc ctgtgatccc agttactcag gaggctgagg tgggaggact gcttgagccc 73860
tggaggttga ggctgcactg agctgtgatc atgccaccgt actccagcct gggtgataga 73920
gtgagaccct atctgaaaaa acaaataaaa ataaaatatt aaaaataaga gtatccagat 73980
gctttgtaag atgacaaaga tgagggggaa agaagggtat gactaaaaga catgagcaaa 74040
cagactagct agactagact agtttctctc tggtgagact ttgttcattc attccttcct 74100
cccttccttt cttctcccct cccttccgga agaaaacaga caggtaaaga tttagaaaga 74160
acaatggaga gtaaatagga tactaagtct gagatactgc aagttacgag aggaaaggat 74220
ggtcacaatt tggaaggtct gacgaagtgg tatgtcctct gggtctatcc ggattttagt 74280
tcgggcaaag agctatgagg aacacttcat aaaagggaga atttggtgac tatgacacat 74340
ttccacagaa cacaatgtgt tttattttaa tttcttcagt ggagattttt tttaaagtac 74400
cttaaaacct tttgttaagg attacacagg ggctacatgc aaatttggag tacaattcta 74460
tagcaaaaac atactggtta ttttagtgaa aatggcctac agcaaatttt cagtttcaga 74520
taacaaaata tttaggtcta cagcttttat attctcattg caggtgacac ttgaattaca 74580
catttagaca gcttaactat ttctgtcttg ccagagaaat cacataaata aacacaaaaa 74640
taactcaatt gttaaattct cttaggtatc ctaactaggc acctaaaaac taacttaaag 74700
gaaagtacga tgacacagag ctgtctctca ggtcttatgg cttacatata tgaaaaatgt 74760
gaggtatctt tgttgaaaat atcctactta atagctcata aagggtaaat actattcata 74820
tagggagacc agccaacata tagtgataga gatgatttaa tttactaatt ggtgttcaca 74880
tcacttcaat ttgttaacct cagaggcaat ttttttttaaa gtcagatctc acaaactaaa 74940
agacacttta gaatgcccat gtcttatctg gatattgtca ttttactttg tcaaacttct 75000
ttaaaactag tatttcgcta gggagggaat aagttagaat gcaacctagt ggggaaatcc 75060
tgcatttgct caatgtccat agctttaca cagttccaga tgttcagtac attaataata 75120
cactaactca aagcctcttt ttgaaacttt agaaagtgca aattgcatta agacctcttt 75180
gccctcatta gtggtattgt tacaatccca gtcctctgga aaatgttttt tggcatgtgt 75240
ttaactttat aatattgcca tttactcact ttaatagcca gtactcaagt cttaatgctt 75300
ttaataggcc tgcctaaaca gactaccatc ttttgtaatc ttactcacat aatttatttc 75360
ataagtagaa cctggtatat ttttccatct aaagatgatt cttcattatt catttcatga 75420
ggtactcata tgttttaaat gtttactttc ttttattcag ttgatatgta cccatatgtg 75480
gcatatgtta gaaataaaaa agagttcctt ttaaatacta ataaaaatga aaaagtagta 75540
gtgtttttta aataactttt ttagttacag taaaaatgaa tgtccttaat ttttgctaaa 75600
tacaaactga aaatataaga aaaccaagaa tatgaatttc tattgaaaca gcttcctgtt 75660
tggtgatatt tgactcagca ctttcacctc aagaggctgg gttatatttc agaatcaaac 75720
tttcaatgaa cacaggtgca gttggaggtg cacttggagg aataatatag acaaatactg 75780
ccctaaagtg aaagtaagac ctctgttggc aatctaactg gaaatgaaga tcctacagcc 75840
ctctctgaaa aggaacactc tctctcataa attagttaaa atactgagga ctctgaaaat 75900
gtcagagctg cactactagt gcagtaatgc actagtgaca cctgatttac agttacttct 75960
tgatacattc tgataaaaat atacaatgat atttacaatg caaaaaactt caaaaaatga 76020
catttgtaaa acaactaaca tatttacaca tgaaattcta ctttcctgaa atcatgtgaa 76080
aagaaaacaa taaaagccag acgcggtggc tcacacctgt aatcccagca ctttgggagg 76140
ccaaggcaga cagatctctt gagctcagga tttcaagacc agcctgggca acatggctaa 76200
accccatctc tacaaaaaaa ataactgggc atggtggtat gttcctgtag tcccagttac 76260
ccaggaggct gaagtgggag gatcacctga gccagggagg taaagactac agtgagccag 76320
tgattgtacc accacattct agcctgggca acagaatgag acactgtctt gaaaaaaaaa 76380
aagagaaaaa agaaaagaaa acaataagaa ctcaatacta tgttatgtat ggaacaaatt 76440
cttaactctt tgaaacataa attctgaatt actgttgttt catcaacagt taattcatca 76500
atgtctaaag taatcattta gttaaaatgt atataatctg acttgttttg ctttttaaaa 76560
atgaagaact gttgactgag ttaataatgt tggctaaaat ctgaatgtat aaacttattc 76620
acacaaattc aactatatgt ttcattcttt tcctgaatag attaactgta ggcgaaaaag 76680

gcaactcgtt ttctatactt ctgtaaatgg gctaataatt aaactataat gaagtcagtg 76740
ctcttgcact attagaattc aggctgaggg aagcagatga ataagaagca accactataa 76800
tgttacctaa aagttactac tttcaagtat tgccatctga atctgctgta aatttgttgc 76860
tctaaaactt gaaagaaatt ttttggagaa agcatcgctt cacaatctag tgaataagat 76920
ccaaaagaaa aatatttaaa ttacagtgca gtgcataaca aacatagaaa ggcctcaagg 76980
cataccacta caggaaacca tcaaatcaca aaggaagaca gcaagagaaa taaagaaaca 77040
aagtacctac acaacaacta gaaaataatt cacaatgctg ggtgggcgtg gtcactcatg 77100
cctgtaattc cagcacttta ggaggctgag gcagaaggat tgcttgaggc caaaggattg 77160
cttgaagcca ggagtttaag accagtgtgg gcaaaaacagc aagaccctat ctctaaaaaa 77220
aaaaaagttt ttaaaaaatg tatctgggtg tggtggcaca tgcctgtagt accagctact 77280
caggaggctg aggttgatcc cttcaaccca ggagttcaag gttatagtga cctatgatca 77340
cacccctgca ctctgacctg ggtgacagag caagaccctg tcataaataa ataaataaat 77400
aaataaataa ataaataaat ggcagtaata actctttacc tttcaatacc taccttgaat 77460
gtaaatgaat tacactctag caaaaagaca tagagtctta attcccagag caatttagaa 77520
agagtaagaa aagaagatcc aactatatgc tgcctacaag agatttgctt ttaaggatgc 77580
acagaggctg aaagtgagca gctgaaaaaa gctattcaac aaaatggaaa ccaaaagaga 77640
gtggggttat ctatacttac ataagacaaa atagactaag tcaaatactg taaaaagaaa 77700
caacgaagaa cattatttaa caaaaaaagg tcaattcatc aagaggatat aacaattgta 77760
aatacacatg cacctaacat caaagtacct aaatatataa agcaagtatt aaaggatctg 77820
aaaagagaga tagattctaa tacaataata gtaggagaca ttgatacccc actttcaaca 77880
atgaacaaat catccagaca gaaaatatat ttaaaaacat tggacttgaa taaatttaga 77940
ccaaatggac ctaacaggca tttacaggca taccttggag atattgtagg ttcagtttca 78000
gaccaccaca ataaagcaaa tatcacaata aagtgagtca tacatttttg ttacccatca 78060
tatataaacg gttaatgttt atactatact gcagtctatt atgtgtacaa gtggcattat 78120
gtctaaaaaa atacatacct taattttaaa atactttatt gctaaaaaaa tgctgacaca 78180
gagacatgaa gtgagtacat agtattggaa aaatggtgac aatagacttg cttgacacag 78240
ggttgccaca aacctttaat ttatgaaaaa aaaaaaaccc agaatatttg caaagtgcga 78300
taaagcaaag cacaataaaa taaggtattc ctgtacagaa tatcccattc aagagcaaca 78360
gaatacacat tcttctcaaa taccataaaa tagtctccag gatagattat atattaggcc 78420
atgaaacaag ttttaacaaa tttaagtttg ttatcatatc aagtatcttt gctggccaca 78480
atggtataaa actagaaatg agtaacagga aaatttggga aaattcacaa atatatggaa 78540
attaaacaac atgttcctga acaaccaaac aaccaagggg gcaatgaaga aattaaaagg 78600
gaaatttaaa aaaaatatat tgagacaaac aaaaatggaa acacaacata ccaaaactta 78660
agggatgcag caaacgtagt cctaagagga aagtttacaa taacaaatgc ctacatcaaa 78720
aaagaagatc ccaaataaat aacattactc ctcaaggatc tagaaaaaga agaactaagc 78780
ccaaaattaa cagaaggaaa gaagtaataa agatcagaga ataaataaat aaagtagaga 78840
ctaggaaaac aacacaaaga tcaacaaaac tatgaattgg tttttgaaaa gataaatgaa 78900
atcaacaaac ctttagctag attaacaaga aaaaaagaga atattcaaat aaaatgagaa 78960
acaaatgagg agatattata actgatacca cagaaataca aaggatcaaa gtgactatta 79020
caattatatg ccaacaaaat aaactggata cctggaagaa atggataaat tcctagacac 79080
atacaaccta ccaagactgc atcataaaga aatagaaaat ttaaacagac caataatgag 79140
ttctttccaa tcaaagaaaa gcccaagacc tgttggctca ctgctgaatt ctacctaaca 79200
gttaaagaac taataccaat cattctcaaa tgctttcaaa gaagttgaaa aggaggatat 79260
acttctaaat tctttttaca tgaccagcat tatcaccctg ataccaaggc cagataagga 79320
caatacaaga aaagaaaact gtaggccaat atccttgata aatagaaaag caaaaatcct 79380
caacaaaata ctagcaaacc aaattcaaca acacattaaa agaacaattc aacatgatca 79440
aatgggattt atcactggat ggaaagatag ttcaacatat gtaaatcaat aaatgtatac 79500
agcacattaa tagaatgaag gacaaaaacc atatgatcat ctcattagat gtagaaaaat 79560
catttgacaa aattcaacat cctttcctga gaaaaacttt tactaaatta ggtatagaag 79620
aaatgtactg catatttggc caataaaggc caaatatgat aagaccacac ttaacattat 79680
attcaatggt gaaaaaattg aaagccttac ctctaagatc tggaacaaga caaggatgcc 79740
cactttcact atgtccactt aacacagtac tgaagtcctt gccagaaaaa tttagaaaga 79800
gaaagaaata aaaagcatcc aaatagaaaa gaaaaaagtg aaactgcccc tgtttgctga 79860
agacataatc ttatatatag aaacccctaa agactacaac aaaaaactgt tagaactaat 79920
aaatgaataa atacagtaaa cttgcaggat acaaaatcaa cacacaaaaa atcagtagca 79980
tttctatata ctaataacag actatccaaa aaagaaatta agagaactcc ttttacaata 80040
gctaccaaaa agagagagaa agaaagaaag aaatttaacc aaggtgaaga aaggcttgta 80100
cgctgaaatc tataaaatgt tgatgagaga aattgtagaa gatacaagta aatagcatga 80160
tattctttgc tcatagactg gaagaatatt gttaaaatgt tcatattacc caaagtgaca 80220
tattgattca atgtaatctc tatcaaaatt ccaatgtcat ttttcataga aatagaaaaa 80280
agaatcctaa atttcatata gagccacaag aagctttgaa tagccatggc agtcatgagt 80340
aaaaagaaca aaaatggagg catcacacga cctgattcca aaccacactg caaagctata 80400
gtaattaaaa cagcatggta ttggcataaa aaacagccac agtgaccaac gaaacaaaga 80460

```
gcacagaaag aaacccatgt atgtgctcaa ctgattttca acaaaggtgc caagtataca     80520
caatgggaaa aaaagactct ctaaaaatgg tgctaagaaa attagatatc cacatgcaga     80580
agaatgaaaa tggaccctta tctcacaata tttaaaaaaa aactcaaaat gaattaaaga     80640
tttaaaccca agaactgaaa ctgtaaaact actagaagaa aacagagggg ggaaaactac     80700
ataacattgg tttaggcaat gattttttgg atttgatccc aaaagcacag gcaacaaaag     80760
caaaaatagg ccaatgcaat tacaacaaag agaaaacaat aaacaaagtg gcaacctaca     80820
gattgggaga aaatatttac aagctatata tccaataagg agttaatgtc caaaatatat     80880
aagaaactca aacaactcat tagcaagaac acaaataact caatttaaaa atgggcaagg     80940
gatttgaata gacatttcta aaagaagaca acaaatgacc aacagataca tgaaaaaatg     81000
ctcaaaatca ctaatcatta gggaaataca aattaaaacc atgatgaaac attaccttat     81060
gcctgtaaca atggctactg tcaaaaagat gaaagataac aaagtgttgg tgaggatgtg     81120
gagaaaaggg aagccttgga cactgttggt aagaatgtaa attagtacag ccattataaa     81180
aaactgaatg gacattactc aaaacactca aaacagaatt accttatgat ctagcaatct     81240
cacttctgag tatttacccc taaaatttga agtcagtttg cagaagaaat atctacactc     81300
cctcgttcac tgtagcattg ttcatttggc tggaattgga gaacattatg ctaagtgaaa     81360
taagccaggc acagaaagat aaatgtctca tgcccttact ttacgtggca tctaaaacaa     81420
ctgaactcaa agaagtgaca gtaaaatggt agttaccaga gtctggggca accacccttc     81480
tactttctat ctctgaattt gactactcta agtacctcat ataagtgtaa tagtgtattt     81540
gccgttttta tgacgagctt atttcactta gtctaatgtc ctccagattc atccatgttg     81600
tagcatgaaa agaaataccc atttattatc tcacagtctc tgtaggtcag aagtccagac     81660
acagggcagc tggtatggga atgtggggaa ggttgtgcat gtgtcgggta aaggggtata     81720
tgagagctgt ctatactgtc cactcaattt tgctgtgaac ctaaaactgg tataaaaaat     81780
accatgtgac ctatggtatt ctctacattg agctgctatc tgtagaatct tttccttgat     81840
gactttttc ctttatgatc tctcatgtta gcatgacttc aagctaacat tatctacact     81900
aggcaagcaa ggtgaatctt atttctaaaa actcctcaga aatagtactt tgtacaaagg     81960
caaacaacca acgcactgat atgagctcta accattcatt caccatgtcc tgtagagact     82020
gatgtatctt ttgaaagact tttcaaaaat gcaataaccc ttgagttaac tagatatctt     82080
aaaatcgaag gctagagatt gggagagttc gcttctataa tttgcatctg ctgtatattt     82140
gagaatatat aaaatacaag aacccaaata gatgggtgaa actcttaaaa tgttaaaatc     82200
gtagctagaa gttagaaacc tgtgtaagcc atattaaaga gataaagtaa gtacagcatt     82260
tctctatgaa catagatgaa aaaggatgga aagacactag atattcaaga tacactacat     82320
atacatgagg tatcaaatat ccaagataca ttaaatatat ctgaggcata taaaatacta     82380
cccaatatcc ataaaatctt attcagccac ccattggtga cacccattct ggctccatgt     82440
atcctctgcc caagaaaggc aaataaaata cattgtagct cagcaggcaa actgatggac     82500
tacagctgtg gccaagtgca tttgcgctgg ccttactcta ataaagtgtg tcaattgtgc     82560
atctgttggg tccccttgca taaacctcat ttctagtctc tgtaagcaca ccatcagcaa     82620
aggagagctg atgtggaaaa gagaaagaca agacataaac attgcacagc ccaggattta     82680
ggtagtgtgg tccttccacc cacttacatt gtattcacat gttaacctac cctcaactct     82740
ctccccttgc ctcccactgt gtgttttaac tatctttact aaactatatg attcaagcat     82800
cccaatgtgg tgtcactttt ttgtcccatg acctctggga taagtatatt tggagactac     82860
cactgcatca agttaacgtc cctcctgaca ccacacaaat catctaaatt caaaaggtgc     82920
caatatgaaa ctgtcagcca aaaaggaaaa tcaattggtt gctattatga agattaactg     82980
ggtagatatt aaagattgta accaagatga aacaactaga acctccaagt ttttctagtc     83040
catatcaagc tcaaaaattg ctttaatatg taaaatgtac agtaaattaa tcataagtta     83100
tatttaacta atttgtctgt catttcccaa aagtgcttcc tatatttatc cttacatcca     83160
tgaaaaatag tttagaacta aggggtgggg gggagcacag ggagttagga tgaattcagc     83220
ttctaaattt gctacttgat ccctgcctgc tatggtttga atgtcccctc ccaaagtcat     83280
gttaaaatgt aattgtcatt gtaacagtat taacaggtgc agcatttaag agaccattag     83340
gccagaaagg ttctgtcctc atggatgggt ttaatgcctt taaaaaataa aggagctttt     83400
tctttctgtg tcacttgccc tttcaccttt cacctcgaga tgatggagca agaaggccct     83460
tactagatgc tggcaacttg atacttggac ttcttagtct ccagaattgt gagaaataaa     83520
tttattttct ttctgtccag tctgtggtat tctgttatag catcaaaaaa ttgaccaaga     83580
cactgccttc tcaaactaag ttacctggac atccttgact ctagtttaat cttaacttct     83640
ctgcctttat tctccctaga tttttcactt aaaaattata atacatatac atacataaac     83700
acacacatac acatatacac atacatatgt atgtgtgtgt gtgtgtgtgt atatattata     83760
tatatatata tatatatata tatatatata tatatatata tttttttttt tttttttttt     83820
tttttttttt gagacagagt ttcactctta ttgcccaggc tggagtgcat ggcgtgatct     83880
tggctcactg caacctccgc ctcctggatt caagcaattc tcctgcctca gcctcccaat     83940
tagcagggat tacaggcatg tgccaccatg tccggttaat tttgaatttt tagtacagac     84000
ggagtttcac catgttggcc aggctggtct tgaactcctg acctcaggtg atccaccctc     84060
cttggcctcc caaagtgctg ggattacagg catgagccag catgcccagc ctaatcaata     84120
tatttttata aagctttttt gaaaccagat gtgatacaaa tatattcatg ttatttatag     84180
aaagtacata aatgactata ttcatgcaca cacacacaca cacacacaca cacacacaga     84240
```

gcaaaaaaca acttggttat taaattgtta ctttccaact gccctactat tttcattcaa 84300
ttgttaaagc agctttttaa catcctcttt cagagtagct cttatatcta tcttcctaac 84360
agtttagctc ataatcaaaa acaaatgtga aaagcaaaca taaatatagg tataccaata 84420
tgtaggtatt tttatataag tgcatattgt tctaaataaa aataataggt caaattaata 84480
tttatcagat caactatatt tcaaacagta gaatactatt acgaggatag cattgaacta 84540
gaaaatcagg agttctaagg caaatgcttt cactaattaa atgtatgacc ttagccatat 84600
gaattgtcta ctcagaatgt agtatgtgcc cagtaaatgt ttgtcaaatg aataaacaaa 84660
ggaacaaaca aaggaatact ttttgtttaa taaccaagag cagaagttct caagaactac 84720
atctaacctg agagatatac aacctctcta ccacatccca aagcctcaat ggcataatga 84780
ttctttctat ttaacactgt agaaaaaaat atttcttcaa aaggctcagt tgatcactta 84840
aagcaaattt atatctatag aaatttcagc cttctacacg aggggaaaaa aaacactttt 84900
taatgtagca atttagtttc tcctttaggt taagtgcggt acagctttaa aaatgagaac 84960
caaaacaaac tagtagcact gcaaatttct taacatacaa atttagtgct gaacaatact 85020
ttaaaaaact acagctagag agtgaacagg aatgtttgca ttttattcct catctgacat 85080
tcagtgatga atgtaaaata tgccctcagg gaaaatggtt tatacatatt actcataaag 85140
atgtattttt ttttaagtcg aagtctgtat tgtatgaata aaaagatcca acttgttact 85200
ttggcaccag agagtctctt ttcaaaatga tttaatagaa aagtttctat tctctttata 85260
tggacagaaa cttctctgtc catataaaga gtttagaaac tcttctaccc ataaaaatga 85320
atttcagaga gttctaaagc aatgctgcta ctacttaaat aacaacagag tcaaaatcaa 85380
atttatctta gtggaagaat ggcattcctc ctagttacca taaactattg gtctgaaaga 85440
atgtggggaa aaaaactaga tgaacaacgt tctcactctt acccttaaac aaatgctgca 85500
gtttacatca aaagaatatt agtttttact cagtgaaaga gtaaagtttt gagtaaatca 85560
cccattccaa ttatctagta caggaagaaa actggccatt aaatataagg gggaagaaat 85620
gaccactaaa atgtatggtt tgaaaaactt tagacatata tttttctttt ttagagatag 85680
ggtcttgcta ttggccaagc tggagtgcag tggtgtgatc acagctcact gcagcctcaa 85740
acttctgggc tcaagcgatc ctcccatctc agcctcctaa gtagctggga ctcacaacac 85800
cacacctggc tgatattttt tgttttgttt ttttaaacac tgggtctctc tatgttaccc 85860
aggctggtct tgaacatctg gcctcaagtg atcctcctat ctcagcctcc caaattgctg 85920
ggattatagg cttctgcact gggcctagac ttctaccata tgtttctttc tttctttttg 85980
atttttagta gagatgaggt ttcaccatgt tgcccaacct gatgtcaaac tcctgagctc 86040
aggcaatcca cccaccttgg cctcccaaag tgctgggatt acaggtgtga gtcaccgcac 86100
ctggccctta tcctatgttt cttttatgaa actctgcaac cagtctttac attacatttt 86160
cacacatgcc aagctaaact ctgtgattat gaaaatattt tctgtgcctt tgctaataca 86220
tgtctccttg gtccagaata ttatattcct cactcccaca tctaccagta gaaatctatc 86280
cacctttctt taatacccac tgaaatatca accacctcct ctaggaaact cctctgcttc 86340
attcaatcac atgtgatctc ttcctactct taattccatt atcattcttc ttaatctctc 86400
taatgattac aaattttgtc aaataatgca aggatgtgta cacttatttt ataccacaac 86460
ccaaactgaa atcttcttga agagacagta acttgtttat ttttgaatct ttaacaggac 86520
ttagtgcagt cctccgcaca aagtatgtac tcaataatga tttcttcaat ggattaagta 86580
aataaaaata aatgtgaagt aaagttatga gatttctcag gaagatctta aatataatat 86640
aagcaaggct ctcaatataa atattttggt attttaaaga aattcaacta aagatattta 86700
aagttctttc tcaatacaaa ataagtatta gtatatggaa ttaggctgta ttgctctacg 86760
ggaaatcaca ggaattttag tttgcagttc taaaatacct ctaacttcaa aggcagtaag 86820
aaagacttgg aacaataaga gttagtctcc atccattcgt gtgtttattc atcaagcatt 86880
tatggaaaga ttattatgtg cagttgtgat gcctttgcta cctaaatttc ataaatatca 86940
tttcacttct attaatattt tcatatgcca tagtcacaaa tctttattct aatggttctt 87000
cctaaccttt ttcttaccct gataaattaa cccctgtgat aaaaagttta tgatagcccc 87060
taagattcct actccctata tacaacctgc ataaacttct ccccgttgtg agataaaaat 87120
aagactttac ttccatgatt agattatatt atgtagtaaa gatgagggga ttttatgaac 87180
gtaattaagg tctacctcaa ttgattctga gttaatcaaa agggagatta tcttgggtgg 87240
acctgactta atcaggtaaa agtccttaaa agatacaatg cacccctaca gaagagacac 87300
ctttgttggc tttgaagaag gaaaaaaaaa agtagtagta gtaataagct gccacgttgt 87360
tgagagggcg atatgactag gacctgaatg caacctttaa gacctaagag tcatccttag 87420
ccaacagccc gcaaaagact gggtatctca ctcagtccta cgactacaag ggataaattc 87480
tgcaaacaaa ctgagggacc ctggaagcta atctttcccc agtcaagcct ttagtgagac 87540
cacaacccca gttgactctg ctgggagcct tgtaatacac tgagaagaaa aacccagata 87600
agccatgccc agactcctga cccacagaaa gagtaagatg ataaatgtgt actgttttac 87660
ggtgctaatt ttgtggtaat ttgttatgca gcaacataga actaatacaa cccatctaat 87720
tatatttcaa tcctggttcc tcatgtcccc ctgaaatgaa ttctaatgta ggctttctgt 87780
cttctgcatg gtcagtatca aacccctctg aaatgaaaac tagatggcat aatattaata 87840
catttaataa ctgtagtcac tgtactatgt gagaattgac aacggtactg acaaaagatt 87900
ataataatgt gctagtttgc tcttatgact agattctgaa actcagtagc aaatttattt 87960
ttgccaattt tattggactt ctgatttcag ctccaatatg taaagaactg gaagtcatca 88020

cacctgtttt acaacagaaa aaagttaaac aaactaaaaa tcaattactt ttctgggatc 88080
catctgagaa ctgaggtcac aaggcaaact gccaccctga aatctggaga gacaggtaaa 88140
tacagagaat caagatatgc ttactgggac agcggccact aaaacataaa ttgacagaaa 88200
tagttaaaag ataatattga tgaattgctg gagataaaat gtgaactagt ttataagtga 88260
gaaactccta ggggctgcag tcttgccagg gggcccacaa tttttaagac attacctcta 88320
aaaatcccac caggttccca cagtaaagag ccaagaaaaa ccctactgtt tcgggcagga 88380
ggagggaaaa aaacaaccat tttgaaatac gttcaacgca ttctctaaaa caatggccag 88440
tctgccagta aggaagattt taccagatcc ttatccaaca tgagggaagg gatcatttcc 88500
taaactgcag ctccctgcca tctaatctta tgatatgaat gaagagctca gaaacacttg 88560
tgaaggtcac aggcaaggga cacagtcatg ctaaaagact gagacataat cataggatta 88620
gaagatgctt cctgttcccc ataccttacc accacatcaa cagggctcca gtataacagc 88680
agaggattac aacagagaga gacagctgca aggctcagat tctatttaag aataagtttt 88740
tagggacatt ggagagtaac tcaagtccac attaagaatt aattaaagag cactggtcaa 88800
gataactaca aggtagttat aaaagacagt ataaaatgtg ttttaacata gatacaaaag 88860
acaatataaa atgtattctg tttttaattt tttttatctg atttgaaagt cagttgcaaa 88920
aggtaataat tgtaaatctg tgttgagaga catacaatgt gtaaagatgt aatttttata 88980
ataatgacac aaagaaggta ggattaaaaa aacagctaca taggattaaa gcttctgcat 89040
tctattgaaa attaaattag tattaatcca aactacataa gatgttaaat ataattccta 89100
ggacaaccac taagaaaata actaaaaaat acacagtaaa agaaaagcca agggaattaa 89160
agtgacacat tagaaaaatt taaatggcac actagaaaat atctatctat tagattcaaa 89220
aggcaatgat agagaaatag aggggggaaaa ggcaccagat atacagaaaa caaacagcaa 89280
agtggcattt gtaaacacta ccttagcact aattacatta aacgtaaatg gagtaaacac 89340
tcaagttaaa aggcagagat aagcagaatg aatttttcaa atgctccaac tatatgctgt 89400
ctataagaga cacactttaa atttagacac aaataggttg aaagtaaaag gaatgttatt 89460
cagccataaa aaggaataaa gtattaattc atcctataat gctgaacatg gaaacattac 89520
actatgtgaa aaagccagac acaaaaagtc acatattgta tgattcaatt tagatgaaat 89580
gttcagaaaa ggcaaatcta tagaaatagc agattagtgg atactaggga atagcaggta 89640
agagtaggca gtgactgtta atgagttagg agtttctttt tggggtcaga aaaatattct 89700
ggaattagac agtggtgatg attacacaac tttgtgaata tactaaaaat gactcaatgc 89760
aaaaatgcgc aaagaatttg aataggcatt tctctaaaga agatgtctga attactaata 89820
agcacatgaa aatatactta acatcactag tcatcaggga actgcaaatc aaaaccacaa 89880
ggagatacta cactttatac ccatttgaaa ggctattatc aaaaaaaaca taaaataaca 89940
agtattggca aaaatgtgga aaaattggaa tccttgtgca ctgctgatgg gaatctaaat 90000
tatgcattga ctataaaaaa aacagtttgg tggttcttca aaaagttaaa tttaggctgg 90060
gcgtggtggt tcacgcctgt gatcccagca ctttgggagg ccgaggcagg cagatcacct 90120
gaggtcagga gttcaagacc agcctggcca acatggtgaa accctgtctc tactaaaaat 90180
acacaaaaat tagccagaca tgttggcgga cacctgtaat cccagctact cgggaggcta 90240
aggcaggaga atcactggaa cctggaaagc ggaggttgca atgagctgag atgatgccac 90300
tgcactccag cctgggtgac agagtgagac tccatctcaa aaaaagaagt tacatttaga 90360
attatcacaa gatccagaaa ttacatttct aggtatatat ccgaatgaat tgaaagcaag 90420
gtcacaaata gatatttgta caccagcgtt catagcagca ttattcacaa caggcaaaag 90480
gtagaaacaa tccaaatacc catcgacaga tgaatggata agcaaatgtg agattatata 90540
tatatatata tatatatata tatatatata tatatatata tatataaaat gaaatattat 90600
tctgccataa aaaggattcc gatgtatgct acaagatgga cgaaccctga aaacattatg 90660
gctaatgaaa taaatcagaa acaaaggaca aatactgtat gattccactt atatgaagta 90720
gctacaatag ccaaattcac agagacagaa agaatactag agggtaacag gaaatgggga 90780
aaaggaatct ggggagttat tttgtgggaa cagagttctt atttggtatg atgaaaggtt 90840
ctgaaaatag atagtggtct tggttgtcta acattgtgaa tgtacttaat tgtacacaca 90900
caaaaaatgc taaggccgaa cgcagtggct cacgcctgta atcccagcac tttgggaggc 90960
caaggcgggc agatcatctg aggtcgggag ttcaagacca gcctgaccaa cctggagaaa 91020
ccccatctct actaaaaata caaaattagc cgggtgtggt ggcacatgac tgcaatccca 91080
gctactcagg aggctgaggc aggagaatca cttgaacccg ggaggcagag gttgcagtga 91140
gccaagatca caccattgca ctccagactg ggcaacaagg gtaaaattcc atctcagaaa 91200
aaaaaaaaaa aaaaaaaaaa cctaaatttt gttatgcatg tttcaccaca attagaaatg 91260
acaataaata tcagaaaaat aaatcaaaga cctgcgaagc ctctggggat gtgggactgg 91320
gaagaaagga ggagtagtat agtgaaggga gctgttgttt tttcattata accattctaa 91380
cattatttaa ttatgtataa gaattatttt gataaattaa gatatttaaa gctaaaaaag 91440
aaattaaaga atttaatgac aaacaacaag gttttaatga tatgtattat aaaggattac 91500
tatccacact atatatttaa aagcccatac aaatcaatta aaaagacaaa tacaaccaag 91560
acaagcagac aataaacaaa aattttaaaa gaattaaaca aaaacctgca gaacaactaa 91620
ttgacaaacc tgacaaaaac aaaacctgac aaaacctgac aaacttgata aaaacaagca 91680
atggggaaag aattccctat ttaataagtg gtgctgggaa aactggctag ccatatgtag 91740
aaagctgaaa ctggatccct tccttacacc ttatacaaaa attaattcaa gatggattaa 91800

agacttaaat cttagaccta aaaccataaa aaccctagaa gaaaacctag gcaataccat 91860
tcagaacata ggcatgggca aggacttcat gactaaaacg ccaaaatcaa tgacaacaaa 91920
agccaaaatt gaaaaatggg atctaattaa actaaagagc ttcagtacag caaaagaaac 91980
taccatcaga gtgaacagcc tacagaacat tctgtaggct cccatcccta cagaatggga 92040
gaaaattttt gcgatctacc catctgacaa agggctaata tctagaatct acaaagaact 92100
taaacaaatt tacaagaaaa aaacaaacaa ccccatcaaa aagtgggcaa aggatatgaa 92160
cagacacttc tcaaaagaag acatttatgc agccaacaga cacatgaaaa aatgctcatc 92220
atcactggtc atcagagaaa tgcaaatcaa aaccacaatg agataccatc tcacaccagt 92280
tagaatggtg atcattaaac agtcaggaaa caacagaagc tggagaggat gtagagaaac 92340
aggaacgctt ttacactgtt ggtgggagta taaattagtt caaccattgt ggaagacagt 92400
gtggcgattc ctcaaggatc taaaactaga aataccattt gacccagtca tcacattact 92460
gggtatatac ccaaaggatt ataaatcatg ctactataaa gacacatgca tacgtatatt 92520
tattgcagcc tattcacaat agcaaagact tggaaccaac ccaaatgtcc atcaatgata 92580
gactggatta agaaaatgtg gcacatatac accatagaat actatgcagc cataaaaaag 92640
gatgagttca tgtcctttgc agggacatgg atgaagctgg aaaccatcat tctcagcaaa 92700
ctatcacaaa gacagaaaac caaacaccgc atgttctcac tcataggtgg gaattgaaca 92760
atgagaacat gtggacgcag ggtagggaac atcacacact ggggcctgtt ctggggtggg 92820
ggcctagggg agggatagca ttaggagaaa tacctaatgt aaatgacgag ttgatgggtg 92880
cagcaaacca gcatggcaca tgtatacgta tgtaacaaac ctgcacgttg tgcacatgta 92940
ccctagaact taaagtataa taataaaaaa agaaactatg agataataaa tgtttactgt 93000
tttaagccac aaaaaaatta aacaaaaacc tgcagaacaa ttaatttcgc aatcaaagaa 93060
ataaactcaa ttggaacaaa gtatattcga aggatttcct gtgtctttat ttttaattga 93120
aaagctagaa gcagaaaaat aatctattca ttttgctaat atatatacag tcactaaaaa 93180
gttatttcaa tgttatagtc tctagtcacc caattataaa tgtctgtaac taaaattcat 93240
actgtctgct atatgcattc caagcctagc agaaaaatgt cacccagaca tctcctatct 93300
cccttttgt gactatgact ggaaaatata aaaaggtttt agcaatgaaa taagattttg 93360
ctttaatcgc ttacataact gtggagtgag tacagtttcc ataatcccat aaataacttt 93420
tcttccaatt tcccggaaga tatttgtctc catatttaag aattaaaagt cctaaaaatt 93480
ttattaggta agaccaactc cctacaagtt ttattgtatg acttagtatc aagcttaatt 93540
actagttttc ctaaatataa ctgtttggtg tagatgttca cactggaata aatcttgttt 93600
tccattaaga attttaaaaa atgtttatgt ccgttttatt tttagatatt cttgtaaaaa 93660
attatctgat taaatatgtt tttgaaaaca aatcctttaa tttatctaca agagactaat 93720
tgctttagtg tatccctagc tatcaaagta catatatata aaaagacagt agaagagtca 93780
aaagtatcaa tattgtcatt caaatatatt acttccaaat gcttctgtcc ttagaggtat 93840
aacttcctgt ggaaaaaaga gaagggaacg ccacttctct acattggatt taggttagat 93900
tttgatgcaa tgtgatgtta ctttcaggaa ttataagtgg tttcaatgct ttgaatgtta 93960
ggaggaccaa aatatactaa ataatttcaa attaactaaa aaaaattatt aaaaatcagt 94020
gactctaaaa acaacaacaa aattcttctg gccagataca tgaagtaaaa tgagggcaac 94080
gaagaaagtt tggcttagat taactttaat gaaaccttca gaaatgtaat ttatgaaaat 94140
aaaaaagaat gctctgtttc tttctactct gaaaatatcg acttgtccct aagaataaaa 94200
atgtacacat aaacacttct cctatataat taacattgct tgaagacttt tattctttca 94260
gattatttta ttttatttta gagagagtct tattctgttg cccacgctgg agtgcagggg 94320
tgcaatcata gctcactgta acctcaacct cctgggctca agcaatcctc ccacctcagc 94380
ctcgcaggta gctgggacta taggtgtgtg ccaccacacc cagctaattt ttgtattttg 94440
tagaggcaga gtttcaccat gttctccagg ctggtctcga actcctgggc tcaagtgatc 94500
ctcccagctc agcctcccaa cctgctggga ttacaagcat gagccaccac gtccagcctt 94560
gctctttcag acttttaagt aaaacagagg taggacaatc aggcagtatc ttaatcaaaa 94620
aataagtaaa ataatatgtg atgtttagaa aactatagtt attaccttgt tgcctctacc 94680
ctaggaagct tactgtggta attttaaaat tagcatgcca atgtttaaaa tgtaaatgat 94740
tatttaaagg caaaagaaac aataaatatt cccttacctt gtaagatggc aagaaacaca 94800
aaattccttg gctcacagtc tggcacacag ataacaaaag tgctcccact tcatcttgga 94860
actcaaatgt ttcagtattc tggaaggtag cacagagatt ccgacccttg gggcctgacc 94920
caatggtacc aacccaaacc tagaatatga atatgtcatt attagagtta tgcctgaaaa 94980
aggcatggaa attagtattt atttggaagt atgtacataa aagatcaagc aacaagttta 95040
acaatttatt tttaaattgt caggggggttt tctattacct tcaattaact gtatacagat 95100
ttaatttata aatgttttgc tttaggcaga tcactaaatt ctcttagagt gaaaattctt 95160
tgaaagcaaa acaggttgta cctgtctctg tttacttaat agctttgcaa aatttaagca 95220
tgtaacaaat ttttgttagg tgaataaatg aacaagtgaa atgatgaata tataccaata 95280
gcagaatcag ataattccaa aatactgggg atgaaggcca gtataatttg ccataggcaa 95340
gtctcaaaaa tgacaggaac tactctttca aaaattcatt ggtgctcaaa gatccttagt 95400
catagaaaaa aatcacaccc accacatctt acagttattt gcaattctat ctcccttata 95460
tgtagagaga gggagagaga cgaaaatttt gcaaaatgtt aacaagtgat gaatctaggt 95520
gaaggatata aagatattca caatattaat attatctttc caacttttct gaaatttcta 95580

atttttttgaa atacagttga aagaatcaat gaatgaatga tcaaaatgta gcattgagta 95640
gaaaaaaaca aactttttcc tttgctctca caccacagca atcaacacag aaaacttctg 95700
tgaccaaatg tgtgggaggt tttttccaca taccaagcaa tcaattctgc aacagacatc 95760
aactgggtgt cctctaattc aattcaattc tgatactatc tactgggaga tgataacttc 95820
agatcccaga ggttgagggc tcagtcccca agactgcccc tgatttctgt ggccagtcac 95880
agttgttttt acttgcactt ctgactgatc agctagaaat cgggcatccc acaaccctct 95940
gcttgggttc aattaatttg gtagagctgc tcacagaacc cagggacaca tttacctaca 96000
cttactgatt tattagaaaa aatattacaa aggatacaga taaagagatg tatgtgggaa 96060
ggggctcaga gcttcaattc ccaggcacat caccctccaa gtgttcagga acctccaagt 96120
gttcagctat ctggaagctc tctgaaccct gtccttctag gtttttatgg aggcttcatt 96180
atgtaggcat gattgattaa accactggcc attaagataa acttaacctt tagcatcatc 96240
tcccccaagc cctccccaga ggttgaggaa tgggggtgaa aatcccaact ctcctatcat 96300
gtcttggtct tcccggaggc cagccctgat cctgatgcta cctaggagcc ccctaccatt 96360
agtcatctca ttagcataca aaatgtcact ttggagattc taaggatttt aggagttgta 96420
tgccaggaaa tggagtggaa gaccaaatat atatattata aatcacaggc atatacacac 96480
aagaaacatt attcaccttt aaagaggaaa ctctggcaca tgctacaaca tagatgatga 96540
actttgaaga cattatctgc aacatggaca aaccttgagg acattactaa gtaaaataag 96600
ccagtcacaa aaacgacaaa tattttatga ttccacttat atgaagtatt tatagtaatc 96660
agattcatag aaacagaaag cagaatggta gttaccagga gctgaggaga gggggagaat 96720
agagagctgt tgtttaatgg gaacagagtt tcagtttttc aagaggagaa agtcttgaga 96780
ttggttgcac aacaacatat acatacttaa taagactgag ctgtatactc aaaaatggtt 96840
aagatggtaa attttatata tattctacca caattaaaag ttttttaaat tttaagttga 96900
aggaaaatat tacagtgtaa cagaatccag ctgcacaact acattattat aattcagcga 96960
cattataggt taaatggact tttgtgcagt aggctgagaa catagagcct gtaataacat 97020
tcttatgaga aaacgtatgc tgaatttacc aacttcccat atttatctgt gtctctaaag 97080
aaagcaactt gaaaacgtgg aacatgtatt cttccttcac tgctgttcat agtcacattt 97140
ctcagcagga acactctggc atttggaaag cacaattctt agttacatag gagtagcatt 97200
ctaggttccc aactaccaaa tgccgtagtt gctcccaagt aattgtgagg accaaaaatg 97260
ttcccaccta taaacctgtg tatatagctt aataaagagc tagcattgtg ttaggcacac 97320
aggtgatgtt aacttaacca ctttagcatt aaccacagct taacatcacc tgtgtgccta 97380
accactttaa aatatttcat aaaaagaaac cagaaggcat aatgaaaaat aacaatctat 97440
acatttccac aaaaattaaa ataacattta gtagccatcg ttataaaaca ttaaaatctt 97500
aaatcatagg actcataaga gtacttttaa ctcatttcag atattcaaac atgctgaaca 97560
cttgctaaat accagctatt aatctaggca cagaggaaga acgtggatgt aaaaggaaga 97620
atcaggatac actgtagtct aatggaaaag agaagcataa ataattatac aagctgggca 97680
tggtagtaca tgcctgtagt cccagctact caggaggctg aggcaggtgg actgcctgag 97740
cctgggagtt tgaagccagc ctgggcaaca tagcaagcga ccttgtctct aaaaagataa 97800
agtctggcca aaatggcaaa accccgtctc tactaaaaat acaaaaatgt gccaggcatg 97860
gtggcataca cccataatcc cagctactca gaaggttgag gcaggagaac cgctggaacc 97920
caggaggcga aggctgcagt gagccaagat cgcaccactg cactccagtc tgggtgacag 97980
agcaaaacct tgtctcaaaa aataaaagta aaaataaaaa tacagagata aaataaataa 98040
aatataaata ggctgggcac agtggctcac gcctgtaatc ccaacacttt gggaggctga 98100
ggcaagtgca tcatttgagg gcaggagttc aagaccagaa tggacaacat gctgaaaccc 98160
catctctacc aaaaaaataa aaaattagcc aggcgtgcgg cgcacacctg taatctcagc 98220
aacttgagag gctgaagcag gagaattgct taaacacgag tgagccaaga tcacaccact 98280
gcactccagc ctgggcaaca gagccagact ctgtctcaaa aatcaatcaa tcaataaata 98340
aaacataatt atacaatgag aattaaagaa atcaaaattt tgttttcatt acaaaactaa 98400
aacttactta gaagaagaag ctgaaataca gccttttgca tcccaagtga ctggattatt 98460
tccttctgtt gacacatcat taagtagctg acagattttc ttttattgta aaactggaat 98520
gttgaatttc ctaccaagat ttacttgctg gcacttcagg tatcttctaa cttgtttaca 98580
tagttatatt gaagtagaaa cactgaaggc cttccaaaaa aaaaaacaac aactaacctg 98640
tgaattttta atgatatgat tagcctccag ctggatagta aatgtaacac caagttctga 98700
cgaaaaggat ttcattggtg ataatgtacc agatgtcaaa acaatggtct gaactttgcc 98760
attaatatct gaaaaggcct aaaagaaaac aacattagat aaataaaatt atctttagaa 98820
gaggctgggc aaagtggctc acacctgtaa tcccagcact ttgggaggct gaagcaggaa 98880
gatcgcttga gtctaggagt ctgagaccag cctgggcaat atggtgaaac cccgtctcta 98940
caaaaaatac aaaaattagc cgggcatggt gacacatgcc tatagtccca gctactcggg 99000
aagctaaggt gggaggatcg cttgagcctg gaaggtcaag gctacagtga gctgagattg 99060
caccactgca ctccagcctg ggtgaagagc aagaccctgc ctcaaaacaa caacaacaac 99120
aacaacaaac aactatcttt aaaagagtca accacattta ttaaaatgct ggtactgagc 99180
aagaagacaa aatttccatt tacatgatga gcttaccaca gctggattta agcaccaaaa 99240
gtttagcaca tgaactgcag ttttctgtcg tgaacgtttc ttattttttg gtagaaccaa 99300
caacccattt ttgtctgaaa tatcaatctg atttgtccag gagtaagtct gttgaatcgc 99360

aattttataa tcatctgcaa atctagatgc aaagaaagtg ctaattaagt ggcaaaactt 99420
ttaaaaccta tgacccagct acatacaata taaaaactga ttacattaaa actcatttga 99480
aagagctggt accttcccat tcaaaaataa cattctcctt accattaatt aaattcagta 99540
attctgatat taataaagcc attaatcata gaatcaactg attctcagag ggaatattag 99600
aaatcatcta accatctatc ttttatcagc tacttcctaa cctcattttc aaacattaca 99660
tatgtcatgg cagaagcaga attaaaatcc aggcctccta actcccagtc cagagctctc 99720
tgcatgctct actacatgat gagttacaaa atagaaaaca gtattatggt taaagagact 99780
aaaaggtggg ttatactttt ttattaaatg agaaaataga aggataattt ttactgaaag 99840
tttttaaagt attagcaaca agttataaaa ttcttacatt cctttgaaac ctgagtctca 99900
gccttcatct acttttaatg aaggtgctga accagatgat atctgtgatc ccatccagtg 99960
tataactgct ttcaataaga atctagaaaa tggttaagct taaaaataat aaaatgtggc 100020
caggcgcggt ggttcacacc tgtaatccca gcactttggg aggccgaggc aggtggatca 100080
cgaggtcagg agttcgagac cagcctggcc aacatggtga aaccccgtct ctactaaaaa 100140
tacaaaaatt agccaggtgt ggtggcagtc gcctgtaacc ccagctactc aggaggctga 100200
ggcaggagaa tcacttgaac ccgggaggtg gaggttgcag tgagctgaga tagcgccact 100260
gcactgtagc ctgggtgacg gagcaagact ccatctcaaa aaaaaaaaaa aaagtaaaat 100320
gtgatattct ctaccatgtc ttcgctatag tatttcctta ttcatttcac tcctagagag 100380
attagatctc aaaattaaag ttgggaactt aacaactgtg gagagaatct tataaaaata 100440
ctgggagcca aaaactgtaa aactcttaaa acaaagggga gaccgggtgc gatggctcac 100500
gcctgtaatc ccagcacttt gggaggccga ggtgggcgga tcacgaggtc aggagatcga 100560
gaccattctg gctaacacgg tgaaaccccg tctctactag aaaatacaaa aaattagccg 100620
ggcttagtgg cgggcgcctg tagtcccagc tactcgggag gctgaggcag gagaatggca 100680
tgaacccggg aggctgagct tgcagtgagc cgagatcgcg ccactgcact ccaacctggg 100740
tgacagagcg agactcccgt ctcagaaaaa aaaaaaaaaa aaggaaaaga aaacataggg 100800
gaaatcttca tatcagattt ggccatgatt tactggatgt gacactaaaa gtacaggcaa 100860
caacaagaaa agagaaagat aaattggact tcaacaagat taaaaacctt ttctgcatca 100920
aaggacacta tgaataaatg aaaaaacaac ccacagaatg agagaaaata tttgtaaagt 100980
atatatctct tacaggactg acatccagaa tatctaaata actacagctc aacaacaaca 101040
acaaaaaaac ccaataattc aaaagtgggc aaaagacttc aatagatatt tctccaaaaa 101100
aagacataca attggccaat aagcacatga aaagattctc aacttctcta gtcattagga 101160
aatacaaata aaaaccagat gatatcactt gatacctatt aggatggcta ttatacaaaa 101220
aataaaataa cgagtgtcag caaaaacaat tctgatatat gctgctacat aaactgttgg 101280
caaggatgtg tagaaattgg aacccttgtg cattgataat gagaatggaa aataatgcag 101340
ttgctgtgag aaacagtatg gtgagtcctc aaaaagttaa atgcagcatt accatatgat 101400
tctgcaattc cactgctagg tacttatata cacccagaag aactgaaagc agaactcaga 101460
tatttgtaca ccaacgttca tagtagcatt gtttacaata ggcaaaagat gaaaacaacc 101520
caaatgtcca ttaacataag aataaattaa caaattgtgg tatatacata caatggagta 101580
ttattcagcc atagaaagga atgaaattct gatatatgct actacataaa tgaactctga 101640
aaacactgtg ccaaatgaat tgaaataagc cagacacaaa aagacaaaca ttgtatgatc 101700
gctcttatgt gaaataccta gaaaggactt cataagatga aaatagaata caggttagca 101760
ggtgatgagg ggaaagactc taggggttat tatttcatgg gtacagaatt cctatttgga 101820
ataataaaaa atttctggaa atctctggaa atgagtacaa tgtacaacat tgcaaatata 101880
ctgtacttaa tggcactgag ttgtatacgt aaaatggtta aaatggtgta ttttatgtat 101940
attttaccac aataaaaaaa aaataccaga actaggtact actataattt aagttacagg 102000
atgagaggga ttggttgttt ttcattttcc acagttcata caataaaaaa gggactttct 102060
gacactgtag gaacataaga aaactttagc aatagaagat aatttaatat gaattaatta 102120
aatatattta tttaatgatt tcaatattca ttaatataaa agactaattt tggccaggca 102180
tgggctcaca cttacaatcc tagcactttg ggaggctgag gtgggaggat cacttgaggc 102240
caggagttca agaccatcct gatcaacata gtgagatctc atctctacaa aaaaaataaa 102300
aattaaaaaa ttagctgggt gtgggtgttg cacacctgta atcccagcta ctcaggaaac 102360
aggtgggagg attgcttaag cccaggaggt caaagttgca gtgagctgtg atcatgcaac 102420
tgcacttcag cctgggtgac agagaagtcc ctgtctcaaa aaagcatcag actaatttca 102480
attcctctaa gtctgtgtca ataagagcaa atatataata atatctataa cacttgtttt 102540
caattctcct atttactcac ccaaaatagg tatgtattaa acacatgcta gcatccaaat 102600
taggctattt ttaaaaggaa aatacatact agttatcttc acttacctgc tattttgcct 102660
aaaaagatag tcaagtacca taaaaagtcc tttaagcatt atttgagttg atgcactaat 102720
aacaggtact tctcttgcct cctctttacc ataaattggt gagatttttt cctcttttttg 102780
aagaacagca gaaaaatgtc cctataagaa attaccatat taagtataga ggggttggga 102840
gggaattgga aaaagaaact tctcaaaaat acaatgaaac ccaacaaaac atgcattgaa 102900
attttaggtg aacagaattg ctatagtttg atagtctgac ttctccaaat ctgatattga 102960
aatttcatcc ccagtaatgg aagtggggca tatcaggagg tttgggacca tgggggtaga 103020
tacctcatga atagattaac gccctcccta agtagagagg gggtgagtta gtgctctgtt 103080
agttcccacg agagctggtt gttagaaaga gcctgacacc tccctccctt ctctctctcg 103140

cttcttctct tgccatatga tctgtacaaa ccagctcccc ttcacctttt gccatcagtg 103200
gaagcaggct aaggtcctca aaattagatg ctggagccat gcttcgtaca agctgcagaa 103260
ctgtgaacca aataaacctc ctttctttgt aagttaccca gcctcaggta tttatagtga 103320
tacaaacaaa gacaattatt aacagcacaa tgctaacaat atattttaga ttgccatatt 103380
agcaaaattc tcctcagagt caaagactct ttaataataa gttaggtatt tagaagattc 103440
aatcagggta ttatttggtg cctttcagat gtaaggaatt aaataaaaat gtagcaaatg 103500
gcttctccaa aaggctttaa agagttgata tgttgcctta aatccatgag ttatttccag 103560
aaagtaattc tgatgctgag aataatatga ctacatcaca agcaaaacta aatgacttaa 103620
ggaaacaata catgctaaat aacaaaatgt atgttaactg gttctttggt ctttacatgg 103680
ctcataagtt ttaatatgga tgaaagtgtg gacattaaaa caaaattata agaaaacaga 103740
agagtatgtt ccccacattg taaaagagaa aattctgaat actgaagcaa taaaagatat 103800
tggtgacaac tctgatgtct gacaataaaa tgtgttcaat gaaaataaaa tggggaaata 103860
ggataaataa gaaagataca ttttaatatt cagatatatc atgaaacaat acaaatctat 103920
aagagtaaaa atcagccttc actgaacaaa acattacaga atacaaataa gtagaaagac 103980
ttaaaaaaca aaccaacaaa gaacaaacaa gaaagacaac atgaacttgg aaaaatattc 104040
agccctgcaa tgaaatacaa atatcaaatt attctttttg aaaaatgaag tcccctcagc 104100
aacaccttat atcaaagctt aatatccttc tccaacactg gcagaacact ggcaatttac 104160
tctgataaaa tatagaaaat ataaactact aaaggtgagg cgctgtacta aaaacagaag 104220
ggctaaatga aatcctacat attcaattat tagagtactc cgagctcaat tccagttagc 104280
ttacctacac attggcagct gggttggggg gggtagaaaa atagaaaact aagcaatgga 104340
aaaataaata aaaagttaat tatggttcct taggaaacat acataaagct gtataaaaat 104400
ataatcaagt aagctacatg cattactatc atgggctata ataatacata cacgtgatga 104460
tatccacgct gaatatcaat ataactatac tggcagaata gagagtgaag agagagaaga 104520
aagaagggca gaaaagcagg aagcaaagga agaagagaga gcgcatgagt gagcgtgtgt 104580
gtgtgtgtgt gacagagaga gaagagtgtg tgtgagtgtg tgcgtgtcta aaggagctaa 104640
atcatcatta tacttaacag aaagtcaaca gacaacatct aaaattgaaa actccaaaga 104700
agcagaaccc acgttatttt tttaaatgtg gttaaatatc agaagaaacc actacaagag 104760
ataaaagtgc actccaaagt gagaattggg attgaggagt gggtcagagg aatgcttttc 104820
tcaacagaac tgactcttta tattctatag gtatactact ttggtaaaaa ttcctaaaaa 104880
tattgataat cataatgatg aatgtcttag ccaactagaa ttagggcaaa tttccttaat 104940
ccaataaaaa taactataaa atatataata taataaaaat ataatatata tattatttaa 105000
cagctctaat taattttta atttaattaa ctgaattgaa ttgaattaaa attaacagct 105060
ttattgagct ataattcaca tgcttacaat ttaccttttt aaagtgagtt ctgtgggtta 105120
tttctatatt atatatttta tatattatat atttatatat aatatagtat atattatata 105180
tttatatatt ttatataaat atattttata taatatattt atataaaata tatattcata 105240
tatgtattaa tatattaata tataaatata ttttatatat aatgtaaata taaaaatata 105300
tatttatata taatatattt atatataata tatttatatt atatttatat ttataaattt 105360
atataaattt ataaataatt ttataaaatt ataattttat ttataaataa atttatataa 105420
atttatataa atttataaat ataattatat ataaatatat tgcatgttta tgtattaata 105480
aatatattaa tatataatat attatatact atataaaata tataaaaata tattatatac 105540
tatataaaat atataaaaat atattatata ctatataaaa tatataaaaa tatattatat 105600
actatataaa atatataaaa atatattata tactatataa aatatataaa aatatattat 105660
atactatata aaatatataa aaatatatta tatactatat aaaatatata aaaatatata 105720
aaacattata tattaatata ttatatatag ttatatacta tatatagtat atatatttat 105780
ataaaatatt tatataaaat atataatata tatactatat attatatata aatatataat 105840
ataaaaataa cccacagaac tcactttaaa aaggtaaatt ttaagcatgt gaattatacc 105900
tcaatagagc tgttaattaa aaaagaattt ttaatcacag caaacatctc aataaagggt 105960
gcaatactag taattcctcc tttaagcaac aaaaccaaga tgtctttttc ttttttttat 106020
tttattttat ttcttttttg agacagagtc tcgctctgtc gcccaggctg gactgcagtg 106080
gcgcgatttc ggctcactgc aagctccgcc tcccaggttt gcgccattct cctgcctcag 106140
cctcctgagt agcggggact acaggcgccc gccaccacgc ccggctaatt ttttgtattt 106200
ttagtagaga cggggtttca ccgtgttagc caggatggtc tcaatctcct gacctcgtga 106260
tctgcccgcc tcggcctccc aaagtgctgg gattacaggc gtgagccact gcgcctggcc 106320
caggatgtct ttttcattgc ctgtgataaa caacgcactg gaagtctgag acagcatagt 106380
aagaaataaa aacatgaaga aatagaaaga aatgagaaag aaagaaaccg gtcattattc 106440
agacaatatg gttatctaca tggaaaattt aagataatct gtgggcaaac ctgtaaaata 106500
attcaacaag gtggttaata agacaattat aagataaata tataaaaatc aaacaccagt 106560
tttatatagc aataacaatt aaaaatgtaa ttttaaaaat atatcatttg taatggaaac 106620
agaaacttta agctacccat gaataaatct aaccaaagcc atctaatctt ttaaaaataa 106680
aattataaaa ttttattgaa agataggaaa aaataatgaa gaactatatc ttgtcatgaa 106740
tgggaaaact tcataatgta aatgcatgaa ttctttccaa attaatctat aaatacagtg 106800
caattacaat aaaaattcta acagaacttt tcctagagat tataagctaa ttctaaaatt 106860
caaatggcag agtaaaacaa aggacagctg atacaattcc aaaggaaaat aataaagtga 106920

gggatatgcc ctactatata taagcacttt tcttaaatca ttaattaaca aatgtgacaa 106980
attagagaaa cagaatggtg gaacacaatc aagtccagat ataaattcac acatatatga 107040
aaattagagg gactgggtgt agtggttcac tcttgtaatc ccagtgcttc aggaggccga 107100
ggtgggagga tcgcctgagg ccaggagttc aagaccagcc taggcaacat agtgagaccc 107160
tgtctctaca aaacataaaa aaaattaagc caggcacggg tggctcatgc ttgtaatcct 107220
agcactttga aagaccgagg cgggtggatc acttgagctc aggagctgga gaccagcctg 107280
ggcaacatga caaaagcccg tctctacaaa aaaatacaaa aaatgagcca ggtgtggtgg 107340
catgcttgtg gtcccaggta cttgggggcg ctgaggcaca agaattgcct gaacctggga 107400
ggcggaggtt gcagtgggct gagatcacac cactgcactc cagcctggtt aacagagtga 107460
gattctgtct caaaataaat aaaaaaaataa gtaattagcc aggtgtggta atgcatgcct 107520
gtagtcccag ctactgagca ggctatggca ggaggatccc ttaagaccag aaatccaagg 107580
ttacagtgag ctatgaatgc accattgcac ttcattccgg gcaacacagc aagactctgt 107640
ctcttaaaaa caaaaaaaaa aagttaaaaa atattttttt aattaaaaaa agaaaaatag 107700
gtagcagagg tagcattaca aattagaggg aaattaatat gcactattta ggaaacagtg 107760
ctgggacaat tctctatcca tacaggaaaa aaattgattc ataaattcac aacataaaca 107820
aaatgaatcc aaggctttgt aaaagtattt ataaatccca tcctttataa acaataaata 107880
ccttaaaatt tttaggagaa atataacata ggataagaaa agactgctta aagtaaattc 107940
aaaaaccaca attcataagg gatgttcata tcagaactgt ttattatact gggaaactgg 108000
aaataaccta tatttcaact tatcagatta atcaatttag aataagtaca ctcataaaaa 108060
tgctacaccg caattaaaaa ttatgtgaag gaatctttac ttaaatagga aagtatacac 108120
aatatattgt taaattaaaa gagggtaaaa caaatgtgtc atatcagaaa tatatttata 108180
ataagcaaat atgggcatag aagaaggcat aagtagacat aaatcacaat gttcacaatg 108240
ttcatctttg agtttatgag tcattttttct ttgtgccatt ctgtattttc cagttttttt 108300
cccttgacat acattttatt tgtacatttt ataactgaca tgtaacatat acagtagaat 108360
gaactaatct tacatgtaca cctcactaaa tacacacacc tccccatgta actaacacct 108420
gaccaggata caaaacattc ctagaatccc agatgttcct ttgtgcccct tctcagccca 108480
aactgcttca aacccccaca tagttgtaac cagtacttca acttctgtca ccataattag 108540
gtttgtctgt tcttcaactt cataaaaata gatttataca gtttataccc ttttgaatct 108600
ggtttccttc acctaacata atatttaaga ttcatctaga gccaggtgcg gtggctcaca 108660
cctgtaatcc tagcactttg ggaggccatg gtgggcggat cacctgaggt caggagttcg 108720
aggccagcct gaccaacatg gagaaactcc gtctctacta aaaatacaaa aaattagcca 108780
ggtgtggtgg cgcatgcctg taatcccagc tacttgggag tctgaggcag gagaatcact 108840
tgaacccagg aggcagaggt tgcggtgagc tgagatcacg ccattgcact ccagtctggg 108900
caacaagggc gaaactccat ctcaaaaaaa tatatatata tatctatatg gttgtgttgg 108960
aagcagttca tttttattg ctatgtagta ttccactata tgaatatgct acaatttatc 109020
cttttctttt tttttaaaca gggtcttgct ctgtgcccca ggctggagtg tagtggtgca 109080
atcatggttc actgtagcct caacctcctg ggttcaggtg atcctcctgc ctcagcctcc 109140
caagtagctg ggactacagc gcatgccacc acaccagcta attttttgta gacaacaggg 109200
tctttgttgc ccaggctggt cttgaactcc tgggctcaag caatccgcca gcctcagcct 109260
gtcaaagtgt aggattactg gcatgcacca ccaccctcag ccccatttta ctgttatata 109320
agtagctttc aaaatgcaaa caatttatgc aaaaaatatt atgaaagatg tcacaatatg 109380
gaaaaattct tacaatagat accacgctgc attttataaa tgcagggcaa aaatttaagt 109440
atatcctgat taaaatatat gttttaatag tcacatgccc cgaatactat tgtgatcatg 109500
ttggcaatta ttttcatcat tttttttgtt caaaaacctc tttgagaaat aacatcagca 109560
agatggaaga atttgagttt tctagccagg cacggtgact cacacctgtg agcccagcac 109620
tttgggaggc caaggcaggt ggatcacttg aggtcaggag tttgacacca gcctggccaa 109680
catggtgaaa ccctgtctct accaaaaata caaatattag ccgggcatgg tggggcatgc 109740
ctgtagtccc agcaactcag gaggcttaga catgaggatc acttgaacac gagaggcaga 109800
ggttgcagtg agccgagatc acgccactgc cctccagcct gggcaacaga gtgagacttc 109860
atctcaaaaa aaaaaaaaaa aaaaaaagaa tttgagtttt ccattgtcat ctcccaacaa 109920
tgaaccaatt ctgacaacca ctcgcacatg agagtacctt tgttaagagt ctaagagtct 109980
agcagagaag ttccaggacc ccattggagc aaaaaatcca agaacagatg cattgaagag 110040
ggcaaaaagt atagtttcat attacctaca tcacccctgc cccatggaag tacagcccag 110100
tgccaagaga gcctccatta gcccacaatt cttcccatgg ggaaaagtga gaatatagtg 110160
agtgattaat gaaataaaga aaatgtggca catatatata taatgaaata ttcagtctta 110220
aaaaagaaga aaattttgcc atttgcaaca acatggatga aactggaagt attatgctat 110280
ctgaaataag cctgatagaa agacaaatac tgcatgctat cactcatatg tgtcatctaa 110340
aaaagttgta ctcgtagaag cagagtcctc agccccaaag ttcactggtg aagatgtaca 110400
acaacaggaa cttttgttca ttgctggtgg gaatgcaaaa tggtacagcc actttttgtc 110460
aagagacaaa gtcttgctat gttgctcagg ctggagagca gtggctattc acaagcatgc 110520
aatcatagtg cactacagcc tcaaattcct gggttcaagc aatcttcctg cctcagcctc 110580
ccaagtagct gggactacag gcgcatacca ccatacccgg cagttacagc cacttttgaa 110640
gactgtttag cagtttcttg caaagctaaa cacagtctta ccatataata caacaattgt 110700

```
gttcctaagt aaatatccaa atgagtagaa agcttgtcac taatagaaat gtttataata 110760
gctttattca caattgccaa aaactggaaa ccatcaatgt ttctttcagt aggtgaatgg 110820
ataaactgtg gtacatccaa ataatgaaat atcattcagc tataagaaga aatgagctat 110880
caaggcaaaa aaatacatgg aggaatctta tatgcatatt gctaagacag aagccagtct 110940
gaaaagacta catactgcac aatttcaact atatggcatt ccagaaaagg caaaacaatg 111000
gagttagtaa aaaaaatctg tggttgccaa gggtttggtg ggtgtgggcg tggtgtggtg 111060
gagggatgaa taggtgtaac acagaagatt tttaggacag tgaacacact ctgtatgata 111120
ctgtagtggt ggattcctag gcaaaaccta attgaactct acaacacaag gggtaaacct 111180
taaggtaacc tatggacttt aatgaacaat aatgtagcaa taatggttca tcaattataa 111240
caaatatact acactattgt aagaggtcaa taatggggaa actatggagt tggtgtgtat 111300
tgtgtatgtg tataggtgtg tagctgggag tgagagtata tgagaattct ggaccttctg 111360
cttaattttt ctctaaacct aaaacaacca ttctaaaata cataatctat taatttaaat 111420
aagtaaatag caacaaaaaa agaaaatata ttcttatcgg caaaaaacat tagaaaatat 111480
aatgaaattt atgcagcctc tatttagaaa aatgtacaaa aggatacgct cataaaattt 111540
tgcatatagc ttcataagtt tcatagtctt ccaaagccta ctcatgatct ctccatgaat 111600
atcaggttta taatttctaa attaagatga acaaaatagg aatttctcct ctttttattt 111660
tctaaaattt taacttttac aattgactaa gtaagtctac aacctaaaaa tattgactcc 111720
ttttagcttt aaacaaagca attaatccct ttaaagacta atttccttca aactaaataa 111780
tgctatattt tcacccacaa tttacccatg ccatcactta aagattatca aatttagata 111840
ataaaatttt taaaaaatta aattgctata tttaacaatt ctgggttact cactagattt 111900
aatctggatt tagtcacgac taaatcactt ctaattcact aaatacgttt cacaggtaga 111960
aaaaatatct tacctgcaaa atgggaaaag tagcagtggt gatacccatt ttgtgtaaag 112020
ttaagagcat ttcatttcca ctccatattt tacaagctga ttcataatct ctttctacaa 112080
gatattcagc gtttgcttct aaccaactga aataaaataa aacaattgtg tcaaccagta 112140
tcatccttac acacactatt tcagcagaac aagagaatca tcattattgt catgcgttga 112200
tctgtatatc ttgacattct taggacatga atgtggatta attaagacac cttttaaaaa 112260
gcattcatgt ctcatattgc aaaataaact ttatataact gaaaagaaag cttttattca 112320
gaagaaacaa acatgcatat gatttcccac ttgttttgtg gattcacatt gctttactta 112380
gctaatgtct cttcaactga gagccaggtc ttaggtactt atcccaaata tatataaaag 112440
gaaatgcaaa acaacctaat aaagaaatta aagtgttaca tattaacaca aaaaagagaa 112500
ttactagaaa atatataaat acattcattg atatatatac acattcatac attaagtgtt 112560
tgaatgccta ttttattcca gacattgttc tgggtagtga aaataacagc agttaaataa 112620
aataggcaag gtccttactc tcatggaaat tacattctac taaagggaga caggaataaa 112680
catcaaaaca aatacataaa caagaaaatg tcaggtagtc atgggatact atatggccat 112740
aaaaaagaac aagaacatgt cctttacggg aacatagatg gagctggaga tcatatcctt 112800
agcaaactaa tgcaggaaca gaaaatactg catgttctca cttataagtg agagctaaat 112860
gatgagacca catggacaag aggggaacag cacatactgg ggcctatcgg atggtgtagc 112920
gttggagacg ggaaaagatc aggaaaaata actaatgagt tataaggctt aatacctggg 112980
tgatgaaata atatgtacaa caaaccccca taacatgttt acctgtataa caaacctgca 113040
catgtacccc tgaacttaaa agttaaaaaa aaaagttcta ataaaatgaa aaaaaaatca 113100
ggtaattata tacaaataaa ttatgaaaat aaattagttt aatataacct agttgcaact 113160
ttaattttaa tgtcaagaat gctctccctg aggtgacatt tattctaata actgaatgca 113220
aaaagtatcc agtcatggga gaatcagaag aaaagaaata tccaagcaga aggaacagct 113280
aggagaaagg tcctgaggtg gttagcaaat tttgcttgtt tgaggatctg aaagaaagcc 113340
aatatgattt gcagcacaat gggcaaaaat gaataatggt atgaacaatg tcagagtgat 113400
aagcagaaat cagtcatctt gagtcatcat aagaaatctg aatttattca aagggcaatg 113460
ggaaggcttg ggttttaatt tttaattttc gtgggtacat agtaggtgta tatatttatg 113520
gggtacatgt tttgatacag gcatgcaata tgtaataatc acatcatgga agatggggta 113580
tccatcctct caagcattaa tcctttgtgt tacaaataat ccaattatat tcttttagtt 113640
atttttaaat gtacagttaa atcattattg actatagtca cactgttgtg ctatcaaata 113700
ctaggcctta ttcattcatt ctatttttct tttttttgta ctcattaacc aacccctcct 113760
cccctgtgat ccccatctac tacccttgcc agcctctggt aaaccatcct tctactctct 113820
atctcaatgg attaaattgt tttcattttt agatcccaca aataagtgag aatatgtgat 113880
atttgtcttt ttgtgcctgg cttatttcac ttaacatagt gacttccaat tttatccatt 113940
ttgttgcaaa tgacaggatc tccttctgtg ttatggctga atggtactcc attgtgtata 114000
tgtaaccaca ttttctttat ccattcatct gttgatggac atttaggctg ctttcaaatc 114060
ttgactattg tgaatagtgc tgcagcaaac atgggagtat agctatacct ttgatatact 114120
gatttccttt attttgggta tatatctagc agtgggattg ctggatcgtt tggtagctct 114180
attttaagtt ttttgaggaa cttccaaatt gttcttcata gtggttgtcc taatttacat 114240
tcccaccaac agtatacgag ggttgtcttt tctccacatc cttgccagca tttgttattg 114300
cctgtctttt gcataaaagt cattttaact ggggtgaggt gatatctcat tgtagttttg 114360
atcagcagag aaatgctgat caatgatgtt gagcactttt catatgtctg attgccattt 114420
gtatgtcttc tttagagaaa tgtctattca aatcttttgt ccatttttaa ttagattatt 114480
```

agatttttc ctacagagtt gtttgagctc cttatatatt ctggttatta atcccttgtc 114540
agatgggtag tttgcaaata ttttctccca ttctgtgggt tgcctcttca ctttgttgat 114600
tgtttccttt gctgtgcagc agctttttaa cttgatgtga tcccatctgt ccatttttgc 114660
tttggttacc tgtgcttgtg gggtattgct caagaagttg ttccccagac cgatgtcctg 114720
gagagttttt ccaatgtttt ctggtagtag tttcatagtt tgaggttgta gatttaagtc 114780
tttaatctat tttgatttga tttttgttaa tggtgaaaga taggggtctt gttttattct 114840
cctgtagatg gatatccagt tttcccggca ccatttattg aagagactgt ctttccctca 114900
gtgcatgttc gtggcacctg tgtcaaaaat gagttcactg taggtgtgtg gatttgtttc 114960
tgagttctct attctgttcc attgatctgt gtgtctgttt ttatgccagt actgtgctgt 115020
tttggtggga aggttttaaa tacagagttt taaataaaag acagactatc tattttacat 115080
gtttaaaaaa tgattctagc taccaagcta gaattgatta ctggagaaag atgagtgaaa 115140
gtagagacct gttaggaggt tgttataata tccttgttga gagaagactg taggatgata 115200
gatatgaaca atcaatgata ttgaggaatt ggggatatac tgtgcaggtg gcatctataa 115260
gacttactca ttaattggat gaggtgaggc agtaaggata ttattaagga tagtatttaa 115320
ctttttact tgaggaactg gtacatactg tttgcttaga tgaggaagac tggggagaaa 115380
acagaacaca ggaagaaaag caattctgtt ttagatgtaa agttgaaatg tctattttga 115440
tatacaaaaa gagctataaa gtagatagtt ggatataaga atggcattct agggagaggc 115500
tgaagctaga gacataggtt caggcctcac cagtactgaa gattatattc taagctatga 115560
tactacaaac tatttaggga aaatagaact acagaagaaa ctaggtccca gtaccaagta 115620
agtcctgagg caccccaaca tttagatatc aaaaagaata agaattatca aaagaaatga 115680
taaaaaaaaa tggactgtga gaaggaggaa aatcagagat gtcacagaag ctaagacaaa 115740
aatgcttttc aaagaggtag tcatcaacta cccaacacca ctgactggct gagataaaag 115800
aaacaataac ctctgtattt ggcaatatag agatcactag tgatcttgat caaaacaatt 115860
tgcgtagaat tgtccagaga gcaacactgc taagatcgat gagggaagaa tgtcaagtga 115920
gaaaatagaa ttagcaacta cagacaactc tttcaacaag ttatgctgta aaataaaaac 115980
cacaaaaatc tgcatggcaa aacaccacca gaaaagacac aaatggcaaa atggggtgga 116040
aagtatttac aactcttatt gcaggcaaag attttaattc cctaatatat gaatggggtt 116100
ctataaatca ttaagaaaaa gaaccacaat aagaacaaaa aaaggacaga gatgtcaaca 116160
ggcagttgac acaaaaggaa atataaatgg cctttaaaga tgcttaattt catgcatatt 116220
aaggaaaatg aatattaaaa ctacattggg ataccacttt tcacctagca ggctagaaga 116280
aaacaatttc catactctta tacattgctg gtagaagcat acattagccc ccctgtcatc 116340
cacctattgt gtaaatgtta gctatatcta ttgaaattac aaatatgtct atgctttcac 116400
ccaacaattc cacttctaaa aagattccta tagatatatt tgcatacaaa tgaaattatg 116460
tatgttcaaa gttattcatt atagcagttt aagagcaaaa aactagaaac aacctcctct 116520
attaatcaga ggctggttaa ataaactatt gcatatctat ctaatggaat tctagatatc 116580
tattttttttt ttaaactgag aaagctttct aggtcttaac atggaaaggt ctccaaaata 116640
cactgggaat tgaaaaaagc aagttgcaga aaaatgtatg tatactatgc catcatttgg 116700
aaaatagtga gggacaagaa aatatatcta atattggttc ataaatgagt aatatgcatt 116760
gaatgataaa cttgtttagg agaacacaga cattaaacag atcagggatg gaactggaag 116820
gaggttttta ctatacaaat ttgtatacat tttgtcttca aaccatgtga ctgtgtaaca 116880
ttcaaatttt tttaaagtgt aaatgtgaag gaaacagaaa aatgctacaa taaatgaaat 116940
gggattcaga gttgggaagt ttttttctat ggtaacaaga ttctagatca acaacttaat 117000
gtctaataaa agaaaaatta gtgtaaattg tcataaagct agtactaaat atattatttt 117060
agagtactga aatcttgatt aattaaaacc catgaaacta gaatcctatt taaattggag 117120
aataaagcaa taaattaaag ccatgatcca gaattttaaa atatattaat taaaagcacg 117180
tttagtttct ggttcaatat cctagacaca tccttcgggg ctattatgtt atcctgccaa 117240
attaggaata agattcacaa ttattcattt ttaaaaatca cttatgttca ttatattctt 117300
gtgaacaaga caaaaggttg gactagcctt gtttttaaag cttaactggc aaggaacaat 117360
tcatttccca agaagcctag ttaaccaaag tttactaact ttaaatactc tggcataatc 117420
aaacatattt ttcatataaa ggcagcacaa atacactaat agacaaatct tcttacttaa 117480
tgaggctaca gcacacagct cgtaggggtt catgatcttt cttccttata ttattgttga 117540
ccatactatc tagttcatcc cgagcaaacc gaagctgaac ttctgttaca ctgtaacttg 117600
ctgattcccg agcacagtcc tcgatgttat gagcttcatc taaaatgaca acctgttctt 117660
tcagatttaa atccatctat aagataaaag aattttcttg taaaacattt ggcaaaatag 117720
atttaacaac agcaggcaag atatttcatt ttaaaattca cactataggc caatattgca 117780
aatgcaatta cataagcaac agagattcta ggtcttaaat aaaacttctg aagcactatg 117840
gccaaccaaa tatcagctct acattattgt gtttgccatt tttttactct taaaatactt 117900
tgcaaaccta tcgacagcaa caaaaaatatg tcagtgagtc tggatcaagg taccacaggc 117960
tacacagaaa ggtataaaat cccactatga aagtgtttcc aacataattt tacaaactaa 118020
ttataagacc tttaaaaata aaataacaat ataagattaa aagcataaga cctttaaaag 118080
taaaataaca atacaagatt aaaagcataa aacctttaaa agtaaaataa caatacaaga 118140
ttaaaatgac tcttaaaata ctaacataat atatatgttt tgttttaatc tgtcctgcaa 118200
tccttctcca acatgggagt tggagaattg ttcttctcca actcccatgt gatgctggca 118260

ggactattaa cctgtgaatc aaaggacatc accactttca caatgggggt aaggcccagg 118320
attgtcagtt aaatagagta ttccatcctt ctgtggtcac agtgactggt tcaggttcag 118380
gaacagacat gtgatccacg gtaggccaac cagagtaatt tggagggact aatatgtggc 118440
catgctcttt tctccaggtg agctagctgc ttatggttat ctttgttcca taagcaagag 118500
tgagaacttg cctgagaata aagccatcac aataaagaga atcaagtatt ttttcttaag 118560
catttttttag ggtaggtttg tgtcacttgc aatagaaaga gtactgacta atacaaggaa 118620
ctgtcacaaa gcaattgtca atgagttatt ttgaggaagt aagaatacac agacagctga 118680
aaagttgaga aaaaaccatg gaagagtagg aaatatattg gggtttaaaa gataagtagg 118740
atttggattt ggacagacaa agagtggaca gggcattcca aagaggtgtc atcaaaagca 118800
aagatagagt aaagaacaag gtatcctcaa gggagtatga ggtaattttt gaaggattca 118860
aattgggcag atccaggtca gactattgag catcttgagt gtaagcttaa gaaatatgaa 118920
ctttatctca tgagcaaagg aagcaataga tttttaggca gataaaagga tgtaataaaa 118980
gcaatatttg gaaaactgac ctggcattga tatgcacagt gtctcagaag gaagagatct 119040
agttaagaga taaccaaata agaggtagga tagaaggggt agtaaacata atgcctatta 119100
atttggatct taaagaaata gaaagctgag atgaattgtg cagtcagatc tgaagacagg 119160
aataacaatt gactatgcca aattattcac tttttgtctt atgctgttaa caattgcatg 119220
tctctaaaaa gaaccaggca aacaaacaca aatgccatta tctgaagttc acttctcaaa 119280
gctcacaagg cataatttta ttcccccaca taagttcttc aaaacatagt tttttcagag 119340
aaatgtttct aaataattct ttttgaatat tcataaatgc aaagagaaat cctgctgtaa 119400
tcccacttca gtcactatgt tccttaaaac tgcttgttat tttggaaatt aaaatgagtg 119460
ggttgatttt aacccatgtt aggatatgtt ttcagtactc taatatgttt acacaaattt 119520
atttacataa attaaagcaa aaaaaattaa aacatctaaa agcttttaca ttcaacattt 119580
acatctccat gagtaggaag aaggttctca tttttacaca tatactcaca ctttccctta 119640
tttgtgcatc tagaagatag ttgtagggac aaaatatgat gtcagcatct tgtattagtt 119700
ctcgggctgt gtaatatgga caggccttta gtttcttccc caggctgaca agttcttcta 119760
tatcccaggc tttgcacatc ccttggaaag tctgtaatgt gtgctgatca ctaattttat 119820
gaactccatg ataaaaatag caggattttc cctagaaaca aatatgcata actgaaatgt 119880
gaaccaatat tagcatagaa ggaataaaat aagcttatct cagaatttga ggaacatcat 119940
taaagccaca aggctaagat tttactggct acgccaccac acctggctaa ttttttgtct 120000
tttttgagac aataccatag ctacaatcac aacgaagtct ggttgactca gacttagata 120060
gaagtccaaa tcggaagagc tccaaatacc ctaaattaac ttgaaaaaaa tttcttttaa 120120
atatgtttgt tatgcctttt tttttctgat taaactgatg ttaggcatga aatacttctg 120180
aaaggattca caaaacaatg gtatcactgg ttgcttccaa ggatgagaat taagtgactg 120240
ggcacaggag tgagagattt ttctctgtag accccttttgt accttttcaa ctttgagcca 120300
tgtgatagta tttcttattc aaatataaat aagaaggaag gaaggtaagt aggtaggttg 120360
gaatgtccta aggggaaaaa aagaaagtaa gagtattcac tacaaaatat tcaggagtac 120420
caaaaaaagc ctaaattatc cataattcta ttacacaaag aatgcactag aaacattttg 120480
gtgtatttca tttcatattt cttctataca tgtgtatata aggatatctc tacaatgtga 120540
cccaatatta aaataacaag aacaagcaag gcacgatgac aggtgtgagc actttgggag 120600
gcttaggcaa aaggactgtt ttaggtgagg agtttgatac cagcctgggc aatgtagcaa 120660
aaccctgtct ctacaaaaaa taaaaaattt tctaggattg gtggcgtaca tcaatggtcc 120720
tagctactca ggaagctgag gcaggaggat cacttcagcc caggagttcg aggatgcagt 120780
gagccatgat caagccactg cactccggcc tggatgacaa aacaaagccc tatctctaaa 120840
tagtaataaa aacaagaaca aagcaaatgt tccccaggca ttgaggatta ttaacaattc 120900
agccacaata aacaaaaatt tttataaagc atattccctt tgttttcttc tgtctagcgt 120960
attaaaaatc atacaaggaa aatcttgaca tcactaagca tcttttcgtt gctgctcata 121020
tatgtacatt gaattaatgt gccatagctg atgaaccagt tttcttatta ctgggcttat 121080
agactgttac caatttctgc tttagacata aggctgcagt gagcattctt atgctggttt 121140
ctaaatattt tcttagtcta tatttttaga aatggcatta tgggatcaga cgctacaagg 121200
tatttttaag tttcttgaaa acatatagtg aaattatcct ccagaaagta tatattgctt 121260
tgcattaccc ttttcccaca tccttatcaa cactggtgtt atcttttgtt atctctatct 121320
taaaagtaaa aaatagaatc ttgttttgat ttgtattttt tatagtttct agcaaagttt 121380
aacatagtgt tgtatgaata gtctaacaag gttcaatagt aatttgtgtt tcttctttta 121440
tgttatctac taatgccctt tgccattcta ttaccttttt ttttttcaga gatgggagtc 121500
tcactctgtc acctggggtg cagtggcacg atccaatctt ggttcactgc aacctccgcc 121560
tcccaggttc aagcaattct tgtgcctcag ccaccaagta gctggaacca caagtgcatc 121620
accacatatg gctccttttt tgtagagaca gggttttgtc atgttgctca ggctggtctt 121680
gaactcctga actcaagcga tccgcctgcc tcggcctccc aaagtgctgg ggattacagg 121740
catgagccac tgcactccac cccattctat cactggaaat aaaaaaattt cattacaggc 121800
tgggcacagt ggctcatgcc tataatccca acattttgag aggccaaggc cggaggactg 121860
catgagccta ggagttcaag accagctggg cagcacaggg agactcaatc tctacaaaaa 121920
aacaaaaaaa attaaaccgc agtgagcagt gaccacgcca ctgcactcca gcctgggcaa 121980
cagagtgaga tcctgtctca aaaaaaaata ctcattatgt gcattaataa aaaaataaaa 122040

tgtttgataa aacccagccc taacaagtgt ctcatatact attaaaaatg tacaaaatta 122100
gaaaagtata tatattttta gtagaagtat aaaattctag tagtagaagt acataggcaa 122160
tttgtcaaaa aactaccaaa atttaaaatt ttaccattaa tgtgaataga tgtaccctgt 122220
actgttaaat agccaaaaaa ttgaaaatta accaaatgtc tacccataag ggacatgtta 122280
aataaattat ggtaaattaa tacaaaaact ttagccctta aaaatgattt agatttgtct 122340
atgtgttgat taggctctct atgttaagtg agaaaaacat gttgaataac gtgtatagaa 122400
tgattaaatt catgtaaatt gcttttttaa agaatatgtg cacatacatt atagcaaata 122460
catttttaa acattctggt agaatagata tataaaatct tacagtagtt accttaaagg 122520
attaaggaga tggaggatct agaaaaagcc tctcttttca ttttctaacc acatacatgt 122580
actacttctc tttttcaatt gtcattggaa ctaagtggta gaataatggg ccacttttat 122640
tttaattaat tctactattt attttaaaac gattttgaaa taaaaatatt tatttttcct 122700
agtttgctat ccttaacaaa aaacaaccat acataaaata tataaattat atcagggata 122760
aaagactttg aggcagctat atacatatgt gtgtgtgtgt gtgtgtgtgt gtgtgtgtgt 122820
gtgtatgtgt gtgtacatac acatacatat atacatatgg gttcaagcac ttctcatgcc 122880
tcagcctccc aagtagctgg gattacaagc acgcatcacc atgcccagct aatttttgt 122940
atttttagta gagacaggtt ttgccatgtt ggccaggcca gtcttgaact cctgacctca 123000
agtgcccacc tcagcctccc agagtgctgg cattacaggc atgagcatca tgcccggcca 123060
atgtattttt tacaaataca taataatttc cccatatata tatattcaaa atatttttgt 123120
ctaataatta ttaaaattat aaaatataaa aataattagt ctaacaaggt tcagcaataa 123180
tttgaatttc ttcttttagg ttatctacta atgtcctttg ccattgtatt actggaaata 123240
aaagatttca ttacaggctg agcgcactgg ctcatgctta taatcctaac actttgagaa 123300
gccaaggcag gatgaaatgt ctctctcttt ctgtgtgtgt gtgtgtgtgt gtgtgtgtgt 123360
gtgtgtgtgt gtgtgtgtaa aattaaattg tatttgtata cgtctgtata ataaattaaa 123420
ttgtatatat atacacacac atatatatgc atatatataa tctttacatg tatttttatc 123480
tatgaattat ccaaaaggag aatccgtgtt ttcaaaacat acatacatca atacatacac 123540
aaatgtatct agatattgct agaaacaaag taaaccataa gagattcata acctataaaa 123600
gaatgtcagc aaatcaaagg acaagacatg aatagctgag gaccagttct cctaaagctt 123660
tgtattcagc catgttaaag gatgatacct gttagtcctc caaagtatta aaaataaaaa 123720
ttaggaggaa aggactcatc taactatatt tatttaggcc attttctaca ttattttcac 123780
tcactttctc taacctctac tgttcttatt cttcatactg tgtcaacttc tccctacctc 123840
aatgccatct ttggattttg tttctagtgt aaccaaaata gcaatggatg aggaatcaga 123900
aaatttgggt tctggtccct ccttgctaat tacttgtctg actgagaaaa ctccagggcc 123960
catttttctc atgtgactaa gcatcaaaag aatatttgaa ccagaaggct tgtaagattc 124020
cttctgactc actgactata tcatctctaa aattccttcc agctctaaaa ttctgtgatt 124080
caggtaaaaa cgtgtcaatc atctttcata ctctcagttt gatacagact gagtttaata 124140
ataggacatt caccatttct tcattccaag cttcttccat acaattctga aagcttacct 124200
ttatacaaaa tgatcactaa agtgccttta gaatctatga ctatgagcta ttaagtctca 124260
ttttaaccaa aaaaagatat atatctaaaa taatatttca gaattgtcat tcctgactct 124320
agttaaaatt attttttctt aactgaaaca aaaattaact tttaacagat aagtgcatac 124380
ttttccagta taagggcact ttagtaaaag aaacaatgaa caaaaacaca tgtgccttgg 124440
atcaagccaa aaaatcagaa agtttacact ttaagggcag ttagaatgac acccctctgc 124500
aggctgacag caaaaccaga aaagctcatc cattttagca gtgtaacaca accctgagga 124560
agtctgtcaa ttgaagtgtt atcacacttt aaacacttcc attcaaactg tcagttatct 124620
atttatcatt tcgctttggt aattcagtca aagtaaacac agacacctat ggtgactttg 124680
taattgccct tcattacaat accaaattac taacagggct tgagaacaag aaagaggatg 124740
ttcatgtatg cccgtgaggt aagcattctg aggcagaatt aggtcaagac aaaccacaag 124800
cagatatctt caattattca tattactaat attaaaaaat aaaatacctg ttcactttaa 124860
gacaaactaa aggccaatga aaaattcgga acattgaagt ctactgaatt cctttttatt 124920
tttacatgat tcaaacttat gagtaaagat caataaaaac gtaaccagca ctgtaggatt 124980
ctaggttaca caaaatgaaa ctgtctgcct tcaatacaca atcttacact ttatcattgc 125040
ttttaccaat gtattttacc aatgtatgtt tttggttggt tggttgtttt tgagacaaga 125100
gccttgctct gttgcccagg ctgcagtgca gtggcgcgat cttggctcac tgcaacctct 125160
gcctcctggg ttcaagcgat tctcatgcct cagcctccca agtagctggg attacaagca 125220
tgcgccacca cgcccagcta attttttgta tttttagtag agatgtgttt tgccatgttg 125280
gccaggccag tctcgaactc ctgacctcaa gtgcccgcct ctgcctccca aagtgctggg 125340
attacaggca tgaaccatca ggcccagcca atgtattttt tacaaataca taattaattt 125400
ccccaataac aagtaaaaca aaaaaagata aaaatatggt gacaatgcca gcaagtcttc 125460
ttgcataggc tatatagcca aaaaataaat ttcaagcatt tgaggaattc agcagatcac 125520
ttagtcacta tggaaagcag tctacttatg catgaaattc atggagtttt accataaaga 125580
gctttatttt attattttg aacacctgac acttaatact ttcatctctc cttgaagaga 125640
agtttttaa tatacagagt aaaggaattt cacaattcct cagttagtta gaaataactc 125700
tttggaaata aatatgactg taggatttca acaatgactt tgattcagta attctgtatt 125760
ctctcctggc tcggaacaag tcactcaagt tttgccattg gaacacctta ctacttatac 125820

tttataatct cgtttgaata tctgttaaag tatcttttga ccatccctaa ataaagttta 125880
aataaatttt catctataaa agagagtatt aaggaagaaa agcataaaac aaaataattc 125940
acattatatg gtagggggtg atatatcaat gaaatgaaac tctgggggct atagctcaca 126000
gtttttgtg tagaaccaac tctaataaga tactagtgaa aaacaaaaca actctaaaaa 126060
tatattaaaa tatcattggt cacttcatag taatcaattt atggttcact gatttggggg 126120
tataatttca tacttgcaac ttcaatttta gagtaaacta attatcacac cagaacatag 126180
gctcccaaac taatactgcc aaaagaaatt atttgaaagt catataagtc tttagtaaga 126240
aagaattctc tatttccacc aagcccaaat tcatattatt ctaatttccc tggaaaattt 126300
caaaaccacc aatgacatct tttgaaagtt aataatttag tataactttt aaaagagggg 126360
tttgtattta tcagaattat gaggtgggat aatcattata tttctcattc aattaagaaa 126420
attcaaactt tgcccagaca gcctaaataa actatctgct tttgaagttt ttaagtatat 126480
aaagtatatt aattagatct aattagacta aaccatttaa acacttctat ctcctcatat 126540
gatatcatcc aagtttgact atttttggta gcaggattta aaagaagaaa ctaagatgtc 126600
tgactacaac agaaattaat ttcaaggaat taaatatccc aagctttaag accaaatccc 126660
atactttaaa acttgactaa agattttatt acaggaaaat tgttttttaa aagatatcaa 126720
tactagcagt taatttgatt ttccgaagtt gattatcact aaaatgtaca tataaaacac 126780
atactgagta atttaaatat tttcagcctt attttttctc taacacaaaa taactttact 126840
cacgtttttc ccatctagca attccatgca cttctcattt ctgttgaagt taccgactac 126900
ctcaggatgg acacaagtat gatccctgct ggaaagaata gtcattggaa cccctgaata 126960
tgccgtcctc cggagctctc tagtaatctg agcaatctgc ttgtgtgtgc gtgtcccaaa 127020
atatattttg ggtatcttgg atttccctgt atgatccttc ttaatggtat tcgatgactc 127080
ttgactgttt ccttgtttag tagaacaaca gcacctagaa cagtggccag gggcctgtaa 127140
gaaaggaaag aaacgataac taatatctaa actaccataa aaaacgttat caaacctcac 127200
atggaatcag aacctgtaag taatgaatca caatggtcaa ataaagagaa ataacaagaa 127260
attatagtat ctaaaacttg ccattaaaga aaaaactaca atttaaaatt ggtcctcatt 127320
ctttctttta aaagttataa atgatatttc caaatttgca ttctttccaa cataccactc 127380
taactctcca attccaaatg ttttgagtac attttaactc tcctctttca taaattataa 127440
aaatgataaa acaaagtagg aacaatttta attttttctt aggcttcata cgaacttgtg 127500
atattatagt ttagagttaa tagttttgtt cacagcatag ttcattcaaa tattccttaa 127560
tacaccaatg cttttgaagc acttttaaat atagtttaca aaagaataga attatcaagt 127620
tgaatctaaa ggatcactta acaacttcaa aaagagaaaa ttcttcttta gcaaaggtac 127680
gaaaataaat gcaaaatatt aatatttaat aatgaaagca ggttgcttgt gtggaacaag 127740
tatatctgaa attcagttac ctgtcatgga gtgtaaacca atcagtaaaa ttatcatcca 127800
gggtagtttt tcctggacaa tttcacttgt aactcagttt cataactgaa atagttatat 127860
aaatgtctct ttaaacaaac catatggaac atcaggaata tagtctactt aatttacctg 127920
atttttctct tctgtttcca aattctcaaa tactaaaaaa aaaagtttaa tgctatatat 127980
tagaactcac aggttcttaa taactacttc cttctaagtc tcaattatta caccaaatga 128040
cacagaacaa agtctcattt tagagaaata gtgtaaaatg gttaatttat agaagtggtc 128100
attgatgggg agggaaaaaa ggtaaatagc attgtaactt aaaagactgt gtgctaaaga 128160
gttttaattt agtagaacaa acactctact tgactgtttt tttttattat tgtaatcagt 128220
aagattagcc ttcataaaca aatgctaagt catagcatgt tacaccacaa tactggggat 128280
gaagctgtct tttagcctca actcagtgga aaagcaagtt caaattactc ccagtctcac 128340
tcaaccttgt ccgccattaa atattacaat acatgataga atggatgtaa atgtgaccct 128400
tctggagctg tcactcactg ggataaaagg ttcactgatg tgccaaataa acagtcagtc 128460
aaagcagata aagttacagc catgctggaa ttgttacatt ctcctctcag gttgccagta 128520
acagcagatt tcattacaga gtgctgccac attaaatagc cagttccaaa tatcctgcct 128580
tctatattga ataattactt attcactaaa aggataaaaa gaagagttat gaatggggct 128640
cttgtcaaca gtgaggcagg aaacagctga catgtggttt atgttacata tgttgcaata 128700
tccaacaatt tacggtcaca gagaaaatgg aatgcaaatt atatcatgat atttctgact 128760
gaattctttt tggcttttgc aatgatcaca ttaatcaatt aacatacaaa aagaattaag 128820
gtaactcaat tagaattaat ttactttttc ataccatcaa ttttttccct caattatact 128880
atttctcttc cttatgatac agttaaattc atgtgaatac tagaatgcta tggcccaatg 128940
cactgttatg tactatatag atacattcta tctaagcata aaagtaatcc aataaagtag 129000
ctttttctca aggctgggaa aacttacttt ttgatgttac acttttcaac atcaattcca 129060
cttaaagctt taccatctac tccctttaat ttcacctttt ctcttggtca aaaaaattca 129120
aacaataata cagaaatatt ttttaaaaat caactatccc atcacgaata atttagtgta 129180
taacttactg accttcatat atacatatct ttcatatctt tttttcaaat agaaagggat 129240
gctattatac agttttacaa ctagatactt tgttttcaaa gtttttaatt tttaaaaggg 129300
gtgaatttgg cacaaataat ttaatgttct acttatcttt aaatgttaca ctagtagtgg 129360
tactaacaaa agcaaaagat caagaactag tctcagaaaa cttttctcac tagagtgact 129420
ttagttagga cttgaaatct gttttttcac tccataattc agaatattat aaaccacagc 129480
taactttata ttaaatgttt ttgtttgttt ttttaagttg taaagtaata tgaacaaatt 129540
ataacatcta tgaacttaag tagactcctc aagaagatgg ttttcttttt ttgtttttct 129600

ttttgttttg cttttttttga gacagacttt cactcttttt gcccaggctg gagtgcaatg 129660
gcatgatctc ggcttaccac aactccatct cccaggttcc agcaagtctc ctgcctcagc 129720
ctcccaagta gctgcgatta caggcgtcca ccaccacgcc tggctaatgt tgtgttttta 129780
ttagagatgg ggtttctcca tgttggtcaa gctggtctcg aactcccgaa atcaggtgat 129840
ccgcctgcat cggcctcccg aagtgctggg attacaggtg taagccacca tgcccggcca 129900
agaagatggt tttcaatagg tacttaggca agtaaggtat aaggtggaaa ttaaaagtta 129960
attcttttcc ctttgcatta ttaaacagga attatttctt cagaagcaat cttctattaa 130020
agtttccatt taaaagattc ggtacaggcc gggcatggtg gctcacatct gtaatcccag 130080
cactctggga ggccgaggca cgtggattgc tttgggtcag gagttcaaga ctagcctggc 130140
caacatggtg aaaccctgtc tctactgaaa aaacaaaaat tagccaggca tggtggcatg 130200
tgcctctagt cccagatact cgggaggctg aggcaggtga atcacttgaa ccagggagac 130260
ggaggttaca gtgagccaag accacgccac tgcactccag cctgggtgac agagcaagat 130320
tccatctcaa aaaataaata aataataaaa gatttggtac aatcatattt gaagacagaa 130380
aaactgattg tagtaatata acaaaggctt caaaaagtca cttataacaa aagataatta 130440
cctgtaccaa aatcagcaac tttcaatgct ctaagaaaac tagaggaagg atcctgatgc 130500
cagcagtgtc aggaggtcag aatatccccc aatcaaatta ccaggctaat actttatggc 130560
ctctctctga aaccctttga cccaggggtc ttaaaacaat tagatgaata aaattaaata 130620
atttgtatc tcttattgag tatttttcct gctaacaaat gatcatgttt atctttcaaa 130680
aagacttttt tattttccct ttgctgttta aattgacaga gatttccttt tctccaggaa 130740
actagagaat aaattttata atattcactc tcatccaata atatatctga aaatcaatac 130800
ataagacatg gggctttcca aaatctgtaa actctaagac ctgtaagtgg ctacacagtg 130860
ccagaaccaa actctttaag aaatggcttc ttctccaagc tagaaactgt tcagagtcct 130920
tttacttcaa gaaaaggcaa gaaaacccaa cccaagtaac tgcaaaaata tactatcatt 130980
aaaaaaaaaa aacaggagat acatgtggat gtgacagcta taatatttcc atgtgcagta 131040
gccaattgtt taaaaataca tttcaaatct acaattatca aaactgttct tagaacatca 131100
gcaaccaagg attttcagaa aatttttctt gtgtttaata aattatattc cctctcttgt 131160
tgtattatat agagacatgt ctacattctt tcaaagatga agagttcgaa gatttgatct 131220
tgaaagaaag atgaattgct gagtccttgc cactgttcct ttcacaagct ataattataa 131280
gttcaaacgg taacatacca caatgtcaac ttctaaagat ttataacttt ctccaaactt 131340
tccctaccac tcacacaaaa aaatactatg cctaaatacc tgaaaaatga gcatagcaat 131400
taaatatttt tagaaatgga aaatgataaa gaaataggaa ctaatttcat tgccaaagaa 131460
gaagttcctt tgctaactgt aaatcaggag agattctcaa aggagactac aaattgaatt 131520
tcaaaagcag gcaggagtac gaaaactaag tatataaaga agtagagaga aaaagagaat 131580
gaagagaaac ctgtttgagt gaaataaaag gaaaactagg tcaatgaaac agtaaaaaaa 131640
aaaaatacaa aaacttgtgt tcttccataa ctttccacat gtgcatgtat tgtctttcca 131700
accacactgc aaattccttg aagacagaaa ctatgtctta catttcttta tatagctcat 131760
tcatcgagag tggtgttttg cacataactg taacttagct ttttttaaac tgatgaatta 131820
tgactaaatg tccctgaacc aaatactgta cccatgcatt acaattaaat ctgacagatg 131880
cttaaatgat gtcctaattt gaagtgctgc acatatcaaa aagatacttc aaaatccaga 131940
ttagctttta aaggaaaaag tacaggacaa ttactatttt tcaaggccaa aaagaaaaac 132000
catcttatgc tagtatttta gtcgtgacaa acatatatcc caatattttc attagtaata 132060
atactgccat ttttaagcct gtccttgtgc taaacatttt tcatctaata tcccatttca 132120
ttcattcatt gactcaagag acattggaat ccctatatat caagccacgg agaagacaat 132180
gacaaggatc acctcatctt actgacaaag agagcttcat tcttaaattg ttctacaaag 132240
tgtagttctt ggagtttatc acaccatttt cactcataaa ccaaggaatt gtttaaagtt 132300
agttatatta ttttatgaat gcaattacaa aatattccta agtatgatct tcataaaaat 132360
tgttttaact tattagttga catttaaatc aaatatgtta taacaagatc catattatgt 132420
cccccaaggg aaaaataaaa aacacaaact ttcaattaat agactcttta gagcaagagt 132480
cagcaaaaga gccagacagt aaacatttca agcttcaaag gctatatggt ctcttcttcc 132540
ataggaagat atcttttatg atcgaaatgt agagaaggat ttcctaaaac aataaaccaa 132600
aaacacaaat gagtgaaaac acagcttcat taacatgtta aacttctatt taaagataca 132660
tattccttaa agaaggaaag aaaaataggt aaaaaggggg cacaggctga cagaagatac 132720
ttgcagtgct tacagtcaag tgaggatcag tatctacaac atataaagaa ttccaataaa 132780
tctattttta aaagacaaac cctacagaaa accaagaaca ggcaaatcac agaagagaaa 132840
agctgaaagg ctaataaata tctgaaaaga tgtgccatct aatgtacagc ttcactagta 132900
attaggaaaa tgcaaattaa aaccataatt agattccatt tcctatccac cagattgggt 132960
actggagagg atatgaagaa actcttattc actgctgatc gaagtataaa ttggcacaac 133020
tacttgagag caaattggtg atctagtgaa atcaaagata catataccct ttgacccagc 133080
atgacactgt taggtataca tgctagaaaa actcttgcac attaagacag atacaagagg 133140
aatattaatt gcagcattgt tttaatagtg agaaaaacaa gctacatgta tcaacagaag 133200
gaatggatga gtaaattgtg ggctctttcc aatcaatgga ataatattat aatgaaaata 133260
tatgaatgac agttgtatgc atcgacatcg ataaacatga acaagcagtt gaacaggctg 133320
agtatggctt aaacaaaatt gtattttccc cataaaattc taaataagtt gatgatagaa 133380

```
ataaaaatat aaaacttcag gtagagagtg aggaatcaag actatggaag ataatcttaa     133440
aagctaacaa ttgtcatgaa aaacatacct gaaaaatgct agaagtgctg gaaaaaaaaa     133500
aaaaaaccca cagtaaatat actctgttct ggtttctcct cagttgtttc atacccagca     133560
gaatagcttt catcttaaca cctcctcctc aaaagttttc aacctaattt attaggaaat     133620
gggttctcaa attagggtcc atgggctatt acagatcacc cagaaactac agacacaatt     133680
ttgtgtgcat ttttagaaag tgaaccccta actttaatca agtgctcaaa gcagattgtg     133740
actgatttgg gttagggctt tattccagaa tttatatctg gtttttaac gaaaatcaaa     133800
tggaaacaac attttcttta taaccaaatt agccagacat agttattctg taaaggaata     133860
aacaacagcc aagagaattt cataaagttt cctccactgc ttctgaactc atttctattt     133920
taatgtttgt taattaccta atattaacat aaggctaaaa tactcagttc actaatgtat     133980
atttctaact tcattttaca gataaaaaag gaaattcaga agaattcaaa ttcccacaaa     134040
atacacaata aatttgtgga gaagttgaaa ttcataacct taaatcttta aagtgaagct     134100
ttaacaggat tgttttcatc taccacttaa gcaaaaaagc aaaacactgc cctttatttc     134160
aaaaagtcat gcatatgaag agctagtggt aaaaggccaa tgaacataga attatactac     134220
aaactttgag acataaaaaa gggtttttta atctttatca accatttgct gttgtccaag     134280
ccatctaaac aggtactgaa tgcccgttgt gcaaaatctc acattagcac acactctagt     134340
aagatagcaa aagtaacata actatacctt ttcaactatt cttttttaa aaattcaaac     134400
agtagtagta ccctactgcc accgtttcat gattagactg cacaaagcag ctgtttatcc     134460
actcacagca accagcaggt gaacctgatt caatatttat cacaggcttt catagatcac     134520
tgctaaccaa gcaaacgttt taattaagct ggaacttgct tcaaagagct cattcaagat     134580
gtcaatcagc aggtagggga tcagatacag tgtcaggtga gagtgtgtaa tacaaatcca     134640
caactttctt catcccatcg ctgtaaatta gcctgctcta aagttcagac tctgattgta     134700
acagactgcc agtctcctgg aatgtgtaac tgaagtcact caaattctat tgtgaaggtt     134760
gtaagcaata tgttaaactg cagtgggcta gcacattaag acagatcctt ttcacggttg     134820
tttttgata ccataagtgt ttgttaccct actctccact tactgtttac tttcttgcct     134880
agtgtaaatg ggaattcctt aacacagttg ctccagtttc tgaaaagtct gtgtttcaat     134940
agagtaaatc aataagaatt atcagtttaa gaacaagcat atcataaaga tgactgacct     135000
gctataaagc aaaaattatc ctggtataat agaactttac atagatgtga ttatagcaat     135060
gagtctcaaa gcagtgatga gtcacaccga tcaacttcat gagaaaaacc cagggtgttt     135120
ataaaaaata aaaaggggaa aagaagact caaacgagca aaatggggaa gtgggcgggt     135180
aggcggaaaa tatcttcttt aaataagaat gccagctaag aattgtaaat aaaatgatag     135240
aattttaaaa atcaccattt tgcaactttc ctgtactaac tgattcaggc aagttacata     135300
aatggatgct gaaatctttc agtgaaaagt tattagggaa cagaatattg acaaagtctc     135360
aaaatatcac cccacagatt attcattaat tacaaaagga aaatgtacct ttacaatgaa     135420
gagacctggc agtgacactc taaccaagtg gtgaaattca gcatcaatgg tattgagact     135480
aactggcttc atatgcctcc tgatataaga aatatactct tgtgcccatc ttcaataaaa     135540
ctacatgatt attaaatcca aacaatcaag acatgaatca aaaagaacct gggaatagag     135600
aaactgtggt aaaaagacat atatgtatta tttagtcggt gaaaattcat ccaattgtaa     135660
tttatgatat gtacacttgg ttatagattg tatttcaaaa aggcttttaa aaaaaaccct     135720
atctcttgag attgcaggta atttttactt tctcctttat ggtatttttc aaactttctt     135780
caatgaacct tttaaaatga gaaaacaatg ctatcctatc atgtcatttt ccacatatta     135840
accaaaatgc tataaacaaa acatatgaca tagagatttg tgcttctatg ggctttaaaa     135900
attaaatggt tattattaca tacaagtctc taaagcagca ctacccaaca ggattttctg     135960
tgatgacagc gtatttgata tctgcactat tcaatatatt tggcattagc cgcatgggca     136020
cttgaaatgt aattagtaca actaactgaa ttttaatttt aattaatcaa aaaacataaa     136080
tacatgtggc tagtggacag aatacattta aagtattatc agagattcta gaaaaccaat     136140
ggttattgca ggtgcattgt atgtgtgaga aattctcttc gtaaattcaa gttagaaagg     136200
aaaaagcaaa agtacactgt aaaaattgtg tgctcatagg taaaaatggg ttctgcaggt     136260
tgcagctcat gaaaactggg ttcacaattt ttgtagacta gtaaactact tttcttaatc     136320
tttttgttc taatttttgt ctgaattcca atcaaaatcc cagcaagatt ttttttggg     136380
taatctaaat ttaatcttga aaataaataa gtgagaataa ccaaaacttt ttggaaaaat     136440
aagatgggga caggtgtgat ctctcacacc tgtaatccca gcactttggg aggccaaggc     136500
aggcatatca cttgtgcttg ggagttcgag accagcctgg gcaacatggc aagacctcat     136560
ctctaaaaag gggggagggg ggggaaagat gggcaagtag ggtatatatt tctatagatg     136620
taagaatttc aaatatatga taaagatgct gtattaggca cctctatgga catgtgtcca     136680
cctacttact ctgaaatcat ctatatcccc tgctgaaagg ctgaacctaa tatccacatt     136740
aataacacat atccaaagac actctcaggc atagcaagat caattagttt cactttccca     136800
agaacttgta tttggggcat ttagactgag ttgattaatt acagaaaata ggccacatca     136860
tttggtagat tttaggcttc cactttgaga tgtcatggta aggtcatatg aaagagtgta     136920
agtatctaga aaaaccaatc tacaaaaaat aatgagaaaa ctttgctgag aaatgcagat     136980
gtcacaccag aggccccaaa gagaacagag aagctgccta aaaactttc atttccaatg     137040
aggggaatta tatctcctgc atgatctttc ttttagcggg tttctatttc ttaaaaagca     137100
gatcactccc ttaaaaatct gatttaaaaa tgggccaaaa atttgaatag acatttctca     137160
```

```
aaagatatac aaatggcaaa cagacatatg aaaaggtgct caacatcact gatcatcaga 137220
gaaatgcaaa tccaaactac aatgagatat aatttcatcc caattaaaat ggcttttatt 137280
ggctgggcac ggtggctcac acttgtaatc ccagcacttt gggaggccga gggggcaga 137340
tcacctgagg tcagtagttc gagaccagcc tggccaactt ggtgaaaccc aatctctacc 137400
aaaaatacaa aaatcagcca ggtgtggtgg tgcacgcctg taatcccagc tactcaggag 137460
gctgaggcag gagaatcact tgaacccggg aggcagaagt tgcagcgagc cgagatcatg 137520
ccactgcact ccagcctggg caacagagtg agaatttgtc tcaaaaaaaa aaaaaaaagg 137580
cttttatcca aaacacaagc aataacaaat gctggcgagg atgtggagaa aagggaaccc 137640
tcatacaccg ttggtgggaa tgtagattag tacaaccact atcgagaaca atttggaaat 137700
tcctcaaaaa actaaaaatt gaattaccat atgatccagc aatcccactg ctaggtgtat 137760
atccaaaaga aagaaaatca gtatatcgag gagatatctg cactctcatg tttattgcag 137820
cattattcac aatagccaag atttggaagc aacctaagtg tccatcaaca gatgaatgaa 137880
tgaagagaat gtggcacata cacaatgaag tactatttag tcataaaaag aatgagatcc 137940
tgtcattttc aacaacataa atggaactgg aggtcattat gttaagtaaa ataagccagg 138000
cacagaaaga caaactttgc atgttctcac tcatttgtga gagctaaaaa ttaaaacagg 138060
tgaactcatg gagagagaga gtagaagggt ggttttcaga ggctgggcaa ggtagtctag 138120
ggaggggaga gagtggagtt gttaatggga acaaaaaagt agttagaaag aataaataag 138180
acttagtatt tgctagcaca acagggtgaa tatagtaaaa aataatgtaa ttgtacattt 138240
caaaataact aagagtataa ttggattatt tgtaacacaa aggataaatg cttcaggtga 138300
tggatacctc atttacccta atatgattat tacacattgc atgcctgtat caaaatgtct 138360
catgtacttc ataaatatat acacctacta tgtacccacc aaaattaaaa attaaaaaaa 138420
aaaaaagcaa accaccctgt ttaaatcagg tggcatttca aatctgtggg ggaaattatt 138480
caactaatat ttagatgaat gactatttgg ggaaaaaaat tagatcctat ctcccacctt 138540
acatcaaaat aatttacagt tgaattaaag atcagtatat aagaactgaa accacgagac 138600
ttccagtcta atatggcaga ttaggtacac acatttgccc tccttcccac ctaaaacacc 138660
actaaaataa acttacttag tagctgaagt tgcaaactgg aggtccacaa atcatacctg 138720
cttcacaatc atgttttgaa tggctacaca gagttttata aatttgaact acttaaaaat 138780
tatgagatga aaatccatag tgctggtttc ttctagaaaa actggttgac tggccacatt 138840
aggcctgtat tcttacagga cagcaatcca ctaacattga ctagattgcc acatttggat 138900
gagacatata ctctcaaggt atcacagtcc ctgccactta aagaatgtaa tcacatgcac 138960
ttatacttag accccttctt tcattcatgc tacctatctg gtgctcattt aagttgccaa 139020
ccctggttac aaaagtgtaa aaagggccag gcacagtggc tcacgcctgt aatcccaaca 139080
ctttgggaga ccgaggggag tggatcactt gaggtcagga gttcaaaatc agcctagcca 139140
acatggtgaa accctgtctc tactaaaaat acaaaaatta gccaggcgtg gtggcatgca 139200
cctgtaatcc caactactcg ggaggctggg gcaggagaat cacttgagcc agggaagcgg 139260
aggttgcagt gggctgagat cccgctattg cactccagcc tgggcaacga agcgagactc 139320
tgtcttaaaa aaaaaaaaag aaagaaagaa agaaagtaca ctccacagag tgggaggggg 139380
cttgagcaag aggctcaaga gcactggtta caaaattttc tggagtttaa atactctcta 139440
gaggtttcca ttggttattt tgttgcacac tatgtaaata aacacgtggc ctgtgaccag 139500
tctgattggt tgtgggaggc aaccaatcag aagctgaagg gaagctacag ttacatccta 139560
tgcaaaggaa gacatggccc atgaccagtc tgattggttg tgagagggga ccaatcagag 139620
gctgaagtga agttacagag ttacacccta tgcacatgaa gactggttgt gggaaggaga 139680
ggtactttcc attctttaac ctgccattca gtagaaaggc agggtgttgc aaacagagta 139740
gcctctgatc cttttgttac ttgggggtgg agaggtgagt ttttcttttt gattcagttt 139800
taggaagtca gtgtgaatcg gccttaggtt ccctgcctcc agaccctatt ctcctgcctc 139860
aatttcattc tcttttttttt tcttgtattt tttacctttt tatttttaat tttatttttt 139920
tttacagaca gggtctcatt ctgttgccca ggctggagta cagtgatata atcatagttc 139980
actgcaacgt tgaactccta ggtttaagca atcctcctgc ctcagcttcc cgagtagctg 140040
ggactatagg cacatgctac cacgcctggc tactttttgc atttttgtc tttttaaaat 140100
ttttcttaag atacaggatc tctctctctc tctcgcccat gctggagttt aatggcacaa 140160
ttatgactca cttcattctc aaccctcaac ctcctcggct caagtgatcc ttccgtctca 140220
gcctcccaag tagctggggc tacaggtgaa tatcaccaca tcaggccacc atttcattct 140280
cagcagatat tgaaggctca atgaatatta ctggtagaaa taagcaacaa aataaataaa 140340
tctcacatta actaaaactg cacatgaaaa catttgttaa tgactaaaaa ttgtcagaag 140400
aatcagtaat agccaccagt aaaagaacta aatgacagaa gacataataa aaacctcgtg 140460
tgactggttt ttgatgtgaa aaatataaat taatgaatga aatgtcttcc ctataagcgt 140520
tacataaaat tagtaaatga caaaaaataa aagacaataa aagaagaaga aaagctattt 140580
gacgaataga ctgataatgg gattctcatc attttttttct gtaaaatctt tgatgttact 140640
agaattttta taagcaaata tcacttttat aaaaacaatt acatttttta aaacatacaa 140700
cagaaaagaa aacaatatac agggtagaga tactaccaaa aggagaagta actagttttt 140760
cagggcttgc agaatcattt tactcaattt gataagattt ttgtcgtact aatagatcca 140820
gttatgtgcc ctctgggaaa aatgcaccaa aaagcgccaa ggaagaaagg gaactggaca 140880
gactatccag taaggccagt caaatcaatc atacaggtta atgagatgac tgaataggaa 140940
```

aacagactgc tctcacatct actattgttc agaatattag attaaggaaa gagctgagac 141000
aaggtttcct tgtggaagga caggagaaaa cataacatgg ttatagctaa ggaaaagaat 141060
caatagaaag gaggaaaaat aattgacaaa catccagtga tgtgtaaatg tgtagtggga 141120
tgtgaatggg gaaaagtttg tgtttaaaaa aaaaaaggcc ctgtatgact aatatgggga 141180
ccaacaagtt ttcagtttaa aggtgggacg gttccaaatg acaacgaagt cccatgtgta 141240
gtggtgctgt tgaattaatg gctaaattag gtcgtaagtt tcaagaaggc tgaggaacta 141300
tggtcaaaat agcagatcat caacacagat catgaagtac cccaggatgg tgactgcttc 141360
tcctactcca gattcactga gtaacaagga gaatctgaca tttttactgc cctgatgtgt 141420
gcctacttca ctaaacctca aaagcatctt tctttaaaag attcatacat gtttccaatg 141480
ctgttgtcaa tatcaattat cttctgaatg aaacgttaac cttgcacaag taatgcaaca 141540
tctggcttat agtagttttc acttagaaca atgcatatat ttgtttaagt tttccaaaag 141600
gccaacattt aaagaaacaa taaatgtcct taaaaatatg attgtatggc cttctgcttt 141660
tagccatacg aaaaaacaga aatcttatct tgcaccataa actactaaca aattggacaa 141720
gatatatgaa acagtacttt tcagacactg aattgtgatc tcagagaaaa ggaaacaaag 141780
aggtgataac tacattactt tacttctaca gatgaaaaca caataactga aaagaaaaat 141840
ccactagtaa gatttaacac caaattagaa atagcagaag agatcaagga gcttgacaaa 141900
tcggcaatag aaggtagaca aaatgaagca caaagaagaa aaaatagctt aaaaaatgaa 141960
catatagtca gtgatgtata atatcaagct gtctaatata cacataattg aagtctgaaa 142020
ggaaaaaaat gagtacttaa gaaaatattt ttaaatggct caaattgttc ataatttgat 142080
gaaaactata aaaccataga tccaaaaagc tcaatgaccc caagcacaca ataaacacac 142140
acacacacac acacacacac acacacacac acaccttagt tgaattgctg aaagcagaga 142200
taaaaataga attttttttt ttgagacgga gtttcgcagt tgttgcccaa agtgatctca 142260
gctcactgca acctccgcct cctgggttca agtgattctc ctgcctcaga ctcctgagca 142320
gcagagatta caggcatgtg ccgccgcacc tgcctaattt tttgtatttt tagtagacac 142380
agggtgtcac catgttagcc aggctggtct caaactcctg acctcaggtg atccacccgc 142440
ctcagcctcc caaagtgctg ggattacagg catgagccac catgcccggc ctaagataga 142500
atttttaaag cagccacaga ttactctata gaaactatgt aagacaagcc aagggcagaa 142560
tatcttttaa gtgctgaaag aaagaaaatg tgaacctaaa cttcaatacc cagtgaaaat 142620
gtgtttcaaa acaaagcgac ggttcctatc aataacccaa tgaagtttct tgcagaaata 142680
gaaaatacca tcctaaaatt cacatggatt ctcaaggaac cccgaataac cacaataatc 142740
ttgaaaaaga acaaaactgg aggtctcaca ttttctgatt tctaaactta ctacaaagct 142800
acagtaagaa aaacagcatg gtactggcat aaaaacagaa atatagacca atggaacaga 142860
atagaaattc caaaagcaaa cccccacata tatggtcaaa taattttcag cagcctatca 142920
agaccattca atgggaaaag aacagttttt tcaacaaatg gtgcagggaa acctggatat 142980
ctacacacaa acaatgaagt tgcaccatta taatatatgg aaaaattaac tctacatgga 143040
tcaaaacctt aaatataaaa gctaaaacca taaaactttt ccaaaagcac tggtgataaa 143100
aaatggactt catgagataa attggactca tgaaaattaa aaacgtttat gtatcataga 143160
ccaccatcaa cagagtaaaa atgcaacaca ccaaatggga gaaaatattt acaaatcatg 143220
tatccaataa ggtattaata tctggaatac ataaagaact tccaccaact caacaacgac 143280
aaaagcaatc caattccaaa atggacaaag gcaacatcat actgaatgag caaaagctag 143340
aagcatttcc cctgaaaacc agcacaagaa aaggaagccc ggccgggtgc agtggctcac 143400
gcctgtaatc ccaacacttt gggaggctga ggcaggcgga tcgcaaggtc aggagatcga 143460
gaccatccta gctaacatgg tgaaatcccg tctctactaa aaatacaaaa aattagctgg 143520
gcaaggtggc gggcacctgt agtctcagct actagggagg ctgaggcagg agaatggcgt 143580
gaacctggga ggcggagctt gcagtgaccc aagattgtgc cactgcactc cagcctgggc 143640
gacagagcaa gactctgtct caaaacgaaa aaaaaaagaa aagaaaagaa aagaaaagga 143700
agccctctct caccactctt attaaacata ttattggaag tcctggccag ggcaatctga 143760
caagagaaag aaataaaggg catccaaata ggaagagagg aagtcaaact atccctcttt 143820
gcagatgaca tgatcctata tctagaaaac tccacagtct cagcccaaaa cctccttaag 143880
ctgataaaca acttgtcagg atactaaatc aatatgcaaa atttactgac attccaattc 143940
accatcaact gtcaagctga gagccaaatc aggaatgcaa tcccattcac aattgccaca 144000
aataaataaa taaataaata aataaataaa taaataaata aataaaataa ctgggaatac 144060
aactaaccag gtaggtgaaa gatctctaca aagggaacta caaagcactg cttgaagaaa 144120
tcagagattt cttacaaaca aatgaaaaaa ccttccatgt tcatggatag gaataatcag 144180
tatcattaaa aaggtcatac tgcccaaagc aatttacaga ttcaatgcta ttcctattaa 144240
actaccactg acattcttca gagaactagg aaaaactctt ttaaaactca tatggaacca 144300
aaaaagagcc caaaaagcca aggcaaccct aagcaaaaag aacaaagctg gaggcatcgc 144360
actacccaac ttcaaactat actacgtggc tacagtaatc aaaacatcat ggtactggta 144420
caaaaacaga tacatagacc aatgaaacag aatagagagc ccagaaataa ggtcacacct 144480
acaactatct gatcttgaac aaacctacca aaaacaagca atggggaaag gattccctat 144540
tcagtaaatg gtgctgggat aactcgctag gcatatgtag aagattgaaa ctggacccct 144600
tccttacacc gtattcaaaa attaagatag attaaagact taaatgtaaa acaaaaacta 144660
taaaaaccct ggaagacaac ctaggcaata ccattctgga cataggaaca ggcaaagatt 144720

tcatgatgaa gacaacagaa gcaattgcaa caaaagcaaa aattgacaaa tgacacccaa 144780
taaaattaaa gagttaatgc acagcaaacg aaactatcaa caaagtaaac agacaaccta 144840
cagaatggga gaaaattttt tgcaaactat gcatctgaca aaggtctaat atccagcttc 144900
cataaggaac ttaaatttac aaaggaaaaa caacctcatt aaaaagtggg caaaggacat 144960
gaatggacac ttttcaaaag aagatatata tgcagccaac aatcctatgg aaaaaagcta 145020
acattactga tcattagaga aatgcaaatc aaaaccacca caatgagata ccatctcaca 145080
ccagtcagaa tggctattat taaaaagtaa aaaaaaaaaa aaaaaaaaaa aaaacagatg 145140
ccggtgaggt tgcagagaaa aaggaaaaag gaacacttgc acactactgg taagaatgta 145200
aattagttca accattgtgg aagacagtag acagtgtggc aattcctcaa agacctaaaa 145260
acagaaacac catttgaccc agcaatccca ttactgggta tatacccaaa gaaatataaa 145320
ttgttctatt ataaagacac atgcacacgt atgttcattg aagcactatg cacaatagca 145380
aagacatgga atcaacctaa atgctaatca atggtagact agattttaaa aatgtggtac 145440
atatacactg tggaatacta tgtagccata aaaaaaatga gactgtgtcc tttacagaaa 145500
cttagataga gctggagacc attatcctta gcaaactaat gcaggaacag aaaaccaaat 145560
actgcatgtt ctcacttata agtaggagct aaatcatgag aacatatgga aacataaagg 145620
ggaaaaacac acaatggggc ctatcagagg gtagagggtg ggagcaggga gaggatcaga 145680
aaaataacta atgggtacta ggcttaatac ctgggtgaca aaataatctg tacaacaaac 145740
cactaccaca caagtttacc tatataacaa acctgagcat gtacccctga acttaaaata 145800
aaaattaaat ttaaaaaaaa tggggctggg agtggtggct tacacctgta atctcagtac 145860
tttgagaggt aaggcaggag gatcacttga gaccagaagt tcaaggctag cctggccaac 145920
atggtgaaac cccatctcta ttaaaaatac aaaaaattag ccaggcgtgg gggcacacac 145980
ctgtaatccc agctattcgg gaggctgagg tgggaggact gcttgatcct gggaggcgga 146040
cactgcagtc agctgagata gtgccactgc actccggcct gggcgacaga gtgaagccct 146100
gtctcaaaaa agggcaaagg acttgaatag acatttctcc aaagaaaata cacaaatggc 146160
taataagcag aagaaaagat gctcaacatc actaatcatt agggaaatgc aaatcaaaaa 146220
cacaagatac cacttcacac ccttaaaaat ggctattctt taaaaaggac aacaaaacag 146280
ataatgaggt tggcaaagat gtggagaaac tggaacccct gtgcattgct gatggaactg 146340
taaaatggta cagccactgt gaaaaacagc ttggcagttc ctcaaaaact taaatataga 146400
attaccatat ggtccagaaa ttccactctt aggtgtatac ccaaacgaat gaaaacagag 146460
actcaaacag atacgtacac caatgttcac agcagcatta ttcaaaatag caaaaagatg 146520
gaaacaaccc agatgtccac tggcagatga acggacaaac aaaatgtggt atatccatac 146580
aatggaatat tattcagcca taaaaagaaa taaaattctg acacatacta catggtggat 146640
gaccttgaaa acattatgct gagtaaaata agccagacac aaaaggacaa atattgcatg 146700
attccactta catgaggtat ctagagtagt caaattcata gagacggaaa ggagaacggc 146760
atttttcctg gggctggaca atagggaaat acagagatat tgtttcatgg gtatggtgtt 146820
tcagtttgga aagatgaaaa ggttctggca ctagataata gtatataaat atgcataata 146880
ccactgaatt tacacttaaa aacagataaa tggtaaattt tatgttatat atattttacc 146940
attaaaaaca tgaaggtaag aaaaaaagct gaaaaaatat ttgcaatgca aatgttaaat 147000
aaagctcttt aagctaatag aaaagttaca caatacagaa acttggacct acaaaatgaa 147060
gggcaccaga agtgaaaaac tggagggtta agaaagaaat tattttcctc atttgtaaat 147120
ttgtacatag ctgatgattt aaactaaaaa taacaaaaca ctgtgagggc tatgacaaat 147180
gtagaagtaa aatatttgac aaaaatggca caaaacatgg gagagtagac acggaagtat 147240
attggcagaa ggttcttaca cattatttgt gagttgaaat attatttgaa ggtacactgt 147300
gataagttta agatgcaaag tatgaagcaa ctcacaaaaa taaaactaag aggtatactt 147360
aagaagacag tggtgaaggt aaagtagaat cctaaaacat gtttaattaa ttcaaagaag 147420
acagaaaagg ggaataaaga aaagttggaa caaacagaaa acaaatagta accacagtga 147480
taattacact gaatgtaaat agtataagtg ttccaattaa aaagaagagg ttgtcagatt 147540
ggataaataa agaagacaat tatttataag aaacttatct taaatctaaa gacacaaata 147600
ggttaaaaat aaaaggatga aaaatatata ctatggaaac attaatccta agaaagctgg 147660
agtggctata tcaatgttag ccaaaataga tctcagaaca aagacatttc ataatgacag 147720
atgggttaat acatcaagaa gacataataa tcttaaatgt gtatacagct aataacagac 147780
catgaaaacc catgaagcaa aaactggtag aattgaaaga aaaggcaaat ccacaattat 147840
aggaccctta cagtattctc taattaattt ataagtagac caaaaaaaac cagcaaggat 147900
acagaagatc ttagcaagat aatcaacaaa cttgacctaa ctgattttta taaatgaata 147960
cccatacttt ccaaaaactc acagagcatt tgccaagaca gatcgtcaaa tttaaaagaa 148020
ctgaaatcat atagaatcta ttctctaacc acacaaaaat aaattagaac tcagtaagag 148080
aaaaatatct aggaaaaaac acctagttag tgtttattta gacttctaaa taaatcttgg 148140
gtcagagaag aaatcacaag ggaaatcaga aaaaaatgta acaacatcaa aatttgtggg 148200
acacagataa agaagtgctt agagggaaat gtatagcttt cattggtgtt ttatttagga 148260
aaaaaaaagg tccaaaatca gttatcaact atttaagttt caatcttttt ttttttcttt 148320
ttttttgaga cggagtttcg ctcttgttgc ccaggcttga gtgcaatggc acgatctcag 148380
ctcactgcag gctccgcctc ctgggttcaa gcaattctcc tgcctcagcc tcccgagtag 148440
ctgggattac aggcgcccac caccatgcct ggctaattgt ttgtatttat aatagagaca 148500

gggttttgcc acgttggcca gcctggtctc aaactcctgg cctcaggtga tacgctcgcc 148560
tcagcctccc aaagtgctgg gattacaggg gtaagccacc atgcctggcc aagtttcaat 148620
cttatgaagc tagcagaaga gaaaattaca tccaaagaaa gtaaaacaat ggtaattaaa 148680
aaaaaaaaaa gcagaaacca acaaaataga aaatggaaaa acagaaagtc aacaaaaact 148740
ttttaaaaga tcaataaaat ggataaacct ctactaccta ttacagattg agcaaaatga 148800
attctagatt aagcaaaaaa aaggagatag acacaaattc acaaattatg aatatcagaa 148860
atgaaagagg gcatatcact acaaatccta cagacactta aagaaataag agaatatttt 148920
gaacattatg ccaataaatt caaaaacttg aatggacaaa tttattgaaa gatactaatt 148980
actaaaactg actcagaaac agaaaatgtg aatactccta tactcatttt taaaattaaa 149040
tttaaaagta aaagtcttcc caaaaataac acataagggc aagatggctt ccctggtgaa 149100
ttctatcaaa aatttaagga agaagtaata tcaatacttc ccaactcatt ttataaagcc 149160
aacattacct tgatatcaaa gccagataaa gacattacag aaaactatag accaatattc 149220
ttatgaacat agatggaaag aatccttaac aaaatttagc aagtcaaatc taacaattat 149280
ataaaatggg tcctatacca tgatcaagtg ggttttattc cactaattta cagtcaggcc 149340
aacattcaaa aactaatcaa tgtaatttac catatcaaca ggatatagga gaaaaaacat 149400
ataatcctac caaggaatgc agtaaaactt taaatataaa atccaacatc cactcattat 149460
aaaaatgttc agccaactag cgatagaagg aaacttcttt agcctgacaa agggcataga 149520
cagaacacct agcacaaatg tcatacagaa cagtgaggga ccagaatgtt ttccaccaca 149580
aggttgtgaa caaggcaagg gtatccactc tcatcactct aattcagcct tgtactgcat 149640
gaaaggtaaa cactgtaaag gaagaagtaa aacagtattt actagagaat gaaacatgca 149700
actttgggta cttagaaagt cctaaagaaa ctccaaaaat atcaactaga accaataagt 149760
gatataacaa gttgcaggtt acagggtaaa tatacaaaaa tgaattctac ttctataata 149820
gtaatgaata actggaagga aattataagt ggaatcatac aatatctgtc cttttatgac 149880
tggcttattt tacttcacat aatgtcttca agtttcatct atgttgtagt acatgtcaga 149940
atttccttcc ttttaaaggc tcaataatat tccattgtat gtatacacca cattttgttt 150000
attcgtttat ttatcaatgg acattttggt tgtttccacc ttttggctat tgtgaataac 150060
actcctatga acactggtgt acaagtatct gagtacctgc tttcaattct tttggatatt 150120
tgcctagaaa tgttatttct ggaccatatg gtaattctgt ttttaacttt cagaggaatc 150180
aggaacaagg tttttcagtt aaacatacag aaaggaagaa aattagaatg ggccctctgg 150240
tactggaatg gaactggatg tattggtata aacactcata attttcagtg tacagacaga 150300
cagatacaga aaaaatatag acataaatgt atatgtctat gagtctgggt ttgtgtacac 150360
aatacattgt gtatattctc tagctgtctg ctgaaagggc ctggagcagc aactccaaca 150420
gcaatgagca aaactaactc ccagaacaca gcgttcaaat gttatttttc actaaatgga 150480
accagggttc cttggagaaa tgtctgaatc cgagactagg gcagggaaaa tataaggtga 150540
gcctggaaca ctatcttttg ccaaaaaata aggaagcgtt ccaagaatga ttgatatatg 150600
tcagaaagac acaagcaact taactggttt cccacttgag taccaaaaca aatcatataa 150660
taattataac tcaccgaata aaatggaaaa tcacaagtcc ataaagatat aaataaaatg 150720
agtaaactga atttttgtaa ggacatgtta tttatataat ttcagagttc ttccccataa 150780
agtcctcttt tataaaggca aaaagataac tgtaaagtta tattttttac ctagcagata 150840
tcatattaat caagttatca atcattaatg agaaaaaatg aaactgcgtg ccaccttata 150900
agatgcaatg agcagaaggc atcacttctg tgacattcct gccaaagata aataacctca 150960
gtctaatgag aaaacccaaa ttaagagaca ttctagataa gctgtaatat tctgaagtgt 151020
caaaatcatg acagtcaagg aaagactgaa gaagtgttcc agactgaaga agttactaaa 151080
gtgataacta aatgcaatgc atggttctga ctggattctt ttgcttttat aaaagcgtta 151140
ttaagacaac tgctaaaatt taaaccggtt ctgagtatta gatggcaaca atgaatcaat 151200
tttaacttcc tgattcttat ggttatattg tgattatgta taagaatgtc tttgcttata 151260
agacatagac actaaagtat ttcagggtga gggaacatca gggcaacaac atactcttaa 151320
atggctcaat aaaaaaagat atttggattg tacttccaat ttctctataa ttttgtgaca 151380
gccaaacttt taaaaagcta aaataaccga aagataaatt tactgggcag acagttggtc 151440
aattaacgaa gtagttttca aaggtaaagt tatctttcca cacccagtat ccatgaatga 151500
aaacgttcta catgacaatt gcagatacta tcaccactga atagctacac tcatgtcgga 151560
tgggttgcta tacatattta tttcattaat gtatctacac atcaagaaaa cttagaggag 151620
tggctgaatt ttgtcaactt tctctgaaat attcaccaag ttcactcaat atgataaaga 151680
agatttgaaa cttttgagaa ttaaacattt ccaccataga gaatactatg ttttctccaa 151740
gctctcaaaa aattataatt actgggccag gcacagtggt tcactcctgt aatcccagca 151800
ctttgggagg ccaaggcaag cggatcattt gaggttggga gttcgaaaca tgcctggcca 151860
acaaggcgaa accctgtctc tactaaaaat aaaagaaatt agccaggcat ggtggcaggc 151920
acctgtaatc ccagctactc gagcggctga ggcaggagaa ttgcttgaac ccaggaggtg 151980
gaggttgcag tgagccaaaa ctgcaccact gcactccagc ctgggtgaca aagcaagact 152040
ccacctcaaa aaaaaaaaaa aaagccataa ttattggagc tgaaggacat actcaagcaa 152100
ttagtcatcc attctatcat cctaccccta ggcaaatttg cagacaaggc aagagattca 152160
cactaactcc actaacctct ggcctaactt aaccaagatt acaatagtat agaacgggct 152220
ggcaaacttt ttctctaaaa ggccagagat tttcctgctg gctgaacatg aaaagaaaga 152280

aaagaaaaat acataaatag acagagtata tattttaggt tttgtgagcc atatggtctc 152340
tgtcacaact actcaacttt actgttataa cgcaaaagtt ccacagacaa tatgtataaa 152400
caaatgtaaa atgcgttcaa aaaaacttta tttataaaag caggcagcag gctgtattct 152460
gcccatgggt catagtttgc caaccactgg cataggaaaa aagattacga caaaaaagta 152520
tccatcaaaa tagacctgct aaattttact atgaccgtga tatattaggt gaaaaataaa 152580
agctatatac tataatccta attttgtgtg tggggaggga gtatatatgt aatatatgtg 152640
taatatatgt catatatact acataacaaa tatatgtaat atatactaca gtatcaaccc 152700
ctttacaggg gttgagctct gtacaacacc tgattgcagg agtgagccac cacgctcggc 152760
ccaataatta cgattttttt gagagcctgg agaaaacata gtagtgttct ctatggtgga 152820
aatgtttaca tatatactca gttgatactg ggtatatatg taatatatac tacgtaacaa 152880
aaatggcaac aaattctact tatctttgta tataagctcc ctttcaaggt tactttgtag 152940
ctcttcctac caagatgtga gctttacccc ttgagtctgg atttggtcat gtgatttgct 153000
tttggtgact ggaacattag caaacctgac acaaacagac tcaaaaggca tttgtacact 153060
ggggcttgtt ctcttgctgc tcttggaacc ctgaggccac catatacata aaccagaatt 153120
agcctgctag atgatgagag agacccacct catggtccca gctgactacc aatcaactcc 153180
ctgaagcagg gctgccttgc tgacttacag ctgacctgca gttgaccaca gacacatgag 153240
taggccacct gagatcagca gaagaaatgc ccagctgagc ccaccctaaa ttgctgaccc 153300
ccacaacagt aagctaaata aatggttggt ttgttacagc aacagataac attgcatgtg 153360
tgtgtgcacg cacatgtgcg cacttaagaa tagacagcag gcagaatatc aggggttgat 153420
attgaggaga cagtttaatt aaggattaat gttaataatt taagtacaca ttttggtggt 153480
tcccaaattt tctcttagat gatgctttta tattcagaaa aaagttattt agaaatataa 153540
aatttaattc aatataaaag agtatttttc attatttcca ctaaatattg aaagtctaaa 153600
aatatggaca tcttgacctg atgtatttaa atgaactgat agattttact ttctaacaga 153660
gagtttggag ataaatagtt acaggtacat aggaaactaa gcaaaggaca agacaactac 153720
tagcaccaaa aaaaataaat actggtttgg ggaaagaact gtaataagtt taaatattta 153780
tagaattata acaatacaaa taccaaatgc tggcaggctg aagggtagaa gtaatgagaa 153840
aggagaaaag aataggagga agacaacaga aagtatctaa aaccgaaaac tcaagagaaa 153900
agcaaaatat gcatgctact tcaaaaacta tcagaataca aaggataaat aattgaaaat 153960
tatttacttc agatagtggg aattggtgaa taatagaatt agggcagggg tctactgctt 154020
ttctgtttga agccaagtgg aattctttga catatttttt cacacatact gttaaaaaaa 154080
aaaaattaaa gagaaaaaaa agtccaggaa ccaatccaag tacatataac aatttagtac 154140
atgataacag caaaacttca aaactggaaa aatgattaag tgacactggg acaactgatc 154200
aactatttgg gaaaatatgc aattagagcc tgacctcata catgcatgaa aataaatccc 154260
ataaacaaga gagttaaatg cacaaacaaa actgtaaaag aactattaaa aatatcctta 154320
taaatttata gaaacggatc tagaaagatc ctcatgaaag tgttcatggt gtttccttct 154380
ggaaagcaga aagggagatg gggttaacat aagggagaac attttaattt ttattctgta 154440
ttggttttta taatgttaaa ggtttttcaa aaaatgttat tactcttttt tttttttttt 154500
tttttttgag acaggttttg ctctgtcacc caagctggaa tgcagtggca tgatcacagc 154560
tcactttaac ctctacctcc cgggctcaag caatcctcct gcctcagcct cctggtagct 154620
gggaccacag gcatgagcca ccacaccaag ctaattttgt ttgtttgttt tttgtagaga 154680
cagggtcttg ctatgttgcc caggctgatc tcaaactcct gggctcaagt gatccaccta 154740
cctcagcctc cccaagtgct gggattacag gcataagcca tcatgcctgg ccaaaaacta 154800
tttttaaatc tgcattaagt cacctgtatt taacaatact gtcataccct cttaggtata 154860
aggatctgag taaaaattgt aagctacaag aaacagtggt gaatcataca tctacttaac 154920
tatggccatt tttttcattc atttattcaa caaataatac aagtagctac taggtgcagg 154980
ctctgtgcca gatgctggtt ataaaaaact gaaaaaaaga gacattacac ttgtccttat 155040
ggtcagtctg gtaatgagac aaacaaaaca aaaaacagtt aaacctacaa ataaataaat 155100
aattataaat ttgggtaagc attgtaaatg cttagatttg gtgttgagac actaaaaaag 155160
ggacgaatct tgaataaata acctgcatgg taatgcatat aatatgacct gtaacagtat 155220
aaaaatgaac tgtcatcaaa aagtcatctg gaagagttta ttcagagaca gaatagtgag 155280
gtggtttaga ggatcaattc cagagccaga ctgcctgggt tttaatccag cttctactca 155340
ccaggtttgt gacatgaaca agttattcaa atactctgtg gctcagtttc ctcagctata 155400
attgcggaaa atattaatac cttcctgata cggttgttat aaagattgag ttaaattctt 155460
ttattttgaa aactgtcaaa cccaaagaaa agttaaaaga acatgacaat aagcatcaac 155520
tcacatgaac ctgtatttgc caggtgaatt aatatttcta aagtgcttag cagagtgtct 155580
ggcacataac acagtaagca ccagtatcag catatatatg cagttatgtt aaatagctat 155640
tttaaataaa agtaaattaa aaccataaag taaaagaaag aagggtagga aggaaggaaa 155700
taaaaatggg gtatgggagg aagaaaaagg aagaaaaata gcctaaggat cgccccagtg 155760
ttaacacttt gtaagaaggt caacagcgca ctctgctgga cccaagtgtc ttccacacac 155820
caggcccaag ttatgaatta aaataatatg ctactgctac attaaatcaa atataattac 155880
tttaattaaa gtatataaag aatgttagca tttatttaat gaataaataa aaagcaggta 155940
taagcataag aatatatctg aagacaaccc tggctattgt gaggatttac taaaaaaaga 156000
acagaatata aataaatcta gagaaaatga ttatgaatga tagaacaaag taatatcaaa 156060

acgttaacat actattctaa aaaacaatgt ttttcttaac tatattttga ccatgtggag 156120
acagatgctt cactagaact ttgacatatg aattgtcaga ttaagaaaat aaatctgaac 156180
tgtaaggaaa gggatcaagc aggggacacc agaaagttaa ttatataaga aaaggtactg 156240
acactatctt ccgacttgaa aagagagtta ggagactaga aatgatagaa aaataaagag 156300
acaacaatct acttctccaa tccactaggt ggaaactaaa tgattttcat aaaaactaag 156360
aaaatttgaa gaacctaaaa agtttaatgt caaattcagg ggaaaacaga aatcatcaaa 156420
ctaatttacc atttaattta tggaagaagg tttgtcaaaa gtaatgcaac tgcagatttg 156480
aaaaacgtga agactaagac acacctttgc agggatgaga attaaaccat taatttatga 156540
atccaaataa atatttctat ttaaaaaata ctgtactccc tagtatcata taataaaaag 156600
caagatagga aaagcgtcct tagaatttaa aggtgttaga attcaattag atataacgta 156660
tctcctgacc tcgtgatccg cccgcctcag cctcccaaag tgctgggatt ataggagtga 156720
gccaccatcc tggctaacac ggtgaaaccc tgtctctact aaaatataaa aaattagctg 156780
ggtgtggtga cgggcgcctg tagtcccagc tacttgggag gctgaggcag gagaacggtg 156840
tgaacccagg aggcggaact tgcagtgagc cgatcgcgcc actgcactcc agcctgggcg 156900
acagagcgag actccctctc aaaaaataaa taaataaata aataaataaa taaataaata 156960
aataaataaa gcatcattaa atctagtctc ccattctttt tccttccata tgaaggaact 157020
ggttttcaca aaaaagttac ttacccaagt ctatcaattt accttattca agattattac 157080
aaggtctaga ttaaaataca acagataaaa tgtgaggcat tttggtctga agaaaaaagc 157140
aaaaataaaa aaatataaaa aaaagtgagg catatgatct gtcagcataa gaaaagttct 157200
aatatatgga ctactttttt ttcaattact ttatccaaaa gttttggcaa taaactcatt 157260
tcaaaagctg ctgtataata aatattaaaa cttacgacat taatacacat gaaactattt 157320
ttcttctact tttcatgaaa ccacctatcc caaaatctac agaaaatact tttatgattt 157380
ataataagtc actttgcaaa gactaagaaa tgttacagca gaaaccaaag taatttccta 157440
caaggaaaaa tatttactcc cctatgacac ccaaaaatcc cattttttaa ataaattacc 157500
tctactcata tccccattat tttttcttat attacattct tggaaataat tagtccttgt 157560
tctctgaaaa cctagatgta ttatttaatg ctagaaatcc atgagaaatt tactaaaaat 157620
ttacttaaca tacaaaaagg agaaacattc atgctcacta aaactataca ttttataata 157680
attatatttc tattataagt acttatccta tttttatgtt aggaagaaaa aaataaagtt 157740
gaaaatacaa atcattttac ctatgtattt ttgggttttt ttttcccata acctttcaga 157800
aagcacataa aattacttta tacgtcatgt ttttcatcat agactaccat gaaaagaaac 157860
aaatattaga gctcaaccat atgttctttg cattgcaaaa gtgctttgat gtctgttaaa 157920
tttactttat gcatgtttaa ttagtatgac atatatctct tacatcttct tgcaaacaaa 157980
aattcagtgt cacactagtg aaatgttatc atttgaggga ctagatttaa ttgagagatt 158040
taacttgttc aagaagtttt cagacttatg agaaattgtc agaggcctat atcttaatgt 158100
tatgagtaat aaatcaccca cttatgaaaa attattccat ttctagagat atgtagagtt 158160
ataccaccta aagccaagga agaaaatatg caatttctag cttaaaaata aaacagatat 158220
aaagatacag atatacaatt atagatacag ctatatttta ccaatgagca gaccataatc 158280
aatgatcaac atatatcagc atactacata accaaaagga gaaatcaatc aaagcattag 158340
gcttaaaatt aaggtactca accaagggca cagtagatgt gcttatggtc aagcggttta 158400
gactcaaaag atttcagcca taaacatctt caacagatat caaaagaatt tttggccagg 158460
ttccgtggct cacgcctgta atcccagcac tttgggaggc cgaggcaaac agatcacccg 158520
aggtcaggcg ttcaaggcca gcctggccaa catggtgaag cctcatcttt actaaaaata 158580
caaaaatgag ctgggcgtgg tgtcaggtgc tacttgagaa gctactcaag aggctgattt 158640
aggagaatca cttgaaccca ggaggcagaa gttgcagtga gccgagatcg cccactgcac 158700
tccatcctgg gtgacagggg agactccgtc tcaaaaaaaa aaaaaaaaaa aagaaaagga 158760
atttctact aaactctaaa cctaactcct caaaatttat aatgttgatc agtattatca 158820
acaataacat taataatagc tttcagcagc atttaaaaat aagaactaca aggccaggtg 158880
cagtagctca cacctataat cctggcattt tgggaggcca aggcaggtgg atcgcttaag 158940
cccaggagtt tgagagcagc ttaggcaaca tagcgaaacc ttgtctctaa aaaagttaac 159000
caaataaata aaatgaaaat aagaactaca tattactcca agcaacctac agattcaatg 159060
taatccctat caaaatacca ataacattct ttacaggaat agaaagaaca atcctaagat 159120
tcatatggaa ccacaaagac cctgaatagc caaagtaata gtatgcaaaa agaaaactgt 159180
aggcaccacg ctatgtacta cacaaagcta tagtaaccaa aacagaatgg tattagtata 159240
aaaatagaca catagaccaa tggaacagaa tagagaaccg agaagtaaat ccaggtattt 159300
acagttgact gattttcaac aaagatgcca agaacacata tcggggaaag gactccctct 159360
tcaataaatg gtgctgagaa cactggatgt ccatatgcaa aaggaaaaaa ctagatccct 159420
ctcaccatct acaaaaacca actcaaaata gattaaagac ttaaacataa gacccaaaac 159480
tataagacta ctagaagaaa acatagggca aaagcttcag gacactgatc taggcagatt 159540
ttatgggtaa gaattcaaaa gtacaggcaa caaaaacaaa aatagacaaa taggacagta 159600
tcaaactaaa aagcttctgc acagcaaagg aaacaacaga gtatagagac aacctgtaga 159660
atgggagaaa atatttccaa actattcatc tgagaaggga ctactatcca gaatatacaa 159720
ggaactcaac agcaaaaaaa aaaaaatctg attttaaaat aggcaaaggg tgtgaataaa 159780
catttctcaa aagatacata aagccaacaa gaacatgaaa aaatgctgaa catcactaat 159840

catcagggaa atataaatca aaagcacaat aagatatcat ctcactcagt tagaatggct 159900
tactatcgaa aagacaaaaa aaaataacaa atgttagtga cgatgtggag aagaaactca 159960
tatactatta gtggaaatgt aaattagtac agacattacg gaaaacagca atggaggctt 160020
ctcaaaaacc taaaaatatt actaccatat aatcatataa tccagcaatc ctactactat 160080
ttatccaaag gaaagaaaat ctagagtaca gtagcatgat catatctcac cacagtctga 160140
aactcctaga gtcaagtgat cgcccatgcc tcagcctccc aagtagctag gactgcaggc 160200
acgtaccacc acgcctggct aattttttta aaaattttgt acagatgggg ttctcgctat 160260
gttgccgggg ctggtctcaa actcctggcc tgagtgattc tcctacctga gccttccaaa 160320
gtgttgggat tactggtgtg agccaccaca cccagccaac accatgttta ttgcagcgct 160380
attcataata gacaaggagt tggcatcaac ctaaatgtcc atcaacagat gaatggataa 160440
agaaaatgtg acacatatac acaatggaat attattcagc catagaaaaa tattgaaacc 160500
ttgttatttg cagcaacatg ggtaagcctg gaagatatta agtgaaataa gtcaggcata 160560
ggcaggtaag tactgtatgt tcttactcat atgtaggagc taaaaaaaat gagctcatag 160620
aagtagagta aattgtggtt atcagaggtt aggaaggggt gaggggaggg gaggataagg 160680
agaggtgggt ttacaaattc ataactacag ctagattgga gaaacaggag gaataagttc 160740
tggtattcta tagcactcta gggtaaatat ggttaacaat aatttcttac atattttcaa 160800
aaagctgtaa gagagaattc tgaatattcg caacacaaag aaatgacaaa tgtttgaggc 160860
gatggatatg ttaattactc tgatttgatc attacacatc atatacatgt atgaaaatta 160920
tcacactgaa tcccataagt atgtataatt attatgtgtc aacaaaaata aacaaaaaat 160980
aaagatcaga actacaaata tgtaatatta ttagtcggta ttatatagaa tcttttatca 161040
gcagcagcta aaggtaagaa ctacaaatat gtatccatct gctgctgtta ctactactac 161100
agctgctgct acttctacta ccacttggcg cttaaactct aaggtatgcc agatactctc 161160
agcactgtat agatatcaac ttgtttaatc ctcctaacgg ccccgtgaga taggtactat 161220
tactaacccc atttaacaaa tagctaaaca taataccaag agtttaaaaa atcaaggtta 161280
tacagttaat aaagagtaga gctatgatta aaatccaagc agtctcaaga tatgaaaata 161340
acctaagtgt ctgtcaatgg atgaataaag aaaatgtggt gtgtatgtgt atgtcaatgg 161400
aatattagcc ctaaaaaaga tcctgctatt tgtgacaaca tggatgaacc tggagggagg 161460
acattatgca aagtgaaata tgtcagatgt agaaggaaaa atactacatg atctcactta 161520
tacgtggaat ctaaaaaagt caaatacata gaaacagaaa tcagaacaat ggttactggg 161580
gcagggaggt gggggatttg aggagatgtt gatcaaagag taaaaagttg aatctacata 161640
ggatgaatca agtctagaga tctaatgaac agcatcatga caacagttaa taatactata 161700
ttctaggctg gatatttcga tattggcttt aaaattttca ggtgttctca acacacacac 161760
acacacaaaa ggtaactatg taaggagatg aatatattaa ttagcttgtg atatgatttg 161820
gaagtgtgtc ccctccaact ctcatgctga aatgtgatcc cccaagttgg aggtggggcc 161880
tagtgtaaag tgcttgggtc atcaatggct ggtgccctcc tcacagtaat gagttgagtt 161940
cttgctctga ggtcacacga gatctggtta tttaaaacag tgtggaacct cctctgctct 162000
ctcctctgtc ctctctttcc atgtgacaag tttgctctct ctttgtcttc tggcatgctt 162060
gtaagcttct tgaggccctc accagaagca gatgctgaca ccatgcttcc tgcacagcct 162120
acagaactgt gagtgaaaat aaacctcttt tctttataaa ttgcccagcc tctggtattg 162180
ctttataaca attcaaacag actatcacag cttggctgta gtaataattt cactatgtat 162240
atatatatta aaacatcatg ttttacacct aaaatatata taatttttat tttatgtatt 162300
tattttttat ttttttttag agacagggtc tcgctctgtc acccagactg gagtacagtg 162360
gttcaatcat agttcaatac agcctcaatc ttctggctca agggatcccc ctccctcagc 162420
ctcctgagta gctaggacta caagtacaca ccaccacaca cagctatttt ttttcttcta 162480
ttctttataa agacagggtc tcgttatgtt gcccaggttg gtcttgaact cctgacctca 162540
agcaatcctc tcaccttggc ctcccaaagt gctgagatta taggcatgag ccatgatgcc 162600
tggcccaatc tttatttaaa aattaagatc aggcagtatg tgtccaaagt ccattctctt 162660
aaccaataaa aacaaaacaa gccaggtgca gtgattcatg cctataatcc caaaactctg 162720
ggaggcccag gcaagaggat cactggaggc catgagtttg agaccaacct gggcaacaca 162780
gtgagacctc atctctacaa aaaataaaaa taaaaaatta gctgggcatg gtgttgcaca 162840
gctgtagtcc ttgctattca ggaggctggg gttggaggat cacttgagcc tgggattttg 162900
aggttacagt gggctatgat cacaccactg cgttacaaca tgggcaacac attgagaccc 162960
tgtgtctaca aataaagcaa acaaaaacaa ttcccaagaa aaaaaaatct taatcacagg 163020
tgactctata cttgatttgt ttatctgtga gcttctacat atttctttcc ttatcttggt 163080
gttagaaccc tatttaggtg gctccaggac tgcaaagagg cattttagag agaatataaa 163140
ataaagtgat tagggcattt gccagaacat gagaatgcct aggcaggatt tagctctact 163200
atttactagc tttgtgatct taggccagtt tcttccttct cagagtttcc tcatctgtac 163260
gatgggaata ccaatatacc tgcttcataa tcattgtaag aactcagtaa cattcaacaa 163320
ataaatattt attagacaac taatatgcac catatgctat tctaggcatg agggacacac 163380
atgaaaaaga ctgacattac atcgtaatag gacagacaaa taataaacaa gtaatcaagt 163440
aatttcagat aatagtaagc ccatgaagga aaggaaagaa aatgaatgag aaaacactgt 163500
ggcagagctc aacatatagc ttacattata atgatgttga tgattattgc aatttaacag 163560
aagaagcttc tacataattt ctccaactat taatataaag cttcagtgaa gatcaccaac 163620

aaaacacaaa ttaaatgtac agtaactgaa aacaaatctg tggttgcctg ggaatggggg 163680
gacagggcag aaaaaaggtc catcagtcta tcttgcactt aggatggacc ttttctaggc 163740
atcattttgc aatatcatgc actggtcatt taaataatat ttagttcgct gagtttaagt 163800
tcattgagtt ttacagatct tacaaatgtt gacacatttc attatataaa tataaaaagt 163860
catattctgt aatatcacca atttcatcag aaaagtctgt agttatcaca aagctgccaa 163920
gctcagagta tatacaaatt ttttaaaatt ctgattttca cttgaaagct caaattttat 163980
ctggctcatt ttatcataaa gaattaccat ggttagtata tcattcactc tttttcaagc 164040
aaaaatgatg ttccatgaca aaaagtggct agtcagctca caatccaaac tatcctacag 164100
gcgcttctcc ccaggacatc catcctgctt ctgtatgcaa aagtgtttta tgagttctat 164160
tcatttcttc agacagaatg ttaaaaacct aaacggctga gattaaataa aattagtaat 164220
ttttactgct tcatcaaaga cattcttaaa tgaaactggt ttttttttcaa aactgtgatc 164280
taatgatgct ggaaaactat tattaacata ggttgggttg aggccaaggt gggcggatca 164340
cgaggtcaga agatcgagac catcctggct aacgtggtga aaccccgtct ctactaaaaa 164400
tacaaaaaat tagccaggcg tggtggcagg cgcctgtagt cccaactact cgggaggttg 164460
aggcaggaga atggcatgaa cccaggaggc ggagcttgca gtgggcggag atcacgccac 164520
cgcacttcca gcctgggtga cagagcgata ctccgtctca aaaaaaaatt aaaataaata 164580
aatttaaaaa attatataca tatagagaga gagagagaga aaaagagaga gagagagaaa 164640
gagagagaga gagaggttgg agccaatacc ttgatttctg ctaaggcacc aacagtttta 164700
cccattcttg cttttgcgcc accagtacaa atgtcaagac aatccaaaag gcaataatgt 164760
attttttcct tttttttttt gagacggggt ctcactctgt catccaggct ggagtgcagt 164820
gtggtgcaat cttggctcag tgcacctttg cctcctgggc tcaagccatc ctcccacctc 164880
agcctcccaa gtagctggga ctacaagtgt gcaccacaac acccggctaa tttttgtatt 164940
ttttgtagaa tcagggtttc gccatgttgc ccaggctggt ttcaaactcg tgaactcaag 165000
tgatccaccc gccttggcct cccaaagtgc tgggattaca ggcgtgagcc accacgcctg 165060
gcctacaaca tcttaatatt attacaaaaa taattttcac ctcacagatc actccccaaa 165120
aaaatctcaa tatctagaac tccttggact actccttgag aactgttgcc ctaagaatcc 165180
tgaaagaact gtaaggagtt ggagaattta caaacattaa aatacagaac agaaaaaaat 165240
gactttcaga tctctctcta gtccaattct cttattttat aaataaggaa acgaggcata 165300
gagagaatag gctttgccat ctcacacatt agtaacagag atgtgacagc attgaggact 165360
tctgagtggg ttgctactgt cctttgtatt atactattgt tcctcttttg tcaaaaattg 165420
gtttagaaaa ttccatatct tccttcttta aaactgaaca atggcattaa tacatacttt 165480
ctgtggcgaa aaggagttta tcttttccag tggagagttg agttttacag tctttcctga 165540
atcaactttt gcatccaaat tgtgtacttc tgttccaaag caatgacgtt ttctaatctg 165600
taaacacaga accaaaatga agtttaaggt gaactagaag tttaactggc tagttgttct 165660
caaaggccaa aacagctcta tgtatttttt tcctgcatcc aaaaaaagac tatttctaac 165720
aggcaataat aaaagaaatt ttaaaaatgc aaataagcac ctcattacaa cacttaaagg 165780
aaatagaaca tagtagtgac tcatgaatct tagccaaact atctttaatt agtgatgtat 165840
caaaattaga gtattgtaaa atgaacttct cataaaatag tcccatcata tggacatcaa 165900
atatagcaat attggaaagt ccaatcttat atctaattgg aaaataaaag taaatcaaaa 165960
tttaagcata attctacatt gaaaagaact cagaataaaa cttttaatgt aaacagtcat 166020
gatttcctac aggatggaat aaattactcg attactatta tcactgttac ttactagata 166080
tttgataaac caagttgcct ttgattaaat ttcttttata aataccacta agttttcttt 166140
ccaaactgta tccatgcttt tcctatttgt gaaaaatcta ttcactatat aattggggag 166200
aaaagcattt ctattgataa tctcattccc gccaaaaatg aaatgagaac cagtgaagtg 166260
agggaataaa tgttttatgt tgagccctac ctgatgacat gcaaatctat gaatacattt 166320
tgtcgggggg aaatctctat aggttcacac ctcatttgtc attggtactt tgataaaata 166380
aactagttca ttctatttct aaaaacaagg ctgaattata gaaatttata aagactgtaa 166440
gtggaacagt taaactacat aagacttcaa gaaccttgag tatactcaat taaaagtacc 166500
cagttactta cctacaaata acatcctaat taataatttt tttattttat gtttttattt 166560
atttattttt tagagacagg gtcttgctct gtcaccaagg ttgaagcata attgcatgat 166620
cttagctcac cacagccttc aactcctagg cttgagcaat cctcctatct cagcctccca 166680
agtagctgga aagtagtctg tagggctaca tgtctgatga gcctgtaggg ctcatgccac 166740
cacacccggt cttttttttt actttttgt ggagacagga gccttgctat gtcacccagg 166800
ctggtctcaa actgctggac tcaagagatc ctcccacctc agcctcccag agtgctggga 166860
ttacagtcgt gagccactgt attcagccta ataattatct tttttatttg taactaaatc 166920
tattaaaatc ccagccttcc tatctcactg ccatgaattc tgagaaaata ttgtgttcat 166980
ctgcatccaa atttcattta aaatatctgg taattcaagc cttttagtaa tttttagaaa 167040
taaatataca tattttatca tgtatcaatc ttaaaaactc agtaagaata catcttgtca 167100
ttacattaaa actatcaatt ccaaaatatc cataacatga ataatcatat caaaggtatt 167160
ttagccaaaa ggaaatgcta ttttaagata aaaacaatgg aggagttaaa aagtacaaag 167220
tagtatatca ctaagatatg ccttaagtaa atattttact atcaaattcc tcatatataa 167280
gaaacaccat gcagtttcac ttgaacgaca tgttactgtg aataatctgc tataataaac 167340
tcctttgtaa caagtaactc tacaaaatga aattacaaca aattattaat ttatggctgt 167400

cacatgaccc aactaatctc cacaagtgca ttaaaaacat gatacttgac taccatgttc 167460
agctgtaact aactgggtta tttactgcca ataaactctg tttacctgct gtgtagtttc 167520
taagggtcga attcttttct tctctacttg aaaatcatca ttttcatctc tgtatatgga 167580
tgcctgtttc ttagcagata actttgcagc cagagtggtt ttttcagggg agtcttatat 167640
aagtaattta aaaaaaacag cataaataac ttacaggtag gcaatttttc tagaagaaaa 167700
ctggaaccag gatgtaaggc ttatttctag aaatttcata ccataatcta aaacatgaaa 167760
cttaaacatg tttaaaaagt acttctctac cattcagttt actttaccaa agattctgag 167820
aaacatgctt tcaataggaa aagtccaatt tgattttttta aatcgcctta tgtacttgta 167880
gactactccc aataacatga tttaaaatgt ataactgcaa ggatctctga agaaaatata 167940
tttcatgtct gtaaaaatct ttctttacag tatcaacgtt tcttccctac taagtttctc 168000
cacgttcttt agagcaaaac cattgattat agaatcaagc tcatataatc tgtcttttaa 168060
tcccataatc ctgaaagctc cttctcaagg tcatccagga cctcttaatc accaaataac 168120
attaattatc ccaatcttac aaatgtgaac actaggtttt taaaagtcta gtagcttgcc 168180
cagtatcata cagttattaa atggtagagc ctagacatga ctgaatcctg ctgtgtgact 168240
ccaacatcca tacatgtaac ctcttaggca tacagccaac actgtcttct tggaacacag 168300
caaacacatt ttcttcttcc ttaatgtctg taacactgct cttaactatt ctcatacatt 168360
ggtttctccc ttttcctaaa tataaatatt cttaaaggta ccctccttcc tctttgcttc 168420
tctctaagca ttctctccac tcccatggct tgaattaatt taacaatctt ttttaaccat 168480
cttttttttt ttttttgaga tggagttttc ctcttgttgc ccaggctgaa gtgcaatggt 168540
gtgatctcag ctcaccacaa cctctacctc ccaggttcaa gcgattctcc tgcctcagcc 168600
tcctgagtag ctgggattac aggcatgtgc caccacgcct ggctaatttt gtatttttaa 168660
tagagacggg gtttctccat gttggtcagg ctggtctcga actcctgacc tcaggtgatc 168720
cacccgccta gcctcccaaa gtgctgggat tacaggcgtg agccaccgtg cccagccaac 168780
aatcatttat tacactgagt ctaacccatg gccagacacc atggaaaaac agtgaaaaag 168840
agaatccttg gccctaaagg atttacagca gagaaagacc tacatgagca ggtccaatac 168900
tgttttcaaa acacaaattt gaggccggcc ccagtggctc acgcctttaa tcccagcact 168960
ttggaaggcc gaggcgagtg gatcacttga gcccaggagt tcaagacctg cctgggcaac 169020
atggcgaaat cccatctcta ttaaaaatac gaaaattagc tgtgcatgtt ggtgcaggcc 169080
tataatccca gctactcggg aggttgaggc atgagaatct cttgaacctg ggaggcagag 169140
gttgcagtga gctgagatcg cgccatgcac tccagcctgg gcaacagaac aagactctat 169200
gtctccaaaa aaaaaagaaa gcttgagatt acataaactc agaaatgaac ctatgccaca 169260
tagcactgat tttcaagttt tttctaaagt gtaggaatat tttcttctta aagaaatgtt 169320
gcagctgggc acggtggctc acacctgtaa tcccagcact ttgggaggcc gaggcaggtg 169380
gatcacctga ggccaggagt ttgagaccag cctggccaac gtgacgaaag cccatctcta 169440
ctaaaaatac aaaaattagc tgggcatggt ggtgcatgcc tgtaatccca gctacttggg 169500
aggctgaggc aggagaatca cttgaacccg ggaggcagag gttgcagtga gccaagatcg 169560
caccactgca ctccagccta ggtgacagaa cgagactcca tctcaaaaaa agaaaaaaaa 169620
agaaatgttg cttaaaattt ccaaaatagg gatgaaagac ccagaacttc cctgatccac 169680
tttccccctt cccttatgag tcatgtttct ttctttataa ggttcttaga aacacagttt 169740
gaaaaccact atactctaaa aatcctaata gttttgccta tcagaagtag gtctttaatc 169800
taccttgaat gggcttttgt atatggtata aagtagggct ccaatttcaa ttttttccat 169860
atggacatct aacaggccta gcaccaccca agttcttcct ttcccctaca agctgtaggg 169920
gcacttctat aaaaatatca agtttctgga tacatgtggg accaaattag ggttctctat 169980
acttttatat gagtctatca ctgtgctaac ctcagtctgt cttaattact ctatatttac 170040
acacaataaa tccgtatatt tggtggatca aatctctcac cttgacctgc ttcttcaaaa 170100
gcatcctggc tatccttagt cctttgcatt tccatatata ttttagataa gataggtaat 170160
tttgagctct gcatgtccag gcaaccagta aaataaccac aaaataaacc tagatcagtg 170220
attctcaacc aggccaattt taacaccacc acttctcatc ccaggcatac tgggcaatgt 170280
ctggagacat ttttcgttgt cacaaaaaga gcagaggtgg aaatactact agcatccagt 170340
gcataaaggc cagaagtatt gctaaacatc tcacaaggga gagctcctac aacaaagagt 170400
tatcccactc aaaatgttaa tagtgccact gtggagaaac tctgtcccaa ataaatacgg 170460
ggcaaactga catgggccta ttaagagggt agggaatgtc cacccaaaat gatcatttag 170520
ttttagaaaa ctgaactgcc atgtgccaaa taagggtaac cccaattcat aaaaataaaa 170580
atcatattat catcacatac aaaaaggctt atcttcctgg tcttgatagc aagggagttt 170640
gaaggaagtt catcaaaggg gtaaaatctt taagatacaa agttaaaaag aaagaagata 170700
tctcccgatt atgttttttt aaaatcttca agaaacctaa gaaataccca atagaaaaat 170760
ggaggctggg cacggtggct cacacttgta attcaagcac tttgggaggc tgaggcgagt 170820
gatcatttga ggtcaggagt tagagatcag cctggccaac atggtgaaac cccatttcta 170880
ctaaaaatac aaaaatcagc cgggcgtggt ggcaggtgcc tgtaatccca gctactcagg 170940
aggttgaagc aggagaattg cttgagcctg ggagctggcc agtgcagtga gttgagatcg 171000
caccactgca ctctagcctg agcgacagag tgagacccca cctcaaataa aaaacaaaag 171060
aaaaatggca aacaacttga ttaggcaatt cactaaagag ggtatccaaa tgaccaataa 171120
acatatgaag tcaccagaga aatactaact aaaacccaat gagaatacca cacatacaca 171180

agaatgacta actttttttt taaactataa atatcaagta ctaacaaaaa tggtgaacaa 171240
ctttcatata ctgctgcagg ggttggaggg atctgaccgg ggacgcataa attgattcaa 171300
ccactttaga aaatgttgtc agtatctact atagctgaac atatgtatat actatgacca 171360
ggtatgtgtc tacagacaat acttacctat ttgcaccctt tgaacaagaa tgttcataac 171420
agcactatta aacaatactg aaaacaaccc aatgcctaac aactgtagaa cagattaata 171480
aactgtggta tattcataca aggaaataat acacagaaac aagaagtact actatatgca 171540
acagcatgaa tgaatctcac aaatacaata ttaagcagaa gacagacaca agattacata 171600
tataattcca tttttttttt ttgagatgga gtttcactct atttctgtaa aaaggcaaaa 171660
ctcatcaatg gtcacaggag gacaagaatt acctatggaa agaagggaca gtgactgaaa 171720
aagaagatga ggagggcctc tggggtgttg gtaatgtctc tctctttttt ttttaatcta 171780
tgtagtagtt atgacagtga aaattcatca agttatatac ttaggatttg catttttcta 171840
tatttaggtt acagattaaa ggatttactt acaaatataa agagcgtatc agaatacaac 171900
cttgattttt cccatttaga ctattctaaa taacactgac tcacagtaat gtcaaaatca 171960
agttgtgtac atatggataa ctcattgttg ataaagacac aaaggcaatg tagtggataa 172020
aggatagcct tttcgatata tccaggtgct gaacaactgg atatccacat gcaaaaaaat 172080
gaacttgaat ccatacctta caccatatac acatattaac tcaaaatgga tcttagacct 172140
aaatgtaaac ctaaaattct atctgatcaa agacttgtat tcaaaatgtt taaagaagtc 172200
tcaaaattta acagtaagaa cacaaactaa taaaaaatag gcaattattc gaacagacat 172260
atcatcaaac aagatacatg gatagctaat aagaacatga aaatatggga aataaaaatt 172320
aaaaccacaa tgagatactg ccacatgcct attagagtag ctaaaattaa ggccaggtat 172380
ggtggctcat gcctataatc ccagtgcttt gggaggccga agaaggcaga tcacttgagc 172440
ccaggggttc aagacaagcc tggggaacac agtaaaacca acaaaaacta taaaaattag 172500
ctaggtgtgg tggtgcatgc cagtagtctc aactactagg gagtgtgaag tgtgaggatc 172560
acttggttcc aggaggcagg ggttgcaatg agccaagtga gccaagatca cagtactgta 172620
cccagcctgg gcaacagaat gaaaccctat ctccaggaaa aagaatggc taaaattaaa 172680
aggactgacc ataccaaggg ttcacaagga tgtgaagaaa ctggaactct cataactgct 172740
gatgggaata taaaatggta tgaccacttc atgaaacagt ttagaaattt cttaaaaagt 172800
tggccaggca cggtggctca cgcctgtaat cctaacactt tgtgaggcac agatgggtgg 172860
atcacttgac gtcaggagct acagagcagc ctgaccaacg tggtgaaacc ccatctctac 172920
taaaaataca aaaattagcc aggtgtggtg gcacattcct gtaatcctag ctacttggga 172980
ggctgaggca ggagaatcac ttgaacccga gaggcagagg ttgcagtgag ctgagattgc 173040
gccactgcac tccagcctgg gcaagagtga ctccgtctca aaaaaaaaaa aaaaaaaaaa 173100
agttaaaaca tacacaccta caaagcaatc tggtcattct gctcctagta ttcacccaag 173160
agaaatgaaa acatacatcc atacaaagat gtagacacaa atgttcacag tatctgtctt 173220
tgtaatagcc cagaactagc aataacccaa atatacataa acagccaata accaatttgt 173280
ggtatatcca tgcacaatta ctcagaaata aaaagaaaca aactattaat acatgcaaca 173340
acatagagga atctcaaaat aattgtcata agtgaaagaa gccagacaaa ataagtatac 173400
atcctgtatg aatccattta tataaactat agaaaatgca aactaatcta tagtaacaaa 173460
aatcagacag gtggttggtt ggggaggtga gtcatgtagg ggtgggaaag agggataaca 173520
aaagtccatg agaaaacttt ttgggaaagt gagcatgttt aaaaagtttt ggataaagtg 173580
aatatggggt tggtgtgtat gttcactgtc ttgattctgg tgaaagtttc atggaggtac 173640
atatgtcaaa atgtatcaaa gtgtaccatt taggctgggc gtggtcgctc acgcctataa 173700
tcccaggact ctgggaggcc gaggcaggtg agtaacttga ggtcaggagt tcgagaccag 173760
cctggccaac atggtaaaat cctgtcccta ctaaaaatac aaaaaattag ctgggtgtgg 173820
tggcaggcgc ctgtcatccc aattacttgg gaggctgagg caggataatt gctcgaaccc 173880
aggaggtgaa ggttgcagtg ggccaaggtc acaccactgc actccagcct gggcaacaga 173940
gtgagattct gtctcaaaaa aaaaaaaaaa aaaagtacca tttaaatata tgcaatttgt 174000
tatgtcaagt aaacgtcaat aaagctgttt ttaaaaggtt gtatataaat attatacacc 174060
agaattccta taattagctg aaaattaatc ccattcattc ctaacaacaa atatttatct 174120
agcacctacc atatgccagg caccctgcta gagttctagg gatacaatgg taagcaaatt 174180
gacaatattc ctaacctcat gaagtagtct gccaaggaag cagagattaa taaaagattt 174240
agacagacac atataataca aactgtaaca agtgctataa aggaaaggta cacaaggaga 174300
agacagacta taataggaaa atcaaatcta ctctgacaga aaaatcagtg atggataccc 174360
caaggaagtc acatgtaaac tgaggaagaa aaggataata aattgaagat ggaactttgc 174420
tcagaagaga cactaataga agcaaaggct ctgaggaagg agaaagcata aatctttgaa 174480
ggaaagggaa gaaatccaaa aggccaaaat caaaacaatg tagacaacac tattggtcac 174540
ctatttaata actgttccac ttctctttcc tcattcctac cagaagttca atcatctacc 174600
ctcctctata tagtcacatg ctttagagga agctgactcc atccttgatt ttaggaaatg 174660
gatactcttt gttttgactt aagtcaatca tggcaacctc tgtctccctg ccagtgactg 174720
gtaaaggaat agcatgagta aaagcagaaa aaagcgttat tggatgctct aaacaagaaa 174780
tggtagtttg gactatggag gtaccaatgg atatggagat gtacatgaag aaataagaaa 174840
gagcaaagtg tccaggatgt tccctaggtt tcttaggtaa ataatagtct atcctttcta 174900
tacagccaaa agcaggtgaa atatttttta aatatattat taataatctt atgttactct 174960

taatgtattt ctctaaaatt agtaaaaatt atttaaagat cacacataaa catacctgat 175020
acttatgtag gatactggcc tcagaaggcc aggcaaactt atcaagaatg aatcaatgag 175080
gctaattaga tttcgtctta ggatacaaac attgttttat agaattctaa catataatta 175140
acaataatgg ctacaaataa catcaatttt ggaaaatcat ttattttgcc ttgccttcct 175200
taaaaaatta tttaggacaa caaaatgtct caataaatta aaagtcaaac caagaattta 175260
aaaacgtcta tggatttttg accactctgt gctattttaa aatcattcca gagaaactag 175320
atagagatat ataatcagtt atgctaacca aacttatata aattagggct tataacagta 175380
ataattaaga ctcttattac agatatcaac tgacccaggc aaaatataaa ttaccttgac 175440
aagttgatga agtgccattt ctttcagaag gtggtgtgct tggatagttg aaatgacgtg 175500
aagttccttg gttcatgtca ttgtttgtaa aatcctttga atggcatgca caacaacatg 175560
acaattgtac ttcagctttt tcacttacgc cctcatctgc tggtttccct aaaaatgaaa 175620
gaacatctat ttataatata tctaattaaa taaacatcaa tcattctcta cagcccagtt 175680
cacccaggat tgggaggttt cctaagataa aagattttta gggctaacac caggaaggta 175740
cctggcaaac ctggacaagc tggtcacttt atctgcagca tcaaattagg acaggagaga 175800
aataagccaa aaacatttgg ttggacacag tggctcacgc ctgttatcct agcactttgg 175860
gaggccgagg caggcacatc aattgaagtc aggagtttga gaccagcctg gccaatatgg 175920
taaaaccctg tctctactaa aaatacaaaa attagccagg cgtggtagtg cacgcctgta 175980
ataccaccta ctcaggaggc tgaggcagga gaatcgcttg aacctgggag gcagaggtag 176040
caatgagcag aggtcatgcc actgcactcc agcctaggcg acagagcaag actctgtttc 176100
aaaaaccaaa acaaaaacat ttatagggaa attatcaccc atttatgtaa tctgtaaaaa 176160
tctaataatc taagattaat aatgtaataa aagtacaact actcaagtat tcatttatat 176220
taagatttat ctaaaattca agaatggatt gaaacaagga cataaagtat atccttaatt 176280
actactcttt ccctaaatga agaatttgaa agttcaatat attagataat aatctaacac 176340
ataagggtta aatcattccc ccaattatac ttctatttca aatagaaaca tgtttttga 176400
tgtattaatt ctgatacttg catcaaaata cattttaagg tcaatattca aatcaataaa 176460
ataggtaatt ttagtgctaa atataatacc aaattgtaat aagtttacat tgtgtgccta 176520
cattgtatac aaatgtacaa ttcatatacc cccaaaaaag ctaaataatt tatttttaaa 176580
aataatcagt attgacattg ttttcccatt tgtaccatat aaatattcct aattctattt 176640
tccagacatc tgccattaag acctgaagtt agtaccgata aactcatttt tcccaaaatt 176700
aacgtcagtg agaatcaaaa tgaacaacca aaagcttatg aagtatttac tgtttttttt 176760
ttttttgag acagagtttc gctcctgttg cccaggctga agtgcaatgg cgcgatctcg 176820
gctcactgca acctccacct cccaggttca agtgattctc ctgtctcagc ctctcgagta 176880
gctgggatta caggcacacg ccacctcacc aggctaattt tgtattttta gtagagacgg 176940
ggtttctcca cgttggtctg gctggtctca aacttccaac ctcaggtgat ccacctgcct 177000
tggcctccca aagtgctggg attacaggcg tgcgccactg tgcccagcct taatatactc 177060
ttattagtaa caaacatttc attttaaaaa tgtatctata tacctatata cacatacata 177120
catacataca cataccatgt ttaacctcaa ttctcctaat actttaggtt aaagccatct 177180
aatgtaacta aaatgtatcc aatattatat ttctgattca ttatgtagtt tatatagtct 177240
ttcatagaag taaatattaa taggcatttg ttagcaaaca aagtaaaaat agtttggcct 177300
ggtggctcat gcctataatc ccaactattt aagaggctga agcaggagaa tcacttgagc 177360
ctgggagttt gaggttacag tgagctatga tcacatcact atgctccatc ccaggtgacg 177420
ccactcaaaa aaaaaaaaaa aaaaaggaaa agtagtggga agtaatataa attcatacaa 177480
acaatattat actaaatttc tgtttatctg gcatttaaac aagtagaaag atcaactaga 177540
atgtattctt ttttatatat tccatgtgct gatgttatga gaaaggaaca ggtaagcaaa 177600
tatattaaag gatttccccc cccaaaaaaa gcaattagta tgtgttacag catcatgaca 177660
gcccatctct tgcatctttt acatacaaat tatcatctaa ttatttttat aataactcta 177720
agcatccaaa actttgtttg tttgttttct ttcacccagg ctggagtgca caatcacagc 177780
tctctgcaac ctcgacctcc tgggctcaag cgatcctacc acctcagtct cccaagtagc 177840
tgggactaca agtgtacatc accatgcctg gctaattgat tgtcaatttt tgtagagatg 177900
gggtatcacc atgctgccca ggctgccaag tctttatgta ctttccgact catcaaaaga 177960
ctaaattatg ttcaatacta ttttagcatt aattaaacat attttgctat attgaaactc 178020
ttttggaaga tttataactt atttcaaaga aacatcttta aacaaatatt taagttagcg 178080
acagcatggc tgaaccagtc tggataaaga atactgtatt atattttctc agatcccagt 178140
aagtaacctg aagatatcaa gcaactactt accactaaga gattgttgcc atgctaaagc 178200
agaacaaagt aaggctaagc tttttccact tcctgtggga ctctccaaca aacaatgttg 178260
cttgctgttt aatcctctga gaatctatga acacagaaac caatgaaaat aataaacata 178320
ttaactttat aaaggtctct ctccatctga agtttaaata gaacagcaag gaaaagtgag 178380
attgcactcc agggaacaca acaatagcag aaggaactca tatttagtta aatccaaact 178440
gtattccttt acacctctcc ctaagattat cttactaaaa tatcaagcac aagagaagaa 178500
atgaaaagag gaatctgtgc cagaattatg caagcatcaa tgatttctgt aaaaccagaa 178560
tacattacta cctatcaata tatttgatga aaacttaatg aaggatactg ctagtgagag 178620
tacaaattgt tacaactact tggtagagaa ataaacaatt tcttataaag tgaagatgcc 178680
cagctccagc aagtccagtt ctatgtgtac acccaaaacc tttcccacat gaacacaagg 178740

aaacatgcat aagaaagttc acagcagcag ccacgcttgt aatcccatca ctttgggagg 178800
ccaaggcagg tggatcattt gaggtcagga gttcgagacc agcctggcca acacggtgaa 178860
aacccgtctc tactaaaaat ccaaaaatta gctgggcgtg gtggcgcatc cctgtaatcc 178920
cagctactgg ggaggctgag gcaggagaat agcttcagcc tgggaggcag ggttgcagt 178980
gagccaagat cgcgccactg cactccaatc tgggcgacag agtgagacgc tgtctcaaaa 179040
acaaaaagaa aaaaggaagt tcacagcagc attgtttgta actgcaaaag aatgtaaaca 179100
acctaaatgt ctaccaataa gaaaatggac agataaattc tggtatattc atccaatgga 179160
atttatacaa caattaaaat ggattaagaa tcaacagggc tatacattaa aaataatgct 179220
aagttaaaaa actcaagttc tgcataaaaa gtaaggtacc gtatcacatc aatgcacagt 179280
ttcaaatatg taaaataata ttctatattg tttatggata catatatatg taataaaagt 179340
ataaaaacat ggctaggaaa ggaaaagaac aaattcagga tactaataat cttgttacca 179400
gtaaataaga tatgaagtac atatggcaaa atagctgtaa caaaactgga aagctggttt 179460
actcaaattt gtatattaat tttctacatg atccaaacaa aacaatggtg agaaaaatag 179520
tattctgtgt aaaagatagt aaatactctg tttttactta aaagtatagc agtttttcat 179580
taaaagtata gaaattccaa atgagctgag aacatttttc agaatgttct tctttgcctt 179640
aaccacagat atatacagtt tagctgcaat cagtagtttc ccagaggtta gatattcttc 179700
caagtgaacc cagaaaatat tctccattta ctgagaatat gaatgcagtc aaatactcaa 179760
tgtactttat gggtcataag tatctatatc ttaataaaaa cttaactgct gaaaaatact 179820
tacagaattc atcatagcaa gctgtgacgg gtaagcttta taaggaaagt aaatcttcac 179880
cccaccaatt gtatattcag accacattga agacatagtg ctttcctgtt tatttcagat 179940
tcctaactac aacagaaatg aaaatgtcaa atattgagac acgccttaca aagaaaacca 180000
gagaaccaaa actaagggag aaatctggaa acagaaactg gaattaataa aagtataaac 180060
aaaacaaatg gaaacaaaat aatttcctag tcttataaat cacagcggtc aagagcatgt 180120
cttagagtct aacaaatcta gatttgtatc cttcactctg ccaggtatta gttgtgtgac 180180
ttcgggaaag ttagttacct tctctaagcc acagtgactg catgtacccc ataggattgt 180240
ggtaatgatt aagtgaaata atgtgcaaat atgataaagt acataccaga ttaaacatga 180300
gctcttgtaa cagttagatc agatgtaatc aacctacaga aacaaataag ttcctgaggc 180360
ctctctctct acaactccgt agcatcactc tttctccagt tcttcccctt tgtgttaccc 180420
ctttctgcag tcctccagtt agaagaaaaa agctgttgac cagaagtcag gaagcatagg 180480
ttctatacca ggttttggta tactgcctgg tatgttatct ttgactcagg tatgttatct 180540
ttgacatatc tttttacttc tctagtctaa gatccttacc tgtgataacc cctaatttaa 180600
tttaaaggct gttataagaa agagaatgcc ttggcacata gtaggcactc aaaaatgtta 180660
tttgaagatc agtagagtat tatagaaaca ggatgggctt tggagtaagg caatcctaag 180720
tttgaattcc actgtgtgaa tcacttaact tctcagagct tgtttctcca tatatggaat 180780
tgaaatacta ctacttacct gacagggttt ttatgaaaat tagccagggc agaatgtacc 180840
taacccagga tctgatacag atgaggatca ctttaatcct cacctcttct aaaccacttt 180900
tcttaaccta ggatccacgg atgtaattca gagacaatga cctcttgaaa ttgcaagcat 180960
aagtttctgt acatgattgc atttttctga agaaaaagtc catagatttc tcttcccgta 181020
aatatatctc aaggaaatca aaatagcggg ggaaattaca tgcactgtta ttaaataagg 181080
aattatttgc agtcgtggga aaaaattgta aattcaacct ggggacgaag agatggttaa 181140
aatagcaatt caatagatta tgaagccaat aataacattc accactgctt gtgtgctcca 181200
aaagaagaac cattattcta gggtattttc cagctctaac attttgcgat tctatctgta 181260
aagctctgag aatgaggtat ggtggaatct aacctcttaa tatgaatcaa caactcaacg 181320
ctggcctagt tactcagata taatctagaa atcagcccgt cctgtacccc ggctgtggca 181380
acaatagtgt caaaatgggg tagcttcccc caaacattgc ataaaggata aacacacccc 181440
tctgcctccc gccaagaatt aacgcagcgc ccgggctaag aacaggcctg cgctcaaagg 181500
aggtaaggat aggctccctc ctcaggtttt ctgccccgta tctccctcct cccccagcca 181560
gttgagatcc ccgagaccct ggtgatccag tcaccaccca tgggcccgca ggctgtgcag 181620
aagaactcaa gccctttccg tggacttccc tccgacttgc ctgtctggag ggaaaccgaa 181680
gcaacgctct gagctccgat tcactgaaga aaaggaaagg aaaccgactt ctgagagcaa 181740
ataaagcgga gccctggaag agaagaaagg gcacgagccc ttcctcctcc ctcttcctgg 181800
gggtgctgct acttccccgg ccttgtgtgc aggactgggg ccgccgttac ctttcctcga 181860
cccaccagac ccctcaacca cacaacccga gacgaattcc cgcctcccgc cccctcgact 181920
cccagcgccc aatagcccag cgagctcgac caatcacccg ccaaggccag gaatcaacca 181980
gtcccggtgc gaggtgaggg ggcgagaact tcggcatcca atggaagtct ccgaaaaaaa 182040
aaaaaaaaaa caagggctca aggtacgcgg cggaaggttg tcgccactcc agcaaatccc 182100
tagtgtttgt ttaaagtaga aagtcgtctc agtttgactt ttgaaaccgg cgatagagtc 182160
tatgcagcaa attccttgga aactggaagt ctcattcttc acgtccgtag accagcggaa 182220
aggaagtatc tttaagcttt gccaagttct ttgagcattt atcagtgcca ctcaatttaa 182280
attacccagc tttgcagtag ccaggatagc gaagactcag gcgggaattg ccagactgct 182340
gagccaaact taagtaagaa ggctctgtga catcgggcta caatctatct ttcataatat 182400
tcaaggcttt cccggatgct aaaataagcc agagacaagc tctcagggtc tccagtatca 182460
gatcacttct acttccatta aggcttatcc gccttccatc cctcctaaga ccagtttatt 182520

```
atttgccagt agtactttaa tttactcaaa gaaaaggagg ttcatcatga tcttgccaaa      182580
tcatgttatt tttttaaaaa atctgtataa cctaaacttg gctcaaataa taatgtctcc      182640
aaaaggcaaa acctcatact gtttctcaaa ttcaagtaac tagtttgggg aaggagtttg      182700
ctgggaagaa agagcaggag ggggctagtt gatttgtttg cctcattgtc aattgtgata      182760
tatcttacca a                                                           182771
```

<210> 16
<211> 1249
<212> PRT
<213> Pan troglodytes

<400> 16

```
Met Ser Ser Met Trp Ser Glu Tyr Thr Ile Gly Gly Val Lys Ile Tyr
 1               5                  10                  15
Phe Pro Tyr Lys Ala Tyr Pro Ser Gln Leu Ala Met Met Asn Ser Ile
            20                  25                  30
Leu Arg Gly Leu Asn Ser Lys Gln His Cys Leu Leu Glu Ser Pro Thr
        35                  40                  45
Gly Ser Gly Lys Ser Leu Ala Leu Leu Cys Ser Ala Leu Ala Trp Gln
    50                  55                  60
Gln Ser Leu Ser Gly Lys Pro Ala Asp Glu Gly Val Ser Glu Lys Ala
65                  70                  75                  80
Glu Val Gln Leu Ser Cys Cys Cys Ala Cys His Ser Lys Asp Phe Thr
                85                  90                  95
Asn Asn Asp Met Asn Gln Gly Thr Ser Arg His Phe Asn Tyr Pro Ser
            100                 105                 110
Thr Pro Pro Ser Glu Arg Asn Gly Thr Ser Ser Thr Cys Gln Asp Ser
        115                 120                 125
Pro Glu Lys Thr Thr Leu Ala Ala Lys Leu Ser Ala Lys Lys Gln Ala
    130                 135                 140
Ser Ile Tyr Arg Asp Glu Asn Asp Asp Phe Gln Val Glu Lys Lys Arg
145                 150                 155                 160
Ile Arg Pro Leu Glu Thr Thr Gln Gln Ile Arg Lys Arg His Cys Phe
                165                 170                 175
Gly Thr Glu Val His Asn Leu Asp Ala Lys Val Asp Ser Gly Lys Thr
            180                 185                 190
Val Lys Leu Asn Ser Pro Leu Glu Lys Ile Asn Ser Phe Ser Pro Gln
        195                 200                 205
Lys Pro Pro Gly His Cys Ser Arg Cys Cys Cys Ser Thr Lys Gln Gly
    210                 215                 220
Asn Ser Gln Glu Ser Ser Asn Thr Ile Lys Lys Asp His Thr Gly Lys
225                 230                 235                 240
Ser Lys Ile Pro Lys Ile Tyr Phe Gly Thr Arg Thr His Lys Gln Ile
                245                 250                 255
Ala Gln Ile Thr Arg Glu Leu Arg Arg Thr Ala Tyr Ser Gly Val Pro
            260                 265                 270
Met Thr Ile Leu Ser Ser Arg Asp His Thr Cys Val His Pro Glu Val
        275                 280                 285
Val Gly Asn Phe Asn Arg Asn Glu Lys Cys Met Glu Leu Leu Asp Gly
    290                 295                 300
Lys Asn Gly Lys Ser Cys Tyr Phe Tyr His Gly Val His Lys Ile Ser
305                 310                 315                 320
Asp Gln His Thr Leu Gln Thr Phe Gln Gly Met Cys Lys Ala Trp Asp
                325                 330                 335
Ile Glu Glu Leu Val Ser Leu Gly Lys Lys Leu Lys Ser Cys Pro Tyr
            340                 345                 350
Tyr Thr Ala Arg Glu Leu Ile Gln Asp Ala Asp Ile Ile Phe Cys Pro
        355                 360                 365
Tyr Asn Tyr Leu Leu Asp Ala Gln Ile Arg Glu Ser Met Asp Leu Asn
    370                 375                 380
Leu Lys Glu Gln Val Val Ile Leu Asp Glu Ala His Asn Ile Glu Asp
385                 390                 395                 400
Cys Ala Arg Glu Ser Ala Ser Tyr Ser Val Thr Glu Val Gln Leu Arg
```

```
                405                 410                 415
Phe Ala Arg Asp Glu Leu Asp Ser Met Val Asn Asn Asn Ile Arg Lys
            420                 425                 430
Lys Asp His Glu Pro Leu Arg Ala Val Cys Cys Ser Leu Ile Asn Trp
        435                 440                 445
Leu Glu Ala Asn Ala Glu Tyr Leu Val Glu Arg Asp Tyr Glu Ser Ala
    450                 455                 460
Cys Lys Ile Trp Ser Gly Asn Glu Met Leu Leu Thr Leu His Lys Met
465                 470                 475                 480
Gly Ile Thr Thr Ala Thr Phe Pro Ile Leu Gln Gly His Phe Ser Ala
                485                 490                 495
Val Leu Gln Lys Glu Glu Lys Ile Ser Pro Ile Tyr Gly Lys Glu Glu
            500                 505                 510
Ala Arg Glu Val Pro Val Ile Ser Ala Ser Thr Gln Ile Met Leu Lys
        515                 520                 525
Gly Leu Phe Met Val Leu Asp Tyr Leu Phe Arg Gln Asn Ser Arg Phe
    530                 535                 540
Ala Asp Asp Tyr Lys Ile Ala Ile Gln Gln Thr Tyr Ser Trp Thr Asn
545                 550                 555                 560
Gln Ile Asp Ile Ser Asp Lys Asn Gly Leu Leu Val Leu Pro Lys Asn
                565                 570                 575
Lys Lys Arg Ser Arg Gln Lys Thr Ala Val His Val Leu Asn Phe Trp
            580                 585                 590
Cys Leu Asn Pro Ala Val Ala Phe Ser Asp Ile Asn Gly Lys Val Gln
        595                 600                 605
Thr Ile Val Leu Thr Ser Gly Thr Leu Ser Pro Met Lys Ser Phe Ser
    610                 615                 620
Ser Glu Leu Gly Val Thr Phe Thr Ile Gln Leu Glu Ala Asn His Ile
625                 630                 635                 640
Ile Lys Asn Ser Gln Val Trp Val Gly Thr Ile Gly Ser Gly Pro Lys
                645                 650                 655
Gly Arg Asn Leu Cys Ala Thr Phe Gln Asn Thr Glu Thr Phe Glu Phe
            660                 665                 670
Gln Asp Glu Val Gly Ala Leu Leu Leu Ser Val Cys Gln Thr Val Ser
        675                 680                 685
Gln Gly Ile Leu Cys Phe Leu Pro Ser Tyr Lys Leu Leu Glu Lys Leu
    690                 695                 700
Lys Glu Arg Trp Leu Ser Thr Gly Leu Trp His Asn Leu Glu Leu Val
705                 710                 715                 720
Lys Thr Val Ile Val Glu Pro Gln Gly Gly Glu Lys Thr Asn Phe Asp
                725                 730                 735
Glu Leu Leu Gln Val Tyr Tyr Asp Ala Ile Lys Tyr Lys Gly Glu Lys
            740                 745                 750
Asp Gly Ala Leu Leu Val Ala Val Cys Arg Gly Lys Val Ser Glu Gly
        755                 760                 765
Leu Asp Phe Ser Asp Asp Asn Ala Arg Ala Val Ile Thr Ile Gly Ile
    770                 775                 780
Pro Phe Pro Asn Val Lys Asp Leu Gln Val Glu Leu Lys Arg Gln Tyr
785                 790                 795                 800
Asn Asp His His Ser Lys Leu Arg Gly Leu Leu Pro Gly Arg Gln Trp
                805                 810                 815
Tyr Glu Ile Gln Ala Tyr Arg Ala Leu Asn Gln Ala Leu Gly Arg Cys
            820                 825                 830
Ile Arg His Arg Asn Asp Trp Gly Ala Leu Ile Leu Val Asp Asp Arg
        835                 840                 845
Phe Arg Asn Asn Pro Ser Arg Tyr Ile Ser Gly Leu Ser Lys Trp Val
    850                 855                 860
Arg Gln Gln Ile Gln His His Ser Thr Phe Glu Ser Ala Leu Glu Ser
865                 870                 875                 880
Leu Ala Glu Phe Ser Lys Lys His Gln Lys Val Leu Asn Val Ser Ile
                885                 890                 895
Lys Asp Arg Thr Asn Ile Gln Asp Asn Glu Ser Thr Leu Glu Met Thr
            900                 905                 910
```

Ser Leu Lys Tyr Ser Thr Pro Pro Tyr Leu Leu Glu Ala Ala Ser His
915 920 925
Leu Ser Pro Glu Asn Phe Val Glu Asp Glu Ala Lys Ile Cys Val Gln
930 935 940
Glu Leu Gln Cys Pro Lys Ile Ile Thr Lys Asn Ser Pro Leu Pro Ser
945 950 955 960
Ser Ile Ile Ser Arg Lys Glu Lys Asn Asp Pro Val Phe Leu Glu Glu
965 970 975
Ala Gly Lys Ala Glu Lys Ile Val Ile Ser Arg Ser Thr Ser Pro Thr
980 985 990
Phe Asn Lys Gln Thr Lys Arg Val Ser Trp Ser Ser Phe Asn Ser Leu
995 1000 1005
Gly Gln Tyr Phe Thr Gly Lys Ile Pro Lys Ala Thr Pro Glu Leu Arg
1010 1015 1020
Ser Ser Glu Asn Ser Ala Ser Ser Pro Pro Arg Phe Lys Thr Glu Lys
1025 1030 1035 1040
Met Glu Ser Lys Thr Val Leu Pro Phe Thr Asp Lys Cys Glu Ser Ser
1045 1050 1055
Asn Leu Thr Val Asn Thr Ser Phe Gly Ser Cys Pro Gln Ser Glu Thr
1060 1065 1070
Ile Ile Ser Ser Leu Lys Ile Asp Ala Thr Leu Thr Arg Lys Asn His
1075 1080 1085
Ser Glu His Pro Leu Cys Ser Glu Glu Ala Leu Asp Pro Asp Ile Glu
1090 1095 1100
Leu Ser Leu Val Ser Glu Glu Asp Lys Gln Ser Thr Ser Asn Arg Asp
1105 1110 1115 1120
Phe Glu Thr Glu Ala Glu Asp Glu Ser Ile Tyr Phe Thr Pro Glu Leu
1125 1130 1135
Tyr Asp Pro Glu Asp Thr Asp Glu Glu Lys Asn Asp Leu Ala Glu Thr
1140 1145 1150
Asp Arg Gly Asn Arg Leu Ala Asn Asn Ser Asp Cys Ile Leu Ala Lys
1155 1160 1165
Asp Leu Phe Glu Ile Arg Thr Ile Lys Glu Val Asp Ser Ala Arg Glu
1170 1175 1180
Val Lys Ala Glu Asp Cys Ile Asp Thr Lys Leu Asn Gly Ile Leu His
1185 1190 1195 1200
Ile Glu Glu Ser Lys Ile Asp Asp Ile Asp Gly Asn Val Lys Thr Thr
1205 1210 1215
Trp Ile Lys Glu Leu Glu Leu Gly Lys Thr His Glu Ile Glu Ile Lys
1220 1225 1230
Asn Phe Lys Pro Ser Pro Ser Lys Asn Lys Gly Met Phe Pro Gly Phe
1235 1240 1245
Lys

<210> 17
<211> 1214
<212> PRT
<213> Canis familiaris

<400> 17

```
Met Ser Ser Leu Trp Ser Glu Tyr Thr Ile Gly Gly Val Lys Ile Thr
 1               5                  10                  15
Phe Pro Tyr Lys Ala Tyr Pro Ser Gln Leu Ala Met Met Asn Ser Ile
            20                  25                  30
Val Arg Gly Leu Asn Ser Lys Gln His Cys Leu Leu Glu Ser Pro Thr
        35                  40                  45
Gly Ser Gly Lys Ser Leu Ala Leu Leu Cys Ser Thr Leu Ala Trp Gln
    50                  55                  60
Gln Ser Val Ser Gly Lys Leu Val Asp Glu Ser Leu Ser Lys Thr Glu
65                  70                  75                  80
Val Ser Ser Ser Cys Cys Cys Ala Cys His Ser Lys Asn Phe Thr Asn
                85                  90                  95
```

```
Ile Asp Leu Asn Gln Gly Thr Ser His His Phe Asn Ser Pro Ser Thr
            100                 105                 110
Pro Pro Ser Glu Arg Tyr Asp Thr Ser Ser Thr Cys Gln Asp Ser Pro
        115                 120                 125
Glu Lys Thr Thr Leu Ala Ala Lys Leu Ser Ala Lys Lys Gln Ala Ser
    130                 135                 140
Ile Lys Arg Asp Glu Asn Asp Asp Phe Gln Val Glu Lys Lys Arg Ile
145                 150                 155                 160
Arg Pro Leu Glu Thr Thr Gln Gln Ile Arg Lys Arg His Cys Phe Glu
                165                 170                 175
Lys Lys Val His His Val Asp Ala Lys Val Ala Ser Gly Lys Thr Thr
            180                 185                 190
Lys Leu Asn Ser Pro Leu Glu Lys Ile Asn Ser Phe Ser Pro Gln Asn
        195                 200                 205
Pro Pro Gly His Cys Ser Arg Cys Cys Cys Ser Thr Lys Gln Gly Ser
    210                 215                 220
Asn Gln Asp Ser Ser Asn Thr Thr Lys Lys Asp His Gly Gly Lys Ser
225                 230                 235                 240
Lys Ile Pro Lys Ile Tyr Phe Gly Thr Arg Thr His Lys Gln Ile Ala
                245                 250                 255
Gln Ile Thr Arg Glu Leu Arg Arg Thr Ala Tyr Ser Gly Val Pro Met
            260                 265                 270
Thr Ile Leu Ser Ser Arg Asp His Thr Cys Val His Pro Glu Val Val
        275                 280                 285
Gly Asn Phe Asn Arg Asn Glu Lys Cys Met Glu Leu Leu Asp Gly Lys
    290                 295                 300
Asn Gly Lys Ser Cys Tyr Phe Tyr His Gly Val His Lys Ile Ser Asn
305                 310                 315                 320
Gln His Thr Leu Gln Thr Leu Gln Gly Met Cys Lys Ala Trp Asp Ile
                325                 330                 335
Glu Glu Leu Val Ser Leu Gly Lys Lys Leu Lys Ala Cys Pro Tyr Tyr
            340                 345                 350
Thr Ala Arg Glu Leu Ile Glu Asp Ala His Ile Ile Phe Cys Pro Tyr
        355                 360                 365
Asn Tyr Leu Leu Asp Ala Gln Ile Arg Glu Ser Met Asp Ile Asn Leu
    370                 375                 380
Lys Glu Gln Val Val Ile Leu Asp Glu Ala His Asn Ile Glu Asp Cys
385                 390                 395                 400
Ala Arg Glu Ser Ala Ser Tyr Ser Val Thr Glu Val Gln Leu Arg Phe
                405                 410                 415
Ala Arg Asp Glu Leu Asp Ser Met Val Asn Asn Asn Ile Arg Lys Lys
            420                 425                 430
Asn His Glu Pro Leu Arg Ala Val Cys Tyr Ser Leu Ile Asn Trp Leu
        435                 440                 445
Glu Ala Asn Ser Glu His Leu Val Glu Arg Asp Tyr Glu Ser Ser Cys
    450                 455                 460
Lys Ile Trp Ser Gly Ser Glu Met Val Leu Asn Leu His Lys Met Gly
465                 470                 475                 480
Ile Thr Thr Ala Thr Phe Pro Ile Leu Gln Gly His Phe Ser Ala Val
                485                 490                 495
Leu Gln Lys Glu Glu Lys Val Leu Pro Ile His Gly Lys Glu Glu Thr
            500                 505                 510
Arg Glu Val Pro Ile Ile Ser Ala Ser Thr Gln Ile Met Leu Lys Gly
        515                 520                 525
Leu Phe Met Val Phe Asp Tyr Leu Phe Arg Gln Asn Ser Arg Phe Ala
    530                 535                 540
Asp Asp Tyr Lys Val Ala Ile Gln Gln Thr Tyr Ser Trp Ile Asn Gln
545                 550                 555                 560
Thr Asp Thr Ser Asp Lys Asn Gly Phe Phe Ala Pro Ala Lys Asn Lys
                565                 570                 575
Lys His Leu Arg Gln Lys Thr Ala Val His Val Leu Asn Phe Trp Cys
            580                 585                 590
Leu Asn Pro Ala Val Ala Phe Ser Asp Ile Asn Gly Lys Val Leu Thr
```

```
            595                 600                 605
Ile Val Leu Thr Ser Gly Thr Leu Ser Pro Met Lys Ser Phe Ser Ser
        610                 615                 620
Glu Leu Gly Val Thr Phe Thr Ile Gln Leu Glu Ala Asn His Val Ile
625                 630                 635                 640
Asn Asn Ser Gln Val Trp Val Gly Thr Ile Gly Ser Gly Pro Lys Gly
                645                 650                 655
Arg Asn Leu Cys Ala Thr Phe Gln His Thr Glu Thr Phe Glu Phe Gln
            660                 665                 670
Asp Glu Val Gly Ala Leu Leu Leu Ser Val Cys Gln Thr Val Asn Gln
        675                 680                 685
Gly Ile Leu Cys Phe Leu Pro Ser Tyr Lys Leu Leu Glu Lys Leu Lys
    690                 695                 700
Glu Arg Trp Leu Tyr Thr Gly Leu Trp His Asn Leu Glu Leu Val Lys
705                 710                 715                 720
Thr Val Ile Val Glu Pro Gln Gly Gly Glu Lys Thr Asp Phe Asn Glu
                725                 730                 735
Leu Leu Gln Val Tyr Tyr Asp Ala Ile Lys Tyr Lys Gly Glu Lys Asp
            740                 745                 750
Gly Ala Leu Leu Val Ala Val Cys Arg Gly Lys Val Ser Glu Gly Leu
        755                 760                 765
Asp Phe Ser Asp Asp Asn Ala Arg Ala Val Ile Thr Ile Gly Ile Pro
    770                 775                 780
Phe Pro Asn Val Lys Asp Leu Gln Val Glu Leu Lys Arg Gln Tyr Asn
785                 790                 795                 800
Asp Gln His Ser Lys Leu Arg Gly Leu Leu Pro Gly Arg Gln Trp Tyr
                805                 810                 815
Glu Ile Gln Ala Tyr Arg Ala Leu Asn Gln Ala Leu Gly Arg Cys Ile
            820                 825                 830
Arg His Lys Asn Asp Trp Gly Ala Leu Ile Leu Val Asp Asp Arg Phe
        835                 840                 845
Arg Ser Asn Pro Ser Arg Tyr Ile Ser Gly Leu Ser Lys Trp Ile Arg
    850                 855                 860
Gln Gln Ile Gln His His Ser Thr Phe Glu Ser Ala Leu Glu Ser Leu
865                 870                 875                 880
Ser Asp Phe Ser Arg Lys His Gln Lys Val Ile Asn Val Ser Lys Glu
                885                 890                 895
Asp Arg Lys Phe Thr Gln Asp Ser Glu Ser Ile Leu Glu Val Thr Cys
            900                 905                 910
Leu Lys Asp Asn Thr Leu Thr Tyr Leu Glu Ala Ala Ser Pro Leu Ile
        915                 920                 925
Pro Glu Asn Pro Arg Lys Gly Glu Ala Lys Ile Arg Val Gln Glu Gln
    930                 935                 940
Gln Cys Leu Thr Ile Thr Glu Ser Pro Pro Leu Pro Arg Gly Ile Ile
945                 950                 955                 960
Ser Lys Lys Glu Lys Asp Asp Pro Val Leu Ser Glu Glu Phe Ala Gln
                965                 970                 975
Ala Val Lys Ala Glu Lys Asn Val Ile Ser Arg Ser Thr Ser Pro Ile
            980                 985                 990
Phe Asn Lys Gln Thr Lys Pro Val Ser Trp Ser Asn Phe Asn Ser Leu
        995                 1000                1005
Glu Arg Tyr Phe Thr Gly Glu Ile Leu Thr Ala Gly Pro Lys Phe Arg
   1010                1015                1020
Ser Pro Glu Asp Cys Ala Ser Ser Ile Ser Thr Leu Glu Thr Glu Glu
1025               1030                1035                1040
Gly Tyr Lys Thr Val Leu Pro Leu Thr Asp Lys Cys Glu Ser Ser Ser
               1045                1050                1055
Pro Met Leu Asn Ala Ser Cys Ser Gln Ser Glu Val Ile Ser Ser Val
           1060                1065                1070
Lys Ile Asp Ser Thr Leu Leu Lys Arg Asn Cys Ser Lys Gln Leu Phe
       1075                1080                1085
Cys Cys Glu Glu Ala Val Asp Pro Asp Ile Glu Leu Ser Leu Leu Gly
   1090                1095                1100
```

```
Glu Glu Ala Lys Ser Ser Thr Ser His Arg Ala Ser Glu Thr Glu Ala
1105                1110                1115                1120
Glu Asp Glu Ser Ile Tyr Phe Ser Pro Glu Leu Tyr Asp Pro Ala Asp
                1125                1130                1135
Thr Asn Glu Glu Lys Asn Glu Leu Val Glu Ser Asp Arg Asp Asn Arg
            1140                1145                1150
Phe Val Asp His Ser Asn Cys Ile Leu Val Glu Asp Leu Phe Glu Ile
        1155                1160                1165
Arg Thr Ile Lys Gly Val Asp Ser Val Gln Glu Met Lys Ala Glu Asp
    1170                1175                1180
Cys Thr Val Thr Thr Leu Asn Arg Leu Met His Ile Lys Glu Ser Lys
1185                1190                1195                1200
Ile Asp Asn Ile Asp Ser Asn Met Lys Lys Asn Ser Tyr Lys
                1205                1210
```

<210> 18
<211> 1174
<212> PRT
<213> Mus musculus

<400> 18

Met Ser Ser Val Leu Ser Asp Tyr Thr Ile Gly Gly Val Lys Ile His
1 5 10 15
Phe Pro Cys Arg Ala Tyr Pro Ala Gln Leu Ala Met Met Asn Ser Ile
20 25 30
Val Arg Gly Leu Asn Ser Ser Gln His Cys Leu Leu Glu Ser Pro Thr
35 40 45
Gly Ser Gly Lys Ser Leu Ala Leu Leu Cys Ser Ala Leu Ala Trp Gln
50 55 60
Gln Ser Leu Ser Glu Lys Pro Val Asp Glu Gly Leu Asn Lys Lys Pro
65 70 75 80
Glu Ala Pro Pro Ser Cys Ser Cys Ala Cys His Ser Lys Asn Phe Thr
85 90 95
Tyr Ser Asp Thr Asn Leu Asp Thr Ser Pro His Phe Asn Ser Pro Ser
100 105 110
Lys Pro Ser Ser Gly Arg Asn Gly Val Ser Thr Pro Cys Gln Asp Ser
115 120 125
Pro Glu Lys Asn Thr Leu Ala Ala Lys Leu Ser Ala Lys Lys Gln Ala
130 135 140
Ser Ile His Arg Asp Glu Asp Asp Asp Phe Gln Val Glu Lys Lys Arg
145 150 155 160
Ile Arg Pro Leu Glu Thr Thr Gln Gln Ile Arg Lys Arg His Cys Leu
165 170 175
Glu Lys Asp Val His His Val Asp Ala Arg Leu Ala Ser Glu Lys Arg
180 185 190
Val Lys Pro Glu Ser Pro Ile Gly Lys Ser Phe Ser Asp Arg Lys Asp
195 200 205
Ser Phe Gln Asn Val Asp Gly Leu Cys Ser Arg Cys Cys Cys Ser Ala
210 215 220
Lys Gln Gly Asn Asn Gln Glu Pro Ala Asn Thr Val Lys Lys Asp His
225 230 235 240
Gly Gly Gln Cys Lys Arg Pro Lys Ile Tyr Phe Gly Thr Arg Thr His
245 250 255
Lys Gln Ile Ala Gln Ile Thr Arg Glu Leu Arg Lys Thr Ala Tyr Ser
260 265 270
Gly Val Pro Met Thr Ile Leu Ser Ser Arg Asp His Ser Cys Val His
275 280 285
Pro Glu Val Val Gly Asn Phe Asn Arg Lys Glu Lys Cys Met Glu Leu
290 295 300
Leu Asp Gly Lys His Gly Lys Ser Cys Tyr Phe Tyr His Gly Val His
305 310 315 320
Lys Ile Ser Asn Gln Gln Thr Leu Gln His Leu Gln Gly Met Ser Arg

```
                    325                 330                 335
Ala Trp Asp Ile Glu Glu Leu Val Ser Leu Gly Arg Lys Leu Lys Ala
                340                 345                 350
Cys Pro Tyr Tyr Thr Ala Arg Glu Leu Ile Glu Asp Ala Asp Ile Val
            355                 360                 365
Phe Cys Pro Tyr Asn Tyr Leu Leu Asp Ser Gln Ile Arg Glu Thr Met
    370                 375                 380
Asp Ile Lys Leu Lys Gly Gln Val Val Ile Leu Asp Glu Ala His Asn
385                 390                 395                 400
Ile Glu Asp Cys Ala Arg Glu Ser Ala Ser Tyr Ser Val Thr Glu Val
                    405                 410                 415
Gln Leu Arg Phe Ala Arg Asp Glu Leu Asp Ser Leu Ile Asn Gly Asn
                420                 425                 430
Ile Arg Lys Lys Ser His Glu Pro Leu Arg Asp Val Cys Tyr Asn Leu
            435                 440                 445
Ile Asn Trp Leu Glu Thr Asn Ser Lys His Leu Val Glu Arg Gly Tyr
    450                 455                 460
Glu Ser Ser Cys Lys Ile Trp Ser Gly Asn Glu Met Leu Leu Asn Leu
465                 470                 475                 480
Tyr Arg Met Gly Ile Thr Thr Ala Thr Phe Pro Val Leu Gln Arg His
                    485                 490                 495
Leu Ser Ala Val Leu Gln Lys Glu Glu Lys Val Thr Pro Ile His Gly
                500                 505                 510
Lys Glu Glu Ala Ile Gln Ile Pro Ile Ile Ser Ala Ser Thr Gln Val
            515                 520                 525
Val Leu Lys Gly Leu Phe Met Val Leu Asp Tyr Leu Phe Arg Glu Asn
    530                 535                 540
Ser Arg Phe Ala Asp Asp Tyr Lys Val Ala Ile Gln Gln Thr Tyr Ser
545                 550                 555                 560
Trp Thr Asn Gln Ile Ala Ile Phe Asp Lys Thr Gly Val Leu Ala Val
                    565                 570                 575
Pro Lys Asn Lys Lys His Ser Arg Gln Lys Ile Gly Val Asn Ala Leu
                580                 585                 590
Asn Phe Trp Cys Leu Asn Pro Ala Val Ala Phe Ser Asp Ile Asn Asp
            595                 600                 605
Lys Val Arg Thr Ile Val Leu Thr Ser Gly Thr Leu Ser Pro Leu Lys
    610                 615                 620
Ser Phe Ser Ser Glu Leu Gly Val Thr Phe Ser Ile Gln Leu Glu Ala
625                 630                 635                 640
Asn His Val Ile Ser Asn Ser Gln Val Trp Val Gly Thr Val Gly Ser
                    645                 650                 655
Gly Pro Lys Gly Arg Asn Leu Cys Ala Thr Phe Gln His Thr Glu Thr
                660                 665                 670
Phe Glu Phe Gln Asp Glu Val Gly Met Leu Leu Leu Ser Val Cys Gln
            675                 680                 685
Thr Val Ser Gln Gly Ile Leu Cys Phe Leu Pro Ser Tyr Lys Leu Leu
    690                 695                 700
Glu Lys Leu Arg Glu Arg Trp Ile Phe Thr Gly Leu Trp His Ser Leu
705                 710                 715                 720
Glu Ser Val Lys Thr Val Ile Ala Glu Pro Gln Gly Gly Glu Lys Thr
                    725                 730                 735
Asp Phe Asp Glu Leu Leu Gln Val Tyr Tyr Asp Ala Ile Lys Phe Lys
                740                 745                 750
Gly Glu Lys Asp Gly Ala Leu Leu Ile Ala Val Cys Arg Gly Lys Val
            755                 760                 765
Ser Glu Gly Leu Asp Phe Ser Asp Asp Asn Ala Arg Ala Val Ile Thr
    770                 775                 780
Val Gly Ile Pro Phe Pro Asn Val Lys Asp Leu Gln Val Glu Leu Lys
785                 790                 795                 800
Arg Gln Tyr Asn Asp His His Ser Lys Ser Arg Gly Leu Leu Pro Gly
                    805                 810                 815
Arg Gln Trp Tyr Glu Ile Gln Ala Tyr Arg Ala Leu Asn Gln Ala Leu
                820                 825                 830
```

```
Gly Arg Cys Ile Arg His Lys Asn Asp Trp Gly Ala Leu Ile Leu Val
        835                 840                 845
Asp Asp Arg Phe Asn Asn Asn Pro Asn Arg Tyr Ile Ser Gly Leu Ser
    850                 855                 860
Lys Trp Val Arg Gln Gln Ile Gln His His Ser Ser Phe Ala Ser Ala
865                 870                 875                 880
Leu Glu Ser Leu Thr Glu Phe Ser Arg Arg His Gln Lys Val Thr Asn
                885                 890                 895
Arg Ser Lys Lys Asp Glu Lys Cys Thr Lys Asp Asn Glu Pro Thr Leu
            900                 905                 910
Glu Val Ala Cys Leu Glu Asp Ser Thr Phe Thr Ser Val Ser Glu Ser
        915                 920                 925
Ser His Gln Ser Pro Glu Asn Ser Thr Glu Glu Ala Glu Val Cys Val
    930                 935                 940
Gln Glu Leu Gln Cys Pro Gln Val Ala Thr Lys Ser Pro Ser Val Ala
945                 950                 955                 960
Ser His Gly Val Ser Arg Arg Lys Lys Ser Asp Pro Gly Leu Arg Gly
                965                 970                 975
Glu Ser Leu Gln Thr Met Lys Thr Glu Lys Asn Glu Ile Ser Arg Ser
            980                 985                 990
Ser Ser Pro Thr Phe Gly Lys Gln Thr Glu Pro Val Asn Trp Pro Ile
        995                 1000                1005
Phe Asn Ser Leu Arg Arg His Phe Asn Ser Lys Val Lys Asn Cys Thr
   1010                1015                1020
Pro Val Leu Lys Ser Ser Lys Asn Arg Ala Pro Gly Ser Ser Thr Phe
1025                1030                1035                1040
Asn Lys Thr Ala Leu Pro Leu Thr Gly Asn Cys Val Pro Ser Asn Glu
               1045                1050                1055
Thr Ala Asp Thr Ser Leu Gly Pro Cys Leu Gln Ser Glu Val Ile Ile
           1060                1065                1070
Ser Pro Val Lys Ile Glu Ala Thr Pro Ala Thr Asn Tyr Ser Lys Gln
       1075                1080                1085
Val Phe Cys Cys Glu Lys Asp Leu Leu Pro Asp Thr Glu Leu Ser Pro
   1090                1095                1100
Gly Thr Glu Glu Ala Lys Cys Pro Ser Ser Asn Lys Ala Ala Glu Thr
1105                1110                1115                1120
Glu Val Asp Asp Asp Ser Glu Cys Phe Thr Pro Glu Leu Phe Asp Pro
               1125                1130                1135
Val Asp Thr Asn Glu Glu Asn Gly Glu Leu Val Glu Thr Asp Arg Ser
           1140                1145                1150
Ser His Ser Ser Asp Cys Phe Ser Ala Glu Glu Leu Phe Glu Thr Ala
       1155                1160                1165
Thr Gly Phe Gly Gln Lys
   1170
```

<210> 19
<211> 1166
<212> PRT
<213> Rattus norvegicus

<400> 19

```
Met Ser Ser Val Leu Ser Glu Tyr Thr Ile Gly Gly Val Lys Ile His
  1               5                  10                  15
Phe Pro Cys Arg Ala Tyr Pro Ala Gln Leu Ala Met Met Asn Ser Ile
             20                  25                  30
Val Arg Gly Leu Asn Ser Ser Gln His Cys Leu Leu Glu Ser Pro Thr
         35                  40                  45
Gly Ser Gly Lys Ser Leu Ala Leu Leu Cys Ser Ala Leu Ala Trp Gln
     50                  55                  60
Gln Ser Leu Thr Gly Lys Pro Val Asp Glu Gly Leu Asn Lys Lys Pro
 65                  70                  75                  80
Glu Ala Pro Ser Ser Cys Ser Cys Ser Cys His Ser Lys Asn Phe Thr
```

```
                85                  90                  95
Tyr Ser Asp Thr Asn Leu Asp Thr Ser Pro His Phe Ser Ser Pro Ser
            100                 105                 110
Lys Pro Ser Ser Glu Arg Asn Ala Val Ser Ser Pro Cys Arg Asp Ser
        115                 120                 125
Pro Glu Arg Asn Ser Leu Ala Ala Lys Leu Ser Ala Lys Lys His Ala
    130                 135                 140
Ser Ile His Glu Asp Asp Asp Phe Gln Val Glu Lys Lys Arg Ile Arg
145                 150                 155                 160
Pro Leu Glu Thr Thr Gln Gln Ile Arg Lys Arg His Cys Leu Glu Lys
                165                 170                 175
Asp Val His His Leu Asp Ala Arg Leu Ala Ser Glu Lys Arg Val Lys
            180                 185                 190
Pro Glu Ser Pro Ile Arg Lys Thr Ser Ser Ser Phe Gln Asn Pro Asp
        195                 200                 205
Gly Leu Cys Ser Arg Cys Cys Cys Ser Ala Asn Gln Gly Ile Asn Lys
    210                 215                 220
Glu Ser Ala Asn Thr Val Lys Lys Asp Asn Gly Asp Gln Ser Lys Arg
225                 230                 235                 240
Pro Lys Ile Tyr Phe Gly Thr Arg Thr His Lys Gln Ile Ala Gln Ile
                245                 250                 255
Thr Arg Glu Leu Arg Lys Thr Ala Tyr Ser Gly Val Pro Met Thr Ile
            260                 265                 270
Leu Ser Ser Arg Asp His Thr Cys Val His Pro Glu Val Val Gly Asn
        275                 280                 285
Phe Asn Arg Asn Glu Lys Cys Met Glu Leu Leu Asp Gly Lys His Gly
    290                 295                 300
Lys Ser Cys Tyr Phe Tyr His Gly Val His Arg Ile Ser Asn Gln Gln
305                 310                 315                 320
Thr Leu Gln Phe Leu His Gly Ile Ser Lys Ala Trp Asp Ile Glu Glu
                325                 330                 335
Leu Val Ser Leu Gly Arg Lys Leu Lys Ala Cys Pro Tyr Tyr Thr Ala
            340                 345                 350
Arg Glu Leu Ile Asp Ser Ala Asp Ile Ile Phe Cys Pro Tyr Asn Tyr
        355                 360                 365
Leu Leu Asp Ser Gln Ile Arg Glu Ser Met Asp Ile Lys Leu Lys Glu
    370                 375                 380
Gln Val Val Ile Leu Asp Glu Ala His Asn Ile Glu Glu Cys Ala Arg
385                 390                 395                 400
Glu Thr Ala Ser Tyr Ser Val Thr Glu Val Gln Leu Arg Phe Ala Arg
                405                 410                 415
Asp Glu Leu Asp Ser Leu Ile Asn Ser Asn Ile Arg Lys Lys Ser His
            420                 425                 430
Glu Pro Leu Arg Asp Val Cys Tyr Asn Leu Ile Asn Trp Leu Glu Thr
        435                 440                 445
Asn Ser Lys Asn Leu Val Glu Arg Asp Tyr Glu Ser Ser Cys Lys Ile
    450                 455                 460
Trp Ser Gly Asn Glu Met Leu Leu Asn Leu His Arg Met Gly Ile Thr
465                 470                 475                 480
Ala Ala Ser Phe Pro Val Leu Gln Lys His Leu Ser Ala Val Leu Gln
                485                 490                 495
Lys Glu Glu Lys Val Thr Ser Thr His Gly Lys Glu Glu Ala Ile Gln
            500                 505                 510
Ile Pro Ile Ile Ser Ala Ser Thr Gln Ile Met Leu Lys Gly Leu Phe
        515                 520                 525
Met Val Leu Asp Tyr Leu Phe Arg Lys Asn Ser Arg Phe Ala Asp Asp
    530                 535                 540
Tyr Lys Val Ala Ile Gln Gln Thr Tyr Ser Trp Thr Asn Gln Ile Ala
545                 550                 555                 560
Ile Phe Asp Lys Asn Ala Leu Leu Pro Val Pro Lys Asn Lys Lys His
                565                 570                 575
Ser Arg Gln Lys Ile Gly Val Asn Val Leu Asn Phe Trp Cys Leu Asn
            580                 585                 590
```

```
Pro Ala Val Ala Phe Ser Asp Ile Asn Asp Lys Val Arg Thr Ile Val
        595                 600                 605
Leu Thr Ser Gly Thr Leu Ser Pro Leu Lys Ser Phe Ser Ser Glu Leu
    610                 615                 620
Gly Val Thr Phe Asn Ile Gln Leu Glu Ala Asn His Val Ile Ser Asn
625                 630                 635                 640
Ser Gln Val Trp Val Gly Thr Val Gly Ser Gly Pro Lys Gly Arg Asn
                645                 650                 655
Leu Cys Ala Thr Phe Gln His Thr Glu Thr Phe Glu Phe Gln Asp Glu
            660                 665                 670
Val Gly Met Leu Leu Leu Ser Val Cys Gln Thr Val Ser Gln Gly Ile
        675                 680                 685
Leu Cys Phe Leu Pro Ser Tyr Lys Leu Leu Glu Lys Leu Arg Glu Arg
    690                 695                 700
Trp Ile Phe Thr Gly Leu Trp His Ser Leu Glu Ser Val Lys Thr Val
705                 710                 715                 720
Ile Ala Glu Pro Gln Gly Gly Glu Lys Thr Asp Phe Asp Glu Leu Leu
                725                 730                 735
Gln Val Tyr Tyr Asp Ala Ile Lys Phe Lys Gly Glu Lys Asp Gly Ala
            740                 745                 750
Leu Leu Ile Ala Val Cys Arg Gly Lys Val Ser Glu Gly Leu Asp Phe
        755                 760                 765
Ser Asp Asp Asn Ala Arg Ala Val Ile Thr Ile Gly Ile Pro Phe Pro
    770                 775                 780
Asn Val Lys Asp Leu Gln Val Glu Leu Lys Arg Gln Tyr Asn Asp His
785                 790                 795                 800
His Ser Lys Leu Arg Gly Leu Leu Pro Gly Arg Gln Trp Tyr Glu Ile
                805                 810                 815
Gln Ala Tyr Arg Ala Leu Asn Gln Ala Leu Gly Arg Cys Ile Arg His
            820                 825                 830
Lys Asn Asp Trp Gly Ala Leu Ile Leu Val Asp Asp Arg Phe Asn Asn
        835                 840                 845
Asn Pro Asp Arg Tyr Ile Ser Gly Leu Ser Lys Trp Val Arg Gln Gln
    850                 855                 860
Ile Gln His His Ser Thr Phe Ala Ser Ala Leu Glu Ser Leu Thr Glu
865                 870                 875                 880
Phe Ser Lys Arg His Gln Lys Val Thr Asn Arg Ser Lys Lys Glu Glu
                885                 890                 895
Lys Cys Thr Lys Glu Asn Gly Ser Thr Leu Asp Val Ala Cys Leu Glu
            900                 905                 910
Gly Ser Thr Leu Thr Ser Val Ser Glu Ala Ser His Gln Thr Pro Glu
        915                 920                 925
Asn Ser Leu Glu Glu Glu Ala Lys Val Cys Val Gln Glu Gln Arg Tyr
    930                 935                 940
Pro Gln Met Ala Ala Glu Asn Pro Ser Gly Pro Ser His Gly Val Ser
945                 950                 955                 960
Arg Arg Lys Glu Ser Asp Pro Gly Leu Arg Glu Lys Ser Val Gln Thr
                965                 970                 975
Met Lys Thr Glu Lys Ser Glu Ile Ser Arg Ser Ser Ser Pro Thr Phe
            980                 985                 990
Gly Lys Gln Thr Glu Pro Val Asn Trp Pro Ile Phe Asn Ser Leu Lys
        995                 1000                1005
Arg His Phe Asn Ser Lys Val Lys Asn Arg Thr Pro Val Leu Lys Ser
    1010                1015                1020
Ser Lys Asn His Ala Ser Ala Ser Ser Ala Phe Asn Lys Thr Ala Leu
1025                1030                1035                1040
Pro Leu Thr Gly Lys Cys Val Ser Ser Ser Ala Pro Ala Asn Thr Pro
                1045                1050                1055
Leu Ala Pro Cys Pro Gln Ser Glu Val Ile Ile Pro Ser Val Lys Ala
            1060                1065                1070
Asp Thr Thr Pro Ala Lys Thr His Cys Lys Gln Asn Glu Lys Asp Pro
        1075                1080                1085
Ser Pro Asp Ala Glu Leu Ser Pro Val Thr Glu Glu Ala Lys Gly Ser
```

1090 1095 1100
Ser Ser Asn Pro Ala Val Gly Thr Glu Val Asp Asp Asp Ser Val Cys
1105 1110 1115 1120
Phe Thr Pro Glu Leu Phe Asp Pro Val Ser Thr Asp Glu Glu Asn Ser
1125 1130 1135
Glu Leu Val Glu Thr Asp Arg Ser Ser Asn Asn Ser Asp Cys Leu Ser
1140 1145 1150
Ala Glu Glu Leu Phe Glu Thr Val Thr Gly Phe Gly Gln Lys
1155 1160 1165

<210> 20
<211> 1252
<212> PRT
<213> Gallus gallus

<400> 20

```
Met Ser Ser Asp Val Ser Gln Tyr Thr Ile Gly Gly Val Lys Ile Met
1               5                   10                  15
Phe Pro Cys Lys Ala Tyr Pro Ser Gln Leu Ala Met Met Asn Ala Ile
            20                  25                  30
Val Lys Gly Leu Asn Asn Arg Gln His Cys Leu Leu Glu Ser Pro Thr
        35                  40                  45
Gly Ser Gly Lys Ser Leu Ala Leu Leu Cys Ser Ala Leu Ser Trp Gln
    50                  55                  60
Gln Ser Leu Tyr Glu Lys Ser Leu Leu Lys Ser Ser Cys Glu Lys Glu
65                  70                  75                  80
Asp Arg Glu Pro Ala Ala Ser Leu Pro Cys Arg Cys Val Cys His Ser
                85                  90                  95
Arg Ser Glu Ser Ser Glu Ala Thr Ala Gly Ala Ser His Gly Ala Ala
            100                 105                 110
Cys Ser Asn Asn Tyr Glu Thr Gly Gly Ser Val Lys His Gly Asp Gln
        115                 120                 125
Leu Ser Asp Thr Glu Cys Lys Glu Asn Asn Thr Leu Ala Ser Lys Leu
    130                 135                 140
Ser Ala Lys Lys Arg Ala Ser Ala Cys Gly Asn Glu Cys Asp Asp Phe
145                 150                 155                 160
Gln Val Glu Arg Lys Arg Ile Arg Pro Leu Glu Thr Glu Gln Gln Val
                165                 170                 175
Arg Lys Arg His Cys Phe Ser Lys Glu Val Gln Leu Val Asp Ala Leu
            180                 185                 190
Glu Val Tyr Asn Gln Arg Lys Asn Gly Glu Leu Ile Val His Ser Glu
        195                 200                 205
Lys Ser Val Lys Asn Thr Ser Pro Gln Thr Leu Phe Ser Ser Cys Thr
    210                 215                 220
Glu Cys Ser Cys Ser Ser Gly Lys Glu Thr Arg Lys Asp Ser Gly Asn
225                 230                 235                 240
Thr Lys Lys Lys Ala Asn Gly Asp Gln Thr Phe Ile Pro Lys Ile Phe
                245                 250                 255
Phe Gly Thr Arg Thr His Lys Gln Ile Ala Gln Ile Thr Arg Glu Leu
            260                 265                 270
Lys Arg Thr Ala Tyr Ser Gly Val Pro Met Thr Ile Leu Ser Ser Arg
        275                 280                 285
Asp Tyr Thr Cys Ile His Pro Val Val Ser Ser Ser Asn Ser Asn Arg
    290                 295                 300
Asn Glu Leu Cys Val Glu Leu Leu Glu Gly Lys His Gly Lys Ser Cys
305                 310                 315                 320
Leu Tyr Tyr His Gly Val His Lys Leu Ser Glu His Tyr Ala Leu Gln
                325                 330                 335
Ser Ala His Asn Thr Tyr Gln Ala Trp Asp Ile Glu Asp Leu Val Ser
            340                 345                 350
Leu Gly Lys Lys Leu Arg Ala Cys Pro Tyr Phe Ala Ala Arg Glu Leu
        355                 360                 365
```

```
Met Val Gly Ala Asp Ile Val Phe Cys Pro Tyr Asn Tyr Leu Leu Asp
    370                 375                 380
Pro Gln Ile Arg Glu Ser Met Glu Ile Asn Leu Lys Gly Gln Val Val
385                 390                 395                 400
Ile Leu Asp Glu Ala His Asn Ile Glu Asp Ser Ala Arg Glu Ala Val
                405                 410                 415
Ser Tyr Ser Val Thr Glu Ser Gln Leu Asn Ala Ala Arg Glu Glu Leu
            420                 425                 430
Asp Phe Met Val Asn Asn Asn Ile Arg Gln Lys Asp His Glu Gln Leu
        435                 440                 445
Arg Ala Met Cys Cys Ser Leu Thr Asn Trp Leu Arg Glu Ser Ser Ser
    450                 455                 460
Gln Leu Val Glu Thr Gly Tyr Glu Thr Ser Cys Lys Val Trp Ser Gly
465                 470                 475                 480
Lys Glu Met Leu Asn His Phe His Asp Met Gly Ile Thr Asn Ile Ser
                485                 490                 495
Phe Pro Ile Leu Gln Lys His Leu Ser Ala Val Leu Glu Lys Glu Glu
            500                 505                 510
Lys Ile Ser Met Phe Gly Lys Glu Glu Leu Val Glu Ile Pro Ile Val
        515                 520                 525
Ser Ser Ala Thr Gln Ile Val Leu Lys Gly Leu Phe Met Val Leu Leu
    530                 535                 540
Tyr Leu Phe Lys Asp Asn Ser Arg Phe Ala Asp Asp Tyr Arg Val Ala
545                 550                 555                 560
Leu Gln Gln Thr Tyr Ala Trp Thr Asn Asp Asn Gln Pro Asp Val Ser
                565                 570                 575
Asp Thr Ser Ala Phe Phe Thr Lys Thr Lys His Lys Arg Asn Leu Arg
            580                 585                 590
His Lys Thr Val Val His Met Leu Asn Phe Trp Cys Leu Asn Pro Ala
        595                 600                 605
Val Ala Phe Ser Asp Leu Asn Asp Val Arg Thr Val Val Leu Thr Ser
    610                 615                 620
Gly Thr Leu Ser Pro Met Asp Ser Phe Ser Ser Glu Leu Gly Val Lys
625                 630                 635                 640
Phe Ser Ile Gln Leu Glu Ala Asn His Val Ile Arg Asn Ser Gln Val
                645                 650                 655
Trp Val Gly Thr Ile Gly Thr Gly Pro Asn Gly Arg Lys Leu Cys Ala
            660                 665                 670
Thr Phe Gln His Thr Glu Thr Phe Glu Phe Gln Asp Glu Val Gly Ala
        675                 680                 685
Leu Leu Leu Ser Val Cys Gln Lys Val Gly Gln Gly Ile Leu Cys Phe
    690                 695                 700
Leu Pro Ser Tyr Lys Leu Leu Asp Lys Leu Lys Asp Arg Trp Ile His
705                 710                 715                 720
Thr Gly Leu Trp Arg Asn Leu Glu Leu Val Lys Thr Val Ile Ala Glu
                725                 730                 735
Pro Gln Gly Gly Ala Lys Ser Asp Phe Asp Glu Leu Leu Lys Ile Tyr
            740                 745                 750
Tyr Asp Ala Ile Lys Phe Lys Gly Glu Lys Asp Gly Ala Leu Leu Ile
        755                 760                 765
Ala Val Cys Arg Gly Lys Val Ser Glu Gly Leu Asp Phe Cys Asp Glu
    770                 775                 780
Asn Ala Arg Ala Val Ile Thr Ile Gly Ile Pro Phe Pro Asn Val Lys
785                 790                 795                 800
Asp Leu Gln Val Glu Leu Lys Arg Lys Tyr Asn Asp Gln His Lys Thr
                805                 810                 815
Thr Arg Gly Leu Leu Pro Gly Ser Gln Trp Tyr Glu Ile Gln Ala Tyr
            820                 825                 830
Arg Ala Leu Asn Gln Ala Leu Gly Arg Cys Ile Arg His Arg Ser Asp
        835                 840                 845
Trp Gly Ala Leu Ile Leu Val Asp Asp Arg Phe Arg Asn Asn Pro Asn
    850                 855                 860
Lys Tyr Ile Thr Gly Leu Ser Lys Trp Ile Arg Gln Gln Val Gln His
```

```
865                 870                 875                 880
His Glu Asn Phe Gly Ser Ala Leu Glu Ser Leu His Ala Phe Ala Glu
                885                 890                 895
Arg Asn Gln Lys Gly Ile Asp Phe Ser Ser Gln Cys Ser Asn Glu Val
            900                 905                 910
Phe His Val Pro Leu Asn Ser Lys Glu Pro Ser Ser Ala Ser Gln Gln
        915                 920                 925
Glu Ala Thr Ile His Leu Ser Pro Asp Val Pro Val Lys Ser Glu Glu
    930                 935                 940
Gln Ser Phe Val Pro Glu Thr His Leu Thr Thr Thr Ile Asn Ser Ile
945                 950                 955                 960
Asn Pro Gly Pro Ser Asn Gln Pro Gly Gly Gln Lys Val Asp Val Glu
                965                 970                 975
Ser Cys Ser His Asn Gly Ile Gln Arg Arg Lys His Met Asp Ser Thr
            980                 985                 990
Pro Arg Arg Pro Ala Asn Lys Thr Glu Lys Lys Ser Asp Arg Thr Asn
        995                 1000                1005
Ser Asp Phe Met Lys Glu His Cys Cys Phe Lys Pro Leu Thr Ser Thr
    1010                1015                1020
Pro Leu Pro Val Ala Thr Asn Cys Val Ser Thr Ala Ser Ser Lys Gln
1025                1030                1035                1040
Arg Lys Asn Val Asn Ser Ala Ser Glu Leu Ile Gly Gly Val Asn Gln
                1045                1050                1055
Cys Gln Ser Ser Phe Thr Leu Glu His Lys Pro Ser Ile Pro Glu Ser
            1060                1065                1070
His Leu Glu Thr Thr Asn Phe Ser Val Lys Ser Thr Glu Ala Pro Val
        1075                1080                1085
Ala Glu Glu His Leu Asp Glu Gln Lys Leu Gln Ile Glu Pro Cys Ser
    1090                1095                1100
Glu Leu Pro Ser Val Gly Gly Arg Pro Glu Leu Ser Val Leu Glu Ile
1105                1110                1115                1120
Ser Ala Glu Asp Glu Asp Glu Ser Leu Tyr Phe Thr Pro Glu Leu Tyr
                1125                1130                1135
Asp Asp Ala Glu Ser Glu Glu Gln Glu Met Arg Pro Leu Asp Pro Asp
            1140                1145                1150
Glu Asn Gln Ile Glu Cys Gly Lys Pro Thr Val Ala Asp Asp Leu Phe
        1155                1160                1165
Val Ile Ser Thr Ser Lys Thr Leu Ser Glu Pro Lys Glu Met Ile Asn
    1170                1175                1180
Asp Asp Gly Arg Asn Thr Ser Leu His Gly Thr Met Leu Ser Asp Ile
1185                1190                1195                1200
Ser Lys Asn Ser Thr Val Asn Ile Glu Lys Met Thr Asn Gly Glu Glu
                1205                1210                1215
Ala Glu Gln Val Glu Ser Gln Glu Val Asp Thr Lys Lys Arg Lys Ile
            1220                1225                1230
Ser Leu Ser Arg Ser Arg Asn Lys Gly Val Ser Pro Phe Leu Leu Asp
        1235                1240                1245
Ser Thr Ser Thr
    1250
```

<210> 21
<211> 39
<212> PRT
<213> Homo sapiens

<400> 21

```
Cys Ala Thr Phe Gln Asn Thr Glu Thr Phe Glu Phe Gln Asp Glu Val
  1               5                  10                  15
Gly Ala Leu Leu Leu Ser Val Cys Gln Thr Val Ser Gln Gly Ile Leu
            20                  25                  30
Cys Phe Leu Pro Ser Tyr Lys
        35
```

<210> 22
<211> 11
<212> PRT
<213> Homo sapiens

<400> 22

```
Ala Lys Glu Phe Cys Val Ser Cys His Leu Thr
  1               5                  10
```